(19) Europäisches Patentamt
European Patent Office
Office européen des brevets

(11) EP 4 317 146 A1

(12) EUROPEAN PATENT APPLICATION
published in accordance with Art. 153(4) EPC

(43) Date of publication:
**07.02.2024 Bulletin 2024/06**

(21) Application number: **22778919.5**

(22) Date of filing: **29.03.2022**

(51) International Patent Classification (IPC):
***C07D 401/14*** (2006.01) ***C07D 403/14*** (2006.01)
***A61P 35/00*** (2006.01) ***A61K 31/4725*** (2006.01)

(52) Cooperative Patent Classification (CPC):
**A61K 31/4725; A61P 35/00; C07D 401/14; C07D 403/14**

(86) International application number:
**PCT/CN2022/083597**

(87) International publication number:
**WO 2022/206737 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2021 CN 202110332995**
**16.06.2021 CN 202110666964**
**30.07.2021 CN 202110870938**
**18.09.2021 CN 202111097026**

(71) Applicants:
- **Jiangsu Hengrui Pharmaceuticals Co., Ltd.**
**Lianyungang, Jiangsu 222047 (CN)**
- **Shanghai Hengrui Pharmaceutical Co., Ltd.**
**Shanghai 200245 (CN)**

(72) Inventors:
- **YANG, Fanglong**
**Shanghai 200245 (CN)**
- **YU, Nan**
**Shanghai 200245 (CN)**
- **LIU, Zhiwei**
**Shanghai 200245 (CN)**
- **HE, Feng**
**Shanghai 200245 (CN)**
- **TAO, Weikang**
**Shanghai 200245 (CN)**

(74) Representative: **Potter Clarkson**
**Chapel Quarter**
**Mount Street**
**Nottingham NG1 6HQ (GB)**

# (54) TETRAHYDRONAPHTHALENE COMPOUND, AND PREPARATION METHOD THEREFOR AND USE THEREOF IN MEDICINE

(57) The present disclosure relates to a tetrahydronaphthalene compound, and a preparation method therefor and the use thereof in medicine. Specifically, the present disclosure relates to a tetrahydronaphthalene compound as represented by general formula (I), a preparation method therefor, a pharmaceutical composition containing the compound, and the use thereof as a therapeutic agent, in particular the use thereof as an estrogen receptor degrader, the use in the preparation of a drug for treating and/or preventing estrogen receptor-mediated or dependent diseases or conditions and the use in the preparation of a drug for treating and/or preventing diseases or conditions by means of degrading a target protein.

EP 4 317 146 A1

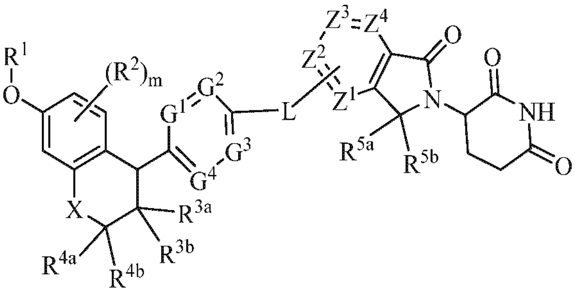

(I)

## Description

### TECHNICAL FIELD

The present disclosure belongs to the field of pharmaceutics and relates to a tetrahydronaphthalene compound, a preparation method therefor and pharmaceutical use thereof. Specifically, the present disclosure relates to a tetrahydronaphthalene compound of general formula (I), a preparation method therefor, a pharmaceutical composition comprising the compound, use thereof as a therapeutic agent, particularly as an estrogen receptor degrader, use thereof in the preparation of a medicament for treating and/or preventing an estrogen receptor mediated or dependent disease or disorder, and use thereof in the preparation of a medicament for treating and/or preventing a disease or disorder by degrading target proteins.

### BACKGROUND

According to the latest global cancer burden report 2020 released by the International Agency for Research on Cancer (IARC) of the World Health Organization, the number of female breast cancer patients exceeded that of lung cancer patients for the first time: breast cancer has become the world's most common cancer. More than 2.26 million women suffer from breast cancer globally, accounting for about 11.7% of all new cancer cases diagnosed and 24.5% of new female cancer cases diagnosed. Breast cancer is the most common cancer in women. One in every eight new cases diagnosed suffers from breast cancer. Meanwhile, more than 680 thousand people died from breast cancer, accounting for about 6.9% of all cancer deaths and 15.5% of worldwide cancer deaths among women. Breast cancer is also the leading cause of cancer death globally among women.

About 70% of breast cancer patients have estrogen receptor (ER)-positive breast cancer. Endocrine therapy plays an important role in the treatment of these breast cancer patients. Endocrine therapy is divided into three major categories, which are: aromatase inhibitors (AIs), which can inhibit the conversion of androgen into estrogen and reduce the estrogen level in the body; selective estrogen receptor modulators (SERMs), which can antagonize the activity of estrogen receptors; and selective estrogen receptor degraders (SERDs), which can not only antagonize the activity of estrogen receptors but also promote the degradation of the receptors (J. Biol. Chem. 2006, 14, 9607-9615). Fulvestrant is the only commercially available drug that acts by degrading estrogen receptors. Better clinical efficacy can be achieved if its clinical dose is increased from 250 mg to 500 mg. In a study using isotopically labeled estrogen to observe the level of estrogen receptor degradation in tumors in patients, the level of estrogen receptor degradation was found to be correlated with the patients' clinical benefit, and the incomplete degradation of estrogen receptors may be associated with early disease progression. However, fulvestrant has poor water solubility and low bioavailability, which make it difficult to further increase doses via intramuscular injection. Therefore, it is necessary to develop a drug that degrades estrogen receptors better than fulvestrant. Protein proteolysis-targeting chimeras (PROTACs) are hybrid bifunctional small-molecule compounds. Their structures contain two different ligands: an E3 ubiquitin ligase ligand and a ligand that binds to target proteins. The two ligands are linked by a linker arm. PROTACs draw target proteins and E3 ubiquitin ligase in cells close to form target protein-PROTAC-E3 ternary complexes. Then E3 ubiquitin ligase labels target proteins with a ubiquitinated protein tag and initiates a powerful ubiquitination hydrolysis process in cells, which specifically degrades target proteins via the ubiquitin-proteasome pathway. PROTACs have unique advantages over traditional small-molecule inhibitors: 1) PROTACs do not need a long and high-strength binding to target proteins, and their degradation of target proteins is similar to catalysis in that they bind and degrade target proteins cyclically, so the systemic exposure of drugs, and thus the toxic and side effects, is reduced; 2) target proteins need to be re-synthesized to recover their function after being degraded; therefore, degrading target proteins shows a more efficient and lasting anti-tumor effect than inhibiting their activity and will not lead to drug resistance caused by mutation of target proteins; 3) PROTACs also have therapeutic potentials for targets which are now considered undruggable, such as transcription factors, scaffold proteins and regulatory proteins. The discovery of cereblon (CRBN)-type E3 ligase ligands is associated with the study of the mechanism of action of thalidomide. In 2010, a study on the toxicity of thalidomide found that the *in vivo* binding of thalidomide to CRBN may be the cause of the teratogenicity of thalidomide (Science, 2010, 327, 1345). Subsequent studies found that thalidomide and its derivatives could be used as anti-inflammatory drugs, anti-angiogenesis drugs and anti-cancer drugs. Among them, lenalidomide and pomalidomide are much safer in that they have significantly lower teratogenicity. A further study has shown that lenalidomide acts by degrading two special B-cell transcription factors-Ikaros family zinc finger proteins 1 and 3 (IKZF1 and IKZF3). This study revealed the mechanism of action of thalidomide and its derivatives: they bind to CRBN-type E3 ubiquitin ligase protein complexes and degrade target proteins (Science, 2014, 343, 301; Science, 2014, 343, 305).

On this basis, CRBN ligands have been widely used in the preparation of protein degraders, and a range of PROTAC molecules based on CRBN ligands have been developed. The present disclosure synthesizes a class of novel tetrahydronaphthalene compounds and demonstrates their use as estrogen receptor degraders in the treatment of

estrogen receptor mediated or dependent diseases.

Published patent applications for PROTAC molecules based on CRBN ligands include WO2015160845A2, WO2016197032A1, WO2016105518A1, WO2017197046A1, WO2017197051A1, WO2018144649A1, US10800770B1, WO2018102725A1, WO2019199816A1, etc.

## SUMMARY

The present disclosure aims to provide a compound of general formula (I) or a pharmaceutically acceptable salt thereof:

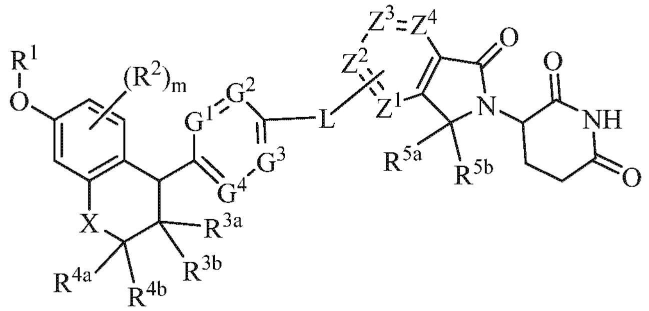

(I)

wherein:

$R^{3a}$ and $R^{3b}$ are identical or different and are each independently selected from the group consisting of hydrogen atom, alkyl, cycloalkyl, heterocyclyl, 8- to 10-membered aryl and heteroaryl, wherein the alkyl, cycloalkyl, heterocyclyl, 8- to 10-membered aryl and

heteroaryl are each independently optionally substituted with one or more substituents selected from the group consisting of halogen, oxo, alkyl, alkoxy, haloalkyl, haloalkoxy, cyano, $-NR^9R^{10}$, nitro, hydroxy, hydroxyalkyl, cycloalkyl, heterocyclyl, aryl and heteroaryl;

or $R^{3a}$ and $R^{3b}$, together with the carbon atom to which they are attached, form cycloalkyl or heterocyclyl, wherein the cycloalkyl or heterocyclyl is each independently optionally substituted with one or more substituents selected from the group consisting of halogen, oxo, alkyl, alkoxy, haloalkyl, haloalkoxy, cyano, $-NR^9R^{10}$, nitro, hydroxy, hydroxyalkyl, cycloalkyl, heterocyclyl, aryl and heteroaryl;

X is an oxygen atom or $CH_2$;

$R^1$ is selected from the group consisting of hydrogen atom, alkyl, haloalkyl, hydroxyalkyl and $-C(O)R^6$;

$G^1$, $G^2$, $G^3$ and $G^4$ are identical or different and are each independently a nitrogen atom or $CR^7$;

one of $Z^1$, $Z^2$, $Z^3$ and $Z^4$ is a carbon atom, and the remaining three are identical or different and are each independently a nitrogen atom or $CR^8$;

L is a linker unit;

$R^{4a}$ and $R^{4b}$ are identical or different and are each independently selected from the group consisting of hydrogen atom, halogen, alkyl, alkoxy, haloalkyl, haloalkoxy and hydroxyalkyl;

$R^{5a}$ and $R^{5b}$ are identical or different and are each independently selected from the group consisting of hydrogen atom, halogen, alkyl, alkoxy, haloalkyl, haloalkoxy and hydroxyalkyl, or together $R^{5a}$ and $R^{5b}$ form oxo;

each $R^2$, $R^7$ and $R^8$ is identical or different and is independently selected from the group consisting of hydrogen atom, halogen, alkyl, alkoxy, haloalkyl, haloalkoxy, hydroxyalkyl, cyano, $-NR^{9a}R^{10a}$, hydroxy, $-C(O)R^6$, $-C(O)OR^6$, $-C(O)NR^{9a}R^{10a}$, $-S(O)_tR^{9a}$, $-S(O)_tNR^{9a}R^{10a}$, cycloalkyl, heterocyclyl, aryl and heteroaryl, wherein the alkyl, alkoxy, cycloalkyl, heterocyclyl, aryl and heteroaryl are each independently optionally substituted with one or more substituents selected from the group consisting of halogen, oxo, alkyl, alkoxy, haloalkyl, haloalkoxy, cyano, $-NR^9R^{10}$, nitro, hydroxy, hydroxyalkyl, cycloalkyl, heterocyclyl, aryl and heteroaryl;

$R^6$ is selected from the group consisting of alkyl, haloalkyl, hydroxyalkyl, cycloalkyl, heterocyclyl, aryl and heteroaryl;

$R^9$, $R^{10}$, $R^{9a}$ and $R^{10a}$ are identical or different and are each independently selected from the group consisting of hydrogen atom, alkyl, hydroxyalkyl, cycloalkyl and heterocyclyl, wherein the alkyl, cycloalkyl and heterocyclyl are each independently optionally substituted with one or more substituents selected from the group consisting of halogen, alkyl, alkoxy, haloalkyl and haloalkoxy;

or $R^9$ and $R^{10}$, together with the nitrogen atom to which they are attached, form heterocyclyl, and the heterocyclyl is optionally substituted with one or more substituents selected from the group consisting of halogen, oxo, alkyl,

alkoxy, haloalkyl, haloalkoxy, cyano, amino, nitro, hydroxy, hydroxyalkyl, cycloalkyl, heterocyclyl, aryl and heteroaryl; or $R^{9a}$ and $R^{10a}$, together with the nitrogen atom to which they are attached, form heterocyclyl, and the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl and heteroaryl;
t is 0, 1 or 2;
m is 0, 1, 2 or 3.

In some preferred embodiments of the present disclosure, the compound of general formula (I) or the pharmaceutically acceptable salt thereof is provided, wherein each $R^2$ is identical or different and is independently selected from the group consisting of hydrogen atom, halogen and $C_{1-6}$ alkyl; preferably, $R^2$ is a hydrogen atom.

In some preferred embodiments of the present disclosure, the compound of general formula (I) or the pharmaceutically acceptable salt thereof is provided, wherein m is 0 or 1; preferably, m is 0.

In some preferred embodiments of the present disclosure, the compound of general formula (I) or the pharmaceutically acceptable salt thereof is provided, wherein $Z^2$ is a carbon atom; $Z^1$, $Z^3$ and $Z^4$ are identical or different and are each independently a nitrogen atom or $CR^8$; $R^8$ is as defined in general formula (I).

In some preferred embodiments of the present disclosure, the compound of general formula (I) or the pharmaceutically acceptable salt thereof is a compound of general formula (II) or a pharmaceutically acceptable salt thereof:

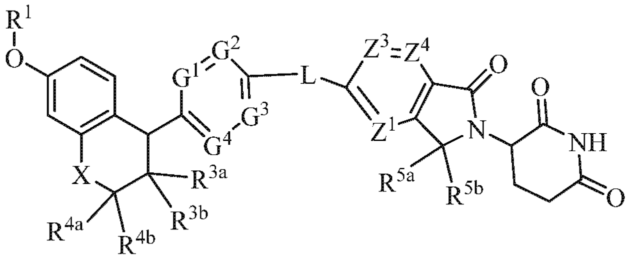

(II)

wherein:

$Z^1$, $Z^3$ and $Z^4$ are identical or different and are each independently a nitrogen atom or $CR^8$;
X, L, $G^1$ to $G^4$, $R^1$, $R^{3a}$, $R^{3b}$, $R^{4a}$, $R^{4b}$, $R^{5a}$, $R^{5b}$ and $R^8$ are as defined in general formula (I).

In some preferred embodiments of the present disclosure, the compound of general formula (I) or general formula (II) or the pharmaceutically acceptable salt thereof is provided, wherein L is selected from the group consisting of $-L^1-$, $-L^2-$, $-R^{1L}-$, $-R^{2L}-$,

$-Q^1-$, $-Q^2-$, 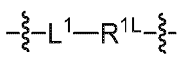 , 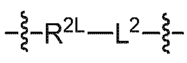 , 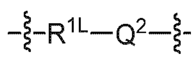 , 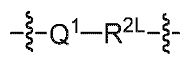 ,

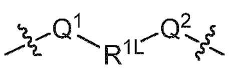 , 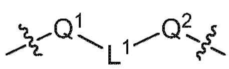 , 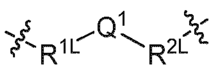 , 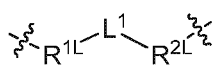 ,

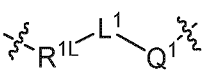 , 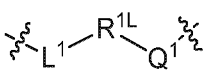 , 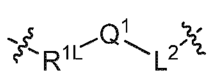 , 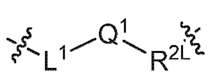 ,

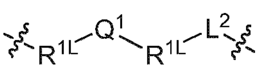 , 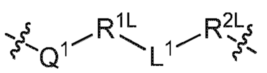 , 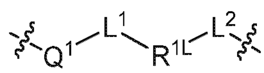 ,

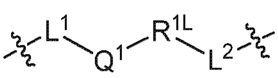 , 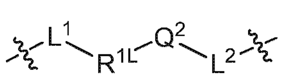 , 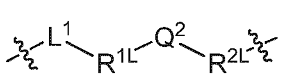 ,

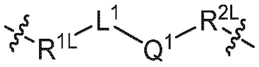 , 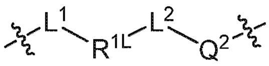 , 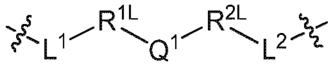 ,

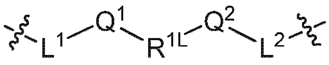 , 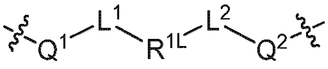 and 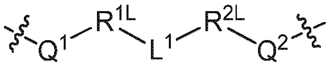 ;

-$L^1$- and -$L^2$- are identical or different and are each independently selected from the group consisting of bond, -O-, -S-, -$NR^{11}$-, -$CR^{12a}R^{12b}$-, -C(O)-, -S(O)-, -$S(O)_2$-, -C(S)-, -C(O)O-, -C(O)$NR^{11}$- and -$NR^{11}$C(O)-;

$R^{1L}$ and $R^{2L}$ are identical or different and are each independently selected from the group consisting of bond, alkylene, heteroalkylene, alkenylene and alkynylene, wherein the alkylene, heteroalkylene, alkenylene and alkynylene are each independently optionally substituted with one or more substituents selected from the group consisting of halogen, alkyl, alkoxy, haloalkyl, hydroxy, hydroxyalkyl, cyano, amino, oxo, cycloalkyl, heterocyclyl, aryl and heteroaryl;

$Q^1$ and $Q^2$ are identical or different and are each independently selected from the group consisting of cycloalkyl, heterocyclyl, aryl and heteroaryl, wherein the cycloalkyl, heterocyclyl, aryl and heteroaryl are each independently optionally substituted with one or more substituents selected from the group consisting of halogen, alkyl, alkoxy, haloalkyl, hydroxy, hydroxyalkyl, cyano, amino, cycloalkyl, heterocyclyl, aryl and heteroaryl;

$R^{11}$ is selected from the group consisting of hydrogen atom, alkyl, heteroalkyl, haloalkyl, cycloalkyl, heterocyclyl, aryl and heteroaryl;

$R^{12a}$ and $R^{12b}$ are identical or different and are each independently selected from the group consisting of hydrogen atom, halogen, alkyl, alkoxy, haloalkyl, hydroxy, hydroxyalkyl, cyano, amino, oxo, cycloalkyl, heterocyclyl, aryl and heteroaryl.

In some preferred embodiments of the present disclosure, the compound of general formula (I) or general formula (II) or the pharmaceutically acceptable salt thereof is provided, wherein L is selected from the group consisting of -$R^{1L}$-,

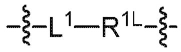 ,

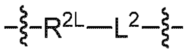 , 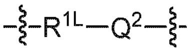 , 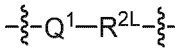 , 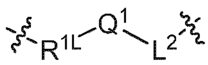 , 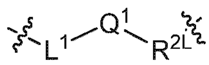 ,

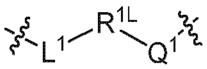 , 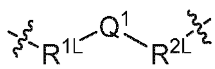 , 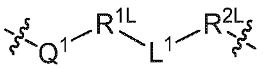 , 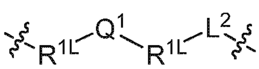 ,

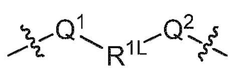 and 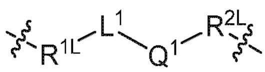 ;

$Q^1$, $Q^2$, $R^{1L}$, $R^{2L}$, $L^1$ and $L^2$ are as defined in general formula (II).

In some preferred embodiments of the present disclosure, the compound of general formula (I) or general formula (II) or the pharmaceutically acceptable salt thereof is provided, wherein L is selected from the group consisting of -$(CH_2)_v$-,

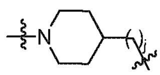 ,

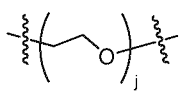 , 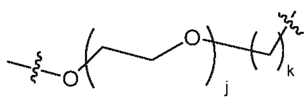 , 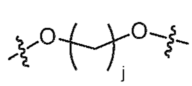 , 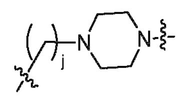 ,

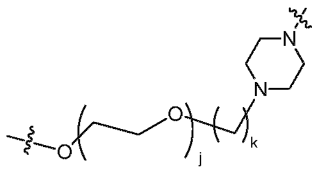, 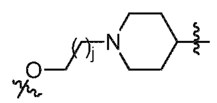, 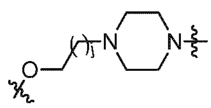, 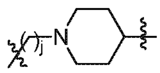,

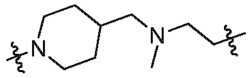, 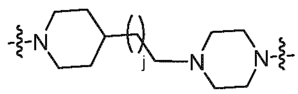, 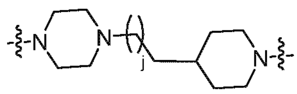,

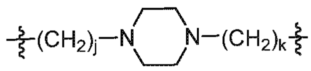, 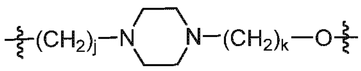, 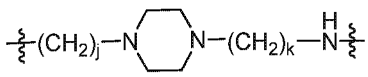

and

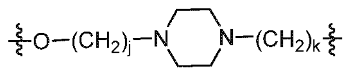;

v is an integer of 1 to 10;
j is an integer of 0 to 10; and
k is an integer of 0 to 10;
preferably, L is

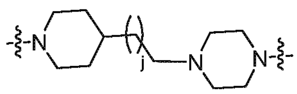,

and j is 0.

In some preferred embodiments of the present disclosure, the compound of general formula (I) or general formula (II) or the pharmaceutically acceptable salt thereof is provided, wherein $R^{5a}$ and $R^{5b}$ are both hydrogen atoms, or together $R^{5a}$ and $R^{5b}$ form oxo; preferably, $R^{5a}$ and $R^{5b}$ are both hydrogen atoms.

In some preferred embodiments of the present disclosure, the compound of general formula (I) or general formula (II) or the pharmaceutically acceptable salt thereof is a compound of general formula (III) or a pharmaceutically acceptable salt thereof:

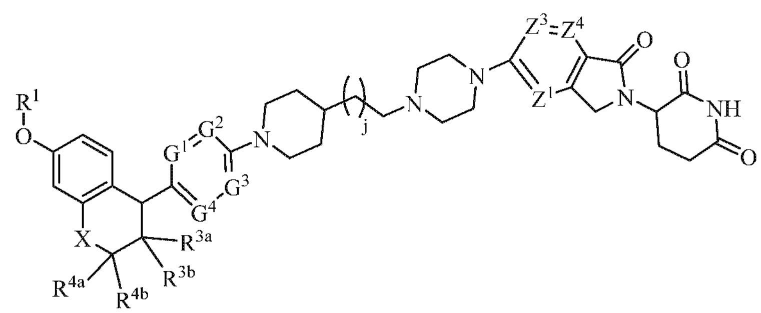

**(III)**

wherein:

j is an integer of 0 to 10;
X, $G^1$ to $G^4$, $Z^1$, $Z^3$, $Z^4$, $R^1$, $R^{3a}$, $R^{3b}$, $R^{4a}$ and $R^{4b}$ are as defined in general formula (II).

In some preferred embodiments of the present disclosure, the compound of general formula (I), general formula (II) or general formula (III) or the pharmaceutically acceptable salt thereof is provided, wherein $R^{3a}$ and $R^{3b}$ are identical or different and are each independently selected from the group consisting of hydrogen atom, $C_{1-6}$ alkyl, 3-to 8-membered cycloalkyl, 3- to 8-membered heterocyclyl, 8- to 10-membered aryl and 5- to 10-membered heteroaryl, wherein the $C_{1-6}$ alkyl, 3- to 8-membered cycloalkyl, 3-to 8-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5- to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl; or $R^{3a}$ and $R^{3b}$, together with the carbon atom to which they are attached, form 3- to 8-membered cycloalkyl or 3- to 8-membered heterocyclyl, wherein the 3- to 8-membered cycloalkyl or 3- to 8-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5-to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl;

preferably, $R^{3a}$ and $R^{3b}$ are identical or different and are each independently selected from the group consisting of hydrogen atom, $C_{1-6}$ alkyl, 3- to 8-membered cycloalkyl, 3-to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8-to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, $C_{1-6}$ hydroxyalkyl, 3- to 8-membered cycloalkyl and 6- to 10-membered aryl; or $R^{3a}$ and $R^{3b}$, together with the carbon atom to which they are attached, form 3- to 8-membered cycloalkyl or 3- to 8-membered heterocyclyl, wherein the 3- to 8-membered cycloalkyl or 3- to 8-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy and $C_{1-6}$ hydroxyalkyl;

more preferably, $R^{3a}$ and $R^{3b}$ are identical or different and are each independently selected from the group consisting of hydrogen atom, $C_{1-6}$ alkyl, 3- to 6-membered cycloalkyl and 3- to 6-membered heterocyclyl, wherein the $C_{1-6}$ alkyl, 3- to 6-membered cycloalkyl and 3- to 6-membered heterocyclyl are each independently optionally substituted with one or more substituents selected from the group consisting of halogen, $C_{1-6}$ alkyl, $C_{1-6}$ haloalkyl, 3- to 6-membered cycloalkyl and phenyl; or $R^{3a}$ and $R^{3b}$, together with the carbon atom to which they are attached, form 3- to 6-membered cycloalkyl or 3- to 6-membered heterocyclyl, wherein the 3- to 6-membered cycloalkyl or 3- to 6-membered heterocyclyl is each independently optionally substituted with one or more substituents selected from the group consisting of halogen, $C_{1-6}$ alkyl, $C_{1-6}$ alkoxy, $C_{1-6}$ haloalkyl, $C_{1-6}$ haloalkoxy and $C_{1-6}$ hydroxyalkyl.

In some preferred embodiments of the present disclosure, the compound of general formula (I), general formula (II) or general formula (III) or the pharmaceutically acceptable salt thereof is provided, wherein $R^{3a}$ and $R^{3b}$ are identical or different and are each independently selected from the group consisting of hydrogen atom, $C_{1-6}$ alkyl, 3-to 8-membered cycloalkyl, 3- to 8-membered heterocyclyl, 8- to 10-membered aryl and 5- to 10-membered heteroaryl, wherein the $C_{1-6}$ alkyl, 3- to 8-membered cycloalkyl, 3-to 8-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5- to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl;

preferably, $R^{3a}$ and $R^{3b}$ are identical or different and are each independently selected from the group consisting of hydrogen atom, $C_{1-6}$ alkyl, 3- to 8-membered cycloalkyl, 3-to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8-to 10-membered aryl and 5- to 10-membered heteroaryl are each independently optionally substituted with one

or more substituents selected from the group consisting of halogen, oxo, $C_{1-6}$ alkyl, $C_{1-6}$ alkoxy, $C_{1-6}$ haloalkyl, $C_{1-6}$ haloalkoxy, $C_{1-6}$ hydroxyalkyl, 3- to 8-membered cycloalkyl and 6- to 10-membered aryl;

more preferably, $R^{3a}$ and $R^{3b}$ are identical or different and are each independently selected from the group consisting of hydrogen atom, $C_{1-6}$ alkyl, 3- to 6-membered cycloalkyl and 3- to 6-membered heterocyclyl, wherein the $C_{1-6}$ alkyl, 3- to 6-membered cycloalkyl and 3- to 6-membered heterocyclyl are each independently optionally substituted with one or more substituents selected from the group consisting of halogen, $C_{1-6}$ alkyl, $C_{1-6}$ haloalkyl, 3- to 6-membered cycloalkyl and phenyl;

most preferably, $R^{3a}$ and $R^{3b}$ are different: one of them is a hydrogen atom and the other is selected from the group consisting of $C_{1-6}$ alkyl, 3- to 6-membered cycloalkyl and 3-to 6-membered heterocyclyl, wherein the $C_{1-6}$ alkyl, 3- to 6-membered cycloalkyl and 3-to 6-membered heterocyclyl are each independently optionally substituted with one or more substituents selected from the group consisting of halogen, $C_{1-6}$ alkyl, $C_{1-6}$ haloalkyl, 3- to 6-membered cycloalkyl and phenyl.

In some preferred embodiments of the present disclosure, the compound of general formula (I), general formula (II) or general formula (III) or the pharmaceutically acceptable salt thereof is provided, wherein $R^{3a}$ and $R^{3b}$ are identical or different and are each independently selected from the group consisting of hydrogen atom, $C_{1-6}$ alkyl, 3-to 6-membered cycloalkyl, 3- to 6-membered heterocyclyl, 8- to 10-membered aryl and 5- to 10-membered heteroaryl, wherein the $C_{1-6}$ alkyl, 3- to 6-membered cycloalkyl, 3-to 6-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy.

In some preferred embodiments of the present disclosure, the compound of general formula (I), general formula (II) or general formula (III) or the pharmaceutically acceptable salt thereof is provided, wherein $R^{3a}$ and $R^{3b}$, together with the carbon atom to which they are attached, form 3- to 8-membered cycloalkyl or 3- to 8-membered heterocyclyl, wherein the 3- to 8-membered cycloalkyl or 3- to 8-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5-to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl;

preferably, $R^{3a}$ and $R^{3b}$, together with the carbon atom to which they are attached, form 3- to 8-membered cycloalkyl or 3- to 8-membered heterocyclyl, wherein the 3- to 8-membered cycloalkyl or 3- to 8-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy and $C_{1-6}$ hydroxyalkyl;

more preferably, $R^{3a}$ and $R^{3b}$, together with the carbon atom to which they are attached, form 3- to 6-membered cycloalkyl or 3- to 6-membered heterocyclyl, wherein the 3- to 6-membered cycloalkyl or 3- to 6-membered heterocyclyl is each independently optionally substituted with one or more substituents selected from the group consisting of halogen, $C_{1-6}$ alkyl, $C_{1-6}$ alkoxy, $C_{1-6}$ haloalkyl, $C_{1-6}$ haloalkoxy and $C_{1-6}$ hydroxyalkyl;

most preferably, $R^{3a}$ and $R^{3b}$, together with the carbon atom to which they are attached, form 3- to 6-membered cycloalkyl or 3- to 6-membered heterocyclyl.

In some preferred embodiments of the present disclosure, the compound of general formula (I), general formula (II) or general formula (III) or the pharmaceutically acceptable salt thereof is provided, wherein $R^{3a}$ and $R^{3b}$, together with the carbon atom to which they are attached, form 3- to 6-membered cycloalkyl or 3- to 6-membered heterocyclyl, wherein the 3- to 6-membered cycloalkyl or 3- to 6-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy.

In some preferred embodiments of the present disclosure, the compound of general formula (I), general formula (II) or general formula (III) or the pharmaceutically acceptable salt thereof is provided, wherein $R^{4a}$ and $R^{4b}$ are both hydrogen atoms.

In some preferred embodiments of the present disclosure, the compound of general formula (I), general formula (II) or general formula (III) or the pharmaceutically acceptable salt thereof is a compound of general formula (IV) or a pharmaceutically acceptable salt thereof:

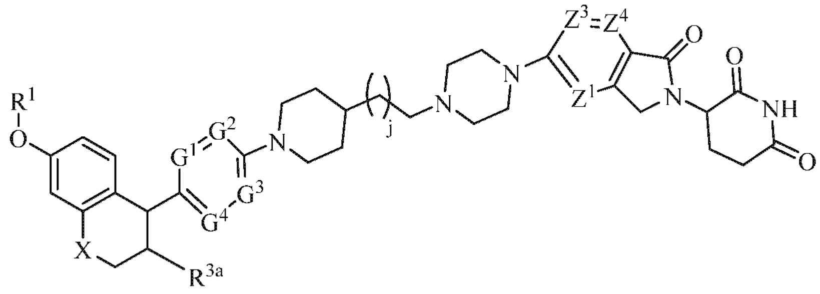

( **IV** )

wherein:

j is an integer of 0 to 10;
$R^{3a}$ is selected from the group consisting of $C_{1-6}$ alkyl, 3- to 8-membered cycloalkyl, 3-to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8-to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5- to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl; preferably, $R^{3a}$ is selected from the group consisting of $C_{1-6}$ alkyl, 3- to 6-membered cycloalkyl, 3- to 6-membered heterocyclyl, 8- to 10-membered aryl and 5-to 10-membered heteroaryl, wherein the $C_{1-6}$ alkyl, 3- to 6-membered cycloalkyl, 3- to 6-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy;
X, $G^1$ to $G^4$, $Z^1$, $Z^3$, $Z^4$ and $R^1$ are as defined in general formula (I).

In some preferred embodiments of the present disclosure, the compound of general formula (I), general formula (II), general formula (III) or general formula (IV) or the pharmaceutically acceptable salt thereof is a compound of general formula (IV-1) or a pharmaceutically acceptable salt thereof:

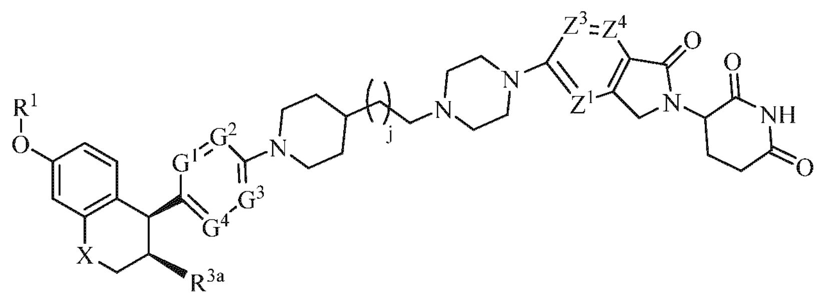

( **IV-1** )

wherein:

j is an integer of 0 to 10;
$R^{3a}$ is selected from the group consisting of $C_{1-6}$ alkyl, 3- to 8-membered cycloalkyl, 3-to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8-to 10-membered aryl and 5- to 10-membered heteroaryl are each independently

optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5- to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl;

X, $G^1$ to $G^4$, $Z^1$, $Z^3$, $Z^4$ and $R^1$ are as defined in general formula (I).

In some preferred embodiments of the present disclosure, the compound of general formula (I), general formula (II), general formula (III), general formula (IV) or general formula (IV-1) or the pharmaceutically acceptable salt thereof is a compound of general formula (V) or a pharmaceutically acceptable salt thereof:

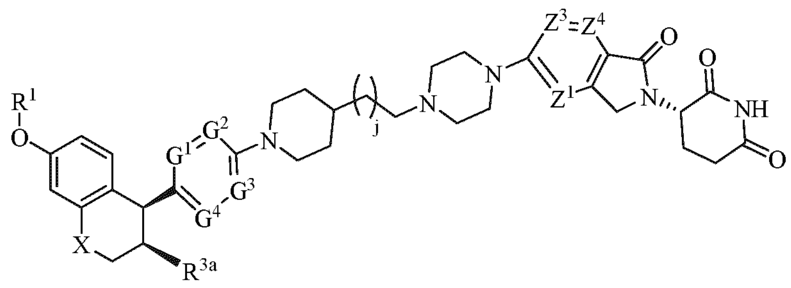

(V)

wherein:

j is an integer of 0 to 10;

$R^{3a}$ is selected from the group consisting of $C_{1-6}$ alkyl, 3- to 8-membered cycloalkyl, 3-to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8-to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5- to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl; preferably, $R^{3a}$ is selected from the group consisting of $C_{1-6}$ alkyl, 3- to 6-membered cycloalkyl, 3- to 6-membered heterocyclyl, 8- to 10-membered aryl and 5-to 10-membered heteroaryl, wherein the $C_{1-6}$ alkyl, 3- to 6-membered cycloalkyl, 3- to 6-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy;

X, $G^1$ to $G^4$, $Z^1$, $Z^3$, $Z^4$ and $R^1$ are as defined in general formula (I).

In some preferred embodiments of the present disclosure, the compound of general formula (I), general formula (II), general formula (III), general formula (IV) or general formula (IV-1) or the pharmaceutically acceptable salt thereof is a compound of general formula (VB) or a pharmaceutically acceptable salt thereof:

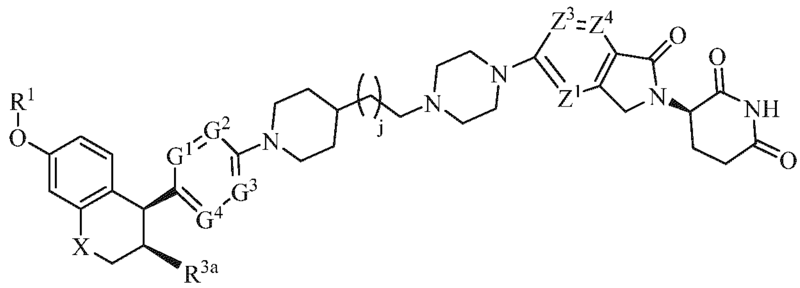

( VB )

wherein:

j is an integer of 0 to 10;

$R^{3a}$ is selected from the group consisting of $C_{1-6}$ alkyl, 3- to 8-membered cycloalkyl, 3-to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8-to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5- to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl; preferably, $R^{3a}$ is selected from the group consisting of $C_{1-6}$ alkyl, 3- to 6-membered cycloalkyl, 3- to 6-membered heterocyclyl, 8- to 10-membered aryl and 5-to 10-membered heteroaryl, wherein the $C_{1-6}$ alkyl, 3- to 6-membered cycloalkyl, 3- to 6-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy;

X, $G^1$ to $G^4$, $Z^1$, $Z^3$, $Z^4$ and $R^1$ are as defined in general formula (I).

In some preferred embodiments of the present disclosure, the compound of general formula (I), general formula (II), general formula (III), general formula (IV), general formula (IV-1), general formula (V) or general formula (VB) or the pharmaceutically acceptable salt thereof is provided, wherein $G^1$, $G^2$, $G^3$ and $G^4$ are all $CR^7$; $R^7$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; preferably, $G^1$, $G^2$, $G^3$ and $G^4$ are all $CR^7$; $R^7$ are identical or different and are each independently a hydrogen atom or a halogen; more preferably, $G^1$, $G^2$, $G^3$ and $G^4$ are all $CR^7$; $R^7$ are identical or different and are each independently a hydrogen atom or a fluorine atom.

In some preferred embodiments of the present disclosure, the compound of general formula (I), general formula (II), general formula (III), general formula (IV), general formula (IV-1), general formula (V) or general formula (VB) or the pharmaceutically acceptable salt thereof is provided, wherein $Z^1$, $Z^3$ and $Z^4$ are all $CR^8$; or $Z^1$ is a nitrogen atom, and $Z^3$ and $Z^4$ are $CR^8$; $R^8$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; preferably, $Z^1$, $Z^3$ and $Z^4$ are all CH, or $Z^1$ is a nitrogen atom, and $Z^3$ and $Z^4$ are CH; more preferably, $Z^1$, $Z^3$ and $Z^4$ are all CH.

In some preferred embodiments of the present disclosure, the compound of general formula (I), general formula (II), general formula (III), general formula (IV), general formula (IV-1), general formula (V) or general formula (VB) or the pharmaceutically acceptable salt thereof is provided, wherein $Z^1$, $Z^3$ and $Z^4$ are all $CR^8$; $R^8$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; preferably, $Z^1$, $Z^3$ and $Z^4$ are all CH.

In some preferred embodiments of the present disclosure, the compound of general formula (I), general formula (II), general formula (III), general formula (IV), general formula (IV-1), general formula (V) or general formula (VB) or the pharmaceutically acceptable salt thereof is provided, wherein $Z^1$ is a nitrogen atom, and $Z^3$ and $Z^4$ are $CR^8$; $R^8$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; preferably, $Z^1$ is a nitrogen atom, and $Z^3$ and $Z^4$ are CH.

In some preferred embodiments of the present disclosure, the compound of general formula (I), general formula

(II), general formula (III), general formula (IV), general formula (IV-1), general formula (V) or general formula (VB) or the pharmaceutically acceptable salt thereof is provided, wherein X is $CH_2$.

In some preferred embodiments of the present disclosure, the compound of general formula (I), general formula (II), general formula (III), general formula (IV), general formula (IV-1), general formula (V) or general formula (VB) or the pharmaceutically acceptable salt thereof is provided, wherein X is an oxygen atom.

In some preferred embodiments of the present disclosure, the compound of general formula (I), general formula (II), general formula (III), general formula (IV), general formula (IV-1), general formula (V) or general formula (VB) or the pharmaceutically acceptable salt thereof is provided, wherein $R^1$ is a hydrogen atom.

In some preferred embodiments of the present disclosure, the compound of general formula (III), general formula (IV), general formula (IV-1), general formula (V) or general formula (VB) or the pharmaceutically acceptable salt thereof is provided, wherein j is 0.

In some preferred embodiments of the present disclosure, the compound of general formula (IV), general formula (IV-1), general formula (V) or general formula (VB) or the pharmaceutically acceptable salt thereof is provided, wherein $R^{3a}$ is selected from the group consisting of $C_{1-6}$ alkyl, 3- to 8-membered cycloalkyl, 3- to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, $C_{1-6}$ hydroxyalkyl, 3- to 8-membered cycloalkyl and 6- to 10-membered aryl;

preferably, $R^{3a}$ is selected from the group consisting of $C_{1-6}$ alkyl, 3- to 6-membered cycloalkyl and 3- to 6-membered heterocyclyl, wherein the $C_{1-6}$ alkyl, 3- to 6-membered cycloalkyl and 3- to 6-membered heterocyclyl are each independently optionally substituted with one or more substituents selected from the group consisting of halogen, $C_{1-6}$ alkyl, $C_{1-6}$ haloalkyl, 3- to 6-membered cycloalkyl and phenyl;

more preferably, $R^{3a}$ is $C_{1-6}$ alkyl or 3- to 6-membered heterocyclyl, wherein the 3- to 6-membered heterocyclyl is optionally substituted with one or more substituents selected from the group consisting of halogen, $C_{1-6}$ alkyl and $C_{1-6}$ haloalkyl;

further preferably, $R^{3a}$ is $C_{1-6}$ alkyl.

In some preferred embodiments of the present disclosure, the compound of general formula (II) or the pharmaceutically acceptable salt thereof is provided, wherein X is an oxygen atom or $CH_2$; $R^1$ is a hydrogen atom; $R^{3a}$ and $R^{3b}$ are identical or different and are each independently selected from the group consisting of hydrogen atom, $C_{1-6}$ alkyl, 3- to 8-membered cycloalkyl, 3- to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, $C_{1-6}$ hydroxyalkyl, 3- to 8-membered cycloalkyl and 6- to 10-membered aryl; or $R^{3a}$ and $R^{3b}$, together with the carbon atom to which they are attached, form 3- to 8-membered cycloalkyl or 3- to 8-membered heterocyclyl, wherein the 3- to 8-membered cycloalkyl or 3- to 8-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy and $C_{1-6}$ hydroxyalkyl; $R^{4a}$ and $R^{4b}$ are both hydrogen atoms; $R^{5a}$ and $R^{5b}$ are both hydrogen atoms, or together $R^{5a}$ and $R^{5b}$ form oxo; $G^1$, $G^2$, $G^3$ and $G^4$ are all $CR^7$; $R^7$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; $Z^1$, $Z^3$ and $Z^4$ are all CH, or $Z^1$ is a nitrogen atom, and $Z^3$ and $Z^4$ are CH; L is

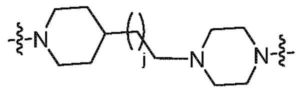,

and j is 0.

In some preferred embodiments of the present disclosure, the compound of general formula (III) or the pharmaceutically acceptable salt thereof is provided, wherein X is an oxygen atom or $CH_2$; $R^1$ is a hydrogen atom; $R^{3a}$ and $R^{3b}$ are identical or different and are each independently selected from the group consisting of hydrogen atom, $C_{1-6}$ alkyl, 3- to 8-membered cycloalkyl, 3- to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, $C_{1-6}$ hydroxyalkyl, 3- to 8-membered cycloalkyl and 6-to 10-membered aryl; or $R^{3a}$ and $R^{3b}$, together with the carbon atom to which they are

attached, form 3- to 8-membered cycloalkyl or 3- to 8-membered heterocyclyl, wherein the 3- to 8-membered cycloalkyl or 3- to 8-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy and $C_{1-6}$ hydroxyalkyl; $R^{4a}$ and $R^{4b}$ are both hydrogen atoms; $G^1$, $G^2$, $G^3$ and $G^4$ are all $CR^7$; $R^7$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; $Z^1$, $Z^3$ and $Z^4$ are all CH, or $Z^1$ is a nitrogen atom, and $Z^3$ and $Z^4$ are CH; j is 0.

In some preferred embodiments of the present disclosure, the compound of general formula (III) or the pharmaceutically acceptable salt thereof is provided, wherein X is $CH_2$; $R^1$ is a hydrogen atom; $R^{3a}$ and $R^{3b}$ are identical or different and are each independently selected from the group consisting of hydrogen atom, $C_{1-6}$ alkyl, 3- to 6-membered cycloalkyl, 3- to 6-membered heterocyclyl, 8- to 10-membered aryl and 5-to 10-membered heteroaryl, wherein the $C_{1-6}$ alkyl, 3- to 6-membered cycloalkyl, 3- to 6-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy; or $R^{3a}$ and $R^{3b}$, together with the carbon atom to which they are attached, form 3- to 6-membered cycloalkyl or 3- to 6-membered heterocyclyl, wherein the 3- to 6-membered cycloalkyl or 3- to 6-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy; $R^{4a}$ and $R^{4b}$ are both hydrogen atoms; $G^1$, $G^2$, $G^3$ and $G^4$ are all $CR^7$; $R^7$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; $Z^1$, $Z^3$ and $Z^4$ are all CH; j is 0.

In some preferred embodiments of the present disclosure, the compound of general formula (III) or the pharmaceutically acceptable salt thereof is provided, wherein X is an oxygen atom or $CH_2$; $R^1$ is a hydrogen atom; $R^{3a}$ and $R^{3b}$ are identical or different and are each independently selected from the group consisting of hydrogen atom, $C_{1-6}$ alkyl, 3- to 6-membered cycloalkyl and 3- to 6-membered heterocyclyl, wherein the $C_{1-6}$ alkyl, 3- to 6-membered cycloalkyl and 3- to 6-membered heterocyclyl are each independently optionally substituted with one or more substituents selected from the group consisting of halogen, $C_{1-6}$ alkyl, $C_{1-6}$ haloalkyl, 3- to 6-membered cycloalkyl and phenyl; or $R^{3a}$ and $R^{3b}$, together with the carbon atom to which they are attached, form 3- to 6-membered cycloalkyl or 3- to 6-membered heterocyclyl, wherein the 3- to 6-membered cycloalkyl or 3- to 6-membered heterocyclyl is each independently optionally substituted with one or more substituents selected from the group consisting of halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; $R^{4a}$ and $R^{4b}$ are both hydrogen atoms; $G^1$, $G^2$, $G^3$ and $G^4$ are all $CR^7$; $R^7$ are identical or different and are each independently a hydrogen atom or a halogen; $Z^1$, $Z^3$ and $Z^4$ are all CH, or $Z^1$ is a nitrogen atom, and $Z^3$ and $Z^4$ are CH; j is 0.

In some preferred embodiments of the present disclosure, the compound of general formula (IV), general formula (IV-1), general formula (V) or general formula (VB) or the pharmaceutically acceptable salt thereof is provided, wherein X is an oxygen atom or $CH_2$; $R^1$ is a hydrogen atom; $R^{3a}$ is selected from the group consisting of $C_{1-6}$ alkyl, 3- to 8-membered cycloalkyl, 3- to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, $C_{1-6}$ hydroxyalkyl, 3- to 8-membered cycloalkyl and 6- to 10-membered aryl; $G^1$, $G^2$, $G^3$ and $G^4$ are all $CR^7$; $R^7$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; $Z^1$, $Z^3$ and $Z^4$ are all CH, or $Z^1$ is a nitrogen atom, and $Z^3$ and $Z^4$ are CH; j is 0.

In some preferred embodiments of the present disclosure, the compound of general formula (IV) or general formula (V) or the pharmaceutically acceptable salt thereof is provided, wherein X is $CH_2$; $R^1$ is a hydrogen atom; $R^{3a}$ is selected from the group consisting of $C_{1-6}$ alkyl, 3- to 6-membered cycloalkyl, 3- to 6-membered heterocyclyl, 8- to 10-membered aryl and 5- to 10-membered heteroaryl, wherein the $C_{1-6}$ alkyl, 3- to 6-membered cycloalkyl, 3- to 6-membered heterocyclyl, 8- to 10-membered aryl and 5-to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy; $G^1$, $G^2$, $G^3$ and $G^4$ are all $CR^7$; $R^7$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; $Z^1$, $Z^3$ and $Z^4$ are all CH; j is 0.

In some preferred embodiments of the present disclosure, the compound of general formula (IV) or general formula (V) or the pharmaceutically acceptable salt thereof is provided, wherein X is $CH_2$; $R^1$ is a hydrogen atom; $R^{3a}$ is selected from the group consisting of 3- to 6-membered cycloalkyl, 3- to 6-membered heterocyclyl, 8- to 10-membered aryl and 5- to 10-membered heteroaryl, wherein the 3- to 6-membered cycloalkyl, 3- to 6-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl and $C_{1-6}$ haloalkoxy; $G^1$, $G^2$, $G^3$ and $G^4$ are all $CR^7$; $R^7$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; $Z^1$, $Z^3$ and $Z^4$

are all CH; j is 0.

In some preferred embodiments of the present disclosure, the compound of general formula (IV), general formula (IV-1), general formula (V) or general formula (VB) or the pharmaceutically acceptable salt thereof is provided, wherein X is an oxygen atom or $CH_2$; $R^1$ is a hydrogen atom; $R^{3a}$ is selected from the group consisting of $C_{1-6}$ alkyl, 3- to 6-membered cycloalkyl and 3- to 6-membered heterocyclyl, wherein the $C_{1-6}$ alkyl, 3- to 6-membered cycloalkyl and 3- to 6-membered heterocyclyl are each independently optionally substituted with one or more substituents selected from the group consisting of halogen, $C_{1-6}$ alkyl, $C_{1-6}$ haloalkyl, 3- to 6-membered cycloalkyl and phenyl; $G^1$, $G^2$, $G^3$ and $G^4$ are all $CR^7$; $R^7$ are identical or different and are each independently a hydrogen atom or a halogen; $Z^1$, $Z^3$ and $Z^4$ are all CH, or $Z^1$ is a nitrogen atom, and $Z^3$ and $Z^4$ are CH; j is 0.

In some preferred embodiments of the present disclosure, the compound of general formula (IV), general formula (IV-1), general formula (V) or general formula (VB) or the pharmaceutically acceptable salt thereof is provided, wherein X is $CH_2$; $R^1$ is a hydrogen atom; $R^{3a}$ is $C_{1-6}$ alkyl or 3- to 6-membered heterocyclyl, wherein the 3- to 6-membered heterocyclyl is optionally substituted with one or more substituents selected from the group consisting of halogen, $C_{1-6}$ alkyl and $C_{1-6}$ haloalkyl; $G^1$, $G^2$, $G^3$ and $G^4$ are all $CR^7$; $R^7$ are identical or different and are each independently a hydrogen atom or a fluorine atom; $Z^1$, $Z^3$ and $Z^4$ are all CH; j is 0.

In some preferred embodiments of the present disclosure, the compound of general formula (IV), general formula (IV-1), general formula (V) or general formula (VB) or the pharmaceutically acceptable salt thereof is provided, wherein X is $CH_2$; $R^1$ is a hydrogen atom; $R^{3a}$ is $C_{1-6}$ alkyl; $G^1$, $G^2$, $G^3$ and $G^4$ are all $CR^7$; $R^7$ are identical or different and are each independently a hydrogen atom or a fluorine atom; $Z^1$, $Z^3$ and $Z^4$ are all CH; j is 0.

Table A. Typical compounds of the present disclosure include, but are not limited to:

| Example No. | Structures and names of compounds |
|---|---|
| 1 | 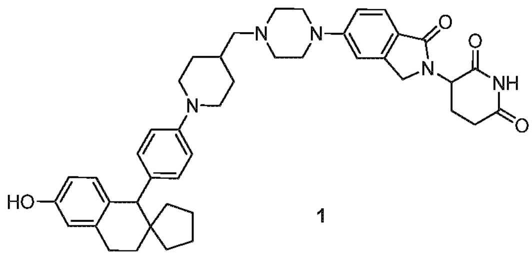 1 |
| | 3-(5-(4-((1-(4-(6'-Hydroxy-3',4'-dihydro-1'*H*-spiro[cyclopentane-1,2'-naphthalen]-1'-yl)phenyl ) piperidin-4yl)methyl)piperazin-1-yl)-1-oxoisoindolin-2-yl)piperidine-2,6-dione **1** |
| 2-1 | 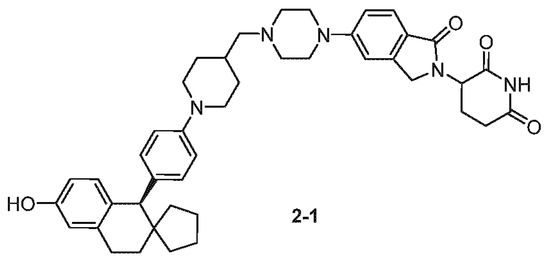 2-1 |
| | 3-(5-(4-((1-(4-((*R*)-6'-Hydroxy-3',4'-dihydro-1'*H*-spiro[cyclopentane-1,2'-naphthalen]-1'-yl)ph enyl) piperidin-4yl)methyl)piperazin-1-yl)-1-oxoisoindolin-2-yl)piperidine-2,6-dione **2-1** (a 1:1 mixture of diastereomers) |

(continued)

| Example No. | Structures and names of compounds |
|---|---|
| | 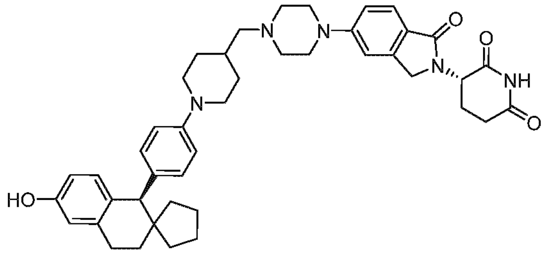 |
| | (*S*)-3-(5-(4-((1-(4-((*R*)-6'-Hydroxy-3',4'-dihydro-1'*H*-spiro[cyclopentane-1,2'-naphthalen]-1'-yl )phenyl) piperidin-4-yl)methyl)piperazin-1-yl)-1-oxoisoindolin-2-yl)piperidine-2,6-dione |
| | 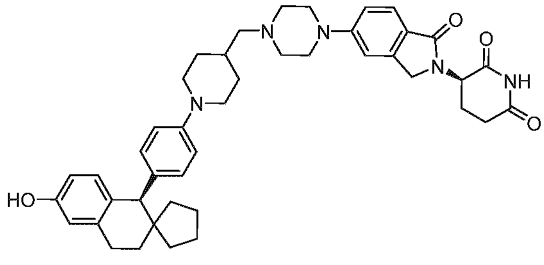 |
| | (*R*)-3-(5-(4-((1-(4-((*R*)-6'-Hydroxy-3',4'-dihydro-1'*H*-spiro[cyclopentane-1,2'-naphthalen]-1'-yl )phenyl) piperidin-4-yl)methyl)piperazin-1-yl)-1-oxoisoindolin-2-yl)piperidine-2,6-dione |
| **2-2** | 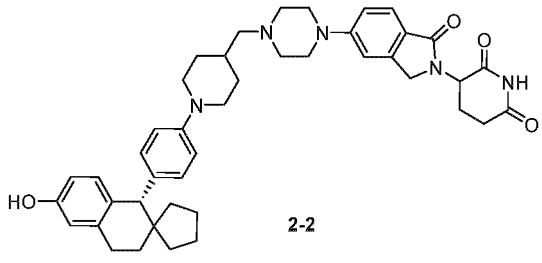 **2-2** |
| | 3-(5-(4-((1-(4-((*S*)-6'-Hydroxy-3',4'-dihydro-1'*H*-spiro[cyclopentane-1,2'-naphthalen]-1'-yl)ph enyl) piperidin-4-yl)methyl)piperazin-1-yl)-1-oxoisoindolin-2-yl)piperidine-2,6-dione **2-2** (a 1:1 mixture of diastereomers) |
| | 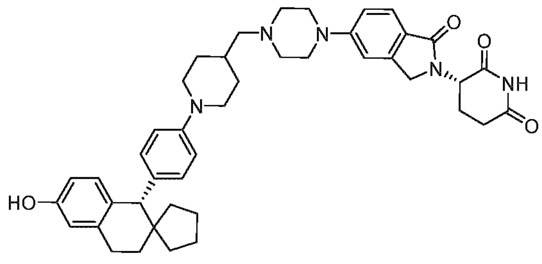 |
| | (*S*)-3-(5-(4-((1-(4-((*S*)-6'-Hydroxy-3',4'-dihydro-1'*H*-spiro[cyclopentane-1,2'-naphthalen]-1'-yl )phenyl) piperidin-4-yl)methyl)piperazin-1-yl)-1-oxoisoindolin-2-yl)piperidine-2,6-dione |

(continued)

| Example No. | Structures and names of compounds |
|---|---|
| | 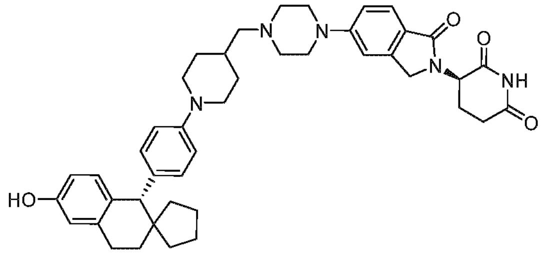<br>(*R*)-3-(5-(4-((1-(4-((*S*)-6'-Hydroxy-3',4'-dihydro-1'*H*-spiro[cyclopentane-1,2'-naphthalen]-1'-yl )phenyl) piperidin-4-yl)methyl)piperazin-1-yl)-1-oxoisoindolin-2-yl)piperidine-2,6-dione |
| | 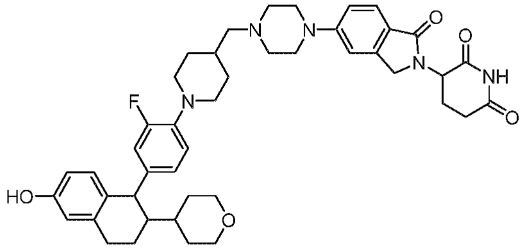 |
| **3** | 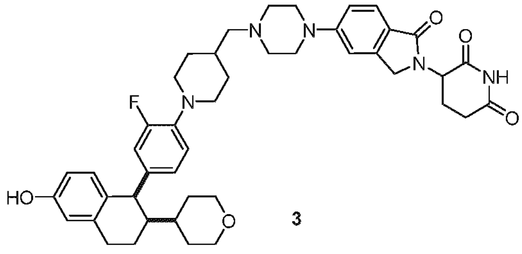<br>**3**<br>3-(5-(4-((1-(2-Fluoro-4-((1,2)-*cis*-6-hydroxy-2-(tetrahydro-2*H*-pyran-4-yl)-1,2,3,4-tetrahydron aphthalen-1-yl)phenyl)piperidin-4-yl)methyl)piperazin-1-yl)-1-oxoisoindolin-2-yl)piperidine-2,6-dione **3** |
| **4-1** | 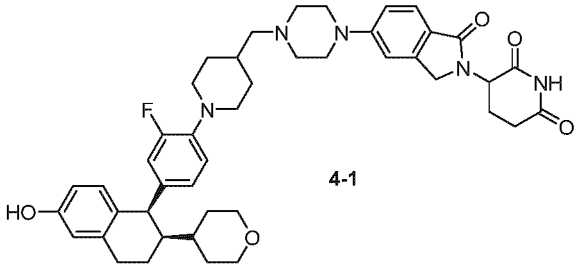<br>**4-1**<br>3-(5-(4-((1-(2-Fluoro-4-((1*R*,2*R*)-6-hydroxy-2-(tetrahydro-2*H*-pyran-4-yl)-1,2,3,4-tetrahydron aphthalen-1-yl)phenyl)piperidin-4-yl)methyl)piperazin-1-yl)-1-oxoisoindolin-2yl)piperidine-2,6-dione **4-1** (a 1:1 mixture of diastereomers) |

(continued)

| Example No. | Structures and names of compounds |
|---|---|
| **4-2** | 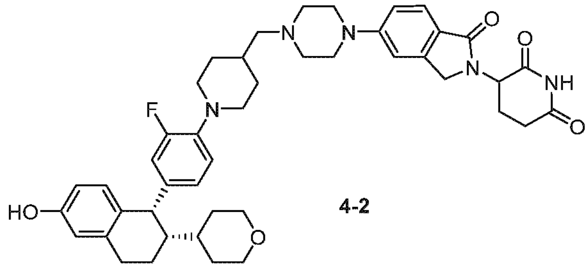 **4-2** |
| | 3-(5-(4-((1-(2-Fluoro-4-((1*S*,2*S*)-6-hydroxy-2-(tetrahydro-2*H*-pyran-4-yl)-1,2,3,4-tetrahydrona phthalen-1-yl)phenyl)piperidin-4-yl)methyl)piperazin-1-yl)-1-oxoisoindolin-2-yl)piperidine-2, 6-dione **4-2** (a 1:1 mixture of diastereomers) |
| | 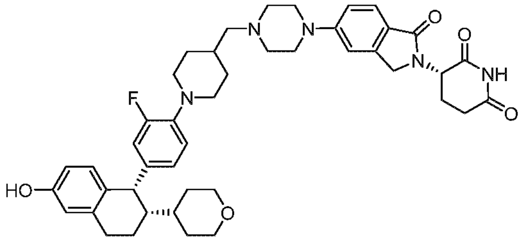 |
| | (*S*)-3-(5-(4-((1-(2-Fluoro-4-((1*S*,2*S*)-6-hydroxy-2-(tetrahydro-2*H*-pyran-4-yl)-1,2,3,4-tetrahyd ronaphthalen-1-yl)phenyl)piperidin-4-yl)methyl)piperazin-1-yl)-1-oxoisoindolin-2-yl)piperidi ne-2,6-dione |
| | 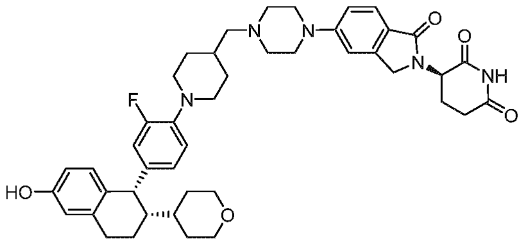 |
| | (*R*)-3-(5-(4-((1-(2-Fluoro-4-((1*S*,2*S*)-6-hydroxy-2-(tetrahydro-2*H*-pyran-4-yl)-1,2,3,4-tetrahyd ronaphthalen-1-yl)phenyl)piperidin-4-yl)methyl)piperazin-1-yl)-1-oxoisoindolin-2-yl)piperidi ne-2,6-dione |
| 5 | 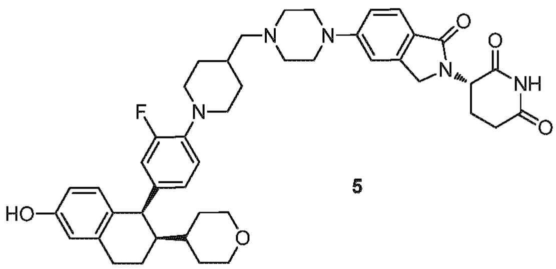 **5** |
| | (*S*)-3-(5-(4-((1-(2-Fluoro-4-((1*R*,2*R*)-6-hydroxy-2-(tetrahydro-2*H*-pyran-4-yl)-1,2,3,4-tetrahyd ronaphthalen-1-yl)phenyl)piperidin-4-yl)methyl)piperazin-1-yl)-1-oxoisoindolin-2-yl)piperidi ne-2,6-dione **5** |

(continued)

| Example No. | Structures and names of compounds |
|---|---|
| | 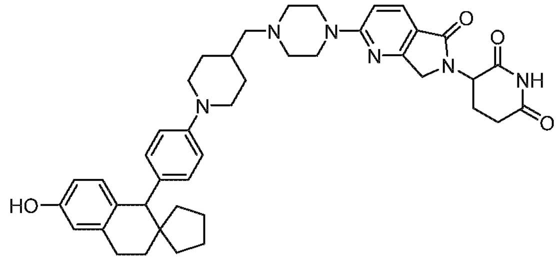 |
| 6 | 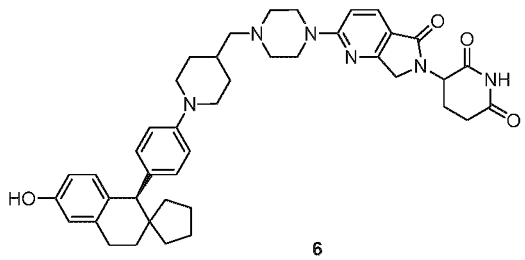<br>**6** |
| | 3-(2-(4-((1-(4-((*R*)-6'-Hydroxy-3',4'-dihydro-1'*H*-spiro[cyclopentane-1,2'-naphthalen]-1'-yl)ph enyl) piperidin-4-yl)methyl)piperazin-1-yl)-5-oxo-5,7-dihydro-6*H*-pyrrolo[3,4-*b*]pyridin-6-yl) piperidine-2,6-dione **6** (a 1:1 mixture of diastereomers) |
| | 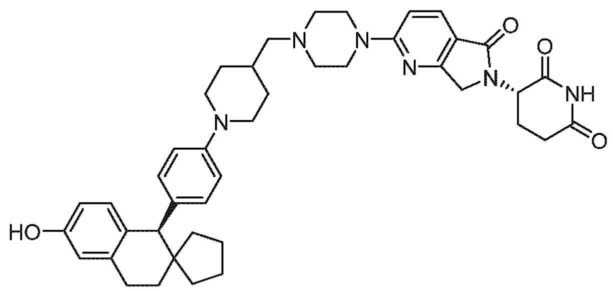 |
| | (*S*)-3-(2-(4-((1-(4-((*R*)-6'-Hydroxy-3',4'-dihydro-1'*H*-spiro[cyclopentane-1,2'-naphthalen]-1'-yl )phenyl) piperidin-4-yl)methyl)piperazin-1-yl)-5-oxo-5,7-dihydro-6*H*-pyrrolo[3,4-*b*]pyridin-6-yl)piperidine-2,6-dione |
| | 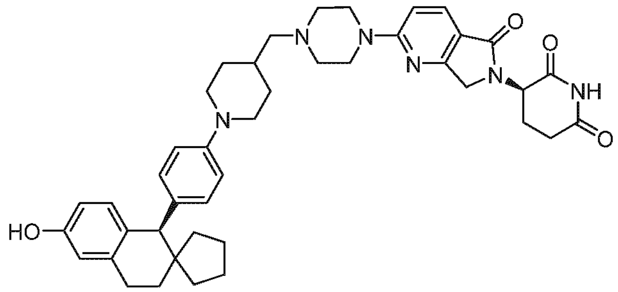 |
| | (*R*)-3-(2-(4-((1-(4-((*R*)-6'-Hydroxy-3',4'-dihydro-1'*H*-spiro[cyclopentane-1,2'-naphthalen]-1'-yl )phenyl) piperidin-4-yl)methyl)piperazin-1-yl)-5-oxo-5,7-dihydro-6*H*-pyrrolo[3,4-*b*]pyridin-6-yl)piperidine-2,6-dione |

(continued)

| Example No. | Structures and names of compounds |
|---|---|
| | 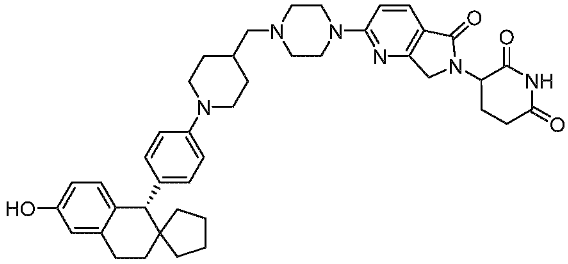 |
| | 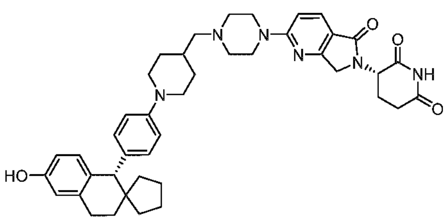 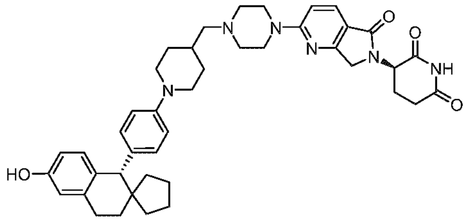 |
| | 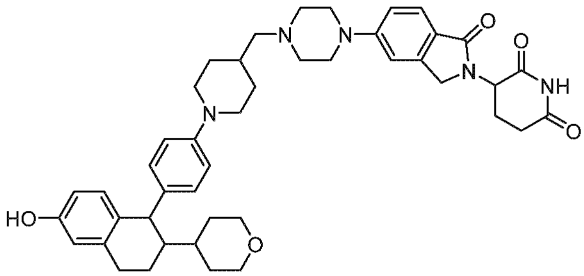 |
| 7 | 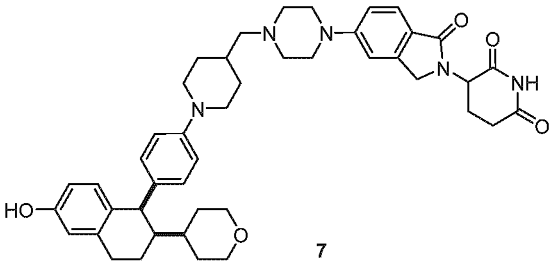 7<br>3-(5-(4-((1-(4-((1,2)-*cis*-6-Hydroxy-2-(tetrahydro-2*H*-pyran-4-yl)-1,2,3,4-tetrahydronaphthale n-1-yl) phenyl)piperidin-4-yl)methyl)piperazin-1-yl)-1-oxoisoindolin-2-yl)piperidine-2,6-dione 7 |
| 8-1 | 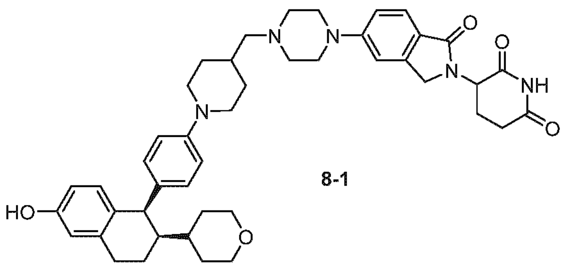 8-1<br>3-(5-(4-((1-(4-((1*R*,2*R*)-6-Hydroxy-2-(tetrahydro-2*H*-pyran-4-yl)-1,2,3,4-tetrahydronaphthale n-1-yl) phenyl)piperidin-4-yl)methyl)piperazin-1-yl)-1-oxoisoindolin-2-yl)piperidine-2,6-dione **8-1** (a 1:1 mixture of diastereomers) |

(continued)

| Example No. | Structures and names of compounds |
|---|---|
| | 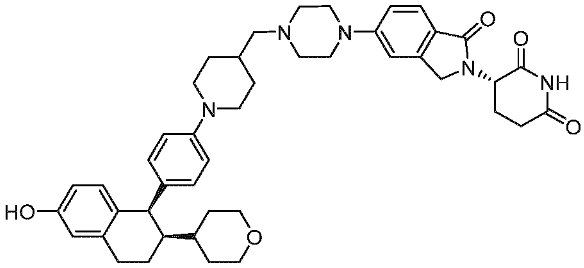 |
| | (*S*)-3-(5-(4-((1-(4-((1*R*,2*R*)-6-Hydroxy-2-(tetrahydro-2*H*-pyran-4-yl)-1,2,3,4-tetrahydronaphth alen-1-yl) phenyl)piperidin-4-yl)methyl)piperazin-1-yl)-1-oxoisoindolin-2-yl)piperidine-2,6-di one |
| | 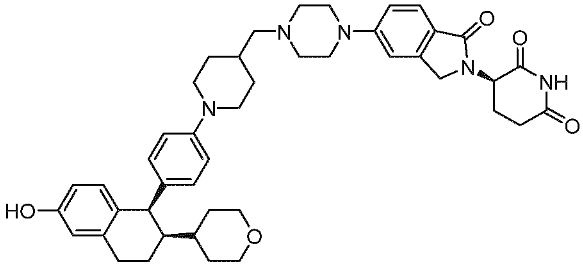 |
| | (*R*)-3-(5-(4-((1-(4-((1*R*,2*R*)-6-Hydroxy-2-(tetrahydro-2*H*-pyran-4-yl)-1,2,3,4-tetrahydronaphth alen-1-yl) phenyl)piperidin-4-yl)methyl)piperazin-1-yl)-1-oxoisoindolin-2-yl)piperidine-2,6-di one |
| 8-2 | 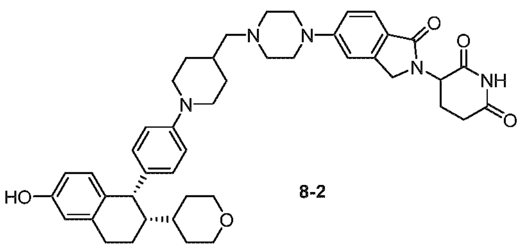 **8-2** |
| | 3-(5-(4-((1-(4-((1*S*,2*S*)-6-Hydroxy-2-(tetrahydro-2*H*-pyran-4-yl)-1,2,3,4-tetrahydronaphthalen -1-yl) phenyl)piperidin-4-yl)methyl)piperazin-1-yl)-1-oxoisoindolin-2-yl)piperidine-2,6-dione **8-2** (a 1:1 mixture of diastereomers) |
| | 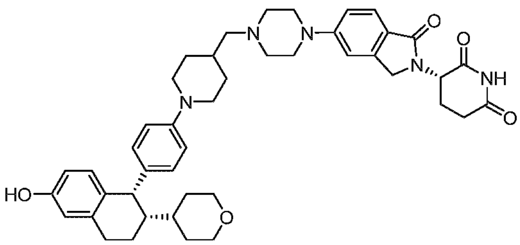 |
| | (*S*)-3-(5-(4-((1-(4-((1*S*,2,*S*)-6-Hydroxy-2-(tetrahydro-2*H*-pyran-4-yl)-1,2,3,4-tetrahydronaphthalen-1yl) phenyl)piperidin-4-yl)methyl)piperazin-1-yl)-1-oxoisoindolin-2-yl)piperidine-2,6-di one |

(continued)

| Example No. | Structures and names of compounds |
|---|---|
| | 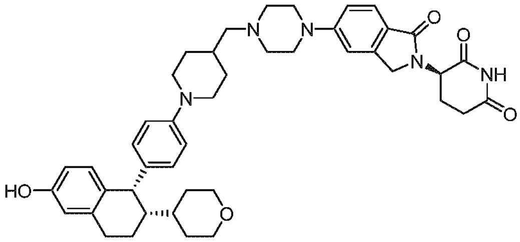 |
| | (*R*)-3-(5-(4-(1-(4-((1*S*,2*S*)-6-Hydroxy-2-(tetrahydro-2*H*-pyran-4-yl)-1,2,3,4-tetrahydronaphth alen-1yl) phenyl)piperidin-4-yl)methyl)piperazin-1-yl)-1-oxoisoindolin-2-yl)piperidine-2,6-di one |
| | 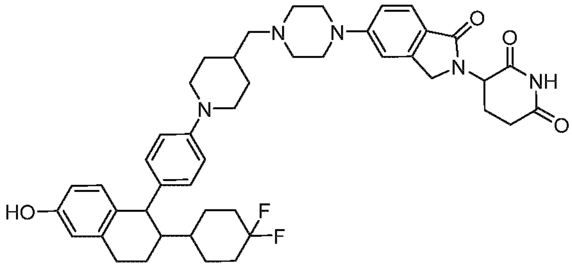 |
| **9** | 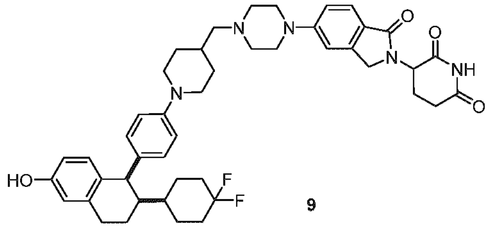 9 |
| | 3-(5-(4-((1-(4-((1,2)-*cis*-2-(4,4-Difluorocyclohexyl)-6-hydroxy-1,2,3,4-tetrahydronaphthalen-1-yl) phenyl)piperidin-4-yl)methyl)piperazin-1-yl)-1-oxoisoindolin-2-yl)piperidine-2,6-dione **9** |
| **10** | 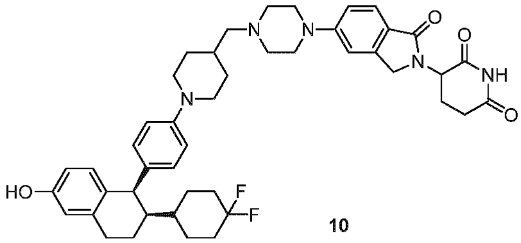 10 |
| | 3-(5-(4-((1-(4-((1*R*,2*R*)-2-(4,4-Difluorocyclohexyl)-6-hydroxy-1,2,3,4-tetrahydronaphthalen-1 -yl) phenyl)piperidin-4-yl)methyl)piperazin-1-yl)-1-oxoisoindolin-2-yl)piperidine-2,6-dione **10** (a 1:1 mixture of diastereomers) |

(continued)

| Example No. | Structures and names of compounds |
|---|---|
| | 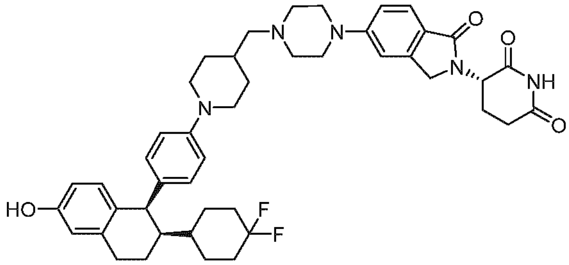 |
| | (*S*)-3-(5-(4-((1-(4-((1*R*,2*R*)-2-(4,4-Difluorocyclohexyl)-6-hydroxy-1,2,3,4-tetrahydronaphthale n-1-yl) phenyl)piperidin-4-yl)methyl)piperazin-1-yl)-1-oxoisoindolin-2-yl)piperidine-2,6-dione |
| | 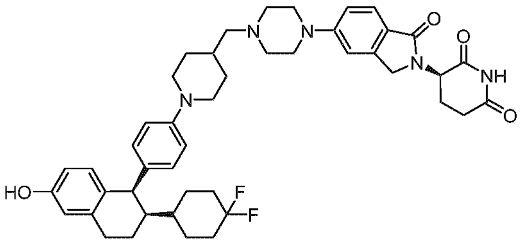 |
| | (*R*)-3-(5-(4-((1-(4-((1*R*,2*R*)-2-(4,4-Difluorocyclohexyl)-6-hydroxy-1,2,3,4-tetrahydronaphthal en-1-yl) phenyl)piperidin-4-yl)methyl)piperazin-1-yl)-1-oxoisoindolin-2-yl)piperidine-2,6-dion e |
| | 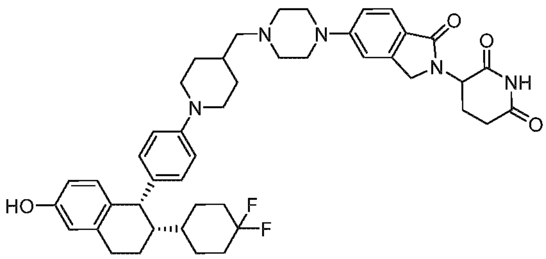 |
| | 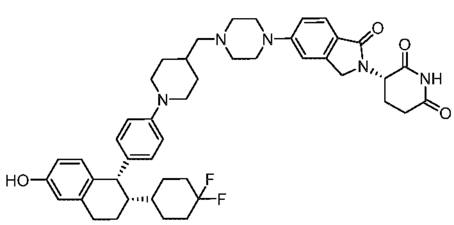 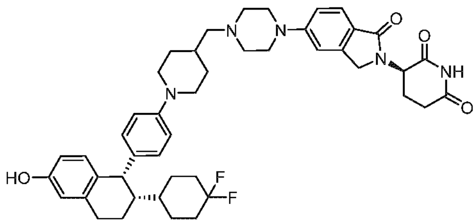 |
| | 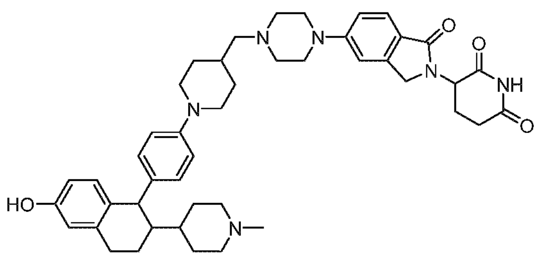 |

(continued)

| Example No. | Structures and names of compounds |
|---|---|
| **11** | 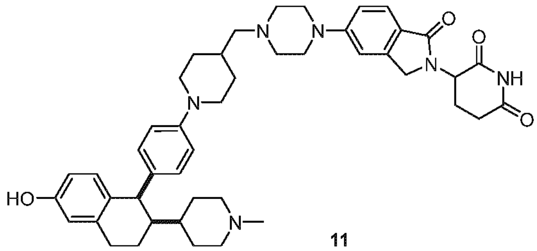 11 |
| | 3-(5-(4-((1-(4-((1,2)-*cis*-6-Hydroxy-2-(1-methylpiperidin-4-yl)-1,2,3,4-tetrahydronaphthalen-1-yl)phenyl) piperidin-4-yl)methyl)piperazin-1-yl)-1-oxoisoindolin-2-yl)piperidine-2,6-dione **11** |
| | 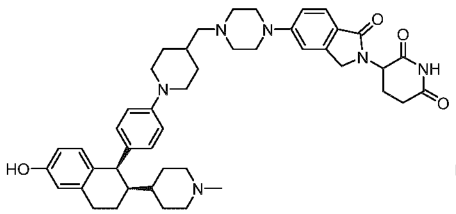 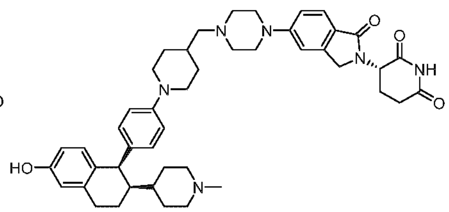 |
| | 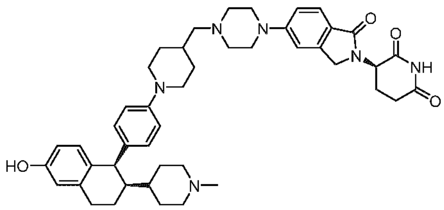 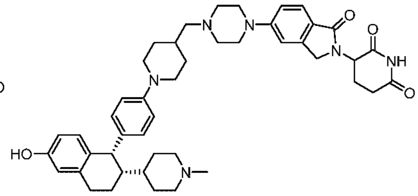 |
| | 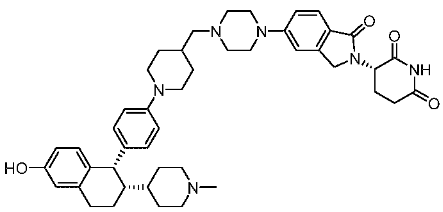 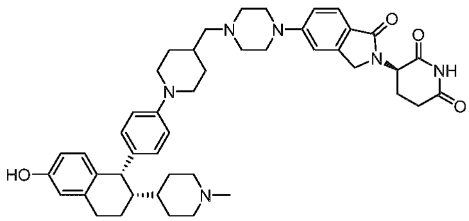 |
| | 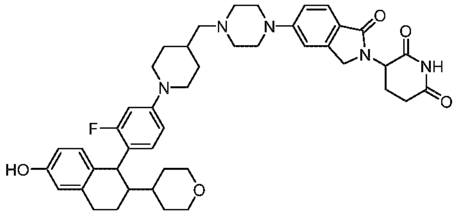 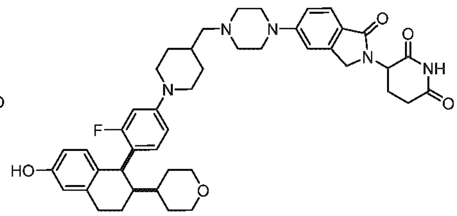 |
| **12-1** | 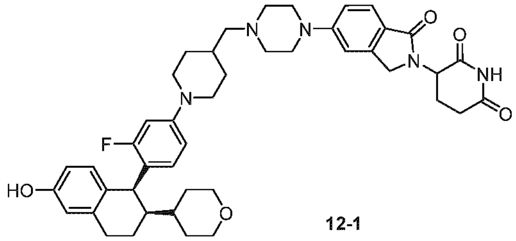 12-1 |
| | 3-(5-(4-((1-(3-Fluoro-4-((1*S*,2*R*)-6-hydroxy-2-(tetrahydro-2*H*-pyran-4-yl)-1,2,3,4-tetrahydron aphthalen-1-yl)phenyl)piperidin-4-yl)methyl)piperazin-1-yl)-1-oxoisoindolin-2-yl)piperidine-2,6-dione **12-1** (a 1:1 mixture of diastereomers) |

(continued)

| Example No. | Structures and names of compounds |
|---|---|
| | 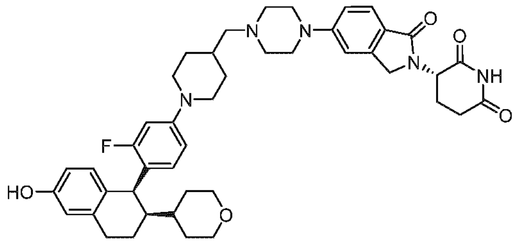<br>(*S*)-3-(5-(4-((1-(3-Fluoro-4-((1*S*,2*R*)-6-hydroxy-2-(tetrahydro-2*H*-pyran-4-yl)-1,2,3,4-tetrahydronaphthalen-1-yl)phenyl)piperidin-4-yl)methyl)piperazin-1-yl)-1-oxoisoindolin-2-yl)piperidi ne-2,6-dione |
| | 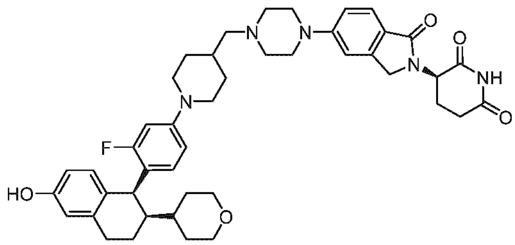<br>(*R*)-3-(5-(4-((1-(3-Fluoro-4-((1*S*,2*R*)-6-hydroxy-2-(tetrahydro-2*H*-pyran-4-yl)-1,2,3,4-tetrahydronaphthalen-1-yl)phenyl)piperidin-4-yl)methyl)piperazin-1-yl)-1-oxoisoindolin-2-yl)piperidi ne-2,6-dione |
| **12-2** | 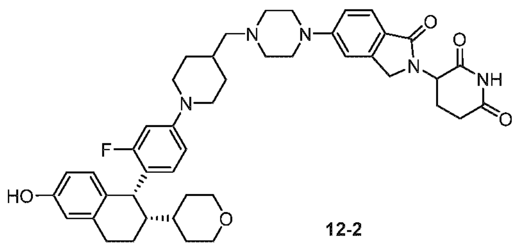 **12-2**<br>3-(5-(4-((1-(3-Fluoro-4-((1*R*,2*S*)-6-hydroxy-2-(tetrahydro-2*H*-pyran-4-yl)-1,2,3,4-tetrahydron aphthalen-1-yl)phenyl)piperidin-4-yl)methyl)piperazin-1-yl)-1-oxoisoindolin-2-yl)piperidine-2,6-dione **12-2** (a 1:1 mixture of diastereomers) |
| | 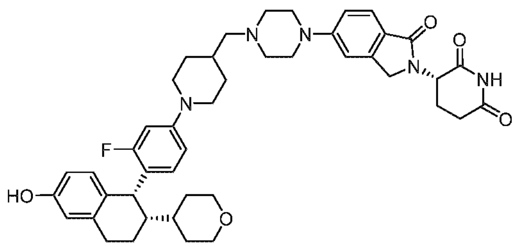<br>(*S*)-3-(5-(4-((1-(3-Fluoro-4-((1*R*,2*S*)-6-hydroxy-2-(tetrahydro-2*H*-pyran-4-yl)-1,2,3,4-tetrahydronaphthalen-1-yl)phenyl)piperidin-4-yl)methyl)piperazin-1-yl)-1-oxoisoindolin-2-yl)piperidi ne-2,6-dione |

(continued)

| Example No. | Structures and names of compounds |
|---|---|
| | 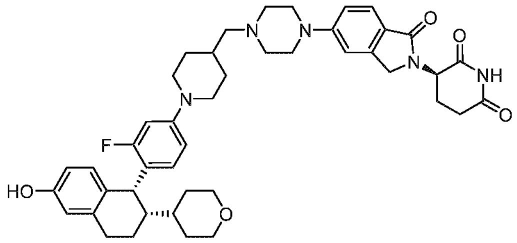 |
| | (*R*)-3-(5-(4-((1-(3-Fluoro-4-((1*R*,2*S*)-6-hydroxy-2-(tetrahydro-2*H*-pyran-4-yl)-1,2,3,4-tetrahyd ronaphthalen-1-yl)phenyl)piperidin-4-yl)methyl)piperazin-1-yl)-1-oxoisoindolin-2-yl)piperidi ne-2,6-dione |
| | 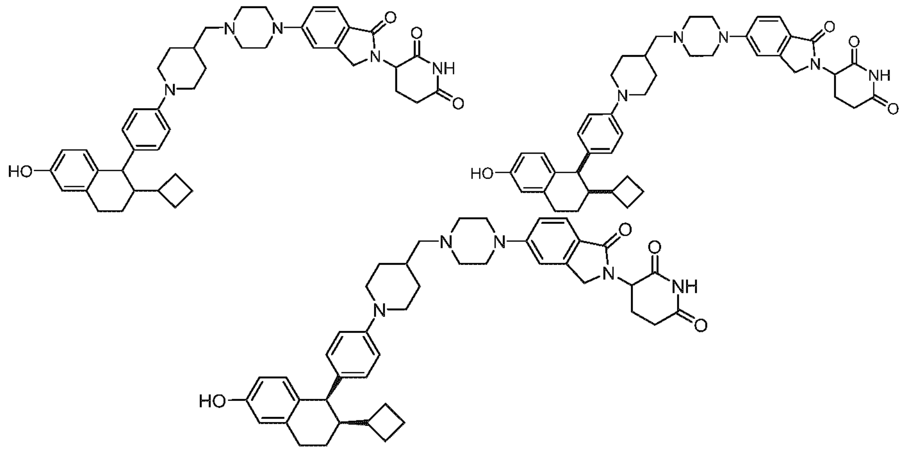 |
| **13-1** | 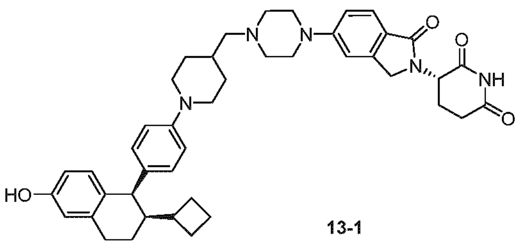 **13-1** |
| | (*S*)-3-(5-(4-((1-(4-((1*R*,2*R*)-2-Cyclobutyl-6-hydroxy-1,2,3,4-tetrahydronaphthalen-1-yl)phenyl ) piperidin-4-yl)methyl)piperazin-1-yl)-1-oxoisoindolin-2-yl)piperidine-2,6-dione **13-1** |
| | 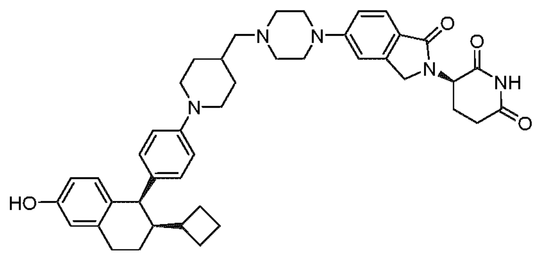 |

(continued)

| Example No. | Structures and names of compounds |
|---|---|
| **13-2** | 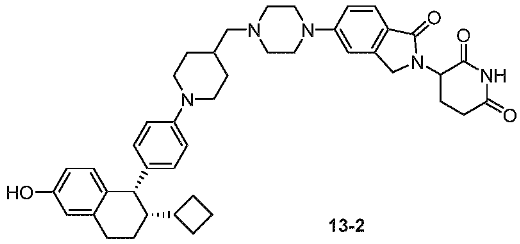 **13-2** |
| | 3-(5-(4-((1-(4-((1*S*,2*S*)-2-Cyclobutyl-6-hydroxy-1,2,3,4-tetrahydronaphthalen-1-yl)phenyl)pip eridin-4-yl)methyl)piperazin-1-yl)-1-oxoisoindolin-2-yl)piperidine-2,6-dione **13-2** (a 1:1 mixture of diastereomers) |
| | 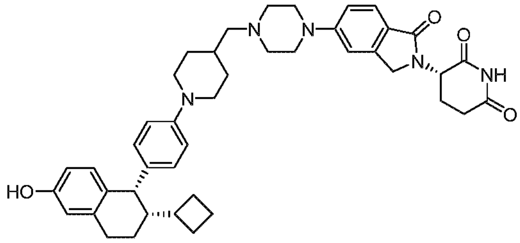 |
| | (*S*)-3-(5-(4-((1-(4-((1*S*,2*S*)-2-Cyclobutyl-6-hydroxy-1,2,3,4-tetrahydronaphthalen-1-yl)phenyl) piperidin-4-yl)methyl)piperazin-1-yl)-1-oxoisoindolin-2-yl)piperidine-2,6-dione |
| | 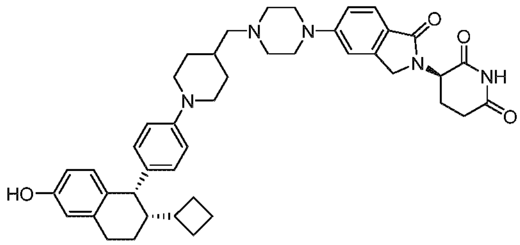 |
| | (*R*)-3-(5-(4-((1-(4-((1*S*,2*S*)-2-Cyclobutyl-6-hydroxy-1,2,3,4-tetrahydronaphthalen-1-yl)phenyl ) piperidin-4-yl)methyl)piperazin-1-yl)-1-oxoisoindolin-2-yl)piperidine-2,6-dione |
| | 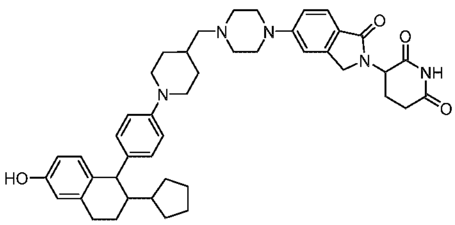 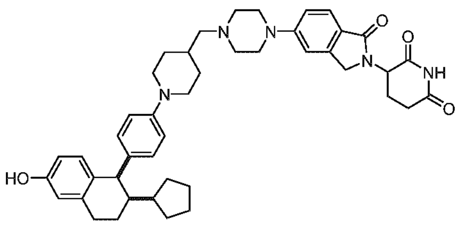 |
| **14-1** | 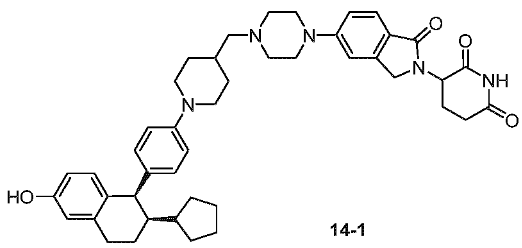 **14-1** |
| | 3-(5-(4-((1-(4-((1*R*,2*R*)-2-Cyclopentyl-6-hydroxy-1,2,3,4-tetrahydronaphthalen-1-yl)phenyl)pi peridin-4-yl)methyl)piperazin-1-yl)-1-oxoisoindolin-2-yl)piperidine-2,6-dione **14-1** (a 1:1 mixture of diastereomers) |

(continued)

| Example No. | Structures and names of compounds |
|---|---|
| | 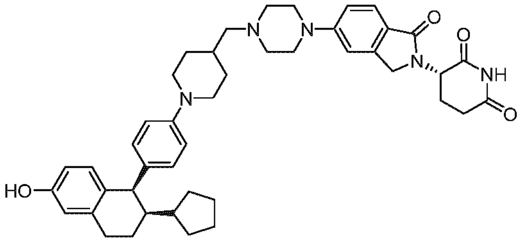 |
| | (*S*)-3-(5-(4-((1-(4-((1*R*,2*R*)-2-Cyclopentyl-6-hydroxy-1,2,3,4-tetrahydronaphthalen-1-yl)phen yl) piperidin-4-yl)methyl)piperazin-1-yl)-1-oxoisoindolin-2-yl)piperidine-2,6-dione |
| | 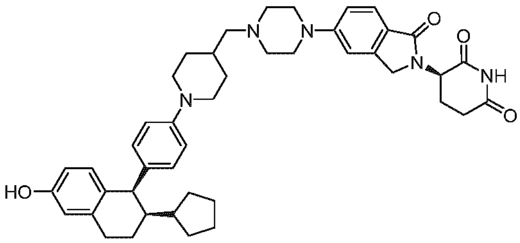 |
| | (*R*)-3-(5-(4-((1-(4-((1*R*,2*R*)-2-Cyclopentyl-6-hydroxy-1,2,3,4-tetrahydronaphthalen-1-yl)phen yl) piperidin-4-yl)methyl)piperazin-1-yl)-1-oxoisoindolin-2-yl)piperidine-2,6-dione |
| **14-2** | 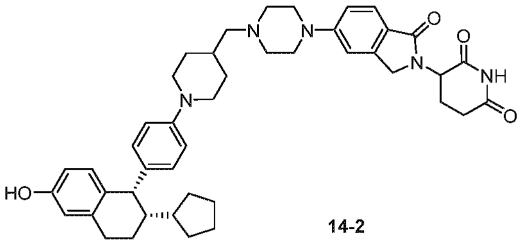 **14-2** |
| | 3-(5-(4-((1-(4-((1*S*,2*S*)-2-Cyclopentyl-6-hydroxy-1,2,3,4-tetrahydronaphthalen-1-yl)phenyl)pi peridin-4-yl)methyl)piperazin-1-yl)-1-oxoisoindolin-2-yl)piperidine-2,6-dione **14-2** (a 1:1 mixture of diastereomers) |
| | 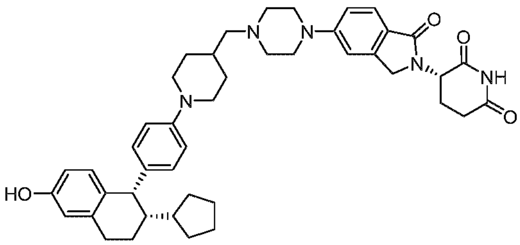 |
| | (*S*)-3-(5-(4-((1-(4-((1*S*,2*S*)-2-Cyclopentyl-6-hydroxy-1,2,3,4-tetrahydronaphthalen-1-yl)pheny l) piperidin-4-yl)methyl)piperazin-1-yl)-1-oxoisoindolin-2-yl)piperidine-2,6-dione |

(continued)

| Example No. | Structures and names of compounds |
|---|---|
| | 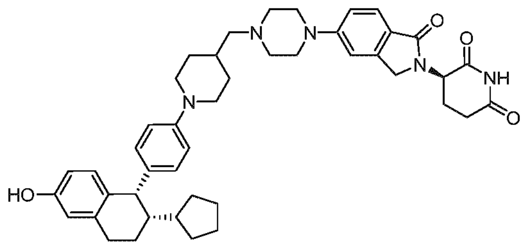 |
| | (*R*)-3-(5-(4-((1-(4-((1*S*,2*S*)-2-Cyclopentyl-6-hydroxy-1,2,3,4-tetrahydronaphthalen-1-yl)pheny l)piperidin-4-yl)methyl)piperazin-1-yl)-1-oxoisoindolin-2-yl)piperidine-2,6-dione |
| | 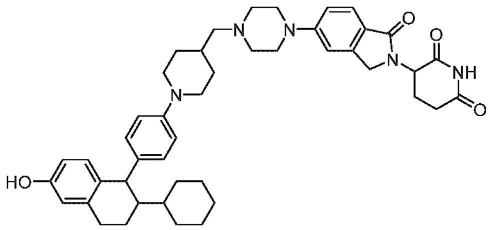 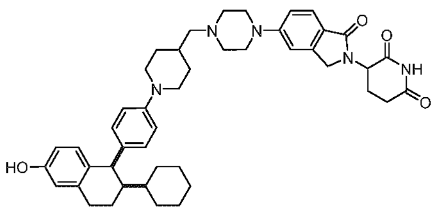 |
| **15-1** | 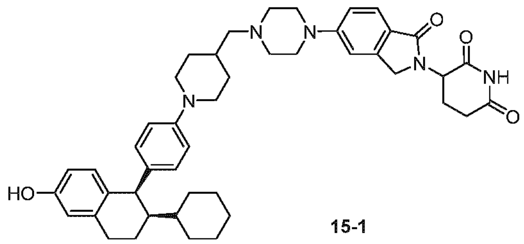 **15-1** |
| | 3-(5-(4-((1-(4-((1*R*,2*R*)-2-Cyclohexyl-6-hydroxy-1,2,3,4-tetrahydronaphthalen-1-yl)phenyl)pi peridin-4-yl)methyl)piperazin-1-yl)-1-oxoisoindolin-2-yl)piperidine-2,6-dione **15-1** (a 1:1 mixture of diastereomers) |
| | 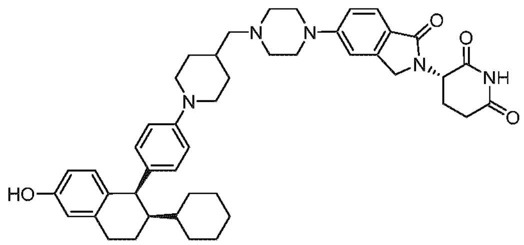 |
| | (*S*)-3-(5-(4-((1-(4-((1*R*,2*R*)-2-Cyclohexyl-6-hydroxy-1,2,3,4-tetrahydronaphthalen-1-yl)pheny l)piperidin-4-yl)methyl)piperazin-1-yl)-1-oxoisoindolin-2-yl)piperidine-2,6-dione |
| | 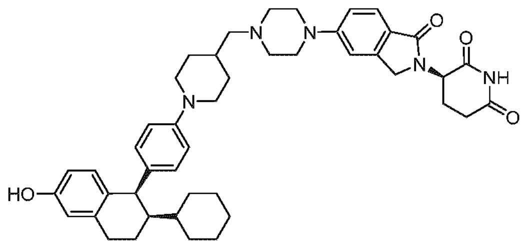 |
| | (*R*)-3-(5-(4-((1-(4-((1*R*,2*R*)-2-Cyclohexyl-6-hydroxy-1,2,3,4-tetrahydronaphthalen-1-yl)pheny l)piperidin-4-yl)methyl)piperazin-1-yl)-1-oxoisoindolin-2-yl)piperidine-2,6-dione |

(continued)

| Example No. | Structures and names of compounds |
|---|---|
| **15-2** | 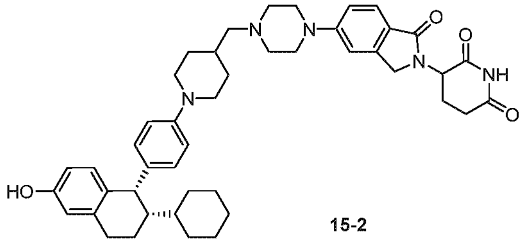 15-2 |
| | 3-(5-(4-((1-(4-((1*S*,2*S*)-2-Cyclohexyl-6-hydroxy-1,2,3,4-tetrahydronaphthalen-1-yl)phenyl)pip eridin-4-yl)methyl)piperazin-1-yl)-1-oxoisoindolin-2-yl)piperidine-2,6-dione **15-2** (a 1:1 mixture of diastereomers) |
| | 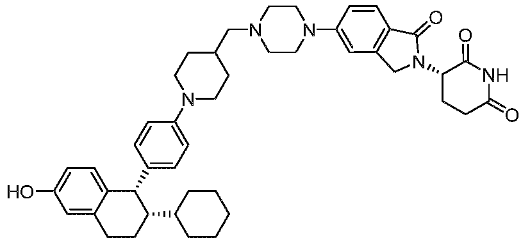 |
| | (*S*)-3-(5-(4-((1-(4-((1*S*,2*S*)-2-Cyclohexyl-6-hydroxy-1,2,3,4-tetrahydronaphthalen-1-yl)phenyl ) piperidin-4-yl)methyl)piperazin-1-yl)-1-oxoisoindolin-2-yl)piperidine-2,6-dione |
| | 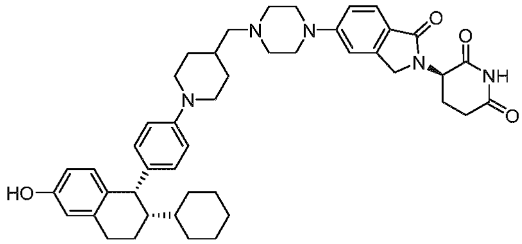 |
| | (*R*)-3-(5-(4-((1-(4-((1*S*,2*S*)-2-Cyclohexyl-6-hydroxy-1,2,3,4-tetrahydronaphthalen-1-yl)phenyl ) piperidin-4-yl)methyl)piperazin-1-yl)-1-oxoisoindolin-2-yl)piperidine-2,6-dione |
| | 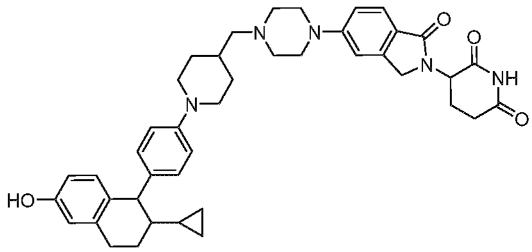 |
| **16** | 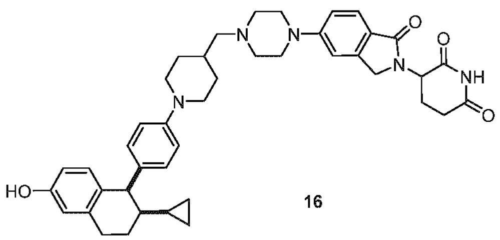 16 |
| | 3-(5-(4-((1-(4-((1,2)-*cis*-2-Cyclopropyl-6-hydroxy-1,2,3,4-tetrahydronaphthalen-1-yl)phenyl)p iperidin-4-yl)methyl)piperazin-1-yl)-1-oxoisoindolin-2-yl)piperidine-2,6-dione **16** |

(continued)

| Example No. | Structures and names of compounds |
|---|---|
| | 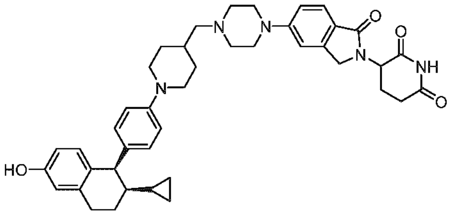 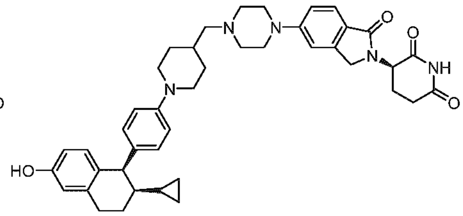 |
| **17-1** | 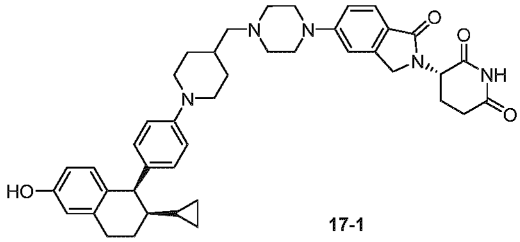 17-1 |
| | (*S*)-3-(5-(4-((1-(4-(((1*R*,2*R*)-2-Cyclopropyl-6-hydroxy-1,2,3,4-tetrahydronaphthalen-1-yl)phen yl)) piperidin-4-yl)methyl)piperazin-1-yl)-1-oxoisoindolin-2-yl)piperidine-2,6-dione **17-1** |
| **17-2** | 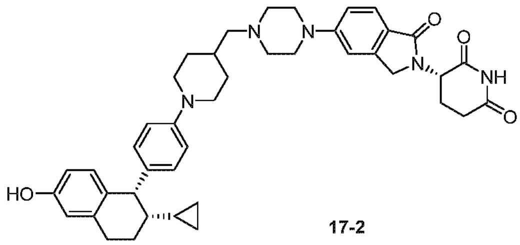 17-2 |
| | (*S*)-3-(5-(4-((1-(4-(((1*S*,2*S*)-2-Cyclopropyl-6-hydroxy-1,2,3,4-tetrahydronaphthalen-1-yl)phen yl)) piperidin-4-yl)methyl)piperazin-1-yl)-1-oxoisoindofin-2-yl)piperidine-2,6-dione **17-2** |
| | 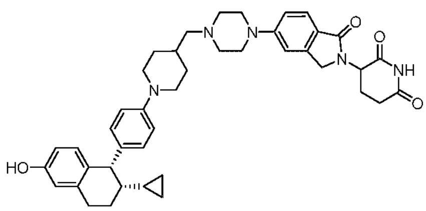 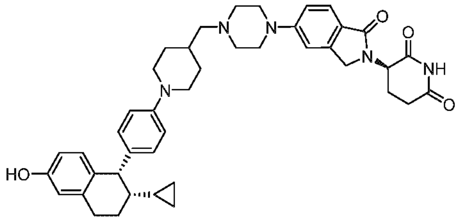 |
| | 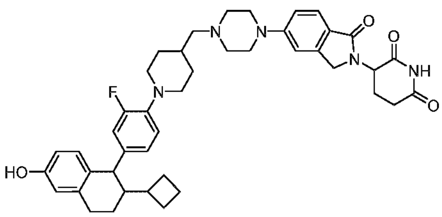 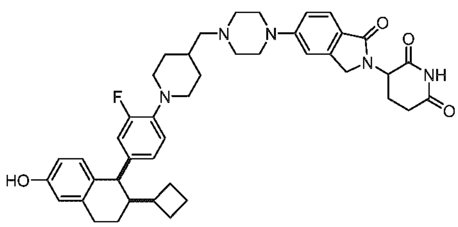 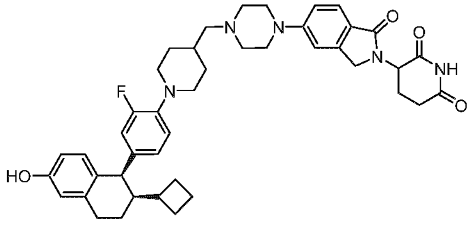 |

(continued)

| Example No. | Structures and names of compounds |
|---|---|
| **18-1** | 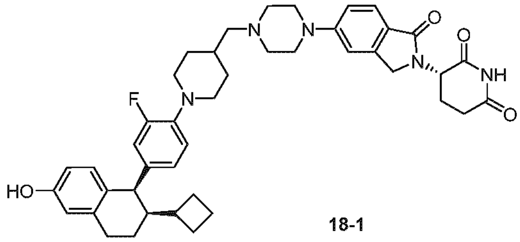 18-1 |
| | (*S*)-3-(5-(4-((1-(4-((1*R*,2*R*)-2-Cyclobutyl-6-hydroxy-1,2,3,4-tetrahydronaphthalen-1-yl)-2-fluo rophenyl) piperidin-4-yl)methyl)piperazin-1-yl)-1-oxoisoindolin-2-yl)piperidine-2,6-dione **18-1** |
| | 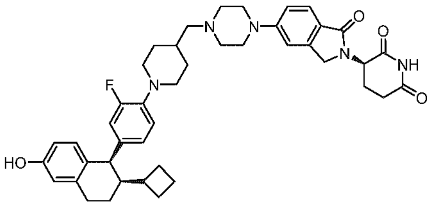 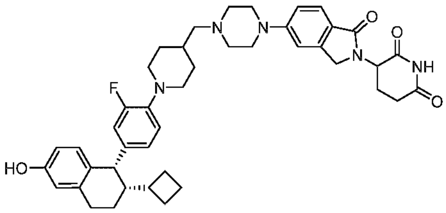 |
| **18-2** | 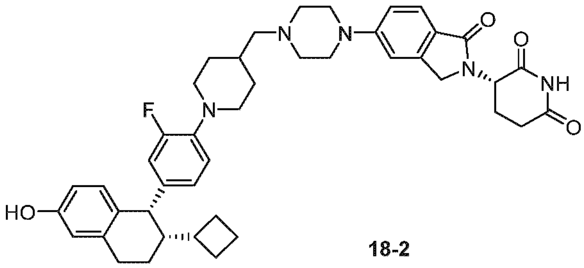 18-2 |
| | (*S*)-3-(5-(4-((1-(4-((1*S*,2*S*)-2-Cyclobutyl-6-hydroxy-1,2,3,4-tetrahydronaphthalen-1-yl)-2-fluo rophenyl) piperidin-4-yl)methyl)piperazin-1-yl)-1-oxoisoindolin-2-yl)piperidine-2,6-dione **18-2** |
| | 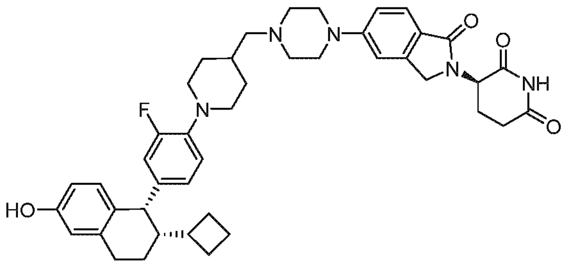 |
| | 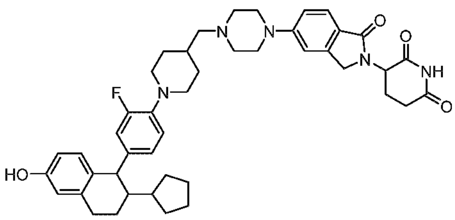 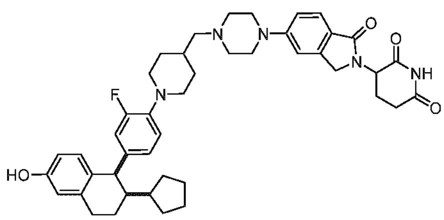 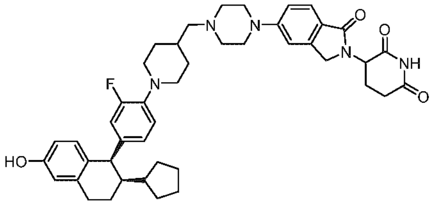 |

(continued)

| Example No. | Structures and names of compounds |
|---|---|
| **19** | 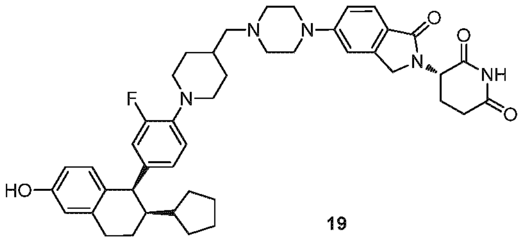 **19** |
| | (*S*)-3-(5-(4-((1-(4-((1*R*,2*R*)-2-cyclopentyl-6-hydroxy-1,2,3,4-tetrahydronaphthalen-1-yl)-2-flu orophenyl) piperidin-4-yl)methyl)piperazin-1-yl)-1-oxoisoindolin-2-yl)piperidine-2,6-dione **19** |
| | 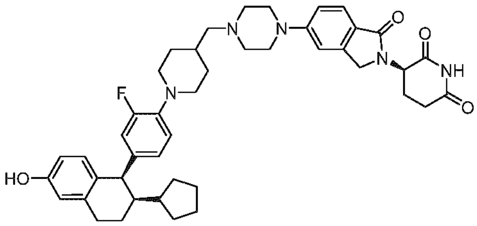 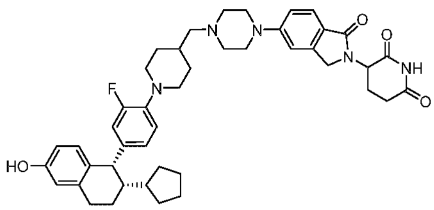 |
| | 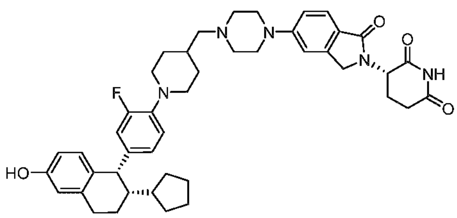 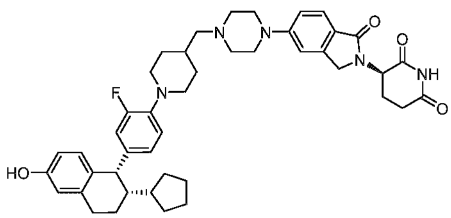 |
| | 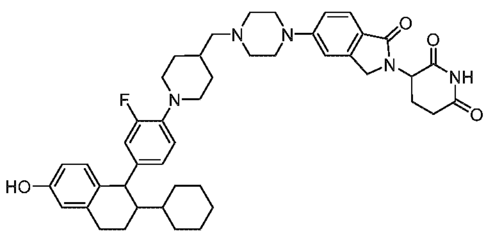 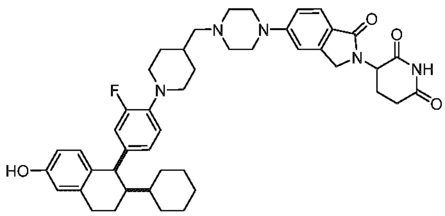 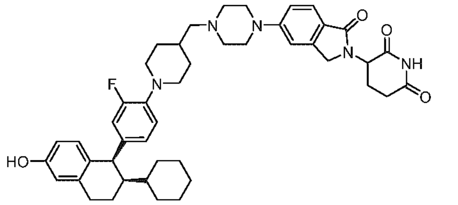 |
| **20** | 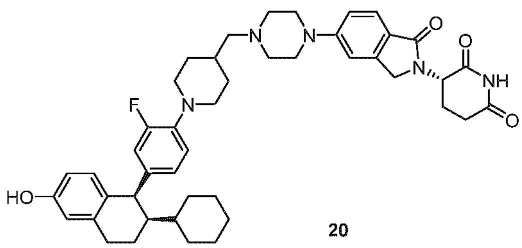 **20** |
| | (*S*)-3-(5-(4-((1-(4-((1*R*,2*R*)-2-Cyclohexyl-6-hydroxy-1,2,3,4-tetrahydronaphthalen-1-yl)-2-flu orophenyl) piperidin-4-yl)methyl)piperazin-1-yl)-1-oxoisoindolin-2-yl)piperidine-2,6-dione **20** |

(continued)

| Example No. | Structures and names of compounds |
|---|---|
| | 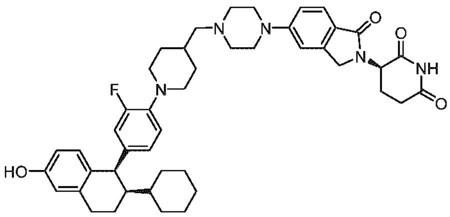 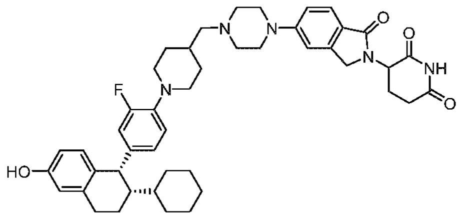 |
| | 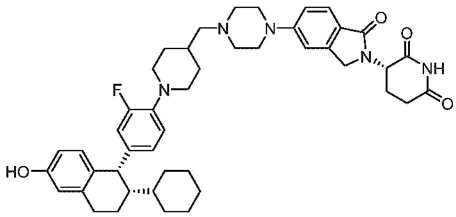 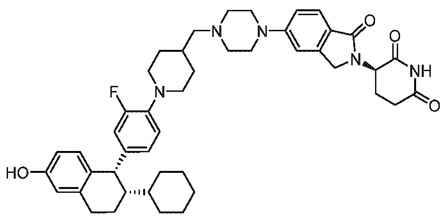 |
| | 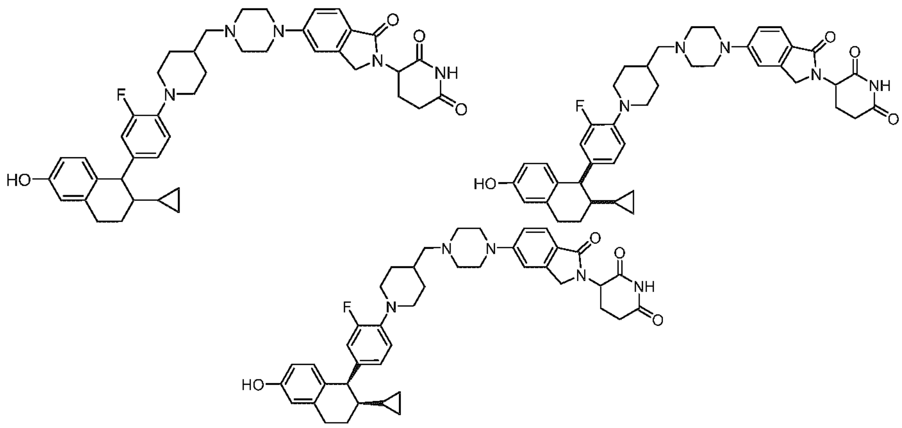 |
| **21** | 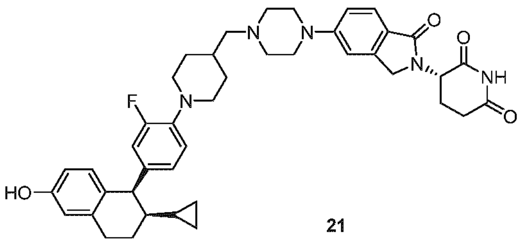 21 |
| | (*S*)-3-(5-(4-((1-(4-((1*R*,2*R*)-2-Cyclopropyl-6-hydroxy-1,2,3,4-tetrahydronaphthalen-1-yl)-2-fluorophenyl)piperidin-4-yl)methyl)piperazin-1-yl)-1-oxoisoindolin-2-yl)piperidine-2,6-dione **21** |
| | 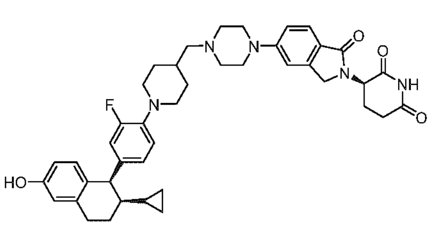 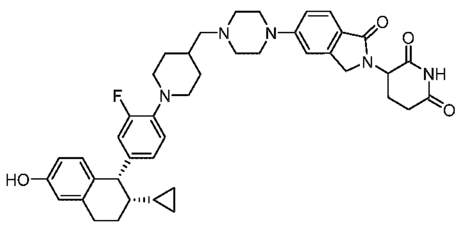 |

(continued)

| Example No. | Structures and names of compounds |
|---|---|
| | 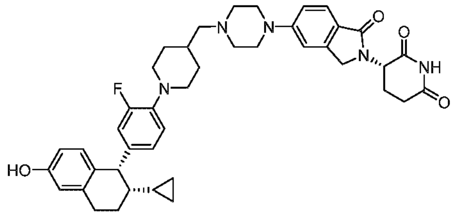 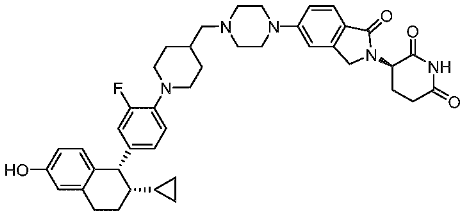 |
| | 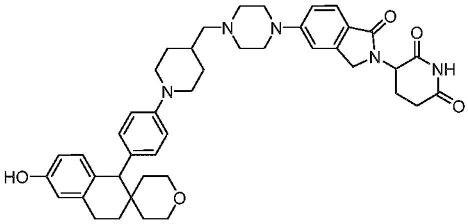 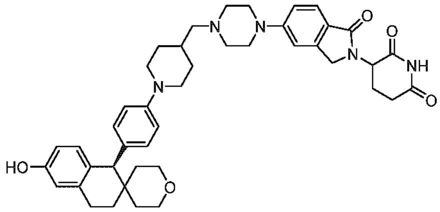 |
| **22** | 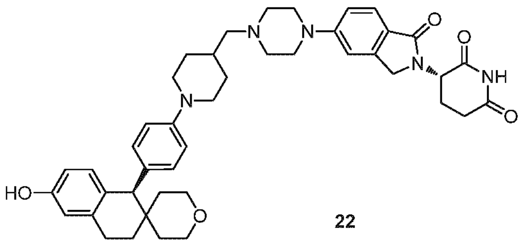 **22** |
| | (*S*)-3-(5-(4-((1-(4-((*R*)-6-Hydroxy-2',3,3',4,5',6'-hexahydro-1*H*-spiro[naphthalene-2,4'-pyran]-1-yl)phenyl)piperidin-4-yl)methyl)piperazin-1-yl)-1-oxoisoindolin-2-yl)piperidine-2,6-dione **22** |
| | 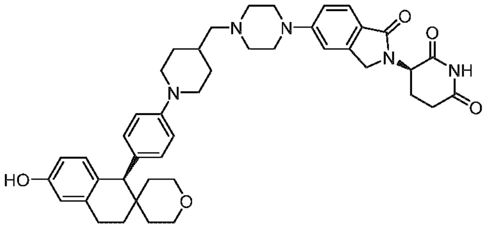 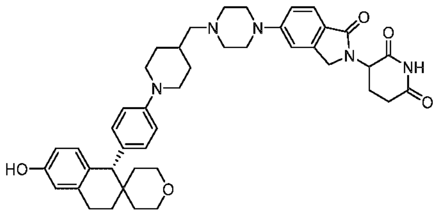 |
| | 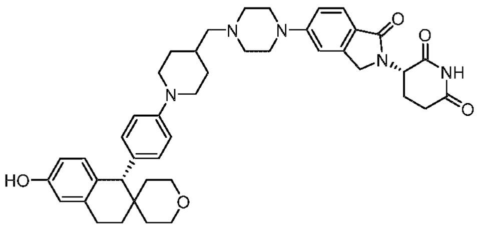 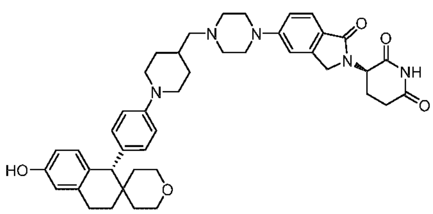 |
| | 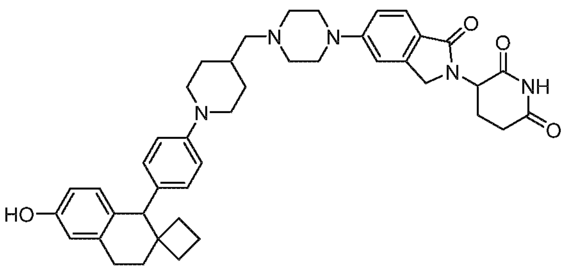 |

(continued)

| Example No. | Structures and names of compounds |
|---|---|
| **23-1** | 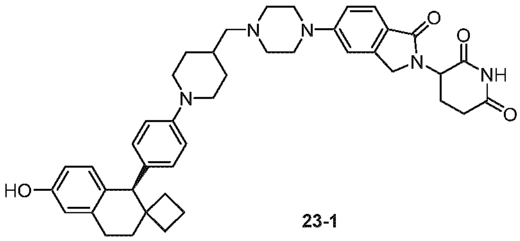 **23-1** |
| | 3-(5-(4-((1-(4-((*R*)-6'-Hydroxy-3',4'-dihydro-1'*H*-spiro[cyclobutane-1,2'-naphthalen]-1'-yl)phe nyl) piperidin-4-yl)methyl)piperazin-1-yl)-1-oxoisoindolin-2-yl)piperidine-2,6-dione **23-1** (a 1:1 mixture of diastereomers) |
| | 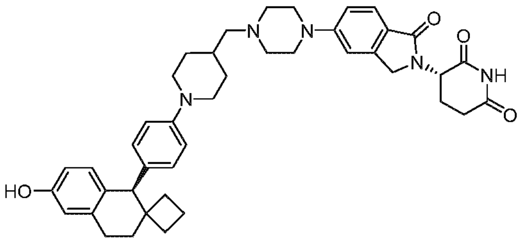 |
| | (*S*)-3-(5-(4-((1-(4-((*R*)-6'-Hydroxy-3',4'-dihydro-1'*H*-spiro[cyclobutane-1,2'-naphthalen]-1'-yl) phenyl) piperidin-4yl)methyl)piperazin-1-yl)-1-oxoisoindolin-2-yl)piperidine-2,6-dione |
| | 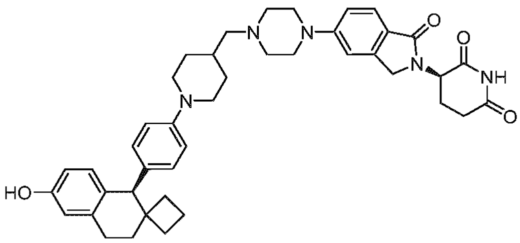 |
| | (*R*)-3-(5-(4-((1-(4-((*R*)-6'-Hydroxy-3',4'-dihydro-1'*H*-spiro[cyclobutane-1,2'-naphthalen]-1'-yl) phenyl) piperidin-4-yl)methyl)piperazin-1-yl)-1-oxoisoindolin-2-yl)piperidine-2,6-dione |
| **23-2** | 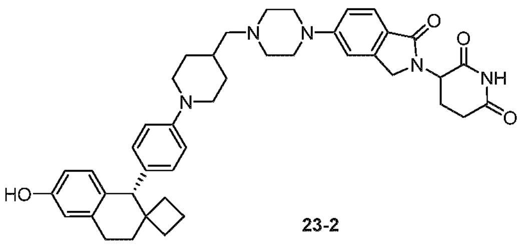 **23-2** |
| | 3-(5-(4-((1-(4-((*S*)-6'-Hydroxy-3',4'-dihydro-1'*H*-spiro[cyclobutane-1,2'-naphthalen]-1'-yl)phe nyl) piperidin-4-yl)methyl)piperazin-1-yl)-1-oxoisoindolin-2-yl)piperidine-2,6-dione **23-2** (a 1:1 mixture of diastereomers) |

(continued)

| Example No. | Structures and names of compounds |
|---|---|
| | 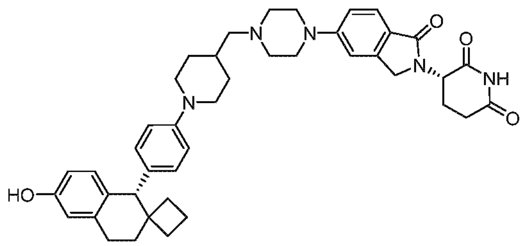 |
| | (*S*)-3-(5-(4-((1-(4-((*S*)-6'-Hydroxy-3',4'-dihydro-1'*H*-spiro[cyclobutane-1,2'-naphthalen]-1'-yl) phenyl) piperidin-4-yl)methyl)piperazin-1-yl)-1-oxoisoindolin-2-yl)piperidine-2,6-dione |
| | 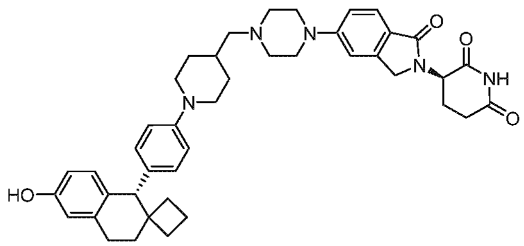 |
| | (*R*)-3-(5-(4-((1-(4-((*S*)-6'-Hydroxy-3',4'-dihydro-1'*H*-spiro[cyclobutane-1,2'-naphthalen]-1'-yl) phenyl) piperidin-4-yl)methyl)piperazin-1-yl)-1-oxoisoindolin-2-yl)piperidine-2,6-dione |
| | 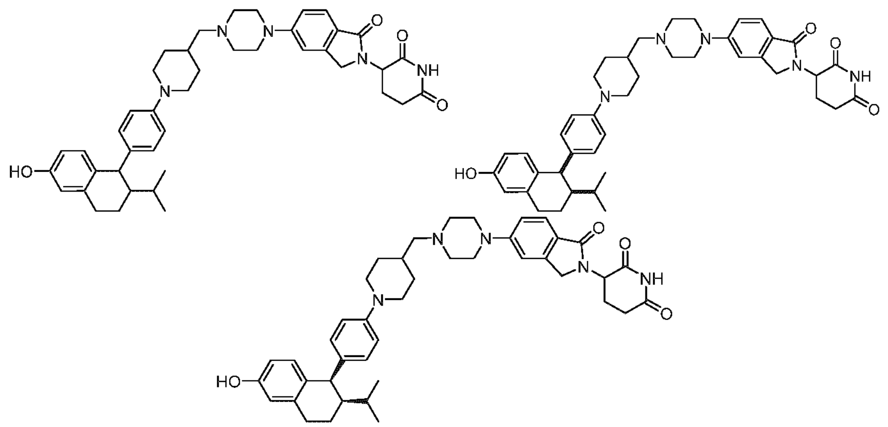 |
| **24** | 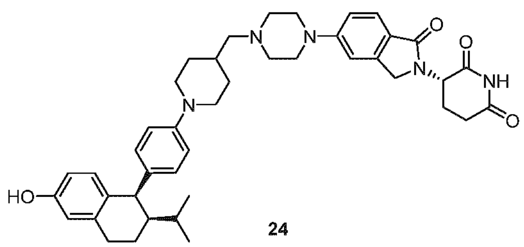 **24** |
| | (*S*)-3-(5-(4-((1-(4-((1*R*,2*R*)-6-Hydroxy-2-isopropyl-1,2,3,4-tetrahydronaphthalen-1-yl)phenyl) piperidin-4-yl)methyl)piperazin-1-yl)-1-oxoisoindolin-2-yl)piperidine-2,6-dione **24** |

(continued)

| Example No. | Structures and names of compounds |
|---|---|
| | 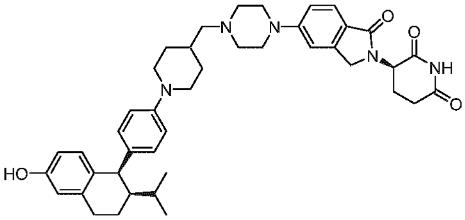 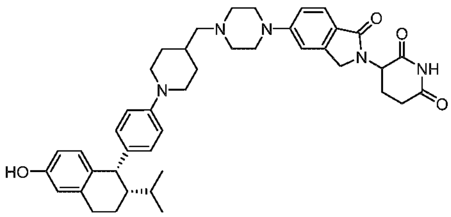 |
| | 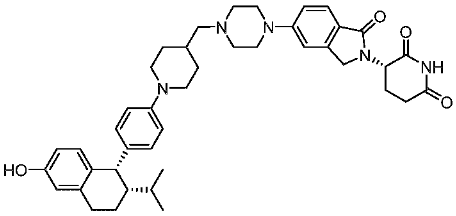 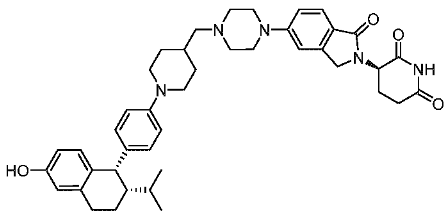 |
| | 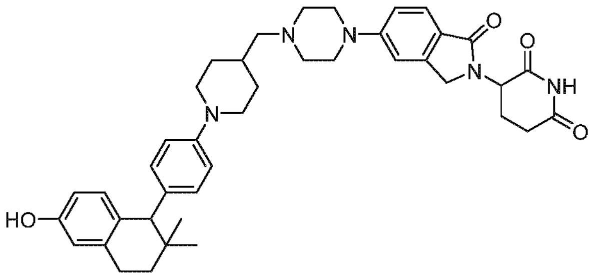 |
| **25-1** | 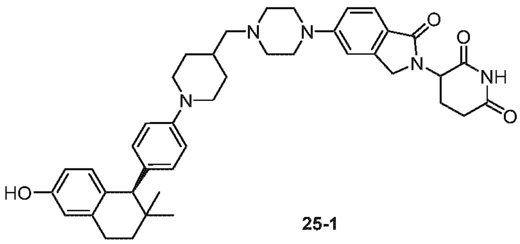 **25-1** |
| | 3-(5-(4-((1-(4-((*R*)-6-Hydroxy-2,2-dimethyl-1,2,3,4-tetrahydronaphthalen-1-yl)phenyl)piperid in-4-yl) methyl)piperazin-1-yl)-1-oxoisoindolin-2-yl)piperidine-2,6-dione **25-1** (a 1:1 mixture of diastereomers) |
| | 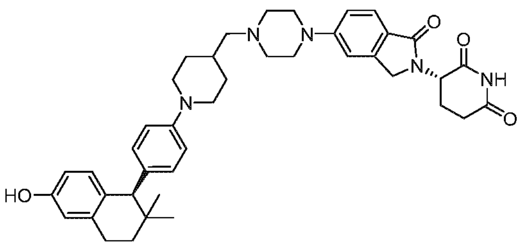 |
| | (*S*)-3-(5-(4-((1-(4-((*R*)-6-Hydroxy-2,2-dimethyl-1,2,3,4-tetrahydronaphthalen-1-yl)phenyl)pip eridin-4-yl) methyl)piperazin-1-yl)-1-oxoisoindolin-2-yl)piperidine-2,6-dione |

(continued)

| Example No. | Structures and names of compounds |
|---|---|
| | 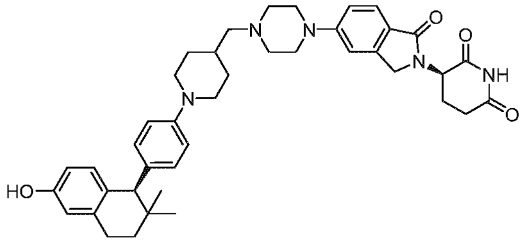 |
| | (*R*)-3-(5-(4-((1-(4-((*R*)-6-Hydroxy-2,2-dimethyl-1,2,3,4-tetrahydronaphthalen-1-yl)phenyl)pip eridin-4-yl) methyl)piperazin-1-yl)-1-oxoisoindolin-2-yl)piperidine-2,6-dione |
| 25-2 | 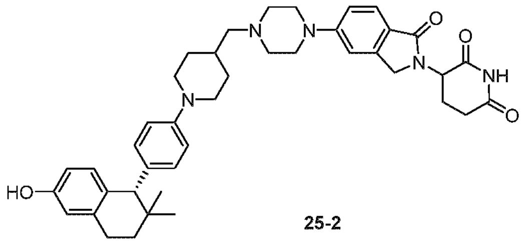 25-2 |
| | 3-(5-(4-((1-(4-((*S*)-6-Hydroxy-2,2-dimethyl-1,2,3,4-tetrahydronaphthalen-1-yl)phenyl)piperidi n-4-yl) methyl)piperazin-1-yl)-1-oxoisoindolin-2-yl)piperidine-2,6-dione **25-2** (a 1:1 mixture of diastereomers) |
| | 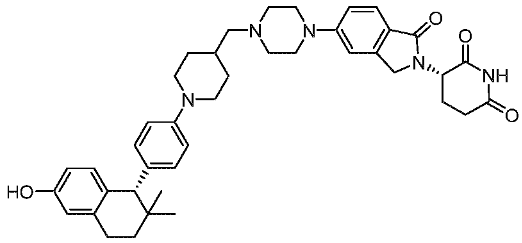 |
| | (*S*)-3-(5-(4-((1-(4-((*S*)-6-Hydroxy-2,2-dimethyl-1,2,3,4-tetrahydronaphthalen-1-yl)phenyl)pip eridin-4-yl) methyl)piperazin-1-yl)-1-oxoisoindolin-2-yl)piperidine-2,6-dione |
| | 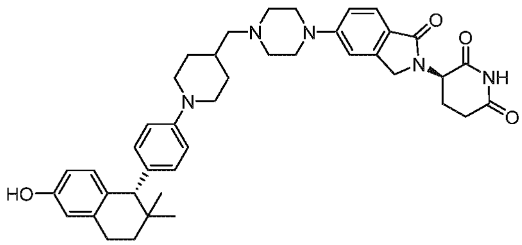 |
| | (*R*)-3-(5-(4-((1-(4-((*S*)-6-Hydroxy-2,2-dimethyl-1,2,3,4-tetrahydronaphthalen-1-yl)phenyl)pip eridin-4-yl) methyl)piperazin-1-yl)-1-oxoisoindolin-2-yl)piperidine-2,6-dione |
| | 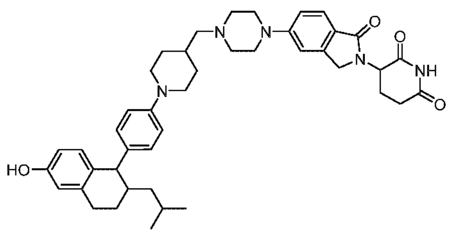 |

(continued)

| Example No. | Structures and names of compounds |
|---|---|
| | 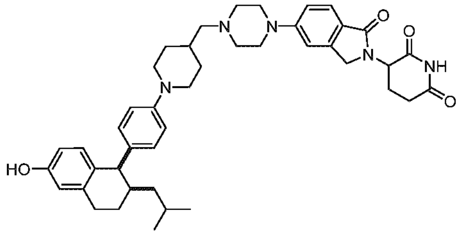 |
| | 3-(5-(4-((1-(4-((1,2)-*cis*-6-Hydroxy-2-isobutyl-1,2,3,4-tetrahydronaphthalen-1-yl)phenyl)piper idin-4-yl) methyl)piperazin-1-yl)-1-oxoisoindolin-2-yl)piperidine-2,6-dione |
| **26-1** | 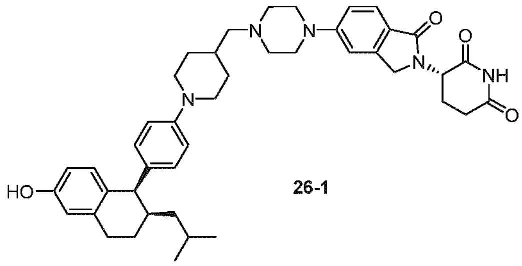 **26-1** |
| | (*S*)-3-(5-(4-((1-(4-((1*R*,2*R*)-6-Hydroxy-2-isobutyl-1,2,3,4-tetrahydronaphthalen-1-yl)phenyl)pi peridin-4-yl)methyl)piperazin-1-yl)-1-oxoisoindolin-2-yl)piperidine-2,6-dione **26-1** |
| **26-2** | 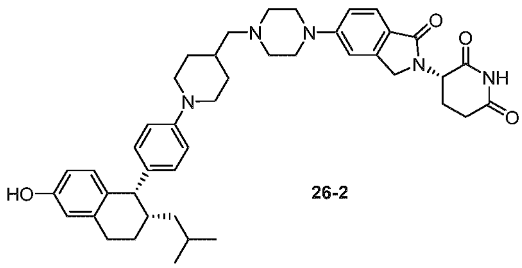 **26-2** |
| | (*S*)-3-(5-(4-((1-(4-((1*S*,2,*S*)-6-Hydroxy-2-isobutyl-1,2,3,4-tetrahydronaphthalen-1-yl)phenyl)pi peridin-4-yl)methyl)piperazin-1-yl)-1-oxoisoindolin-2-yl)piperidine-2,6-dione **26-2** |
| | 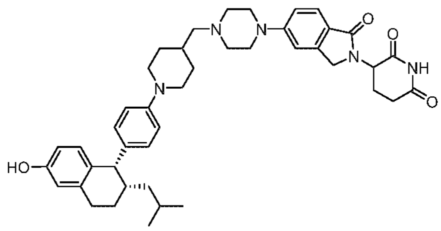 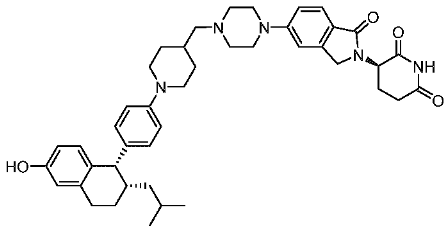 |

(continued)

| Example No. | Structures and names of compounds |
|---|---|
| | 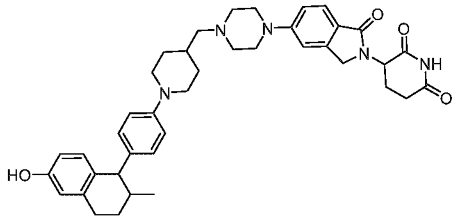 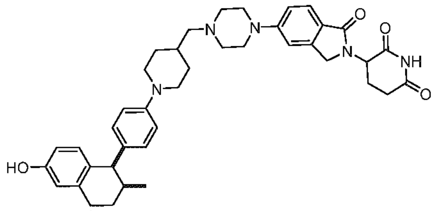 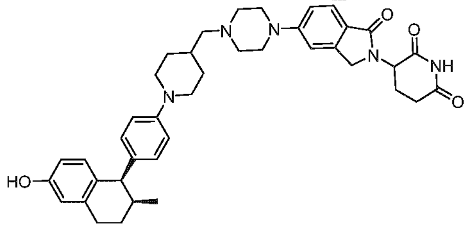 |
| **27** | 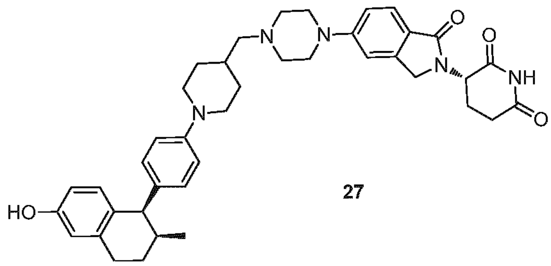 **27** |
| | (*S*)-3-(5-(4-((1-(4-((1*R*,2*S*)-6-Hydroxy-2-methyl-1,2,3,4-tetrahydronaphthalen-1-yl)phenyl)pip eridin-4-yl)methyl)piperazin-1-yl)-1-oxoisoindolin-2-yl)piperidine-2,6-dione **27** |
| | 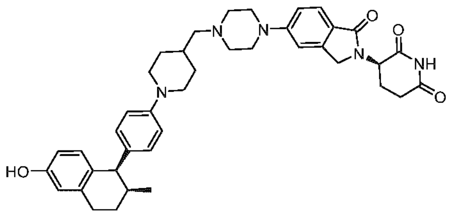 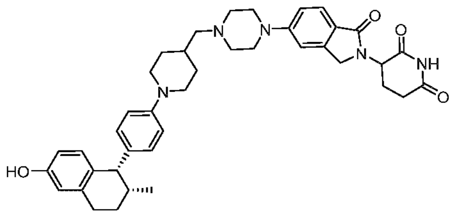 |
| | 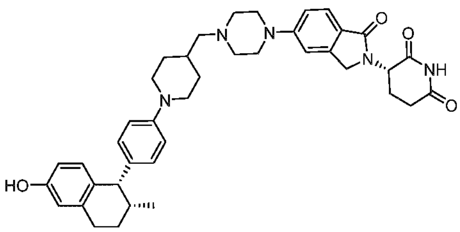 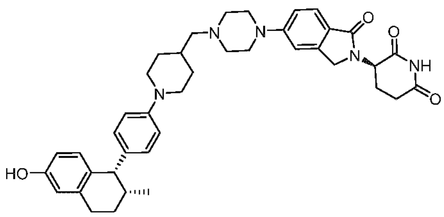 |

(continued)

| Example No. | Structures and names of compounds |
|---|---|
| | 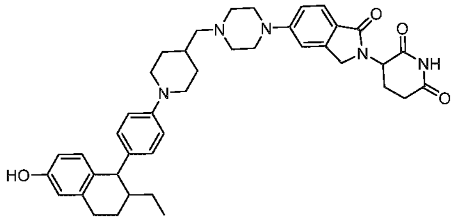 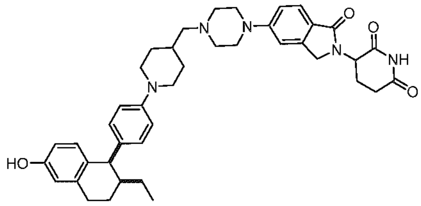 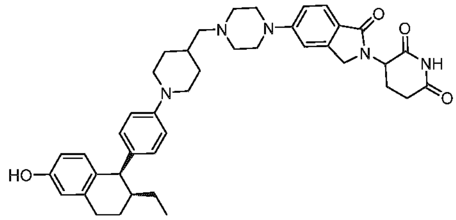 |
| 28 | 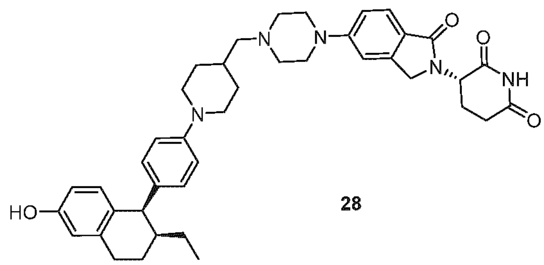 28 |
| | (*S*)-3-(5-(4-((1-(4-((1*R*,2*S*)-6-Hydroxy-2-ethyl-1,2,3,4-tetrahydronaphthalen-1-yl)phenyl)piper idin-4-yl) methyl)piperazin-1-yl)-1-oxoisoindolin-2-yl)piperidine-2,6-dione **28** |
| | 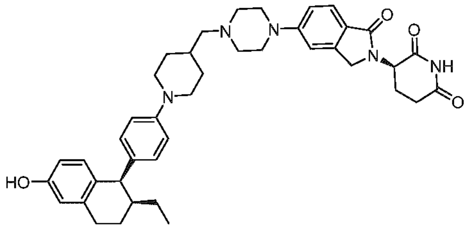 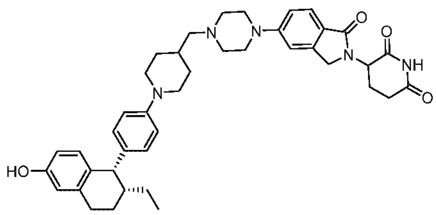 |
| | 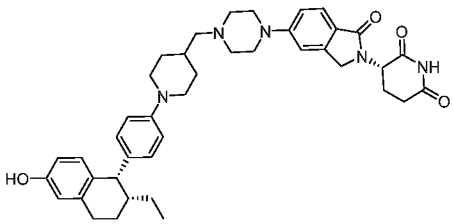 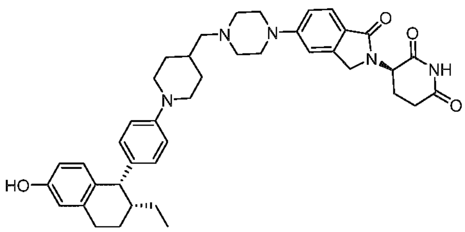 |

(continued)

| Example No. | Structures and names of compounds |
|---|---|
| | 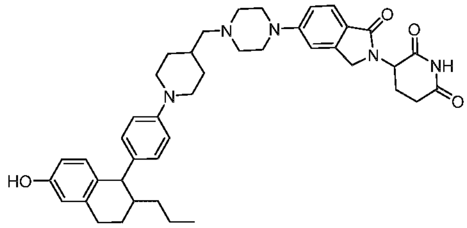 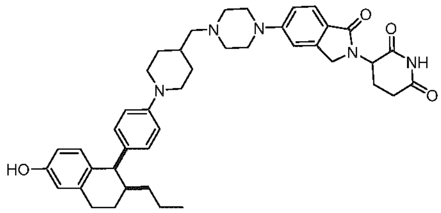 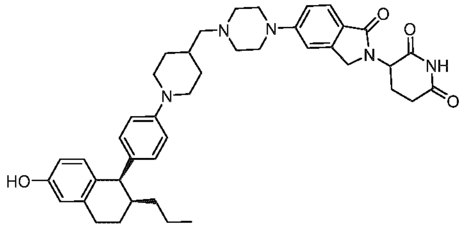 |
| 29 | 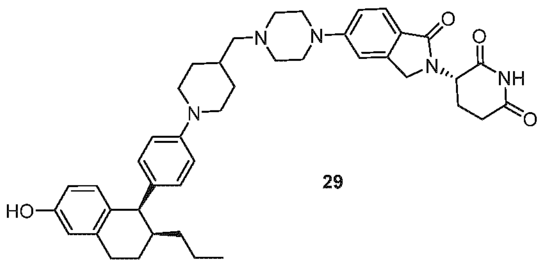 29 |
| | (*S*)-3-(5-(4-((1-(4-((1*R*,2*S*)-6-Hydroxy-2-propyl-1,2,3,4-tetrahydronaphthalen-1-yl)phenyl)pip eridin-4-yl)methyl)piperazin-1-yl)-1-oxoisoindolin-2-yl)piperidine-2,6-dione **29** |
| | 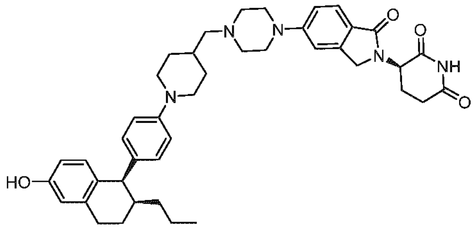 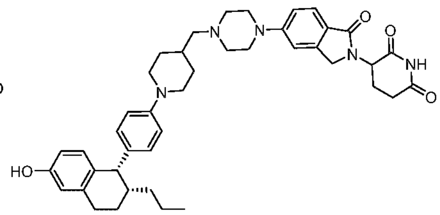 |
| | 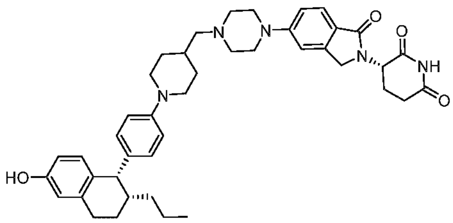 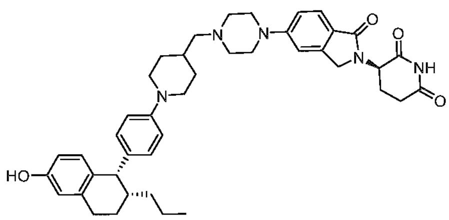 |

(continued)

| Example No. | Structures and names of compounds |
|---|---|
| | 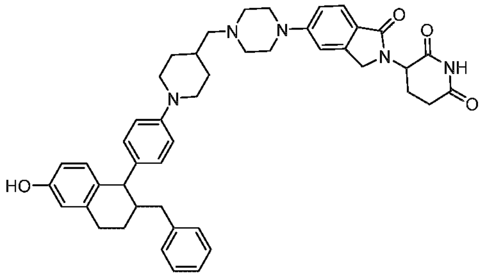 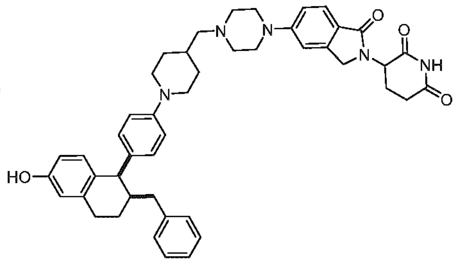 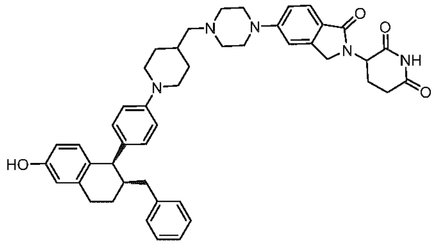 |
| **30** | 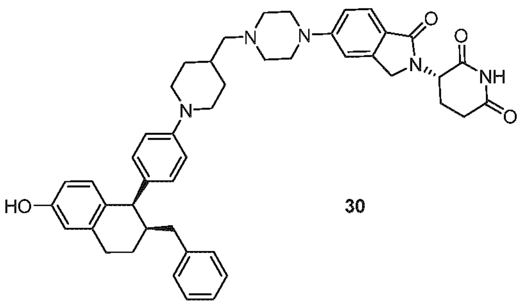 30 |
| | (*S*)-3-(5-(4-((1-(4-((1*R*,2*R*)-2-Benzyl-6-hydroxy-1,2,3,4-tetrahydronaphthalen-1-yl)phenyl)pip eridin-4-yl)methyl)piperazin-1-yl)-1-oxoisoindolin-2-yl)piperidine-2,6-dione **30** |
| | 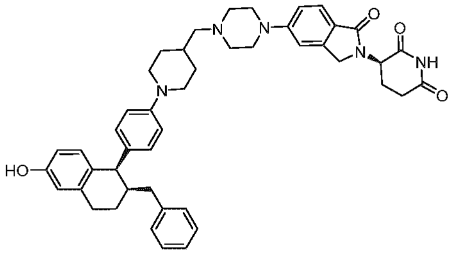 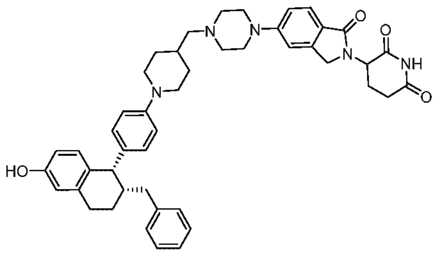 |
| | 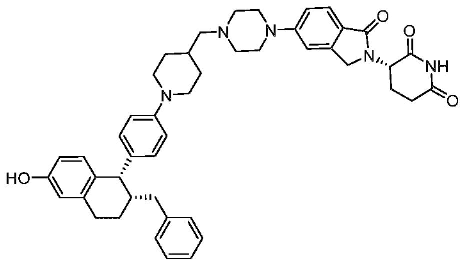 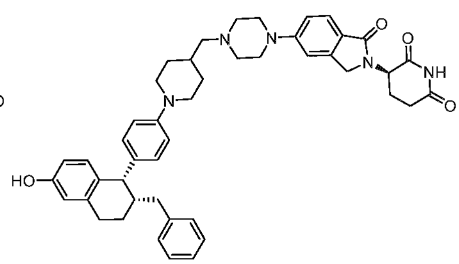 |

(continued)

| Example No. | Structures and names of compounds |
| --- | --- |
| | 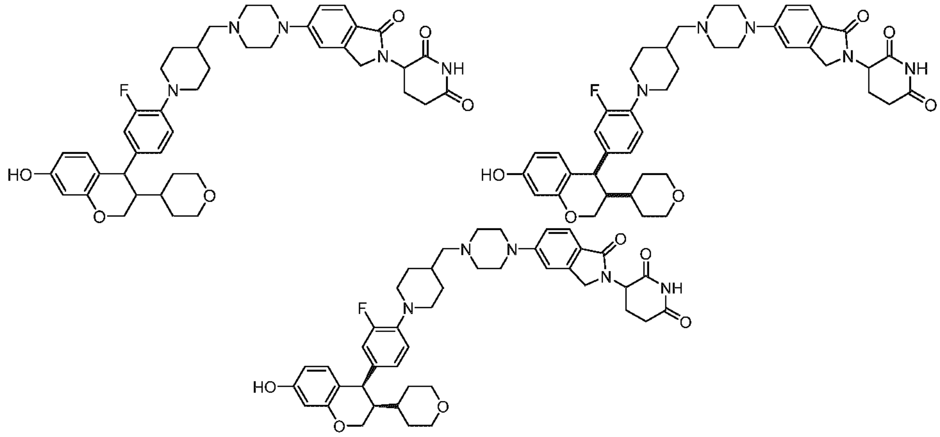 |
| 31 | 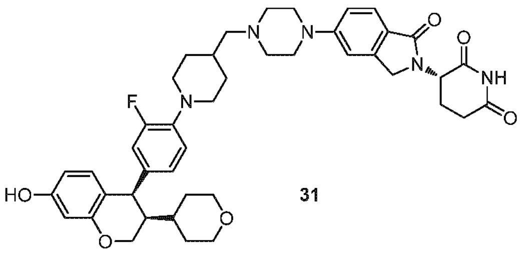 31 |
| | (*S*)-3-(5-(4-((1-(2-Fluoro-4-((3*R*,4*R*)-7-hydroxy-3-(tetrahydro-2*H*-pyran-4-yl)chroman-4-yl)ph enyl) piperidin-4-yl)methyl)piperazin-1-yl)-1-oxoisoindofin-2-yl)piperidine-2,6-dione 31 |
| | 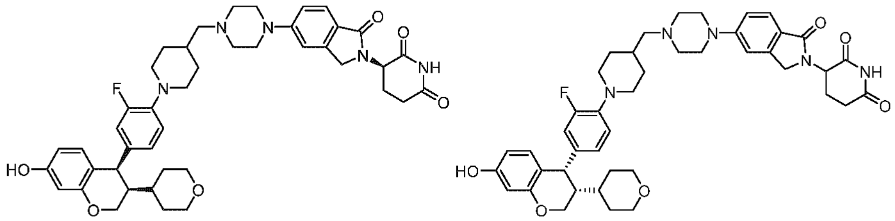 |
| | 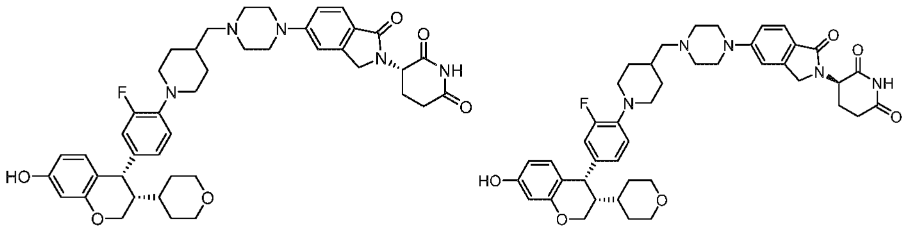 |

(continued)

| Example No. | Structures and names of compounds |
|---|---|
| | 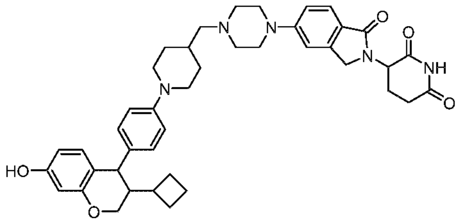 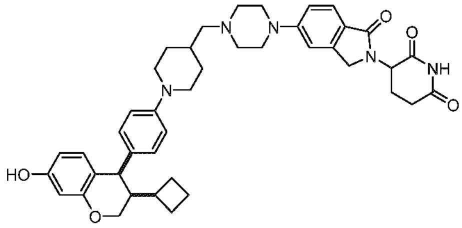 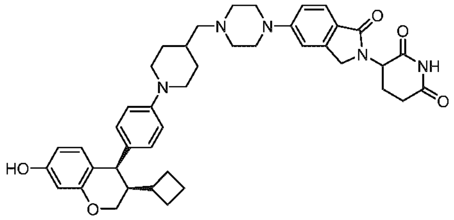 |
| **32** | 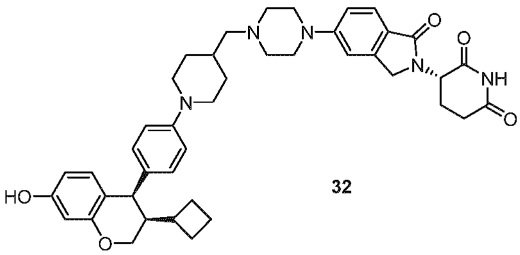 32 |
| | (*S*)-3-(5-(4-((1-4-((3*R*,4*R*)-3-Cyclobutyl-7-hydroxychroman-4-yl)pheny1)piperidin-4-yl)methy l) piperazin-1-yl)-1-oxoisoindolin-2-yl)piperidine-2,6-dione **32** |
| | 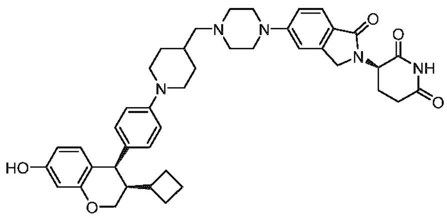 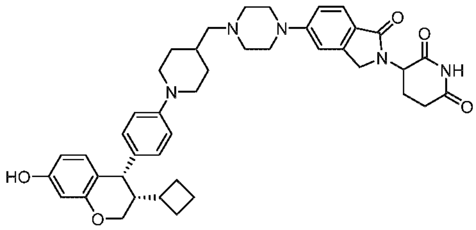 |
| | 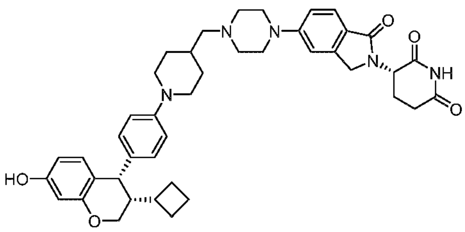 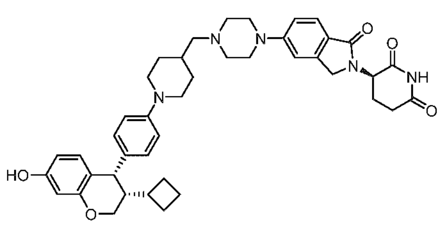 |

(continued)

| Example No. | Structures and names of compounds |
|---|---|
| **33** | 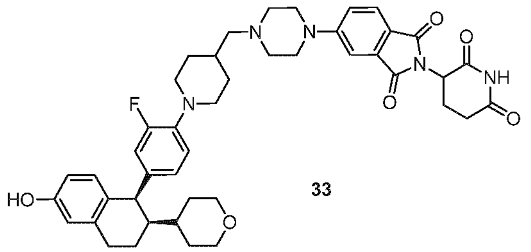 33 |
| | 2-(2,6-Dioxopiperidin-3-yl)-5-(4-((1-(2-fluoro-4-((1*R*,2*R*)-6-hydroxy-2-(tetrahydro-2*H*-pyran-4-yl)-1,2,3,4-tetrahydronaphthalen-1-yl)phenyl)piperidin-4-yl)methyl)piperazin-1-yl)isoindoli ne-1,3-dione **33** (a 1:1 mixture of diastereomers) |
| | 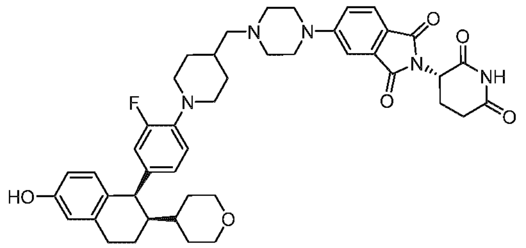 |
| | (*S*)-2-(2,6-Dioxopiperidin-3-yl)-5-(4-((1-(2-fluoro-4-((1*R*,2*R*)-6-hydroxy-2-(tetrahydro-2*H*-py ran-4-yl)-1,2,3,4-tetrahydronaphthalen-1-yl)phenyl)piperidin-4-yl)methyl)piperazin-1-yl)isoin doline-1,3-dione |
| | 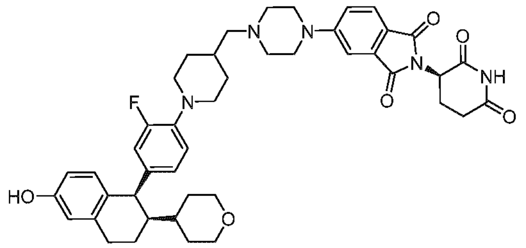 |
| | (*R*)-2-(2,6-Dioxopiperidin-3-yl)-5-(4-((1-(2-fluoro-4-((1*R*,2*R*)-6-hydroxy-2-(tetrahydro-2*H*-py ran-4-yl)-1,2,3,4-tetrahydronaphthalen-1-yl)phenyl)piperidin-4-yl)methyl)piperazin-1-yl)isoin doline-1,3-dione |
| | 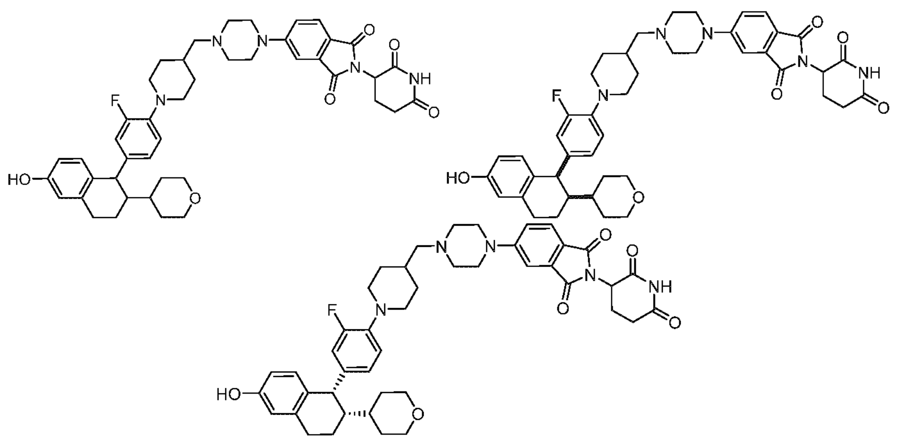 |

(continued)

| Example No. | Structures and names of compounds |
|---|---|
| | 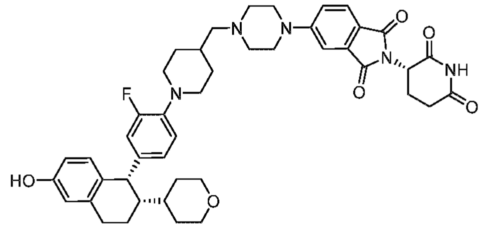 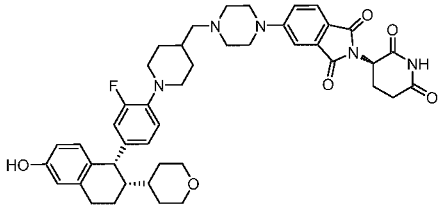 |
| | 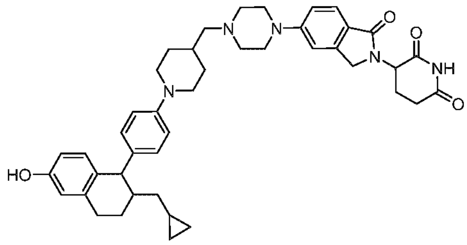 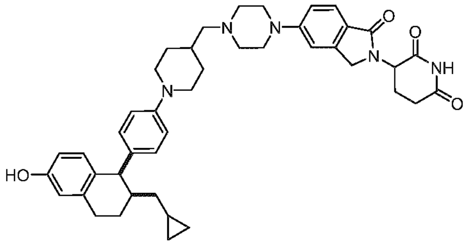 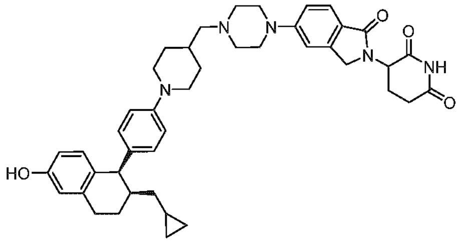 |
| **34** | 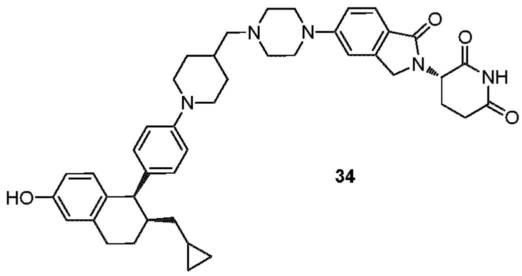 **34** |
| | (*S*)-3-(5-(4-((1-(4-((1*R*,2*R*)-2-(Cyclopropylmethyl)-6-hydroxy-1,2,3,4-tetrahydronaphthalen-1 -yl) phenyl)piperidin-4-yl)methyl)piperazin-1-yl)-1-oxoisoindolin-2-yl)piperidine-2,6-dione **34** |
| | 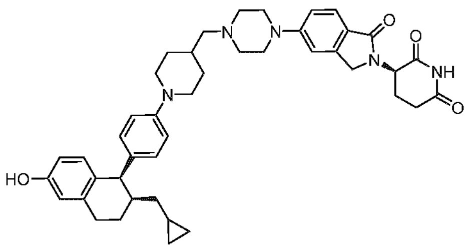 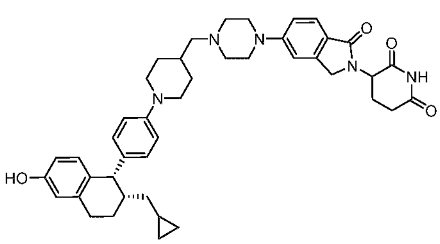 |
| | 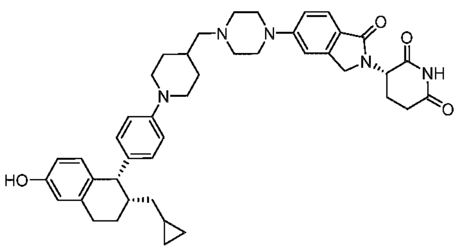 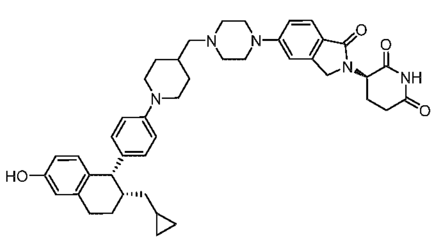 |

(continued)

| Example No. | Structures and names of compounds |
|---|---|
| | 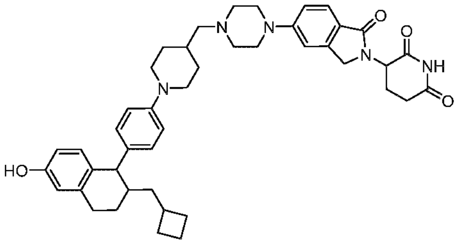 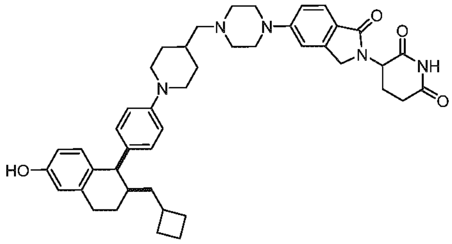 |
| | 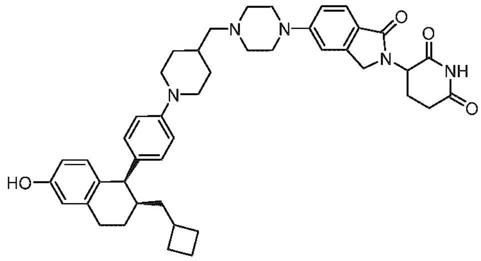 |
| 35 | 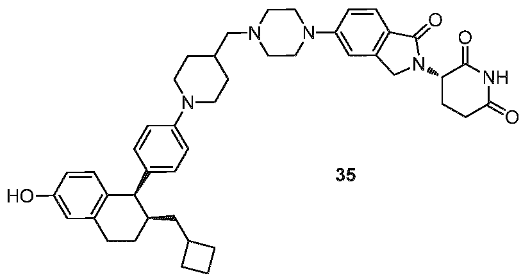 35 |
| | (*S*)-3-(5-(4-((1-(4-((1*R*,2*R*)-2-(Cyclobutylmethyl)-6-hydroxy-1,2,3,4-tetrahydronaphthalen-1-y 1)phenyl) piperidin-4-yl)methyl)piperazin-1-yl)-1-oxoisoindolin-2-yl)piperidine-2,6-dione **35** |
| | 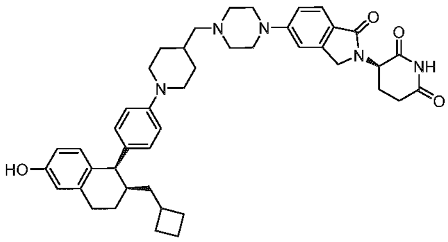 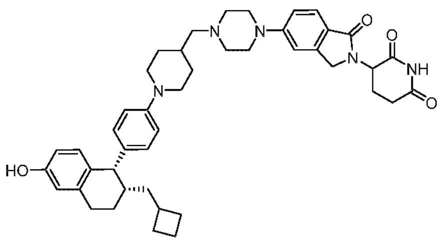 |
| | 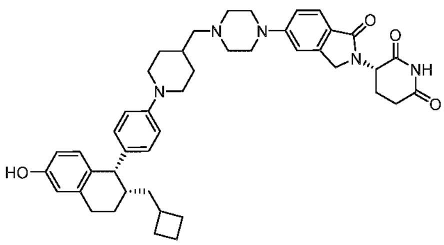 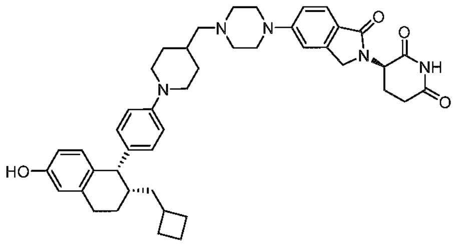 |

(continued)

| Example No. | Structures and names of compounds |
|---|---|
| | 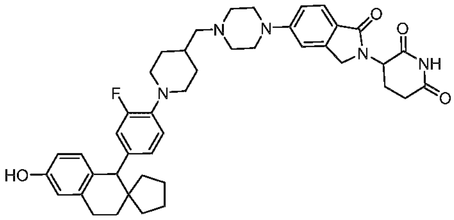 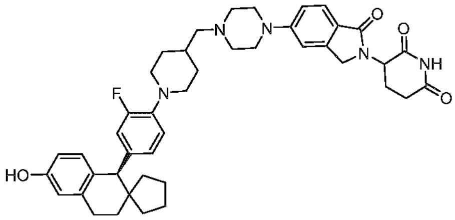 |
| **36** | 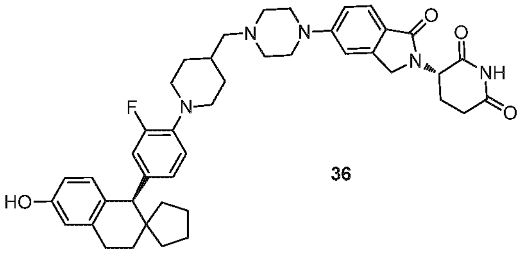 **36** |
| | (*S*)-3-(5-(4-((1-(2-Fluoro-4-((*R*)-6'-hydroxy-3',4'-dihydro-1'*H*-spiro[cyclopentane-1,2'-naphtha len]-1'-yl) phenyl)piperidin-4-yl)methyl)piperazin-1-yl)-1-oxoisoindofin-2-yl)piperidine-2,6-di one **36** |
| | 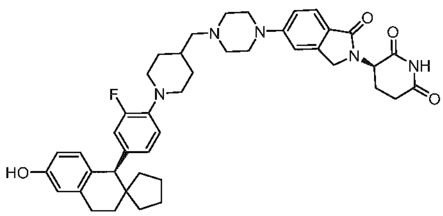 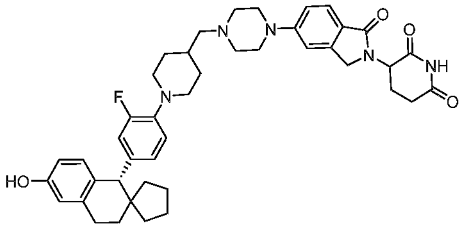 |
| | 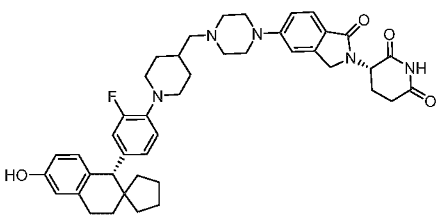 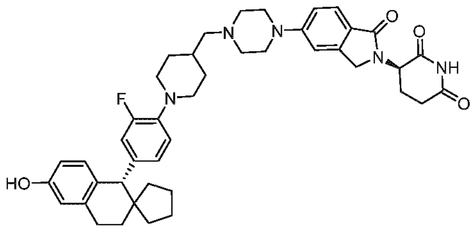 |
| | 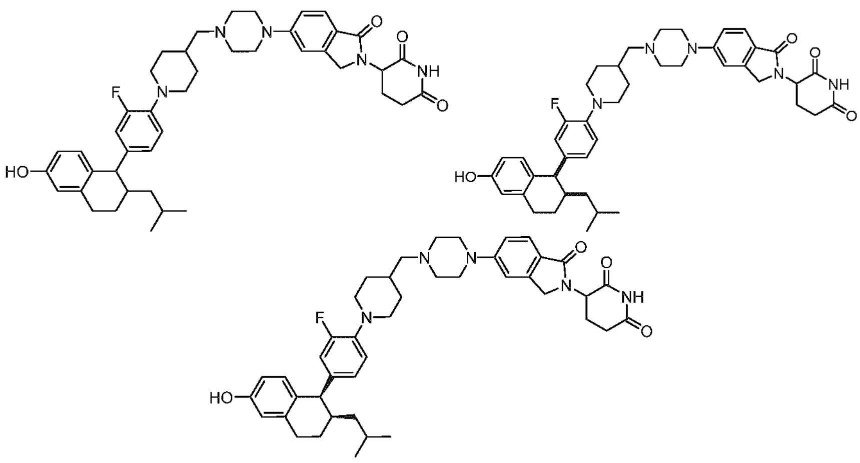 |

(continued)

| Example No. | Structures and names of compounds |
|---|---|
| **37** | 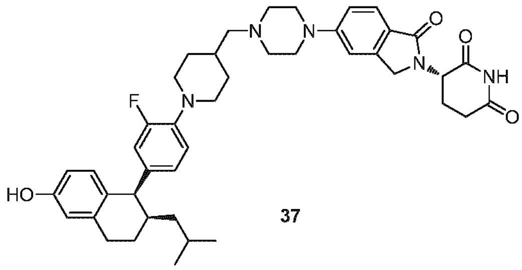 37 |
| | (*S*)-3-(5-(4-((1-(2-Fluoro-4-((1*R*,2*R*)-6-hydroxy-2-isobutyl-1,2,3,4-tetrahydronaphthalen-1-yl) phenyl) piperidin-4-yl)methyl)piperazin-1-yl)-1-oxoisoindolin-2-yl)piperidine-2,6-dione **37** |
| | 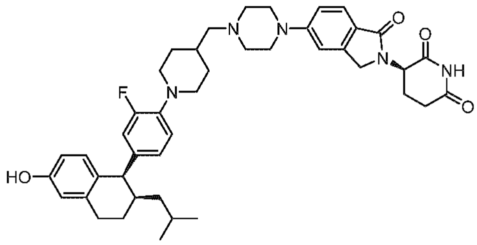 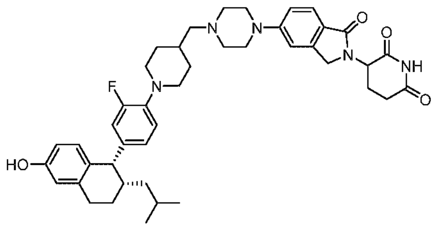 |
| | 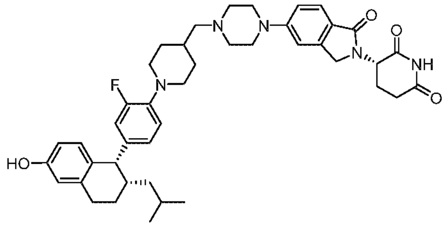 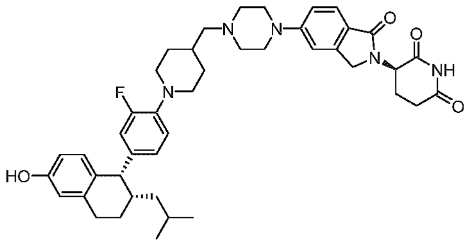 |
| | 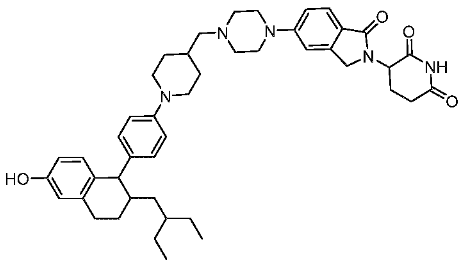 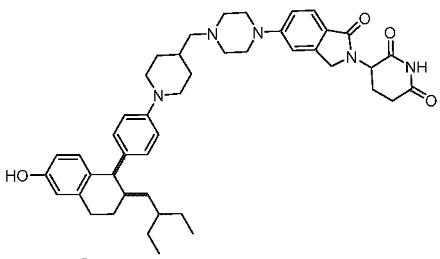 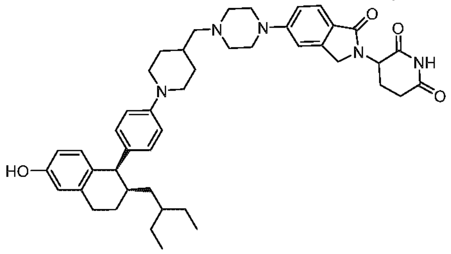 |

(continued)

| Example No. | Structures and names of compounds |
|---|---|
| **38** | 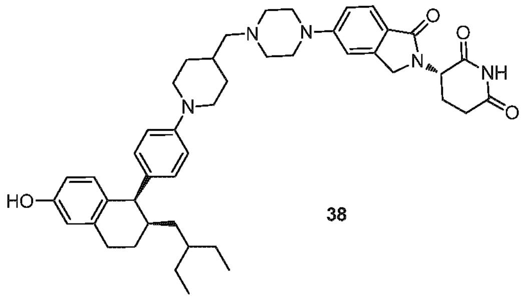 38 |
| | (*S*)-3-(5-(4-((1-(4-((1*R*,2*R*)-6-Hydroxy-2-(2-ethylbutyl)-1,2,3,4-tetrahydronaphthalen-1-yl)phe nyl) piperidin-4-yl)methyl)piperazin-1-yl)-1-oxoisoindolin-2-yl)piperidine-2,6-dione **38** |
| | 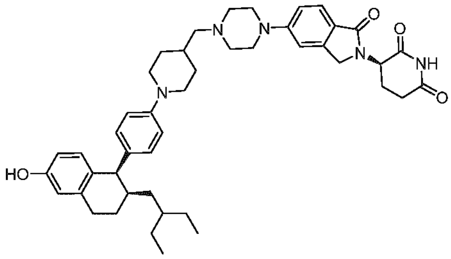 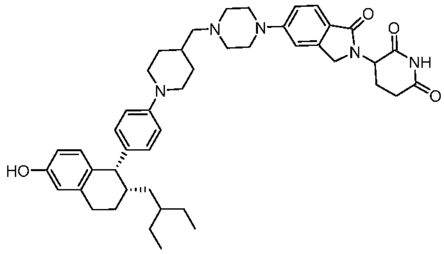 |
| | 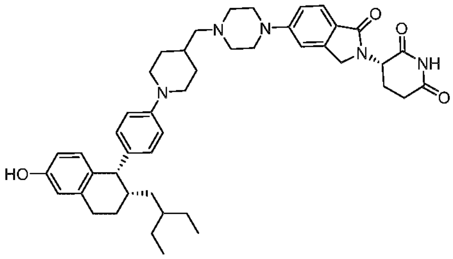 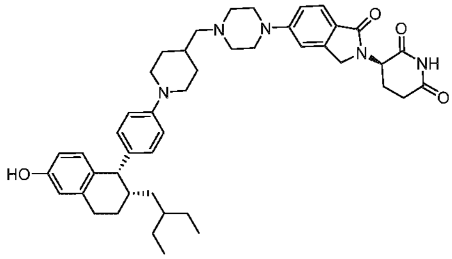 |
| | 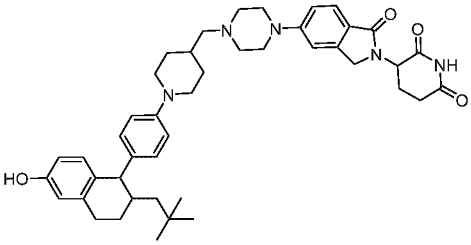 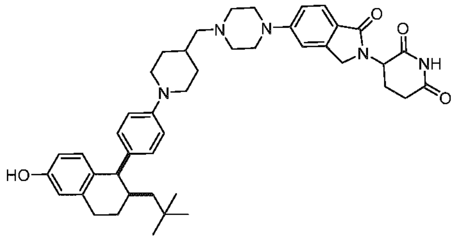 |
| | 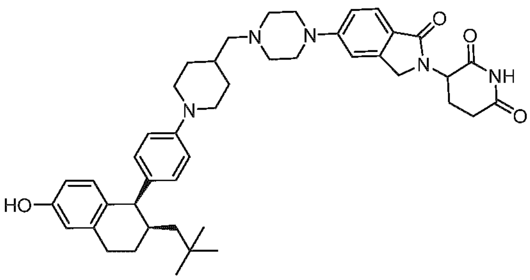 |

(continued)

| Example No. | Structures and names of compounds |
|---|---|
| **39** | 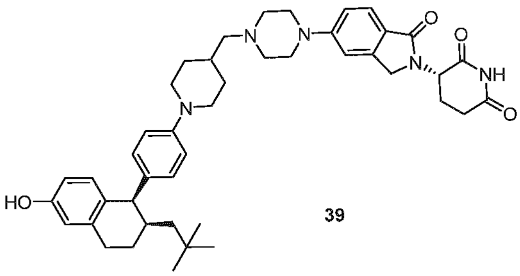 39 |
| | (*S*)-3-(5-(4-((1-(4-((1*R*,2*R*)-6-Hydroxy-2-neopentyl-1,2,3,4-tetrahydronaphthalen-1-yl)phenyl) piperidin-4-yl)methyl)piperazin-1-yl)-1-oxoisoindolin-2-yl)piperidine-2,6-dione **39** |
| | 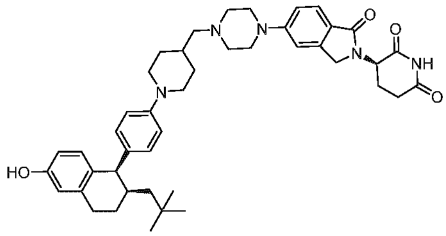 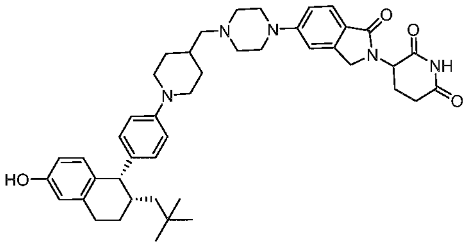 |
| | 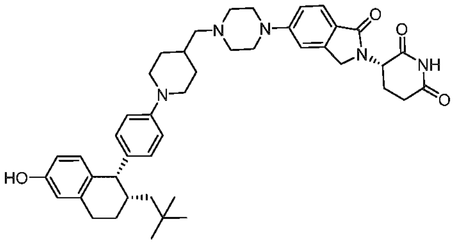 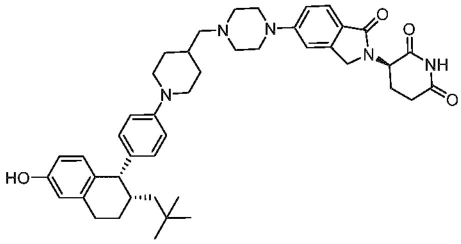 |
| | 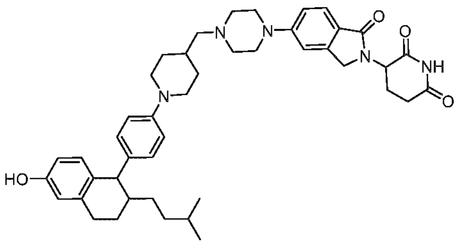 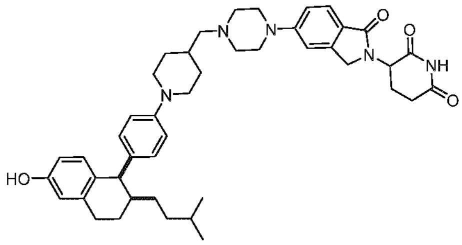 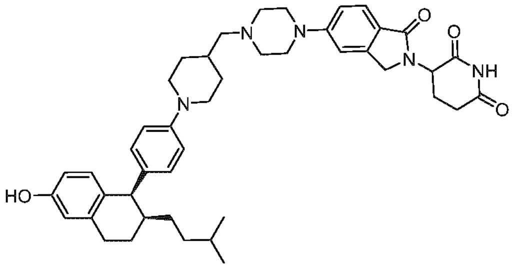 |

(continued)

| Example No. | Structures and names of compounds |
|---|---|
| **40** | 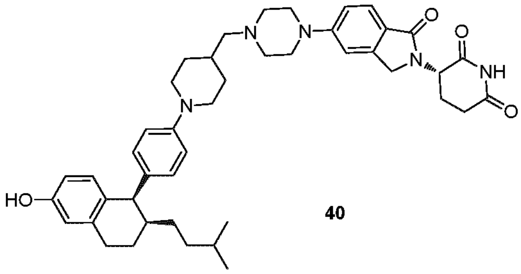 **40** |
| | (*S*)-3-(5-(4-((1-(4-((1*R*,2*S*)-6-Hydroxy-2-isopentyl-1,2,3,4-tetrahydronaphthalen-1-yl)phenyl) piperidin-4-yl)methyl)piperazin-1-yl)-1-oxoisoindolin-2-yl)piperidine-2,6-dione **40** |
| | 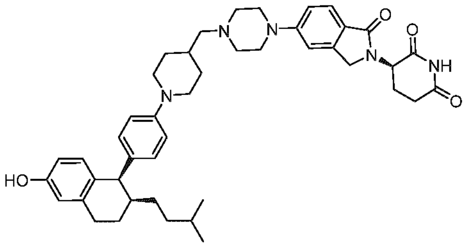 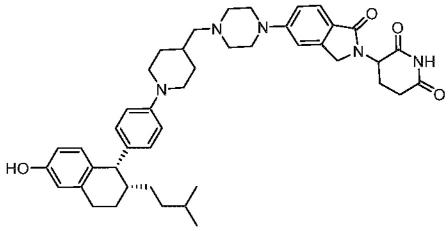 |
| | 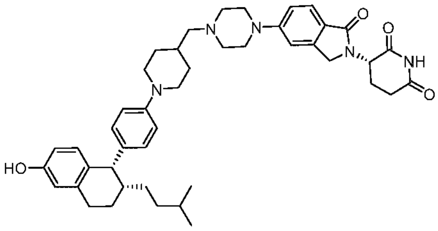 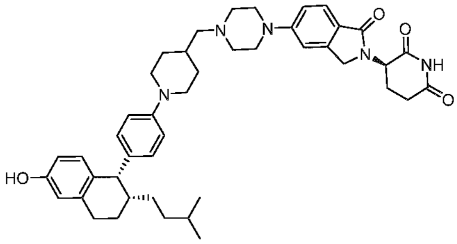 |
| **41** | 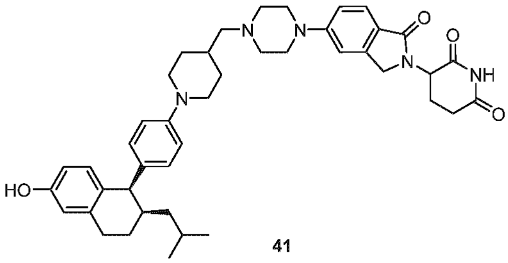 **41** |
| | 3-(5-(4-((1-(4-((1*R*,2*R*)-6-Hydroxy-2-isobutyl-1,2,3,4-tetrahydronaphthalen-1-yl)phenyl)piper idin-4-yl) methyl)piperazin-1-yl)-1-oxoisoindolin-2-yl)piperidine-2,6-dione **41** (a 1:1 mixture of diastereomers) |

(continued)

| Example No. | Structures and names of compounds |
|---|---|
| **42** | 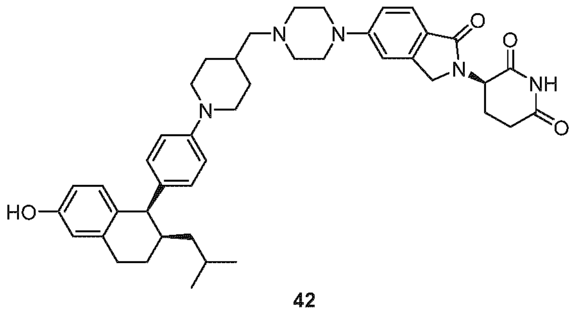 42 |
| | (*R*)-3-(5-(4-((1-(4-((1*R*,2*R*)-6-Hydroxy-2-isobutyl-1,2,3,4-tetrahydronaphthalen-1-yl)phenyl)p iperidin-4-yl)methyl)piperazin-1-yl)-1-oxoisoindolin-2-yl)piperidine-2,6-dione **42** |
| **43** | 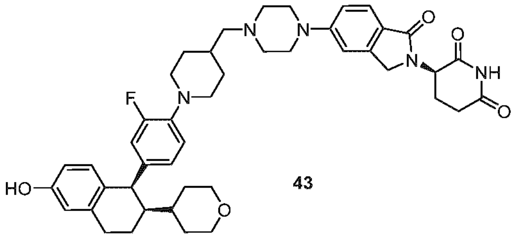 43 |
| | (*R*)-3-(5-(4-((1-(2-Fluoro-4-((1*R*,2*R*)-6-hydroxy-2-(tetrahydro-2*H*-pyran-4-y1)-1,2,3,4-tetrahy dronaphthalen-1 yl)phenyl)piperidin-4-yl)methyl)piperazin-1-yl)-1-oxoisoindofin-2-yl)piperid ine-2,6-dione **43** |

Table C. Typical compounds of the present disclosure include, but are not limited to:

| Example No. | Names of compounds |
|---|---|
| 3 | 3 -(5 -(4-((1-(2-Fluoro-4-((1,2)-*cis*-6-hydroxy-2-(tetrahydro-2*H*-pyran-4-yl)-1,2,3,4-tetrahydronaphthalen-1-yl)phenyl)piperidin-4-yl)methyl)piperazin-1 -yl)-1-oxoisoindolin-2-yl) piperidine-2,6-dione **3** |
| 7 | 3-(5-(4-((1-(4-((1,2)-*cis*-6-Hydroxy-2-(tetrahydro-2*H*-pyran-4-yl)-1,2,3,4-te trahydronaphthalen-1-yl)phenyl)piperidin-4-yl)methyl)piperazin-1-yl)-1-ox oisoindolin-2-yl) piperidine-2,6-dione **7** |
| 9 | 3-(5-(4-((1-(4-((1,2)-*cis*-2-(4,4-Difluorocyclohexyl)-6-hydroxy-1,2,3,4-tetra hydronaphthalen-1-yl)phenyl)piperidin-4-yl)methyl)piperazin-1-yl)-1-oxois oindolin-2-yl)piperidine-2,6-dione **9** |
| 11 | 3-(5-(4-((1-(4-((1,2)-*cis*-6-Hydroxy-2-(1-methylpiperidin-4-yl)-1,2,3,4-tetra hydronaphthalen-1-yl)phenyl)piperidin-4-yl)methyl)piperazin-1-yl)-1-oxois oindolin-2-yl)piperidine-2,6-dione **11** |
| 16 | 3-(5-(4-((1-(4-((1,2)-*cis*-2-Cyclopropyl-6-hydroxy-1,2,3,4-tetrahydronaphth alen-1-yl)phenyl) piperidin-4-yl)methyl)piperazin-1-yl)-1-oxoisoindolin-2-yl )piperidine-2,6-dione **16** |
| | 3-(5-(4-((1-(4-((1,2)-*cis*-6-Hydroxy-2-isobutyl-1,2,3,4-tetrahydronaphthalen -1-yl)phenyl) piperidin-4-yl)methyl)piperazin-1-yl)-1-oxoisoindolin-2-yl)pip eridine-2,6-dione |

Another aspect of the present disclosure relates to a compound of general formula (IIIa) or a salt thereof,

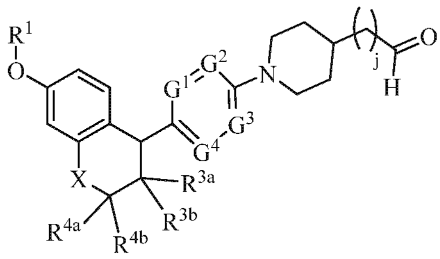

( **IIIa** )

wherein:

X, $G^1$ to $G^4$, $R^1$, $R^{3a}$, $R^{3b}$, $R^{4a}$, $R^{4b}$ and j are as defined in general formula (III).
Another aspect of the present disclosure relates to a compound of general formula (IVa) or a salt thereof

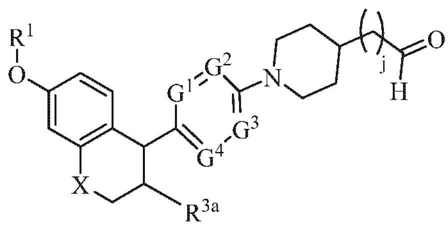

( **IVa** )

wherein:
X, $G^1$ to $G^4$, $R^{3a}$, $R^1$ and j are as defined in general formula (IV).

Another aspect of the present disclosure relates to a compound of general formula (Va) or a salt thereof:

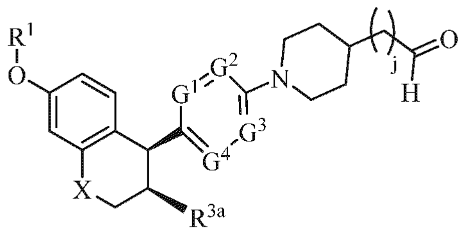

( **Va** )

wherein:
X, $G^1$ to $G^4$, $R^{3a}$, $R^1$ and j are as defined in general formula (V).

Table B. Typical intermediate compounds of the present disclosure include, but are not limited to:

| Example No. | Structures and names of compounds |
|---|---|
| **1n** | 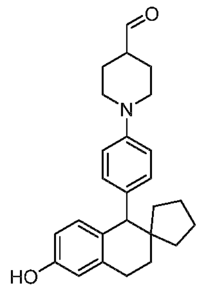 **1n** |
| | 1'-(4-(6'-Hydroxy-3',4'-dihydro-1'*H*-spiro[cyclopentane-1,2'-naphthalen]-1'-yl)phenyl) piperi dine-4-carbaldehyde **1n** |
| **2b-1** | 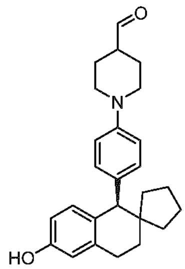 **2b-1** |
| | (*R*)-1'-(4-(6'-Hydroxy-3',4'-dihydro-1'*H*-spiro[cyclopentane-1,2'-naphthalen]-1'-yl)phenyl) p iperidine-4-carbaldehyde **2b-1** |
| **2b-2** | 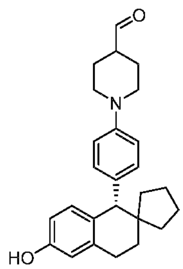 **2b-2** |
| | (*S*)-1'-(4-(6'-Hydroxy-3',4'-dihydro-1'*H*-spiro[cyclopentane-1,2'-naphthalen]-1'-yl)phenyl) pi peridine-4-carbaldehyde **2b-2** |
| **3o** | 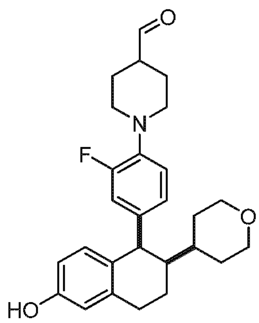 **3o** |
| | 1-(2-Fluoro-4-((1,2)-*cis*-6-hydroxy-2-(tetrahydro-2*H*-pyran-4-yl)-1,2,3,4-dihydronaphthale n-1-yl)phenyl)piperidine-4-carbaldehyde **3o** |

(continued)

| Example No. | Structures and names of compounds |
|---|---|
| **4b-1** | 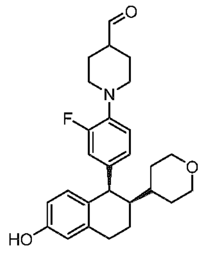 4b-1 |
| | 1-(2-Fluoro-4-((1*R*,2*R*)-6-hydroxy-2-(tetrahydro-2*H*-pyran-4-y1)-1,2,3,4-tetrahydronaphtha len-1-yl)phenyl)piperidine-4-carbaldehyde **4b-1** |
| **4b-2** | 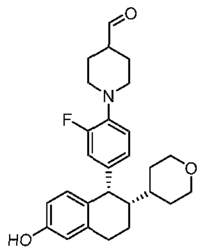 4b-2 |
| | 1-(2-Fluoro-4-((1*S*,2*S*)-6-hydroxy-2-(tetrahydro-2*H*-pyran-4-yl)-1,2,3,4-tetrahydronaphtlial en-1-yl)phenyl)piperidine-4-carbaldehyde **4b-2** |
| **7f** | 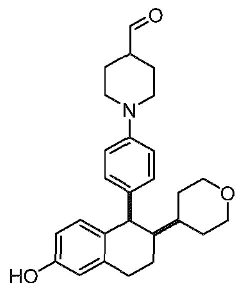 7f |
| | 1-(4-((1,2)-*cis*-6-Hydroxy-2-(tetrahydro-2*H*-pyran-4-yl)-1,2,3,4-tetrahydronaphthalen-1-yl) phenyl)piperidine-4-carbaldehyde **7f** |
| | 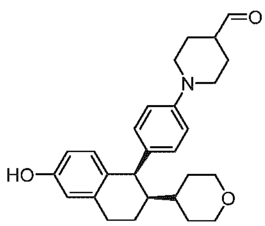 |
| | 1-(4-((1*R*,2*R*)-6-Hydroxy-2-(tetrahydro-2*H*-pyran-4-yl)-1,2,3,4-tetrahydronaphthalen-1-yl) phenyl)piperidine-4-carbaldehyde |

(continued)

| Example No. | Structures and names of compounds |
|---|---|
| | 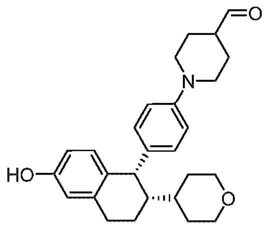 |
| | 1-(4-((1*S*,2*S*)-6-Hydroxy-2-(tetrahydro-2*H*-pyran-4-yl)-1,2,3,4-tetrahydronaphthalen-1-yl)p henyl)piperidine-4-carbaldehyde |
| **9c** | 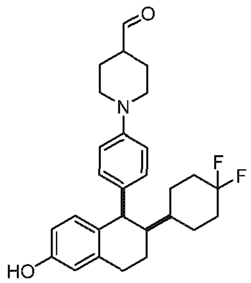 9c |
| | 1-(4-((1,2)-*cis*-2-(4,4-Difluorocyclohexyl)-6-hydroxy-1,2,3,4-tetrahydronaphthalen-1-yl)ph enyl)piperidine-4-carbaldehyde **9c** |
| **10b** | 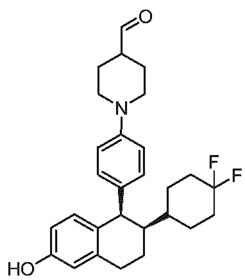 10b |
| | 1-(4-((1*R*,2*R*)-2-(4,4-Difluorocyclohexyl)-6-hydroxy-1,2,3,4-tetrahydronaphthalen-1-yl)ph enyl)piperidine-4-carbaldehyde **10b** |
| | 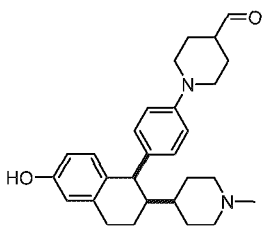 |
| | 1-(4-((1,2)-*cis*-6-Hydroxy-2-(1-methylpiperidin-4-yl)-1,2,3,4-tetrahydronaphthalen-1-yl)ph enyl)piperidine-4-carbaldehyde |

(continued)

| Example No. | Structures and names of compounds |
|---|---|
| **12h-1** | 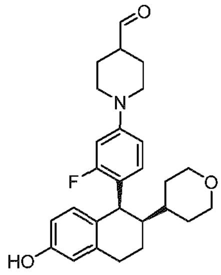<br>**12h-1** |
| | 1-(3-Fluoro-4-((1*S*,2*R*)-6-hydroxy-2-(tetrahydro-2*H*-pyran-4-yl)-1,2,3,4-tetrahydronaphthal en-1-yl)phenyl)piperidine-4-carbaldehyde **12h-1** |
| **12h-2** | 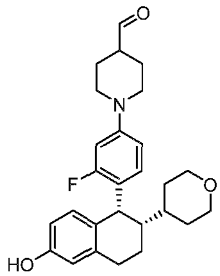<br>**12h-2** |
| | 1-(3-Fluoro-4-((1*R*,2*S*)-6-hydroxy-2-(tetrahydro-2*H*-pyran-4-yl)-1,2,3,4-tetrahydronaphthal en-1-yl)phenyl)piperidine-4-carbaldehyde **12h-2** |
| **13c-1** | 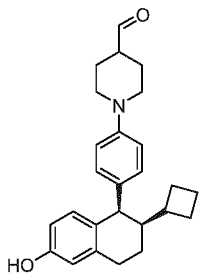<br>**13c-1** |
| | 1-(4-((1*R*,2*R*)-2-Cyclobutyl-6-hydroxy-1,2,3,4-tetrahydronaphthalen-1-yl)phenyl)piperidin e-4-carbaldehyde 13c-1 |
| **13c-2** | 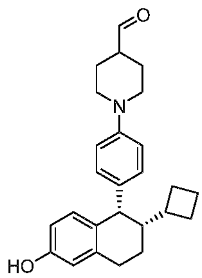<br>**13c-2** |
| | 1-(4-((1*S*,2*S*')-2-Cyclobutyl-6-hydroxy-1,2,3,4-tetrahydronaphthalen-1-yl)phenyl) piperidine -4-carbaldehyde **13c-2** |

(continued)

| Example No. | Structures and names of compounds |
|---|---|
| **14c-1** | 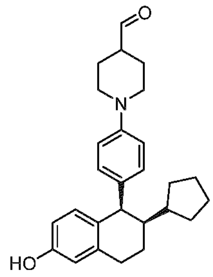 14c-1 |
| | 1-(4-((1*R*,2*R*)-2-Cyclopentyl-6-hydroxy-1,2,3,4-tetrahydronaphthalen-1-yl)phenyl)piperidine-4-carbaldehyde **14c-1** |
| **14c-2** | 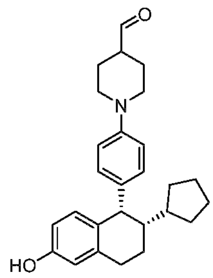 14c-2 |
| | 1-(4-((1*S*,2*S*)-2-Cyclopentyl-6-hydroxy-1,2,3,4-tetrahydronaphthalen-1-yl)phenyl) piperidin e-4-carbaldehyde **14c-2** |
| | 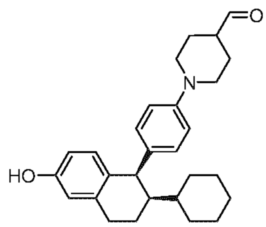 |
| | 1-(4-((1*R*,2*R*)-2-Cyclohexyl-6-hydroxy-1,2,3,4-tetrahydronaphthalen-1-yl)phenyl)piperidin e-4-carbaldehyde |
| | 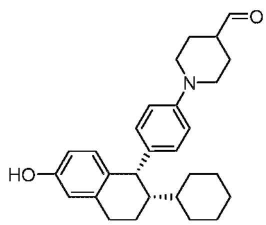 |
| | 1-(4-((1*S*,2*S*)-2-Cyclohexyl-6-hydroxy-1,2,3,4-tetrahydronaphthalen-1-yl)phenyl)piperidin e-4-carbaldehyde |

(continued)

| Example No. | Structures and names of compounds |
|---|---|
| | 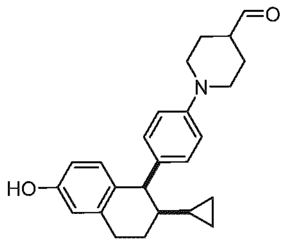 |
| | 1-(4-((1,2)-*cis*-2-Cyclopropyl)-6-hydroxy-1,2,3,4-tetrahydronaphthalen-1-yl)phenyl)pipeiid ine-4-carbaldehyde |
| | 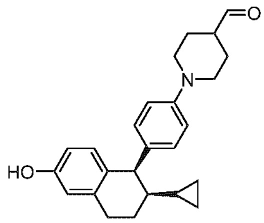 |
| | 1-(4-((1*R*,2*R*)-2-Cyclopropyl-6-hydroxy-1,2,3,4-tetrahydronaphthalen-1-yl)phenyl)piperidi ne-4-carbaldehyde |
| | 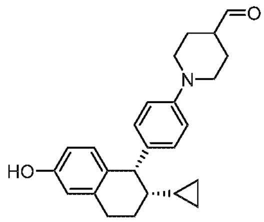 |
| | 1-(4-((1*S*',2*S*)-2-Cyclopropyl-6-hydroxy-1,2,3,4-tetrahydronaphthalen-1-yl)phenyl) piperidin e-4-carbaldehyde |
| | 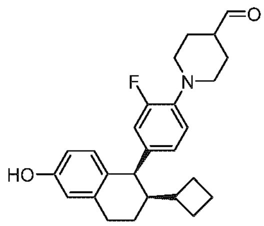 |
| | 1-(4-((1*R*,2*R*)-2-Cyclobuiyl-6-hydroxy-1,2,3,4-tetrahydronaphthalen-1-yl)-2-fluorophenyl) piperidine-4-carbaldehyde |
| | 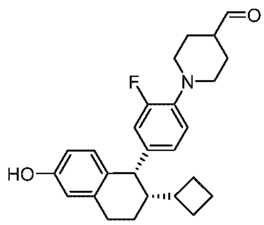 |
| | 1-(4-((1*S*,2*S*)-2-Cyclobutyl-6-hydroxy-1,2,3,4-tetrahydronaphthalen-1-yl)-2-fluorophenyl) p iperidine-4-carbaldehyde |

(continued)

| Example No. | Structures and names of compounds |
|---|---|
| **19d** | 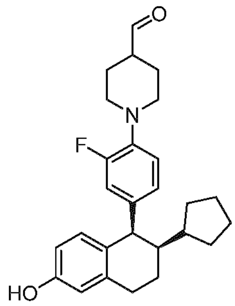 **19d** |
| | 1-(4-((1*R*,2*R*)-2-Cyclopentyl-6-hydroxy-1,2,3,4-tetrahydronaphthalen-1-yl)-2-fluorophenyl )piperidine-4-carbaldehyde 19d |
| | 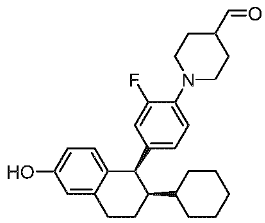 |
| | 1-(4-((1*R*,2*R*)-2-Cyclohexyl-6-hydroxy-1,2,3,4-tetrahydronaphthalen-1-yl)-2-fluorophenyl) piperidine-4-carbaldehyde |
| | 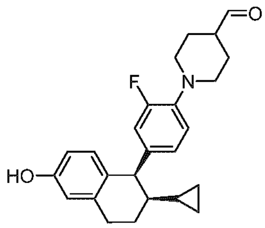 |
| | 1-(4-((1*R*,2*R*)-2-Cyclopropyl-6-hydroxy-1,2,3,4-tetrahydronaphthalen-1-yl)-2-fluorophenyl )piperidine-4-carbaldehyde |
| **22g** | 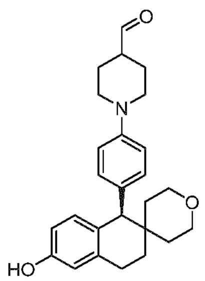 **22g** |
| | (*R*)-1-(4-(6-Hydroxy-2',3,3',4,5',6'-hexahydro-1*H*-spiro[naphthalene-2,4'-pyran]-1-yl) phenyl)piperidine-4-carbaldehyde 22g |

(continued)

| Example No. | Structures and names of compounds |
|---|---|
| **23j-1** | 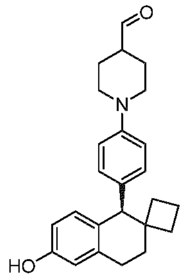 **23j-1** |
| | (*R*)-1-(4-(6'-Hydroxy-3 ',4'-dihydro-1'*H*-spiro[cyclobutane-1,2'-naphthalen]-1'-yl)phenyl) pip eridine-4-carbaldehyde 23j-1 |
| **23j-2** | 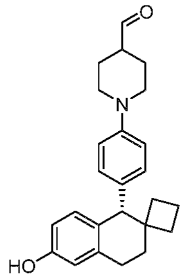 **23j-2** |
| | (*S*)-1-(4-(6'-Hydroxy-3',4'-dihydro-1'*H*-spiro[cyclobutane-1,2'-naphthalen]-1'-yl)phenyl)pip eridine-4-carbaldehyde 23j-2 |
| | 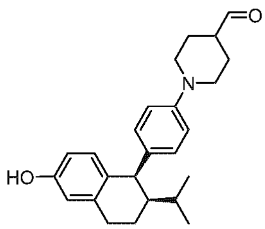 |
| | 1-(4-((1*R*,2*R*)-6-Hydroxy-2-isopropyl-1,2,3,4-tetrahydronaphthalen-1-yl)phenyl) piperidine-4-carbaldehyde |
| | 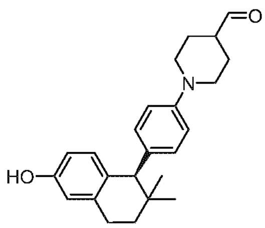 |
| | (*R*)-1-(4-(6-Hydroxy-2,2-dimethyl-1,2,3,4-tetrahydronaphthalen-1-yl)phenyl)piperidine-4- c arbaldehyde |

(continued)

| Example No. | Structures and names of compounds |
|---|---|
| | 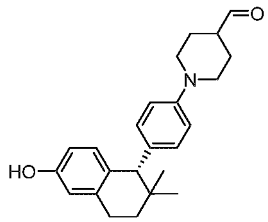 |
| | (*S*)-1-(4-(6-Hydroxy-2,2-dimethyl-1,2,3,4-tetrahydronaphthalen-1-yl)phenyl)piperidine-4-c arbaldehyde |
| **26c-1** | 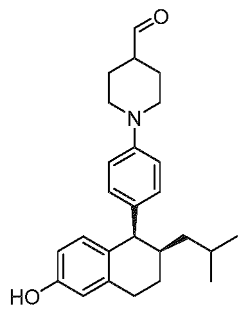 **26c-1** |
| | 1-(4-((1*R*,2*R*)-6-Hydroxy-2-isobutyl-1,2,3,4-tetrahydronaphthalen-1-yl)phenyl)piperidine-4 -carbaldehyde 26c-1 |
| **26c-2** | 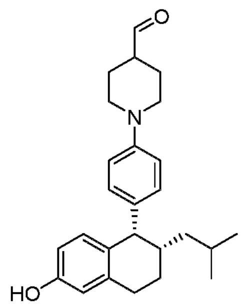 **26c-2** |
| | 1-(4-((1*S*,2*S*)-6-Hydroxy-2-isobutyl-1,2,3,4-tetrahydronaphthalen-1-yl)phenyl)piperidine-4 -carbaldehyde 26c-2 |
| | 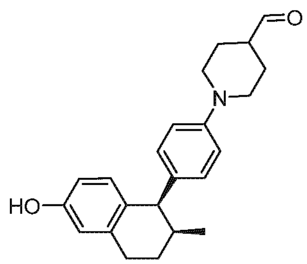 |
| | 1-(4-((1*R*,2*S*)-6-Hydroxy-2-methyl-1,2,3,4-tetrahydronaphthalen-1-yl)phenyl)piperidine-4-carbaldehyde |

(continued)

| Example No. | Structures and names of compounds |
|---|---|
| | 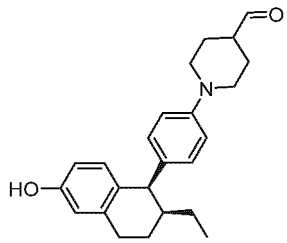 |
| | 1-(4-((1*R*,2*S*)-2-Ethyl-6-hydroxy-1,2,3,4-tetrahydronaphthalen-1-yl)phenyl)piperidine-4-ca rbaldehyde |
| | 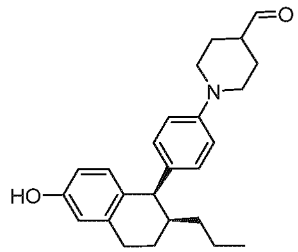 |
| | 1-(4-((1*R*,2*S*)-6-Hydroxy-2-propyl-1,2,3,4-tetrahydronaphthalen-1-yl)phenyl)piperidine-4-c arbaldehyde |
| | 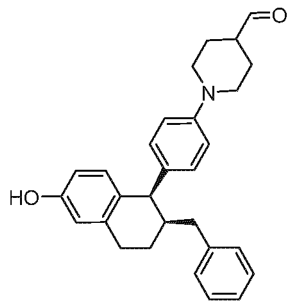 |
| | 1-(4-((1*R*,2*R*)-2-Benzyl-6-hydroxy-1,2,3,4-tetrahydronaphthalen-1-yl)phenyl)piperidine-4-carbaldehyde |
| **31i** | 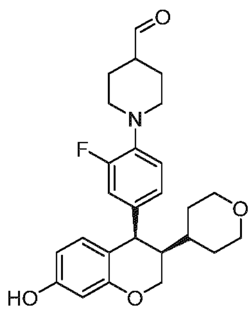 **31i** |
| | 1-(2-Fluoro-4-((3*R*,4*R*)-7-hydroxy-3-(tetrahydro-2*H*-pyran-4-yl)chroman-4-yl)phenyl)piper idine-4-carbaldehyde **31i** |

(continued)

| Example No. | Structures and names of compounds |
|---|---|
| **32i** | 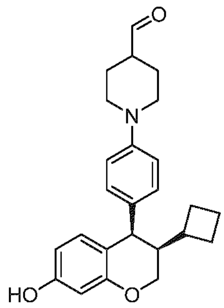<br>**32i** |
| | 1-(4-((3*R*,4*R*)-3-Cyclobutyl-7-hydroxychroman-4-yl)phenyl)piperidine-4-carbaldehyde **32i** |
| **34e** | 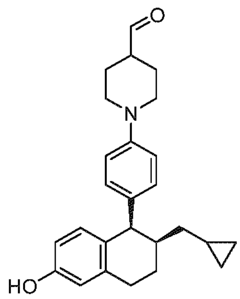<br>**34e** |
| | 1-(4-((1*R*,2*R*)-2-(Cyclopropylmethyl)-6-hydroxy-1,2,3,4-tetrahydronaphthalen-1-yl) phenyl )piperidine-4-carbaldehyde **34e** |
| **35g** | 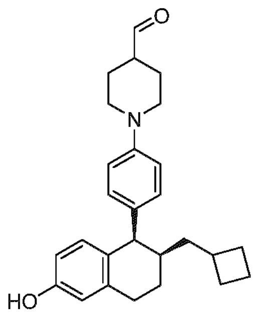<br>**35g** |
| | 1-(4-((1*R*,2*R*)-2-(Cyclobutylmethyl)-6-hydroxy-1,2,3,4-tetrahydronaphthalen-1-yl)phenyl) p iperidine-4-carbaldehyde **35g** |

(continued)

| Example No. | Structures and names of compounds |
|---|---|
| **36e** | 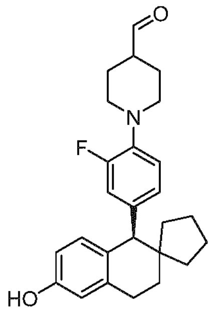 **36e** |
| | (*R*)-1-(2-Fluoro-4-(6'-hydroxy-3',4'-dihydro-1'*H*-spiro[cyclopentane-1,2'-naphthalen]-1'-yl) phenyl)piperidine-4-carbaldehyde **36e** |
| **37d** | 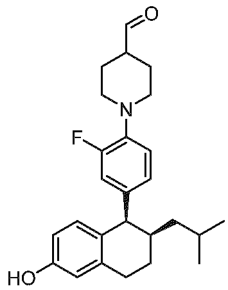 **37d** |
| | 1-(2-Fluoro-4-((1*R*,2*R*)-6-hydroxy-2-isobutyl-1,2,3,4-tetrahydronaphthalen-1-yl)phenyl) pip eridine-4-carbaldehyde **37d** |
| | 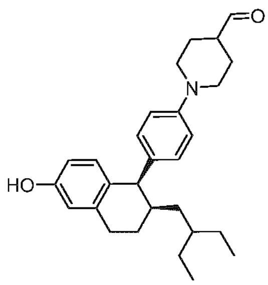 |
| | 1-(4-((1*R*,2*R*)-2-(2-Ethyl-tert-butyl)-6-hydroxy-1,2,3,4-tetrahydronaphthalen-1-yl)phenyl)p iperidine-4-carbaldehyde |
| | 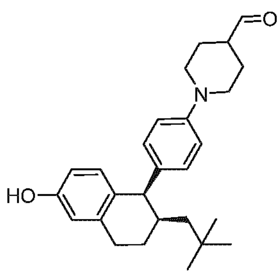 |
| | 1-(4-((1R,2R)-6-Hydroay-2-neopentyl-1,2,3,4-tetrahydronaphthalen-1-yl)phenyl) piperidine -4-carbaldehyde |

(continued)

| Example No. | Structures and names of compounds |
|---|---|
| | 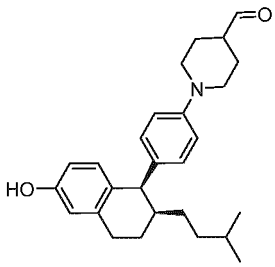 |
| | 1-(4-((1*R*,2*S*-6-Hydroxy-2-isopentyl-1,2,3,4-tetrahydronaphthalen-1-yl)phenyl)piperidine-4-carbaldehyde |

Another aspect of the present disclosure relates to a method for preparing a compound of general formula (III) or a pharmaceutically acceptable salt thereof, which comprises:

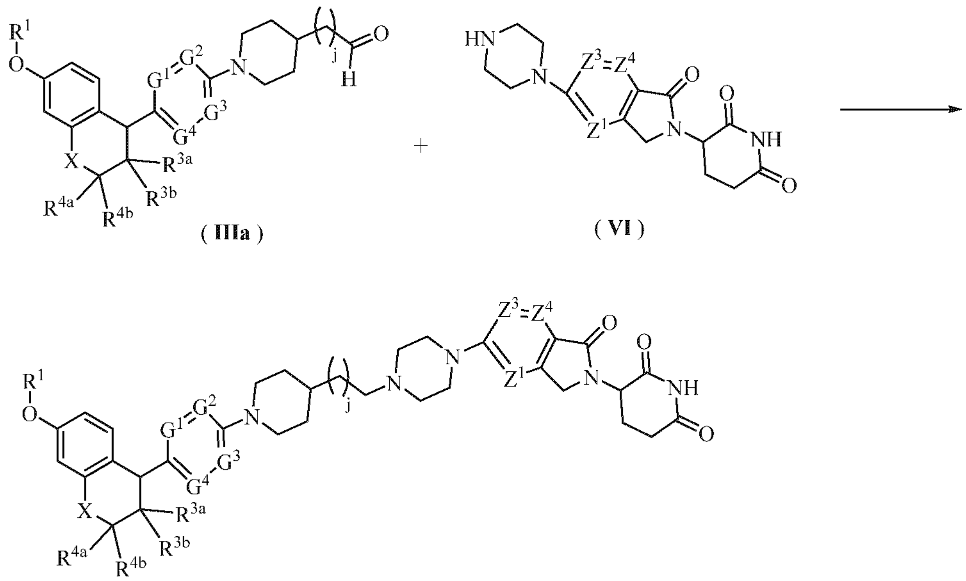

(IIIa) (VI) (III)

conducting a reductive amination reaction of a compound of general formula (IIIa) with a compound of general formula (VI) or a salt thereof to give the compound of general formula (III) or the pharmaceutically acceptable salt thereof; wherein:

X, $G^1$ to $G^4$, $Z^1$, $Z^3$, $Z^4$, $R^1$, $R^{3a}$, $R^{3b}$, $R^{4a}$, $R^{4b}$ and j are as defined in general formula (III).

Another aspect of the present disclosure relates to a method for preparing a compound of general formula (IV) or a pharmaceutically acceptable salt thereof, which comprises:

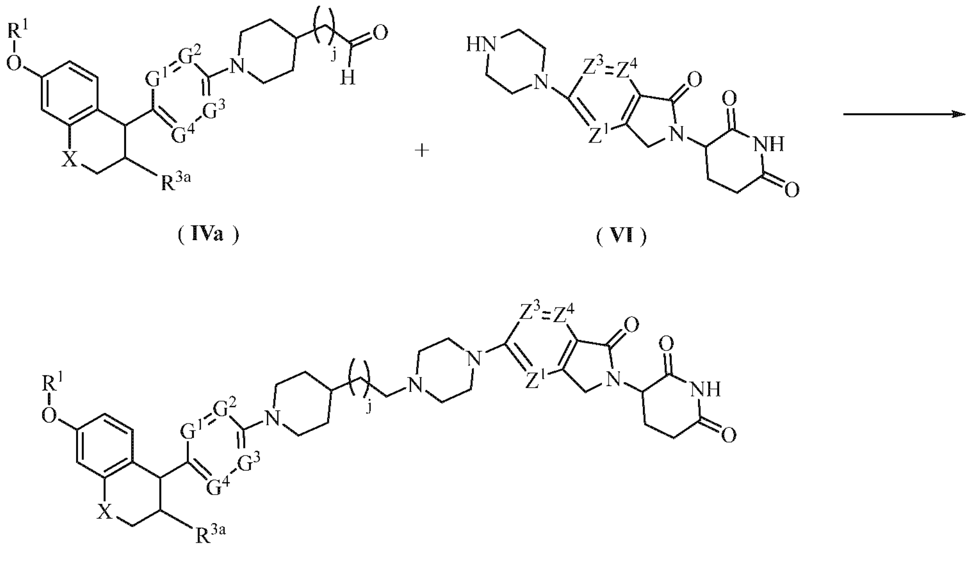

( IVa ) ( VI )

( IV )

conducting a reductive amination reaction of a compound of general formula (IVa) with a compound of general formula (VI) or a salt thereof to give the compound of general formula (IV) or the pharmaceutically acceptable salt thereof; wherein:

X, $G^1$ to $G^4$, $Z^1$, $Z^3$, $Z^4$, $R^1$, $R^{3a}$ and j are as defined in general formula (IV).

Another aspect of the present disclosure relates to a method for preparing a compound of general formula (IV-1) or a pharmaceutically acceptable salt thereof, which comprises:

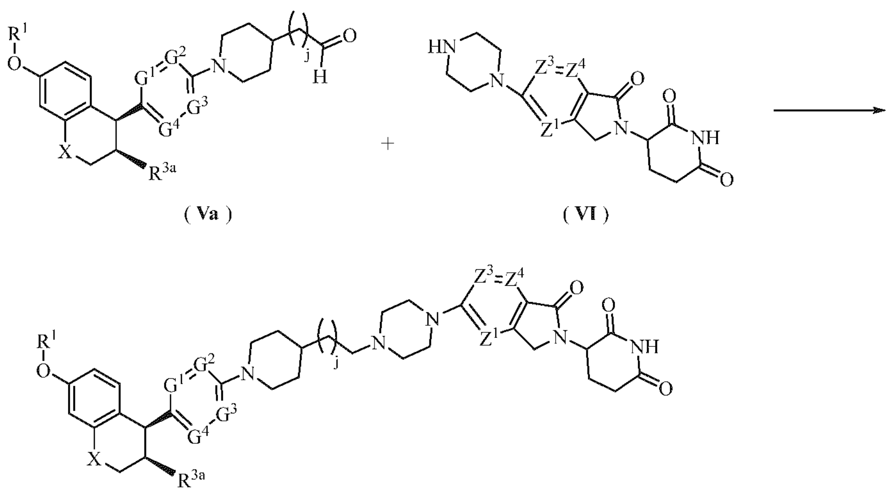

( Va ) ( VI )

( IV-1 )

conducting a reductive amination reaction of a compound of general formula (Va) with a compound of general formula (VI) or a salt thereof to give the compound of general formula (IV-1) or the pharmaceutically acceptable salt thereof; wherein:

X, $G^1$ to $G^4$, $Z^1$, $Z^3$, $Z^4$, $R^1$, $R^{3a}$ and j are as defined in general formula (IV-1).

Another aspect of the present disclosure relates to a method for preparing a compound of general formula (V) or a pharmaceutically acceptable salt thereof, which comprises:

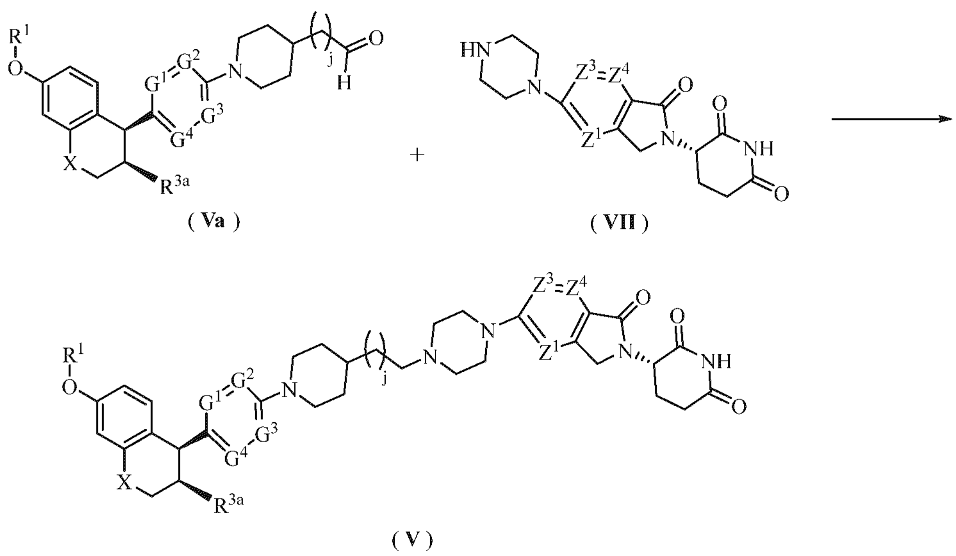

(Va) (VII) (V)

conducting a reductive amination reaction of a compound of general formula (Va) with a compound of general formula (VII) or a salt thereof to give the compound of general formula (V) or the pharmaceutically acceptable salt thereof; wherein:

X, $G^1$ to $G^4$, $Z^1$, $Z^3$, $Z^4$, $R^1$, $R^{3a}$ and j are as defined in general formula (V).

Another aspect of the present disclosure relates to a method for preparing a compound of general formula (VB) or a pharmaceutically acceptable salt thereof, which comprises:

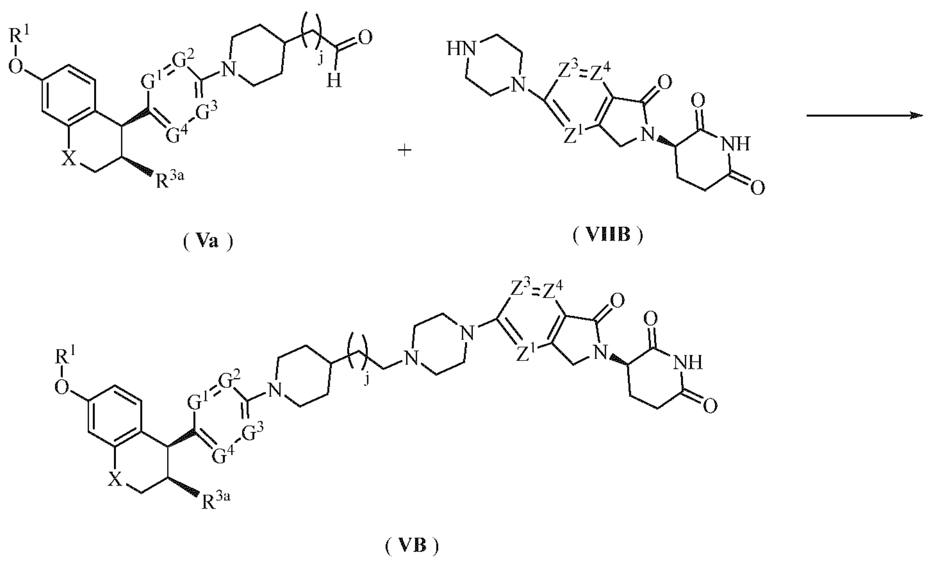

(Va) (VIIB) (VB)

conducting a reductive amination reaction of a compound of general formula (Va) with a compound of general formula (VIIB) or a salt thereof to give the compound of general formula (VB) or the pharmaceutically acceptable salt thereof; wherein:

X, $G^1$ to $G^4$, $Z^1$, $Z^3$, $Z^4$, $R^1$, $R^{3a}$ and j are as defined in general formula (VB).

Another aspect of the present disclosure relates to a pharmaceutical composition comprising the compound of general formula (I), general formula (II), general formula (III), general formula (IV), general formula (IV-1), general formula (V) or general formula (VB) of the present disclosure and a compound shown in Table A or a pharmaceutically acceptable salt thereof, and one or more pharmaceutically acceptable carriers, diluents or excipients.

The present disclosure further relates to use of the compound of general formula (I), general formula (II), general formula (III), general formula (IV), general formula (IV-1), general formula (V) or general formula (VB) and a compound shown in Table A or a pharmaceutically acceptable salt thereof or the pharmaceutical composition comprising same in the preparation of a medicament for treating and/or preventing a disease or disorder by degrading a target protein.

The present disclosure further relates to use of the compound of general formula (I), general formula (II), general

formula (III), general formula (IV), general formula (IV-1), general formula (V) or general formula (VB) and a compound shown in Table A or a pharmaceutically acceptable salt thereof or the pharmaceutical composition comprising same in the preparation of a medicament for treating and/or preventing an estrogen receptor mediated or dependent disease or disorder.

The present disclosure also relates to a method for treating and/or preventing a disease or disorder by degrading a target protein, which comprises administering to a patient in need thereof a therapeutically effective amount of the compound of general formula (I), general formula (II), general formula (III), general formula (IV), general formula (IV-1), general formula (V) or general formula (VB) and a compound shown in Table A or a pharmaceutically acceptable salt thereof, or the pharmaceutical composition comprising same.

The present disclosure also relates to a method for treating and/or preventing an estrogen receptor mediated or dependent disease or disorder, which comprises administering to a patient in need thereof a therapeutically effective amount of the compound of general formula (I), general formula (II), general formula (III), general formula (IV), general formula (IV-1), general formula (V) or general formula (VB) and a compound shown in Table A or a pharmaceutically acceptable salt thereof, or the pharmaceutical composition comprising same.

The present disclosure further relates to a compound of general formula (I), general formula (II), general formula (III), general formula (IV), general formula (IV-1), general formula (V) or general formula (VB) and a compound shown in Table A or a pharmaceutically acceptable salt thereof, or a pharmaceutical composition comprising same, for use as a medicament.

The present disclosure further relates to the compound of general formula (I), general formula (II), general formula (III), general formula (IV), general formula (IV-1), general formula (V) or general formula (VB) and a compound shown in Table A or a pharmaceutically acceptable salt thereof, or the pharmaceutical composition comprising same, for use in treating and/or preventing a disease or disorder by degrading a target protein.

The present disclosure further relates to the compound of general formula (I), general formula (II), general formula (III), general formula (IV), general formula (IV-1), general formula (V) or general formula (VB) and a compound shown in Table A or a pharmaceutically acceptable salt thereof, or the pharmaceutical composition comprising same, for use in treating and/or preventing an estrogen receptor mediated or dependent disease or disorder.

The disease or disorder described above in the present disclosure which is treated and/or prevented by degrading a target protein is preferably selected from the group consisting of abnormal cell proliferation, a tumor, an immune disease, diabetes, a cardiovascular disease, an infectious disease and an inflammatory disease; and more preferably a tumor and an infectious disease; wherein the tumor is a cancer preferably selected from the group consisting of breast cancer, endometrial cancer, testicular cancer, cervical cancer, prostate cancer, ovarian cancer, fallopian tube tumors, leukemia, skin cancer, squamous cell carcinoma, basal cell carcinoma, bladder cancer, colorectal cancer (such as colon cancer and rectal cancer), esophageal cancer, head and neck cancer, kidney cancer, liver cancer, lung cancer, pancreatic cancer, gastric cancer, lymphoma (such as non-Hodgkin lymphoma and Hodgkin lymphoma), melanoma, sarcoma (such as angiosarcoma and meningeal sarcoma), peripheral neuroepithelioma, neuroglioma (such as oligodendroglioma and ganglioglioma), astrocytoma, ependymoma, glioblastoma, neuroblastoma, gangliocytoma, medulloblastoma, pineocytoma, meningioma, neurofibroma, neurilemmoma, thyroid cancer, Wilms tumor and teratocarcinoma; and more preferably selected from the group consisting of breast cancer, endometrial cancer, testicular cancer, cervical cancer, prostate cancer, ovarian cancer and fallopian tube tumors; wherein the infectious disease is selected from the group consisting of viral pneumonia, influenza, avian influenza, meningitis, gonorrhea, and diseases caused by infection with HIV, HBV, HCV, HSV, HPV, RSV, CMV, ebolaviruses, flaviviruses, pestiviruses, rotaviruses, coronaviruses, EBV, drug-resistant viruses, RNA viruses, DNA viruses, adenoviruses, poxviruses, picornaviruses, togaviruses, orthomyxoviruses, retroviruses, hepadnaviruses, gram-negative bacteria, gram-positive bacteria, atypical bacteria, staphylococci, streptococci, *Escherichia coli, Salmonella, Helicobacter pylori, Chlamydiaceae, Mycoplasmataceae,* fungi, protozoa, helminths, worms, prions and parasites.

The estrogen receptor mediated or dependent disease or disorder described above in the present disclosure is a tumor; preferably, the disease or disorder is a cancer; more preferably, the disease or disorder is selected from the group consisting of breast cancer, endometrial cancer, testicular cancer, cervical cancer, prostate cancer, ovarian cancer and fallopian tube tumors; most preferably, the disease or disorder is breast cancer.

The active compound may be formulated into a form suitable for administration by any suitable route, preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder, or liquid formulation.

As a general guide, a suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 wt.% to 99 wt.% of the active compound.

The pharmaceutical composition comprising the active ingredient may be in a form suitable for oral administration, for example, in the form of a tablet, a dragee, a lozenge, an aqueous or oil suspension, a dispersible powder or granule, an emulsion, a hard or soft capsule, or a syrup or elixir. Oral compositions can be prepared according to any method known in the art for preparing pharmaceutical compositions and may comprise one or more ingredients selected from the group consisting of sweetener, flavoring agent, colorant and preservative, so as to provide a pleasant-to-eye and palatable pharmaceutical formulation. The tablet comprises the active ingredient and a non-toxic pharmaceutically acceptable excipient that is used for mixing and is suitable for the preparation of the tablet. Such an excipient may be an inert excipient, a granulating agent, a disintegrant, a binder, and a lubricant. Such a tablet may be uncoated or may be coated by known techniques for masking the taste of the drug or delaying the disintegration and absorption of the drug in the gastrointestinal tract and thus enabling a sustained release of the drug over a longer period.

An oral formulation in a soft gelatin capsule where the active ingredient is mixed with an inert solid diluent or with a water-soluble carrier or oil vehicle may also be provided. An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more flavoring agents, and one or more sweeteners.

An oil suspension may be formulated by suspending the active ingredient in a vegetable oil, or in a mineral oil. The oil suspension may comprise a thickening agent. The sweeteners and flavoring agents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a flavoring agent, a preservative, and an antioxidant. Such a formulation may also comprise a palliative, a preservative, a colorant, and an antioxidant.

The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using suitable dispersants or wetting agents and suspending agents. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

As is well known to those skilled in the art, the dose of the drug administered depends on a variety of factors, including but not limited to: the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of administration, the daily dose of the compound, or the type of pharmaceutically acceptable salts, can be verified according to conventional treatment regimens.

## Description of the terms

Unless otherwise stated, the terms used in the specification and claims have the following meanings.

The term "alkyl" refers to a saturated linear or branched aliphatic hydrocarbon group containing 1 to 20 carbon atoms. Alkyl preferably has 1 to 12 (e.g., 1, 2, 3, 4, 5, 6, 7, 8, 9, 10, 11 and 12) carbon atoms (i.e., $C_{1-12}$ alkyl), and more preferably has 1 to 6 carbon atoms (i.e., $C_{1-6}$ alkyl). Non-limiting examples of alkyl include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various side-chain isomers thereof, and the like. Alkyl may be substituted or unsubstituted. When substituted, it may be substituted at any accessible point of attachment, and the substituent is preferably selected from one or more of deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroalkyl" refers to alkyl in which one or more (preferably 1, 2, 3, 4 or 5) $-CH_2-$ are replaced by heteroatoms selected from the group consisting of N, O and S, wherein the alkyl is as defined above. Heteroalkyl may be substituted or unsubstituted. When substituted, it may be substituted at any accessible point of attachment, and the substituent is preferably selected from one or more of deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkylene" refers to a saturated linear or branched aliphatic hydrocarbon group which is a residue derived from the parent alkane by removal of two hydrogen atoms from the same carbon atom or two different carbon atoms. Alkylene is a linear or branched group containing 1 to 20 carbon atoms. Alkylene preferably has 1 to 12 (e.g., 1, 2, 3, 4, 5, 6, 7, 8, 9, 10, 11 and 12) carbon atoms (i.e., $C_{1-12}$ alkylene), and more preferably has 1 to 6 carbon atoms (i.e., $C_{1-6}$ alkylene). Non-limiting examples of alkylene include, but are not limited to, methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), and the like. Alkylene may be substituted or unsubstituted. When substituted, it may be substituted at any accessible point of attachment, and the substituent is preferably selected from one or more of deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroalkylene" refers to alkylene in which one or more (preferably 1, 2, 3, 4 or 5) $-CH_2-$ are replaced by heteroatoms selected from the group consisting of N, O and S, wherein the alkylene is as defined above. Heteroalkylene may be substituted or unsubstituted. When substituted, it may be substituted at any accessible point of attachment, and the substituent is preferably selected from one or more of deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkenyl" refers to an alkyl compound having at least one carbon-carbon double bond in the molecule, wherein the alkyl is as defined above. Alkenyl preferably has 2 to 12 (e.g., 2, 3, 4, 5, 6, 7, 8, 9, 10, 11 and 12) carbon atoms (i.e., $C_{2-12}$ alkenyl), and more preferably has 2 to 6 carbon atoms (i.e., $C_{2-6}$ alkenyl). Alkenyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkenylene" refers to alkenyl in which one or more (preferably 1, 2, 3, 4 or 5) $-CH_2-$ are replaced by heteroatoms selected from the group consisting of N, O and S, wherein the alkenyl is as defined above. Alkenylene may be substituted or unsubstituted. When substituted, it may be substituted at any accessible point of attachment, and the substituent is preferably selected from one or more of deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to an alkyl compound having at least one carbon-carbon triple bond in the molecule, wherein the alkyl is as defined above. Alkynyl preferably has 2 to 12 (e.g., 2, 3, 4, 5, 6, 7, 8, 9, 10, 11 and 12) carbon atoms (i.e., $C_{2-12}$ alkynyl), and more preferably has 2 to 6 carbon atoms (i.e., $C_{2-6}$ alkynyl). Alkynyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynylene" refers to alkynyl in which one or more (preferably 1, 2, 3, 4 or 5) $-CH_2-$ are replaced by heteroatoms selected from the group consisting of N, O and S, wherein the alkynyl is as defined above. Alkynylene may be substituted or unsubstituted. When substituted, it may be substituted at any accessible point of attachment, and the substituent is preferably selected from one or more of D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent. The cycloalkyl ring contains 3 to 20 carbon atoms (i.e., 3- to 20-membered cycloalkyl), preferably 3 to 12 (e.g., 3, 4, 5, 6, 7, 8, 9, 10, 11 and 12) carbon atoms (i.e., 3- to 12-membered cycloalkyl), preferably 3 to 8 carbon atoms (i.e., 3- to 8-membered cycloalkyl), and more preferably 3 to 6 carbon atoms (i.e., 3- to 6-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl and bridged cycloalkyl.

The term "spiro cycloalkyl" refers to a 5- to 20-membered (i.e., 5- to 20-membered spiro cycloalkyl) polycyclic group in which monocyclic rings share one carbon atom (referred to as the spiro atom), and it may have one or more double bonds. Spiro cycloalkyl is preferably 6- to 14-membered (i.e., 6- to 14-membered spiro cycloalkyl), and is more preferably 7- to 10-membered (e.g., 7-, 8-, 9- or 10-membered) (i.e., 7- to 10-membered spiro cycloalkyl). According to

the number of the spiro atoms shared among the rings, spiro cycloalkyl may be monospiro cycloalkyl or polyspiro cycloalkyl (e.g., bispiro cycloalkyl), preferably monospiro cycloalkyl and bispiro cycloalkyl, and more preferably 3-membered/5-membered, 3-membered/6-membered, 4-membered/4-membered, 4-membered/5-membered, 4-membered/6-membered, 5-membered/5-membered, 5-membered/6-membered, 6-membered/6-membered, 6-membered/4-membered, 6-membered/5-membered or 6-membered/6-membered monospiro cycloalkyl. Non-limiting examples of spiro cycloalkyl include:

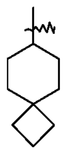 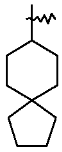 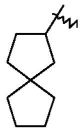 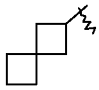 and 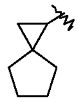 .

The term "fused cycloalkyl" refers to a 5- to 20-membered (i.e., 5- to 20-membered fused cycloalkyl) all-carbon polycyclic group in which rings share a pair of adjacent carbon atoms, wherein one or more rings may have one or more double bonds. Fused cycloalkyl is preferably 6- to 14-membered (i.e., 6- to 14-membered fused cycloalkyl), and is more preferably 7- to 10-membered (e.g., 7-, 8-, 9- or 10-membered) (i.e., 7- to 10-membered fused cycloalkyl). According to the number of constituent rings, fused cycloalkyl may be polycyclic, e.g., bicyclic, tricyclic or tetracyclic, preferably bicyclic or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused cycloalkyl. Non-limiting examples of fused cycloalkyl include:

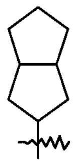 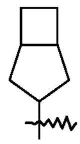 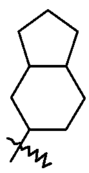 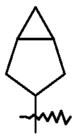 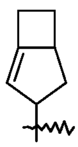 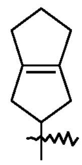 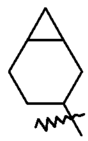 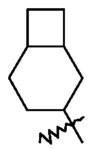 and 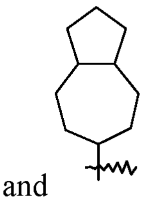 .

The term "bridged cycloalkyl" refers to a 5- to 20-membered (i.e., 5- to 20-membered bridged cycloalkyl) all-carbon polycyclic group in which any two rings share two carbon atoms that are not directly connected, and it may have one or more double bonds. Bridged cycloalkyl is preferably 6- to 14-membered (i.e., 6- to 14-membered bridged cycloalkyl), and is more preferably 7- to 10-membered (e.g., 7-, 8-, 9- or 10-membered) (i.e., 7- to 10-membered bridged cycloalkyl). According to the number of constituent rings, bridged cycloalkyl may be polycyclic, e.g., bicyclic, tricyclic or tetracyclic, preferably bicyclic, tricyclic or tetracyclic bridged cycloalkyl, and more preferably bicyclic or tricyclic bridged cycloalkyl. Non-limiting examples of bridged cycloalkyl include:

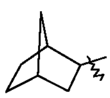 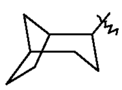 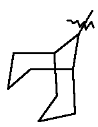 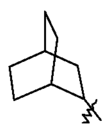 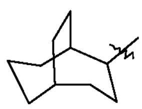

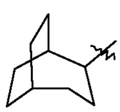 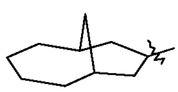 and 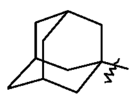 .

The cycloalkyl ring includes those in which the cycloalkyl described above (including monocyclic, spiro, fused and bridged ones) is fused to an aryl, heteroaryl or heterocycloalkyl ring, wherein the ring connected to the parent structure is cycloalkyl. Non-limiting examples include

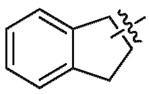, 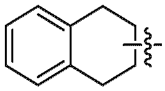, 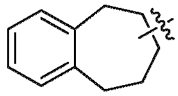,

and the like;

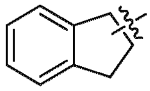 and 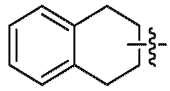

are preferred.

Cycloalkyl may be substituted or unsubstituted. When substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples of alkoxy include: methoxy, ethoxy, propoxy and butoxy. Alkoxy may be optionally substituted or unsubstituted. When it is substituted, the substituent is preferably selected from one or more of deuterium atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic substituent containing 3 to 20 ring atoms (i.e., 3- to 20-membered heterocyclyl), wherein one or more of the ring atoms are heteroatoms selected from the group consisting of nitrogen, oxygen and sulfur, the sulfur optionally being substituted with oxo (i.e., to form sulfoxide or sulfone), but excluding a cyclic portion of -O-O-, -O-S- or -S-S-; the other ring atoms are carbon. Preferably, heterocyclyl contains 3 to 12 (e.g., 3, 4, 5, 6, 7, 8, 9, 10, 11 and 12) ring atoms, of which 1-4 (e.g., 1, 2, 3 and 4) are heteroatoms (i.e., 3- to 12-membered heterocyclyl); more preferably, heterocyclyl contains 3 to 8 ring atoms (e.g., 3, 4, 5, 6, 7 and 8), of which 1-3 are heteroatoms (e.g., 1, 2 and 3) (i.e., 3- to 8-membered heterocyclyl); more preferably, heterocyclyl contains 3 to 6 ring atoms, of which 1-3 are heteroatoms (i.e., 3- to 6-membered heterocyclyl); most preferably, heterocyclyl contains 5 or 6 ring atoms, of which 1-3 are heteroatoms (i.e., 5- or 6-membered heterocyclyl). Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5- to 20-membered (i.e., 5- to 20-membered spiro heterocyclyl) polycyclic heterocyclic group in which monocyclic rings share one atom (referred to as the spiro atom), wherein one or more ring atoms are heteroatoms selected from the group consisting of nitrogen, oxygen and sulfur, and the sulfur may optionally be substituted with oxo (i.e., to form sulfoxide or sulfone); the other ring atoms are carbon. Spiro heterocyclyl may have one or more double bonds. Spiro heterocyclyl is preferably 6- to 14-membered (i.e., 6- to 14-membered spiro heterocyclyl), and is more preferably 7- to 10-membered (e.g., 7-, 8-, 9- or 10-membered) (i.e., 7- to 10-membered spiro heterocyclyl). According to the number of spiro atoms shared among the rings, spiro heterocyclyl may be monospiro heterocyclyl, bispiro heterocyclyl or polyspiro heterocyclyl, preferably monospiro heterocyclyl and bispiro heterocyclyl, and more preferably 3-membered/5-membered, 3-membered/6-membered, 4-membered/4-membered, 4-membered/5-membered, 4-membered/6-membered, 5-membered/5-membered, 5-membered/6-membered or 6-membered/6-membered monospiro heterocyclyl. Non-limiting examples of spiro heterocyclyl include:

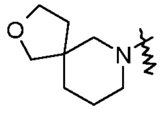 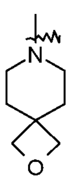 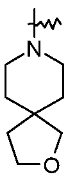 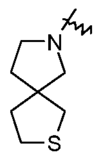 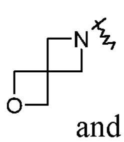 and 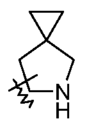.

The term "fused heterocyclyl" refers to a 5- to 20-membered (i.e., 5- to 20-membered fused heterocyclyl) polycyclic heterocyclic group in which rings share a pair of adjacent atoms and one or more rings may have one or more double bonds, wherein one or more ring atoms are heteroatoms selected from the group consisting of nitrogen, oxygen and sulfur, and the sulfur may optionally be substituted with oxo (i.e., to form sulfoxide or sulfone); the other ring atoms are carbon. Fused heterocyclyl is preferably 6- to 14-membered (i.e., 6- to 14-membered fused heterocyclyl), and is more preferably 7- to 10-membered (e.g., 7-, 8-, 9- or 10-membered) (i.e., 7- to 10-membered fused heterocyclyl).

According to the number of constituent rings, fused heterocyclyl may be polycyclic, e.g., bicyclic, tricyclic or tetracyclic, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3 -membered, 6-membered/4-membered, 6-membered/5-membered, 6-membered/6-membered, 6-membered/7-membered, 7-membered/5-membered or 7-membered/6-membered bicyclic fused heterocyclyl. Non-limiting examples of fused heterocyclyl include:

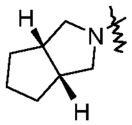 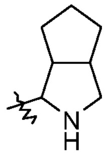 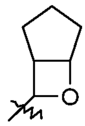 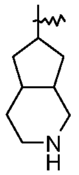 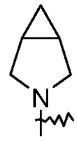 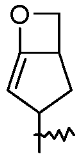 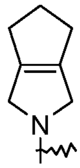 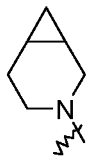

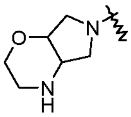, 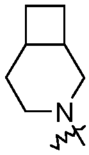, 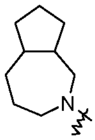, 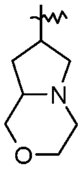, 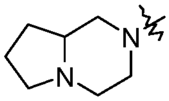 and 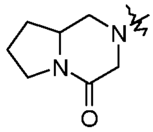.

The term "bridged heterocyclyl" refers to a 5- to 14-membered (i.e., 5- to 14-membered bridged heterocyclyl) polycyclic heterocyclic group in which any two rings share two atoms that are not directly connected, and it may have one or more double bonds, wherein one or more ring atoms are heteroatoms selected from the group consisting of nitrogen, oxygen and sulfur, and the sulfur may optionally be substituted with oxo (i.e., to form sulfoxide or sulfone); the other ring atoms are carbon. Bridged heterocyclyl is preferably 6- to 14-membered (i.e., 6- to 14-membered bridged heterocyclyl), and is more preferably 7- to 10-membered (e.g., 7-, 8-, 9- or 10-membered) (i.e., 7- to 10-membered bridged heterocyclyl). According to the number of constituent rings, bridged heterocyclyl may be polycyclic, e.g., bicyclic, tricyclic or tetracyclic, preferably bicyclic, tricyclic or tetracyclic bridged heterocyclyl, and more preferably bicyclic or tricyclic bridged heterocyclyl. Non-limiting examples of bridged heterocyclyl include:

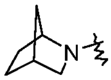 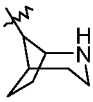 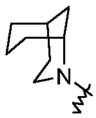 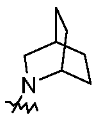

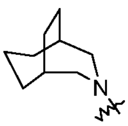 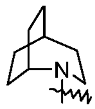 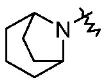 and 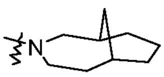.

The heterocyclyl ring includes those in which the heterocyclyl described above (including monocyclic, spiro, fused and bridged ones) is fused to an aryl, heteroaryl or cycloalkyl ring, wherein the ring connected to the parent structure is heterocyclyl; its non-limiting examples include:

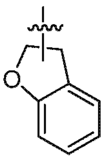, 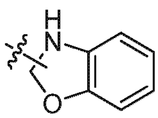, 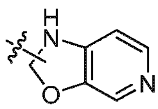, 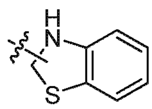,

and the like.

Heterocyclyl may be substituted or unsubstituted. When substituted, it may be substituted at any accessible

point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "aryl" refers to a 6- to 14-membered all-carbon monocyclic or fused polycyclic (in which the rings share a pair of adjacent carbon atoms) group having a conjugated $\pi$-electron system (i.e., 6- to 14-membered aryl), preferably 6- to 10-membered (e.g., 6-, 7-, 8-, 9- or 10-membered) (i.e., 6- to 10-membered aryl), e.g., phenyl and naphthyl. The aryl ring includes those in which the aryl ring described above is fused to a heteroaryl, heterocyclyl or cycloalkyl ring, wherein the ring connected to the parent structure is the aryl ring; its non-limiting examples include:

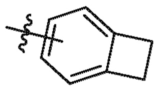, 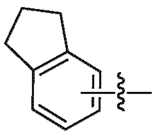, 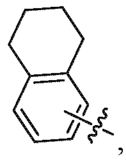, 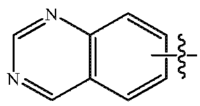, 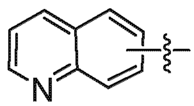, 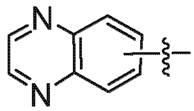,

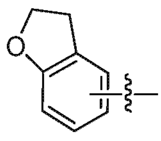, 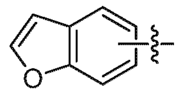, 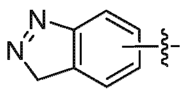, 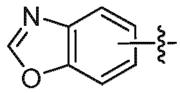, 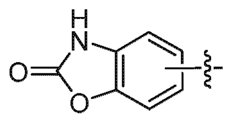,

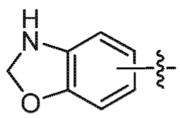, 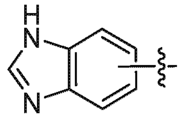, 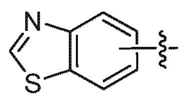, 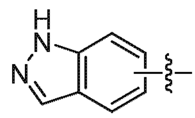, 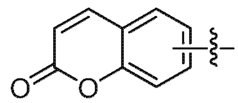,

and

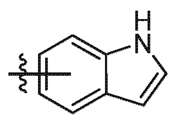.

Aryl may be substituted or unsubstituted. When substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3 and 4) heteroatoms and 5 to 14 ring atoms (i.e., 5- to 14-membered heteroaryl), wherein the heteroatoms are selected from the group consisting of oxygen, sulfur and nitrogen. Heteroaryl is preferably 5- to 10-membered (e.g., 5-, 6-, 7-, 8-, 9- or 10-membered) (i.e., 5- to 10-membered heteroaryl), and is more preferably 5-membered or 6-membered (i.e., 5- or 6-membered heteroaryl), e.g., furyl, thienyl, pyridinyl, pyrrolyl, N-alkylpyrrolyl, pyrimidinyl, pyrazinyl, pyridazinyl, imidazolyl, pyrazolyl, triazolyl or tetrazolyl. The heteroaryl ring includes those in which the heteroaryl described above is fused to an aryl, heterocyclyl or cycloalkyl ring, wherein the ring connected to the parent structure is the heteroaryl ring; its non-limiting examples include:

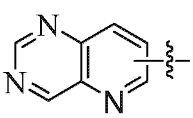 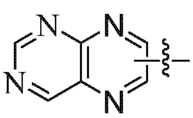 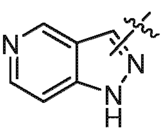 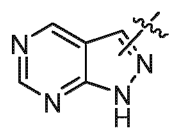 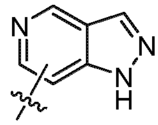 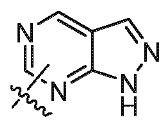

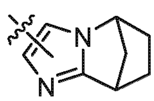 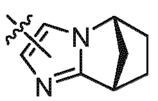 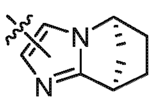 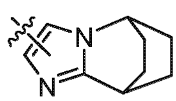 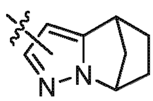 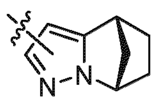

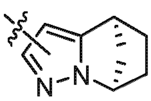 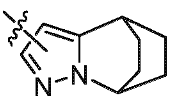 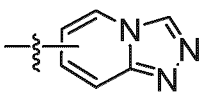 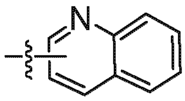 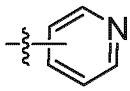 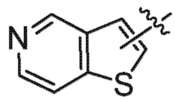

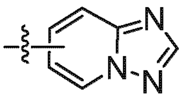 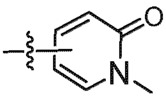 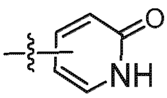 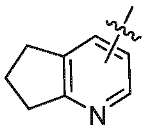 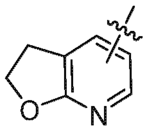 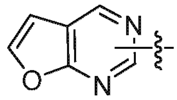

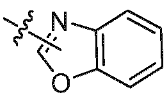 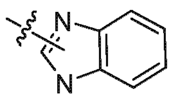 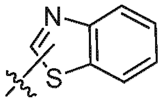 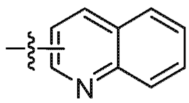 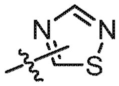 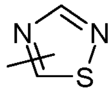

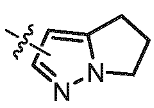 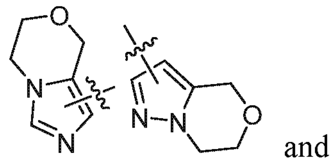 and 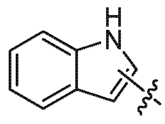.

Heteroaryl may be substituted or unsubstituted. When substituted, it may be substituted at any accessible point of attachment, and the substituent is preferably selected from one or more of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The cycloalkyl, heterocyclyl, aryl and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene" and "heteroarylene".

The term "hydroxy protecting group" refers to a group that is introduced onto a hydroxy group, for blocking or protecting the hydroxy group so that reactions take place on other functional groups of the compound, and the group can be easily removed. Non-limiting examples include: trimethylsilyl (TMS), triethylsilyl (TES), triisopropylsilyl (TIPS), tert-butyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine, or iodine.

The term "hydroxy" refers to -OH.

The term "sulfhydryl" refers to -SH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O- or (cycloalkyl)C(O)O-, wherein the alkyl and cycloalkyl are as defined above.

The term "ubiquitin ligase" refers to a family of proteins that facilitate the transfer of ubiquitin to a specific substrate protein, targeting the substrate protein for degradation. For example, cereblon is an E3 ubiquitin ligase protein that alone or in combination with an E2 ubiquitin conjugating enzyme causes the attachment of ubiquitin to a lysine on a target protein and subsequently targets the specific protein substrate for degradation by the proteasome. Thus, E3 ubiquitin ligase alone or in complex with an E2 ubiquitin conjugating enzyme is responsible for the transfer of ubiquitin to target proteins. In general, the ubiquitin ligase is involved in polyubiquitination such that a second ubiquitin is attached to the first ubiquitin, a third ubiquitin is attached to the second ubiquitin, and so forth. Polyubiquitination marks proteins for degradation by the proteasome. However, there are some ubiquitination events that are limited to mono-ubiquitination, in which only a single ubiquitin is added by the ubiquitin ligase to a substrate molecule. Mono-ubiquitinated proteins are not targeted to the proteasome for degradation, but may instead be altered in their cellular location or function, for example, via binding other proteins that have domains capable of binding ubiquitin. To complicate matters further, different lysines on ubiquitin can be targeted by E3 to prepare chains. The most common lysine is Lys48 on the ubiquitin chain. This is the lysine used to prepare polyubiquitin, which is recognized by the proteasome.

The term "target proteins" refers to proteins and peptides having any biological function or activity (including structural, regulatory, hormonal, enzymatic, genetic, immunological, contractile, storage, transportation and signal transduction). In some embodiments, target proteins include structural proteins, receptors, enzymes, cell surface proteins, and proteins associated with the integrated function of a cell, including proteins involved in: catalytic activity, aromatase activity, motor activity, helicase activity, metabolic processes (anabolism and catrabolism), antioxidant activity, proteolysis, biosynthesis, proteins with kinase activity, oxidoreductase activity, transferase activity, hydrolase activity, lyase activity, isomerase activity, ligase activity, enzyme regulator activity, signal transducer activity, structural molecule activity, binding activity (protein and lipid carbohydrate), receptor activity, cell motility, membrane fusion, cell communication, regulation of biological processes, development, cell differentiation, response to stimulus, behavioral proteins, cell adhesion proteins, proteins involved in cell death, proteins involved in transport (including protein transporter activity, nuclear transport, ion transporter activity, channel transporter activity, and carrier activity), permease activity, secretion activity, electron transporter activity, pathogenesis, chaperone regulator activity, nucleic acid binding activity, transcription regulator activity, extracellular organization and biogenesis activity, and translation regulator activity. The proteins include proteins derived from eukaryotes and prokaryotes, including microbes, viruses, fungi and parasites, among numerous others; including humans, other animals, microbes, viruses, fungi and parasites as targets for drug therapy; and also including microbes for determining targets for antibiotics and plants of other antimicrobials, and even viruses, among numerous others.

The compound of the present disclosure may include atropisomers. The term "atropisomers" refers to conformational stereoisomers that result from hindered or greatly slowed rotation about a single bond in a molecule (as a result of the steric interactions with other parts of the molecule and the asymmetry of the substituents at both ends of the single bond), which interconvert sufficiently slowly to allow separation and isolation under predetermined conditions. For example, certain compounds of the present disclosure may exist in the form of a mixture of atropisomers (e.g., an equal ratio mixture, a mixture enriched in one atropisomer) or a purified atropisomer.

The compounds of the present disclosure may exist in specific stereoisomeric forms. The term "stereoisomer" refers to isomers that are structurally identical but differ in the arrangement of the atoms in space. It encompasses cis and trans (or *Z* and *E*) isomers, (-)- and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. Furthermore, separation of enantiomers and diastereomers is generally accomplished by chromatography.

In the chemical structure of the compound of the present disclosure, a bond "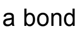" represents an unspecified configuration; that is, if chiral isomers exist in the chemical structure, the bond "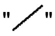" may be "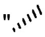" or "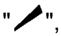", or contains both the configurations of "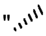" and "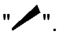". In the chemical structure of the compound of the present disclosure, a bond "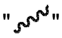" indicates that no configuration is specified; that is, a Z configuration or an E configuration is possible, or both configurations are included.

In the chemical structure of the compound of the present disclosure, two groups on two adjacent carbon atoms are connected to the two carbon atoms respectively with bonds "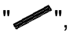", which indicate that the two groups are in a *cis* configuration. For example, 3-(5-(4-((1-(2-fluoro-4-((1,2)-*cis*-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione,

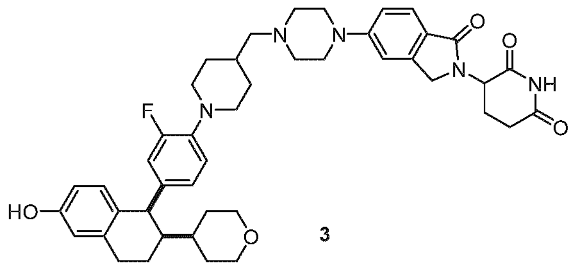

3

,

is in a (1,2)-*cis* configuration; that is, the two groups connected by "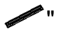" are on the same side: they are both above the paper plane or below the paper plane.

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It encompasses all possible tautomers; that is, it is present in the form of a single isomer or in the form of a mixture of the tautomers in any ratio. Non-limiting examples include, for example, keto-enol tautomerism, imine-enamine tautomerism, and lactam-lactim tautomerism. An example of a lactam-lactim equilibrium is present between A and B as shown below:

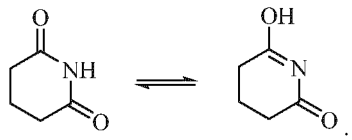

For example, reference to pyrazolyl is understood to include any one of the following two structures or a mixture of the two tautomers:

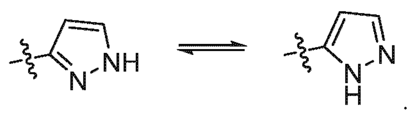

All tautomeric forms fall within the scope of the present disclosure, and the nomenclature of the compounds does not exclude any tautomers.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as $^{2}H$ (deuterium, D), $^{3}H$ (deuterium, T), $^{11}C$, $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$, and $^{131}I$, respectively; deuterium is preferred.

Compared with non-deuterated drugs, deuterated drugs have the advantages of reduced toxic and side effects, increased drug stability, enhanced curative effect, prolonged biological half-lives, and the like. All isotopic variations of the compounds of the present disclosure, whether radioactive or not, are intended to be included within the scope of the present disclosure. Each available hydrogen atom linked to a carbon atom may be independently replaced with a deuterium atom, wherein replacement of deuterium may be partial or complete, and replacement of partial deuterium refers to replacement of at least one hydrogen atom with at least one deuterium atom.

When a position is specifically assigned deuterium (D), the position should be construed as deuterium with an abundance that is at least 1000 times greater than the natural abundance of deuterium (which is 0.015%) (i.e., at least 15% deuterium incorporation). The deuterium of the compounds in the examples with an abundance greater than the natural abundance of deuterium may be deuterium with an abundance that is at least 1000 times greater (i.e., at least 15% deuterium incorporation), at least 2000 times greater (i.e., at least 30% deuterium incorporation), at least 3000 times greater (i.e., at least 45% deuterium incorporation), at least 3340 times greater (i.e., at least 50.1% deuterium incorporation), at least 3500 times greater (i.e., at least 52.5% deuterium incorporation), at least 4000 times greater (i.e., at least 60% deuterium incorporation), at least 4500 times greater (i.e., at least 67.5% deuterium incorporation), at least

5000 times greater (i.e., at least 75% deuterium incorporation), at least 5500 times greater (i.e., at least 82.5% deuterium incorporation), at least 6000 times greater (i.e., at least 90% deuterium incorporation), at least 6333.3 times greater (i.e., at least 95% deuterium incorporation), at least 6466.7 times greater (i.e., at least 97% deuterium incorporation), at least 6600 times greater (i.e., at least 99% deuterium incorporation), or at least 6633.3 times greater (i.e., at least 99.5% deuterium incorporation), or deuterium with a higher abundance.

"Optionally" or "optional" means that the event or circumstance subsequently described may, but does not necessarily, occur and that the description includes instances where the event or circumstance occurs or does not occur. For example, "$C_{1-6}$ alkyl that is optionally substituted with a halogen or cyano" means that the halogen or cyano may, but does not necessarily, exist, and the description includes the instance where alkyl is substituted with a halogen or cyano and the instance where alkyl is not substituted with a halogen or cyano.

"Substituted" means that one or more, preferably 1 to 6, and more preferably 1 to 3, hydrogen atoms in the group are independently substituted with a corresponding number of substituents. Those skilled in the art can determine (experimentally or theoretically) possible or impossible substitution without undue effort. For example, it may be unstable when amino or hydroxy having free hydrogen is bound to a carbon atom having an unsaturated (e.g., olefinic) bond.

"Pharmaceutical composition" refers to a mixture containing one or more of the compounds described herein or a physiologically/pharmaceutically acceptable salt or pro-drug thereof, and other chemical components, and other components, for example, physiologically/pharmaceutically acceptable carriers and excipients. The pharmaceutical composition is intended to promote the administration to an organism, so as to facilitate the absorption of the active ingredient, thereby exerting biological activity.

"Pharmaceutically acceptable salt" refers to a salt of the compound of the present disclosure, which may be selected from the group consisting of inorganic and organic salts. The salts are safe and effective for use in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of a medicament or an agent that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

The term "pharmaceutically acceptable" used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

Synthesis Methods for Compounds of the Present Disclosure

To achieve the purpose of the present disclosure, the following technical schemes are adopted in the present disclosure:

Scheme 1

A method for preparing the compound of general formula (III) or the pharmaceutically acceptable salt thereof of the present disclosure, which comprises the following step:

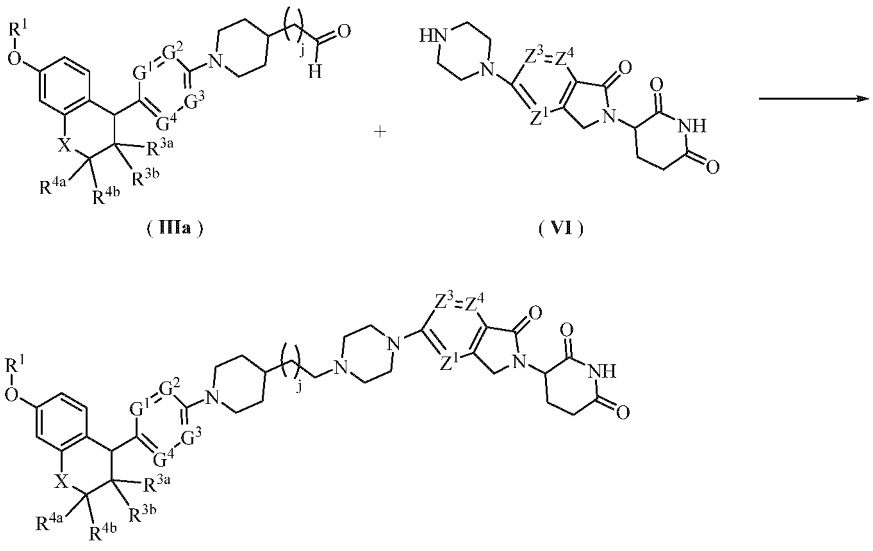

( IIIa ) ( VI )

( III )

conducting a reductive amination reaction of a compound of general formula (IIIa) with a compound of general formula (VI) or a salt thereof under an acidic condition (preferably acetic acid) in the presence of a reductant to give the compound of general formula (III) or the pharmaceutically acceptable salt thereof; or conducting a reductive amination reaction of a compound of general formula (IIIa) with a salt (preferably hydrochloride and benzenesulfonate) of a compound of general formula (VI) under an alkaline condition (preferably sodium acetate) in the presence of a reductant to give the compound of general formula (III) or the pharmaceutically acceptable salt thereof;
wherein:
X, $G^1$ to $G^4$, $Z^1$, $Z^3$, $Z^4$, $R^1$, $R^{3a}$, $R^{3b}$, $R^{4a}$, $R^{4b}$ and j are as defined in general formula (III).

Scheme 2

A method for preparing the compound of general formula (IV) or the pharmaceutically acceptable salt thereof of the present disclosure, which comprises the following step:

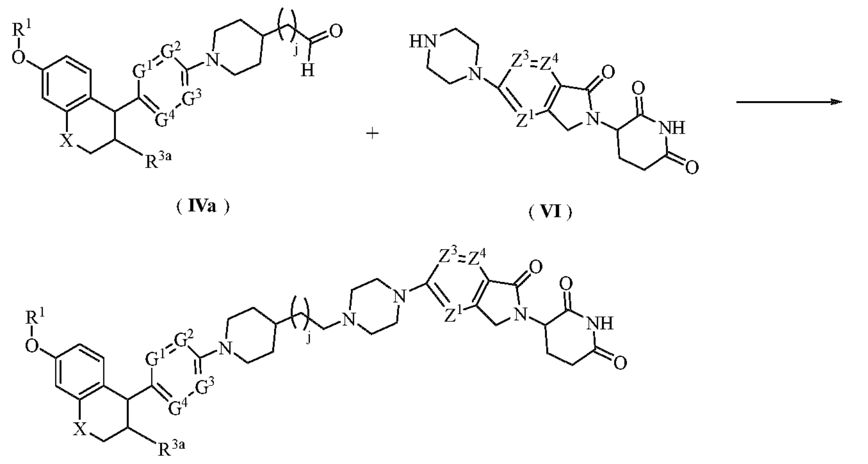

( IVa ) ( VI )

( IV )

conducting a reductive amination reaction of a compound of general formula (IVa) with a compound of general formula (VI) or a salt thereof under an acidic condition (preferably acetic acid) in the presence of a reductant to give the compound of general formula (IV) or the pharmaceutically acceptable salt thereof; or conducting a reductive amination reaction of a compound of general formula (IVa) with a salt (preferably hydrochloride and benzenesulfonate) of a compound of general formula (VI) under an alkaline condition (preferably sodium acetate) in the presence of a reductant to give the compound of general formula (IV) or the pharmaceutically acceptable salt thereof;
wherein:

X, $G^1$ to $G^4$, $Z^1$, $Z^3$, $Z^4$, $R^1$, $R^{3a}$ and j are as defined in general formula (IV).

Scheme 3

A method for preparing the compound of general formula (IV-1) or the pharmaceutically acceptable salt thereof of the present disclosure, which comprises the following step:

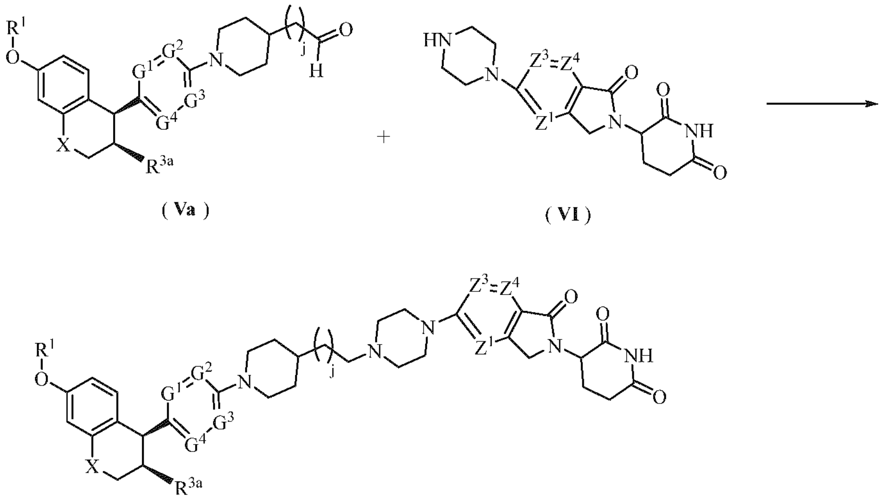

(Va) (VI)

(IV-1)

conducting a reductive amination reaction of a compound of general formula (Va) with a compound of general formula (VI) or a salt thereof under an acidic condition (preferably acetic acid) in the presence of a reductant to give the compound of general formula (IV-1) or the pharmaceutically acceptable salt thereof; or conducting a reductive amination reaction of a compound of general formula (Va) with a salt (preferably hydrochloride and benzenesulfonate) of a compound of general formula (VI) under an alkaline condition (preferably sodium acetate) in the presence of a reductant to give the compound of general formula (IV-1) or the pharmaceutically acceptable salt thereof;
wherein:
X, $G^1$ to $G^4$, $Z^1$, $Z^3$, $Z^4$, $R^1$, $R^{3a}$ and j are as defined in general formula (IV-1).

Scheme 4

A method for preparing the compound of general formula (V) or the pharmaceutically acceptable salt thereof of the present disclosure, which comprises the following step:

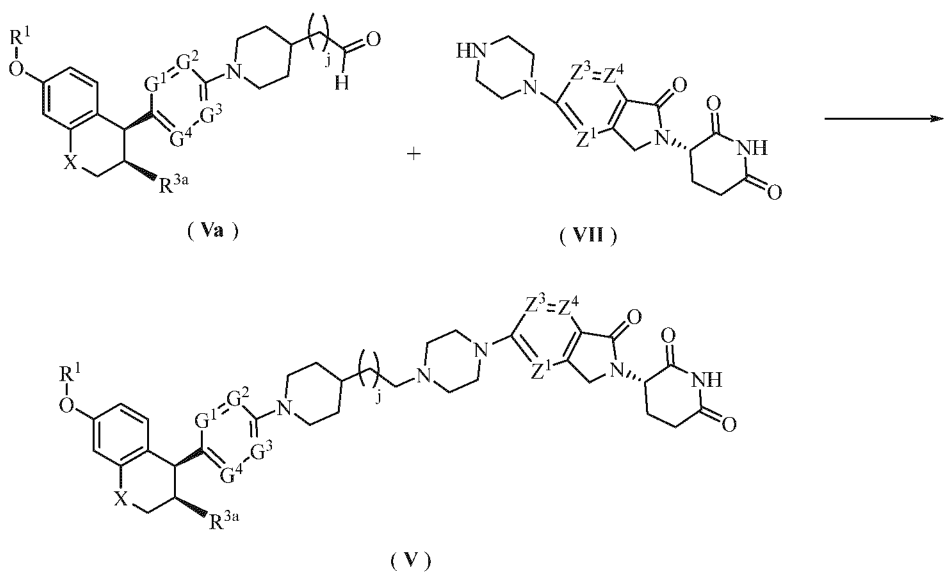

( Va ) ( VII ) ( V )

conducting a reductive amination reaction of a compound of general formula (Va) with a compound of general formula (VII) or a salt thereof under an acidic condition (preferably acetic acid) in the presence of a reductant to give the compound of general formula (V) or the pharmaceutically acceptable salt thereof; or conducting a reductive amination reaction of a compound of general formula (Va) with a salt (preferably hydrochloride and benzenesulfonate) of a compound of general formula (VII) under an alkaline condition (preferably sodium acetate) in the presence of a reductant to give the compound of general formula (V) or the pharmaceutically acceptable salt thereof;

wherein:

X, $G^1$ to $G^4$, $Z^1$, $Z^3$, $Z^4$, $R^1$, $R^{3a}$ and j are as defined in general formula (V).

Scheme 5

A method for preparing the compound of general formula (VB) or the pharmaceutically acceptable salt thereof of the present disclosure, which comprises the following step:

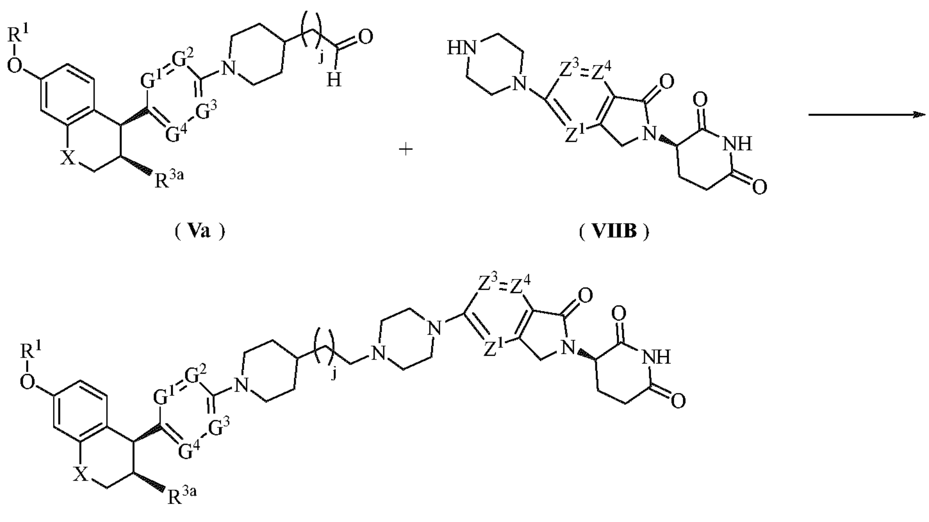

( Va ) ( VIIB ) ( VB )

conducting a reductive amination reaction of a compound of general formula (Va) with a compound of general formula (VIIB) or a salt thereof under an acidic condition (preferably acetic acid) in the presence of a reductant to give the compound of general formula (VB) or the pharmaceutically acceptable salt thereof; or conducting a reductive amination reaction of a compound of general formula (Va) with a salt (preferably hydrochloride and benzenesulfonate) of a compound of general formula (VIIB) under an alkaline condition (preferably sodium acetate) in the presence of a reductant to give the compound of general formula (VB) or the pharmaceutically acceptable salt thereof;

wherein:

X, $G^1$ to $G^4$, $Z^1$, $Z^3$, $Z^4$, $R^1$, $R^{3a}$ and j are as defined in general formula (VB).

Scheme 6

A method for preparing the compound of general formula (Va) or the salt thereof of the present disclosure, which comprises the following steps:

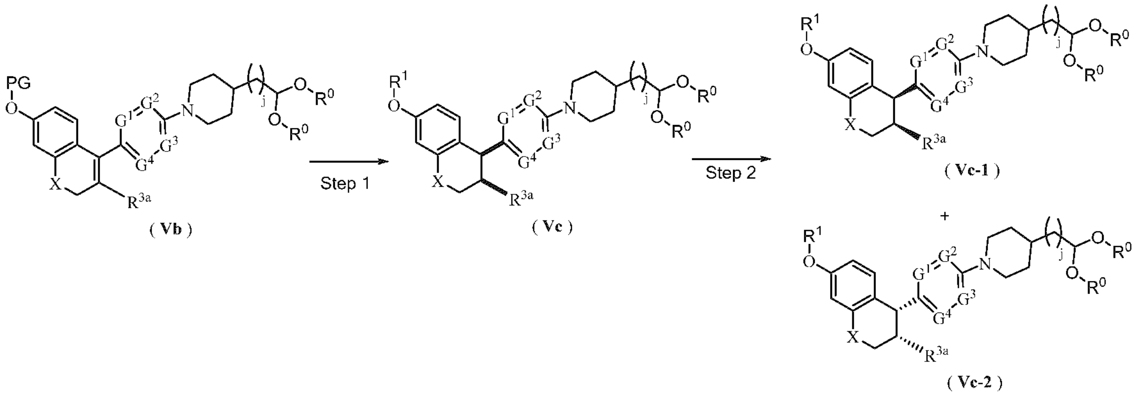


(Vb) (Vc) (Vc-1) (Vc-2)

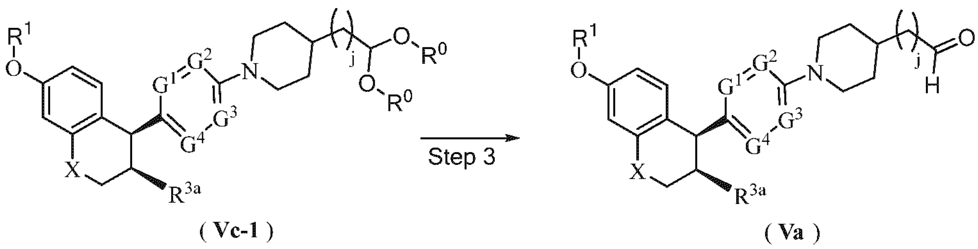


(Vc-1) (Va)

Step 1: conducting a catalyzed hydrogenation reaction of a compound of general formula (Vb) or a salt thereof under the action of palladium on carbon and removing the protecting group under conditions for protecting group removal to give a compound of general formula (Vc) or a salt thereof;

Step 2: performing chiral resolution of the compound of general formula (Vc) or the salt thereof to give compounds of general formula (Vc-1) and general formula (Vc-2) or salts thereof; and

Step 3: conducting a deprotection reaction of the compound of general formula (Vc-1) or the salt thereof under an acidic condition to give the compound of general formula (Va) or the salt thereof;

wherein:

PG is a hydroxy protecting group, preferably benzyl;

$R^0$ is $C_{1-6}$ alkyl, preferably methyl;

or $R^0$ is $C_{1-6}$ alkylene, and two $R^0$ are linked to form a 5- to 12-membered heterocyclic ring; preferably, $R^0$ is methylene, and two $R^0$ are linked to form a 5-membered heterocyclic ring;

$R^1$ is a hydrogen atom; and

X, $G^1$ to $G^4$, $R^{3a}$ and j are as defined in general formula (Va).

Reagents that provide the acidic conditions in the above synthesis schemes can be organic or inorganic acids. The organic acids include, but are not limited to, formic acid, acetic acid, trifluoroacetic acid, methanesulfonic acid, trifluoromethanesulfonic acid and p-toluenesulfonic acid. The inorganic acids include, but are not limited to, hydrogen chloride, solutions of hydrogen chloride in 1,4-dioxane, hydrochloric acid, sulfuric acid, nitric acid and phosphoric acid. Preferably, the reagents that provide the acidic conditions in schemes 1 to 5 are acetic acid. Preferably, the reagent that provides the acidic condition in scheme 6 is sulfuric acid, more preferably dilute sulfuric acid.

Reagents that provide the alkaline conditions in the above synthesis schemes include organic bases and inorganic bases. The organic bases include, but are not limited to, triethylamine, N,N-diisopropylethylamine, n-butyllithium, lithium diisopropylamide, potassium acetate, sodium acetate, sodium ethoxide, sodium tert-butoxide or potassium tert-butoxide; sodium acetate is preferred. The inorganic bases include, but are not limited to, sodium hydride, potassium

phosphate, sodium carbonate, potassium carbonate, cesium carbonate, sodium hydroxide, lithium hydroxide monohydrate, lithium hydroxide and potassium hydroxide.

The reductants in the above synthesis schemes include, but are not limited to, sodium triacetoxyborohydride, sodium borohydride, lithium borohydride, sodium cyanoborohydride, sodium acetylborohydride, and the like; sodium triacetoxyborohydride and sodium cyanoborohydride are preferred.

The above synthesis schemes are preferably carried out in solvents, including but not limited to: ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylacetamide, N,N-dimethylformamide, and mixtures thereof.

## BRIEF DESCRIPTION OF THE DRAWINGS

FIG. 1 shows data on the efficacy of the compound of Example 26-1 against MCF-7 (Y537S) xenograft tumors in BEIGE SCID mice.

FIG. 2 shows the effect of the compound of Example 26-1 on the body weight of BEIGE SCID mice.

## DETAILED DESCRIPTION

The following examples further illustrate the present disclosure, but the present disclosure is not limited thereto.

Examples

The structures of the compounds were determined by nuclear magnetic resonance (NMR) spectroscopy and/or mass spectrometry (MS). NMR shifts (δ) were given in $10^{-6}$ (ppm). NMR analyses were performed on a Bruker AVANCE-400 or Bruker AVANCE NEO 500M nuclear magnetic resonance instrument, with deuterated dimethyl sulfoxide (DMSO-$d_6$), deuterated chloroform ($CDCl_3$) and deuterated methanol ($CD_3OD$) as solvents and tetramethylsilane (TMS) as an internal standard.

Mass spectrometry (MS) analyses were performed on Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q Exactive).

High performance liquid chromatography (HPLC) analyses were performed on Agilent HPLC 1200DAD, Agilent HPLC 1200VWD, and Waters HPLC e2695-2489 high performance liquid chromatographs.

Chiral HPLC analyses were performed on an Agilent 1260 DAD high performance liquid chromatograph.

Preparative high-performance liquid chromatography used Waters 2545-2767, Waters 2767-SQ Detecor2, Shimadzu LC-20AP, and Gilson GX-281 preparative chromatographs.

Preparative chiral chromatography used a Shimadzu LC-20AP preparative chromatograph.

The CombiFlash preparative flash chromatograph used was CombiFlash Rf200 (TELEDYNE ISCO).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Silica gel column chromatography generally used 200- to 300-mesh silica gel (Huanghai, Yantai) as the carrier.

The mean inhibition of kinase and $IC_{50}$ values were determined on a NovoStar microplate reader (BMG, Germany).

Known starting materials in the present disclosure may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions could all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were performed using a Parr 3916EKX hydrogenator and a Qinglan QL-500 hydrogenator, or a HC2-SS hydrogenator.

A hydrogenation reaction generally involved 3 cycles of vacuumization and hydrogen purging.

Microwave reactions were performed on a CEM Discover-S 908860 microwave reactor. In the examples, a

solution refers to an aqueous solution unless otherwise specified.

In the examples, the reaction temperature was room temperature, i.e., 20 °C to 30 °C, unless otherwise specified.

The monitoring of the reaction progress in the examples was conducted by thin-layer chromatography (TLC). The developing solvent for reactions, the eluent system for column chromatography purification and the developing solvent system for thin-layer chromatography include: A: dichloromethane/methanol system, and B: n-hexane/ethyl acetate system. The volume ratio of the solvents was adjusted according to the polarity of the compound, or by adding a small amount of basic or acidic reagents such as triethylamine and acetic acid.

Example 1

3-(5-(4-((1-(4-(6'-Hydroxy-3',4'-dihydro-1'*H*-spiro[cyclopentane-1,2'-naphthalen]-1'-yl) phenyl)piperidin-4-yl)methyl)piperazin-1-yl)-1-oxoisoindolin-2-yl)piperidine-2,6-dione 1

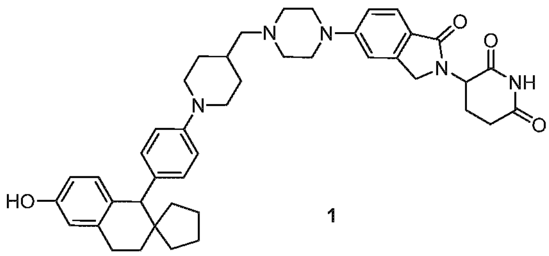

**1**

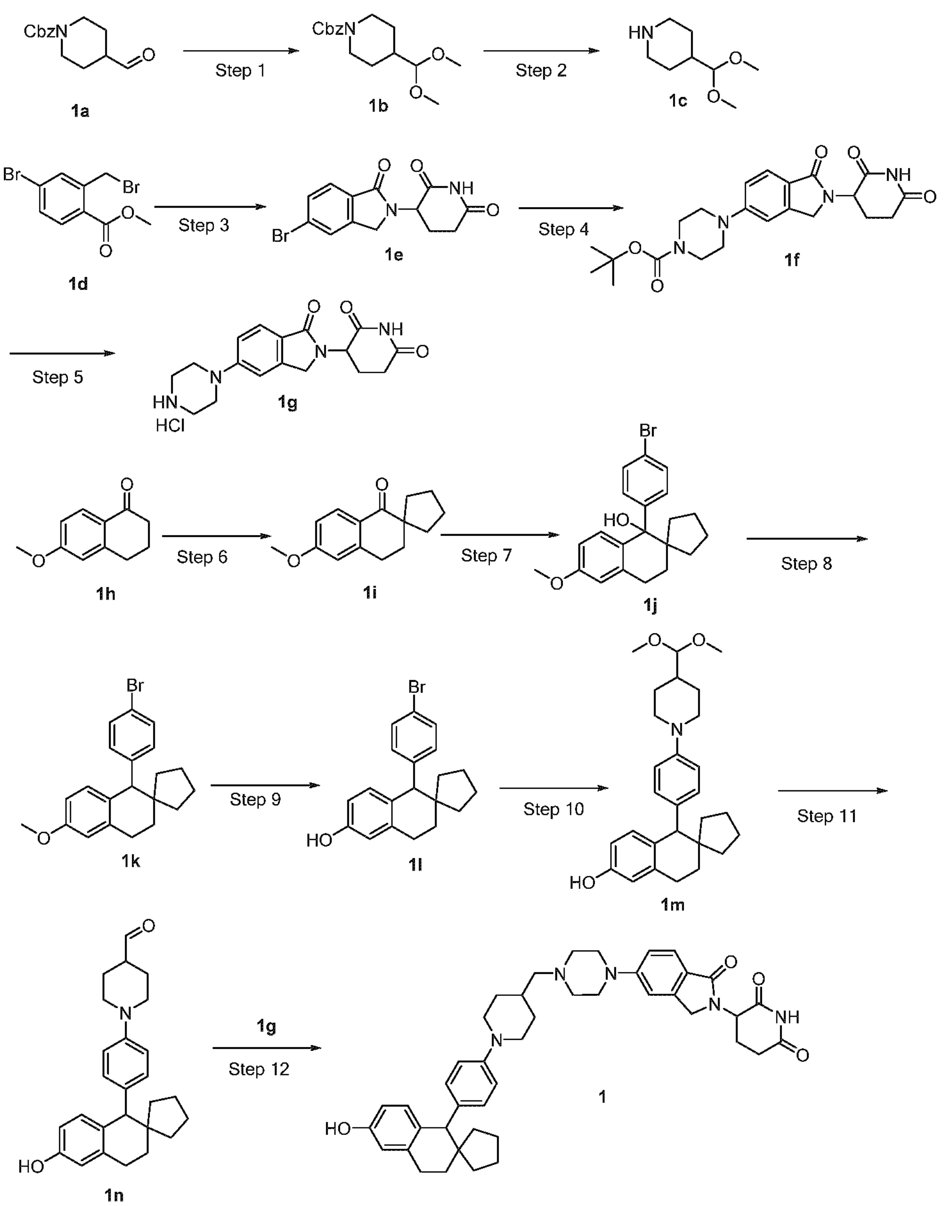


Step 1

Benzyl 4-(dimethoxymethyl)piperidine-1-carboxylate **1b**

Benzyl 4-formylpiperidine-1-carboxylate **1a** (10 g, 40.4 mmol, Shanghai Bide Pharmatech Ltd.) was dissolved in methanol (80 mL), and trimethyl orthoformate (40 mL) and p-toluenesulfonic acid monohydrate (385 mg, 2 mmol) were added. The reaction was stirred for 16 h. The reaction mixture was concentrated under reduced pressure, and a saturated solution of sodium bicarbonate (80 mL) was added. The mixture was extracted with ethyl acetate (80 mL × 3). The organic phases were combined, washed with a saturated solution of sodium chloride (80 mL × 3), dried over anhydrous sodium sulfate, filtered, and concentrated under reduced pressure to give the title compound **1b** (12 g, crude). The product was directly used in the next step without purification.

Step 2

4-(Dimethoxymethyl)piperidine **1c**

Compound **1b** (12 g, 40.9 mmol) was dissolved in methanol (100 mL), and palladium on carbon (1.3 g, 10 wt%) was added. The reaction was stirred in a hydrogen atmosphere for 3 h. The reaction mixture was filtered. The filtrate

was concentrated under reduced pressure to give the title compound **1c** (6 g, crude). The product was directly used in the next step without purification.

Step 3

3-(5-Bromo-1-oxoisoindolin-2-yl)piperidine-2,6-dione **1e**

3-Aminopiperidine-2,6-dione hydrochloride (5.88 g, 35.7 mmol, Shanghai Bide Pharmatech Ltd.) and potassium carbonate (13.5 g, 97.7 mmol) were dissolved in *N*,*N*-dimethylformamide (200 mL). The reaction was heated to 70 °C and stirred for 3 h. Then, methyl 4-bromo-2-(bromomethyl)benzoate **1d** (10 g, 32.5 mmol, Shanghai Bide Pharmatech Ltd.) was added, and the reaction was stirred at 70 °C for another 16 h. The reaction mixture was filtered through celite. The filtrate was concentrated under reduced pressure to remove the solvent. To the residue, 50 mL of a mixed solution of n-hexane and ethyl acetate (V/V = 1/1) was added. The resulting mixture was stirred and filtered. The filter cake was collected and dried *in vacuo* to give the title compound **1e** (5.7 g, 54% yield).
MS m/z (ESI): 324.5[M+1].

Step 4

tert-Butyl 4-(2-(2,6-dioxopiperidin-3-yl)-1-oxoisoindolin-5-yl)piperazine-1-carboxylate **1f**

Compound **1e** (1 g, 3.1 mmol) and tert-butyl piperazine-1-carboxylate (692 mg, 3.7 mmol, Shanghai Bide Pharmatech Ltd.) were dissolved in *N*,*N*-dimethylformamide (20 mL), and cesium carbonate (3.02 g, 9.3 mmol) and methanesulfonato(2-dicyclohexylphosphino-2',6'-diisopropoxy-1,1'-biphenyl)(2-amino-1,1'-biphenyl-2-yl)palladium(II) (RuPhos Pd G3, 260 mg, 0.3 mol) were added. The reaction was heated to 95 °C and stirred for 16 h. The reaction mixture was concentrated under reduced pressure to remove the solvent. The residue was purified by silica gel column chromatography using eluent system A to give the title compound **1f** (800 mg, 60% yield).
MS m/z (ESI): 429.2[M+1].

Step 5

3-(1-Oxo-5-(piperazin-1-yl)isoindolin-2-yl)piperidine-2,6-dione hydrochloride **1g** Compound **1f** (800 mg, 1.9 mmol) was dissolved in dichloromethane (20 mL), and the solution was cooled in an ice bath. A 4 M solution of hydrogen chloride in 1,4-dioxane (5 mL, 20 mmol) was added dropwise. The mixture was allowed to react at room temperature for 16 h. The reaction mixture was concentrated under reduced pressure to remove the solvent, giving the title compound **1g** (600 mg, 88% yield).
MS m/z (ESI): 329.1[M+1].

Step 6

6'-Methoxy-3',4'-dihydro-1'H-spiro[cyclopentane-1,2'-naphthalen]-1'-one **1i**

6-Methoxy-3,4-dihydronaphthalen-1(2*H*)-one **1h** (7 g, 39.7 mmol, Shanghai Bide Pharmatech Ltd.), 1,4-dibromobutane (10.3 g, 47.7 mmol) and potassium tert-butoxide (11.2 g, 99.8 mmol) were dissolved in toluene (100 mL). The reaction was heated to 100 °C and stirred for 16 h. The reaction mixture was concentrated under reduced pressure to remove the solvent. The residue was purified by silica gel column chromatography using eluent system B to give the title compound **1i** (6.5 g, 71% yield). MS m/z (ESI): 231.1[M+1].

Step 7

1'-(4-Bromophenyl)-6'-methoxy-3',4'-dihydro-1'*H*-spiro[cyclopentane-1,2'-naphthalen]-1'-ol **1j**

1,4-Dibromobenzene (5.7 g, 24.2 mmol) was dissolved in dry tetrahydrofuran (60 mL). The reaction solution was cooled to -78 °C in a nitrogen atmosphere. n-Butyllithium (2.5 M, 11.9 mL, 30 mmol) was slowly added dropwise. After the dropwise addition, the mixture was allowed to react at -78 °C for 30 min. A solution of compound **1i** (5 g, 21.7 mmol) in tetrahydrofuran was added dropwise. After the dropwise addition, the reaction was stirred at -78 °C for 1 h. The reaction mixture was quenched with a saturated solution of ammonium chloride and extracted with ethyl acetate (60 mL × 3). The organic phase was collected, dried over anhydrous sodium sulfate and filtered. The reaction mixture was concentrated under reduced pressure to remove the solvent. The residue was purified by silica gel column chromatography using eluent system B to give the title compound **1j** (7.1 g, 84% yield).
MS m/z (ESI): 387.1[M-1].

Step 8

1'-(4-Bromophenyl)-6'-methoxy-3',4'-dihydro-1'H-spiro[cyclopentane-1,2'-naphthalen] **1k**

Compound **1j** (3 g, 7.8 mmol) was dissolved in dichloromethane (15 mL), and the solution was cooled in an ice bath. Triethylsilane (1.81 g, 15.6 mmol) was added, and trifluoroacetic acid (972 mg, 8.5 mmol) was added dropwise. The reaction was cooled in the ice bath and stirred for 10 min. The reaction mixture was made neutral with a saturated solution of sodium bicarbonate and extracted with dichloromethane (30 mL × 3). The organic phases were combined, dried over anhydrous sodium sulfate and filtered. The filtrate was concentrated under reduced pressure to give the title compound **1k** (3 g, crude). The product was directly used in the next step.

Step 9

1'-(4-Bromophenyl)-3',4'-dihydro-1'*H*-spiro[cyclopentane-1,2'-naphthalen]-6'-ol **1l**

Compound **1k** (3 g, 8.1 mmol) was dissolved in dichloromethane (20 mL), and the solution was cooled in an ice bath. Boron tribromide (1 M, 8.4 mL, 8.4 mmol) was added dropwise. The mixture was allowed to react at room temperature for 3 h. The reaction mixture was quenched with a saturated solution of ammonium chloride and extracted with dichloromethane (30 mL × 3). The organic phase was collected, dried over anhydrous sodium sulfate and filtered. The filtrate was concentrated under reduced pressure. The residue was purified by silica gel column chromatography using eluent system B to give the title compound **1l** (2.1 g, 73% yield).
MS m/z (ESI): 357.1[M+1].

Step 10

1'-(4-(4-(Dimethoxymethyl)piperidin-1-yl)phenyl)-3',4'-dihydro-1*H*-spiro[cyclopentane -1,2'-naphthalen]-6'-ol **1m**

Compound **1l** (600 mg, 1.7 mmol) and compound **1c** (300 mg, 1.9 mmol) were dissolved in 1,4-dioxane (3 mL), and sodium tert-butoxide (323 mg, 3.4 mmol) and methanesulfonato(2-dicyclohexylphosphino)-3,6-dimethoxy-2',4',6'-triisopropoxy-1,1'-b iphenyl)(2'-amino-1,1'-biphenyl-2-yl)palladium(II) (BrettPhos Pd G3, 153 mg, 0.17 mmol) were added. The reaction was heated to 85 °C and stirred for 16 h in a nitrogen atmosphere. The reaction mixture was concentrated under reduced pressure to remove the solvent. The residue was purified by silica gel column chromatography using eluent system A to give the title compound **1m** (320 mg, 43% yield).
MS m/z (ESI): 436.3[M+1].

Step 11

1'-(4-(6'-Hydroxy-3',4'-dihydro-1'*H*-spiro[cyclopentane-1,2'-naphthalen]-1'-yl)phenyl)pi peridine-4-carbaldehyde **1n**

Compound **1m** (119 mg, 0.27 mol) was dissolved in tetrahydrofuran (10 mL), and dilute sulfuric acid (2 M, 1.5 mL, 1.8 mmol) was added. The mixture was allowed to react at room temperature for 16 h. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to give the title compound **1n** (101 mg, 95% yield).
MS m/z (ESI): 390.3[M+1].

Step 12

3-(5-(4-((1-(4-(6'-Hydroxy-3',4'-dihydro-1'H-spiro[cyclopentane-1,2'-naphthalen]-1'-yl) phenyl)piperidin-4-yl)methyl)piperazin-1-yl)-1-oxoisoindolin-2-yl)piperidine-2,6-dione **1**

Compound **1g** (142 mg, 0.39 mmol) was added to 10 mL of a mixed solution of dichloromethane and methanol (V/V = 4/1), and sodium acetate (171 mg, 2.1 mmol) was added. After 10 min of reaction, compound **1n** (101 mg, 0.26 mmol) was added. After 15 min of reaction, sodium triacetoxyborohydride (110 mg, 0.52 mmol) was added. The mixture was allowed to react at room temperature for 1 h. The reaction mixture was concentrated under reduced pressure to

remove the solvent. The residue was purified by preparative high performance liquid chromatography (Waters 2767-SQ Detecor2; elution system: 10 mM ammonium bicarbonate, 60% water, 40% acetonitrile) to give the title compound **1** (65 mg, 36% yield).

MS m/z (ESI): 702.4[M+1].

$^1$H NMR (500 MHz, DMSO-$d_6$) $\delta$ 9.00 (s, 1H), 7.52 (d, 1H), 7.20 (s, 1H), 7.07-7.05 (m, 1H), 6.80-6.76 (m, 3H), 6.66 (s, 1H), 6.58 (d, 1H), 6.52 (d, 1H), 6.44-6.41(m, 1H), 5.07-5.04 (m, 1H), 4.32, 4.22 (dd, 2H), 3.59 (d, 2H), 3.56 (s, 1H), 3.30-3.27 (m, 4H), 2.93-2.87 (m, 2H), 2.80-2.74 (m, 2H), 2.65-2.57 (m, 3H), 2.41-2.33 (m, 2H), 2.21 (d, 2H), 1.95-1.90 (m, 2H), 1.80-1.76 (m, 3H), 1.74-1.67 (m, 4H), 1.64-1.50 (m, 4H), 1.35-1.30 (m, 2H), 1.24-1.19 (m, 2H), 0.83-0.80 (m, 1H).

Examples 2-1 and 2-2

3-(5-(4-((1-(4-((*R*)-6'-Hydroxy-3',4'-dihydro-1'*H*-spiro[cyclopentane-1,2'-naphthalen]-1' -yl)phenyl)piperidin-4-yl)methyl)piperazin-1 -yl)-1 -oxoisoindolin-2-yl)piperidine-2,6-di one **2-1** (a 1:1 mixture of diastereomers) 3-(5-(4-((1-(4-((*S*)-6'-Hydroxy-3',4'-dihydro-1'*H*-spiro[cyclopentane-1,2'-naphthalen]-1' -yl)phenyl)piperidin-4-yl)methyl)piperazin-1 -yl)-1 -oxoisoindolin-2-yl)piperidine-2,6-di one **2-2** (a 1:1 mixture of diastereomers)

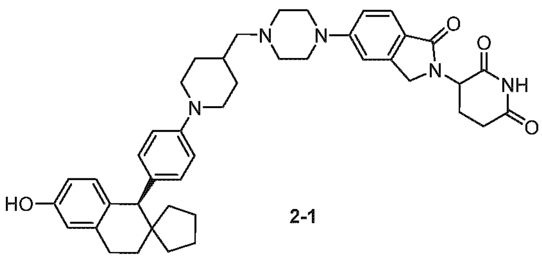

**2-1**

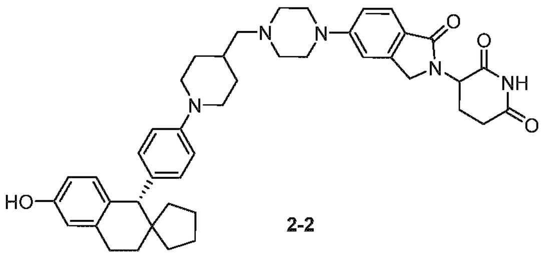

**2-2**

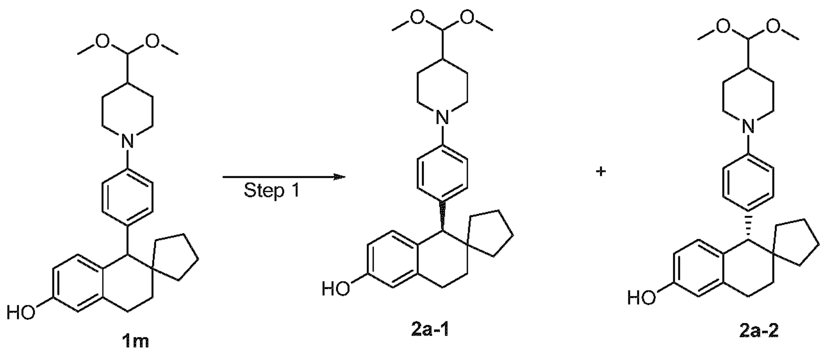


**1m** **2a-1** **2a-2**

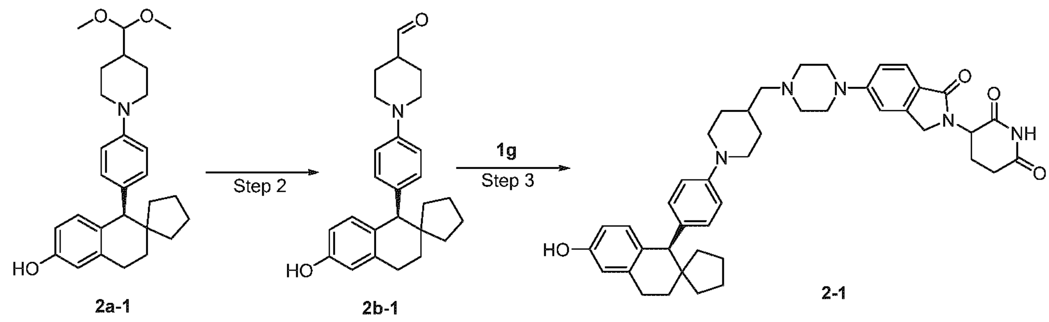


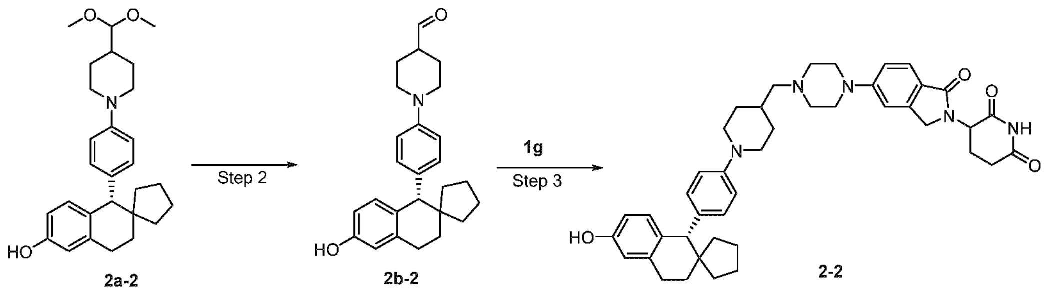


Step 1

(*R*)-1'-(4-(4-(Dimethoxymethyl)piperidin-1-yl)phenyl)-3',4'-dihydro-1'*H*-spiro[cyclopen tane-1,2'-naphthalen]-6'-ol **2a-1** (*S*)-1'-(4-(4-(Dimethoxymethyl)piperidin-1-yl)phenyl)-3',4'-dihydro-1*H*-spiro[cyclopent ane-1,2'-naphthalen]-6'-ol **2a-2**

Compound **1m** (320 mg, 0.73 mmol) was resolved by preparative chiral chromatography (resolution conditions: column: CHIRALPAK IE, 250 mm × 21.2 mm, 5 μm; mobile phases: A: n-hexane, B: ethanol (+ 10 nM $NH_3$), A: 70%, B: 30%) into the title compound **2a-1** (80 mg) and **2a-2** (80 mg).

**2a-2:**

MS m/z (ESI): 436.3[M+1].

Chiral HPLC analysis method: retention time 3.43 min, (column: CHIRALPAK IE A: n-hexane, B: EtOH (+ 0.1% DEA), A: 70%, B: 30%).

**2a-1:**

MS m/z (ESI): 436.3[M+1].

Chiral HPLC analysis method: retention time 8.11 min, (column: CHIRALPAK IE A: n-hexane, B: EtOH (+ 0.1% DEA), A: 70%, B: 30%).

Step 2

(*R*)-1'-(4-(6'-Hydroxy-3',4'-dihydro-1*H*-spiro[cyclopentane-1,2'-naphthalen]-1'-yl)phen yl)piperidine-4-carbaldehyde **2b-1** (*S*)-1'-(4-(6'-Hydroxy-3',4'-dihydro-1*H*-spiro[cyclopentane-1,2'-naphthalen]-1'-yl)pheny l)piperidine-4-carbaldehyde **2b-2**

Compound **2a-2** (RT = 3.43 min) (80 mg, 0.18 mol) was dissolved in tetrahydrofuran (5 mL), and dilute sulfuric acid (2 M, 0.9 mL, 1.8 mmol) was added. The mixture was allowed to react at room temperature for 16 h. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to give the title compound **2b-2** (58 mg, 81% yield).

MS m/z (ESI): 390.3[M+1].

Compound **2a-1** (RT = 8.11 min) (80 mg, 0.18 mol) was dissolved in tetrahydrofuran (5 mL), and dilute sulfuric acid (2 M, 0.9 mL, 1.8 mmol) was added. The mixture was allowed to react at room temperature for 16 h. The reaction

mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to give the title compound **2b-1** (60 mg, 84% yield).

MS m/z (ESI): 390.3[M+1].

Step 3

3-(5-(4-((1-(4-((*R*)-6'-Hydroxy-3',4'-dihydro-1'*H*-spiro[cyclopentane-1,2'-naphthalen]-1' -yl)phenyl)piperidin-4-yl)methyl)piperazin-1 -yl)-1 -oxoisoindolin-2-yl)piperidine-2,6-di one **2-1** (a 1:1 mixture of diastereomers) 3-(5-(4-((1-(4-((*S*)-6'-Hydroxy-3',4'-dihydro-1'*H*-spiro[cyclopentane-1,2'-naphthalen]-1' -yl)phenyl)piperidin-4-yl)methyl)piperazin-1 -yl)-1 -oxoisoindolin-2-yl)piperidine-2,6-di one **2-2** (a 1:1 mixture of diastereomers)

Compound **1g** (66 mg, 0.18 mmol) was added to 10 mL of a mixed solution of dichloromethane and methanol (V/V = 4/1), and sodium acetate (98 mg, 1.2 mmol) was added. After 10 min of reaction, compound **2b-2** (58 mg, 0.15 mmol) was added. After 15 min of reaction, sodium triacetoxyborohydride (66 mg, 0.3 mmol) was added. The mixture was allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **2-2** (35 mg, a 1:1 mixture of diastereomers, 64% yield).

$^1$H NMR (500 MHz, DMSO-$d_6$) $\delta$ 10.95 (s, 1H), 9.00 (s, 1H), 7.52 (d, 1H), 7.20 (s, 1H), 7.07-7.05 (m, 1H), 6.80-6.76 (m, 3H), 6.66 (s, 1H), 6.58 (d, 1H), 6.52 (d, 1H), 6.44-6.41(m, 1H), 5.07-5.04 (m, 1H), 4.32, 4.22 (dd, 2H), 3.59 (d, 2H), 3.56 (s, 1H), 3.30-3.27 (m, 4H), 2.93-2.87 (m, 2H), 2.80-2.74 (m, 2H), 2.65-2.57 (m, 3H), 2.41-2.33 (m, 2H), 2.21 (d, 2H), 1.95-1.90 (m, 2H), 1.80-1.76 (m, 3H), 1.74-1.67 (m, 4H), 1.64-1.50 (m, 4H), 1.35-1.30 (m, 2H), 1.24-1.19 (m, 2H), 0.83-0.80 (m, 1H).

MS m/z (ESI): 702.4[M+1].

Compound **1g** (38 mg, 0.1 mmol) was added to 5 mL of a mixed solution of dichloromethane and methanol (V/V = 4/1), and sodium acetate (50 mg, 0.6 mmol) was added. After 10 min of reaction, compound **2b-1** (30 mg, 0.08 mmol) was added. After 15 min of reaction, sodium triacetoxyborohydride (33 mg, 0.16 mmol) was added. The mixture was allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **2-1** (22 mg, a 1:1 mixture of diastereomers, 40% yield).

$^1$H NMR (500 MHz, DMSO-$d_6$) $\delta$ 10.95 (s, 1H), 9.00 (s, 1H), 7.52 (d, 1H), 7.20 (s, 1H), 7.07-7.05 (m, 1H), 6.80-6.76 (m, 3H), 6.66 (s, 1H), 6.58 (d, 1H), 6.52 (d, 1H), 6.44-6.41(m, 1H), 5.07-5.04 (m, 1H), 4.32, 4.22 (dd, 2H), 3.59 (d, 2H), 3.56 (s, 1H), 3.30-3.27 (m, 4H), 2.93-2.87 (m, 2H), 2.80-2.74 (m, 2H), 2.65-2.57 (m, 3H), 2.41-2.33 (m, 2H), 2.21 (d, 2H), 1.95-1.90 (m, 2H), 1.80-1.76 (m, 3H), 1.74-1.67 (m, 4H), 1.64-1.50 (m, 4H), 1.35-1.30 (m, 2H), 1.24-1.19 (m, 2H), 0.83-0.80 (m, 1H).

MS m/z (ESI): 702.4[M+1].

Example 3

3-(5-(4-((1-(2-Fluoro-4-((1,2)-*cis*-6-hydroxy-2-(tetrahydro-2*H*-pyran-4-yl)-1,2,3,4-tetra hydronaphthalen-1-yl)phenyl)piperidin-4-yl)methyl)piperazin-1-yl)-1-oxoisoindolin-2-y l)piperidine-2,6-dione **3**

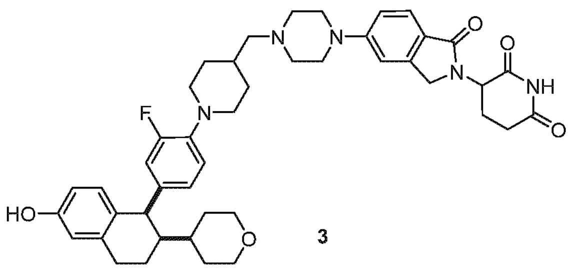

3

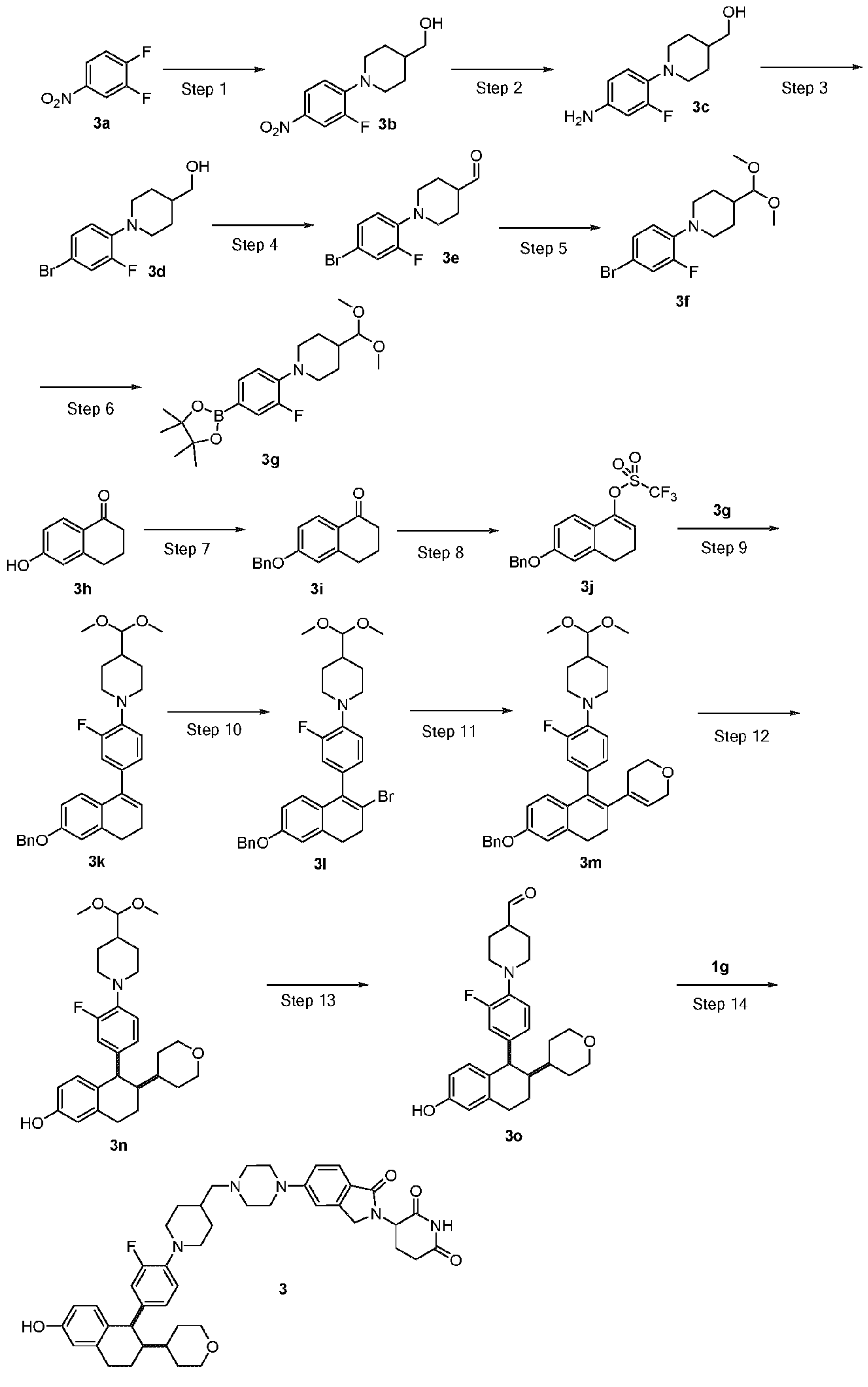


Step 1

(1-(2-Fluoro-4-nitrophenyl)piperidin-4-yl)methanol **3b**

1,2-Difluoro-4-nitrobenzene **3a** (5 g, 31.4 mmol, Shanghai Bide Pharmatech Ltd.) and 4-piperidinemethanol (3.81 g, 33.1 mmol, Shanghai Bide Pharmatech Ltd.) were added to *N*,*N*-dimethylformamide (20 mL), and the mixture was cooled in an ice bath. Potassium carbonate (6.51 g, 47.2 mmol) was added. The mixture was allowed to react at room temperature for 16 h. The reaction mixture was filtered through celite. The filtrate was concentrated under reduced pressure to remove the solvent. The resulting residue was purified by silica gel column chromatography using eluent system B to give the title compound **3b** (7.9 g, 99% yield).

MS m/z (ESI): 255.1[M+1].

Step 2

(1-(4-Amino-2-fluorophenyl)piperidin-4-yl)methanol **3c**

Compound **3b** (7.8 g, 30.7 mmol) was dissolved in methanol (100 mL), and palladium on carbon (1.5 g, 10 wt%) was added. The mixture was purged with hydrogen three times and was allowed to react in a hydrogen atmosphere for 16 h. The reaction mixture was filtered through celite. The filtrate was concentrated under reduced pressure to remove the solvent, giving the title compound **3c** (6.8 g, crude). The product was directly used in the next step without purification.

MS m/z (ESI): 225.1[M+1],

Step 3

(1-(4-Bromo-2-fluorophenyl)piperidin-4-yl)methanol **3d**

Cupric bromide (6.4 g, 28.7 mmol) was added to acetonitrile (30 mL), and tert-butyl nitrite (3.7 g, 35.9 mmol, Shanghai Titan Scientific Co., Ltd.) was added dropwise. The mixture was allowed to react for 10 min. A solution of compound 3c (5.3 g, 23.6 mmol) in acetonitrile was added dropwise. After the dropwise addition, the mixture was allowed to react at room temperature for 1 h. The reaction mixture was filtered through celite. The filtrate was concentrated under reduced pressure to remove the solvent. The resulting residue was purified by silica gel column chromatography using eluent system B to give the title compound **3d** (4.1 g, 60% yield).

MS m/z (ESI): 288.1[M+1].

Step 4

1 -(4-Bromo-2-fluorophenyl)piperidine-4-carbaldehyde **3e**

Compound **3d** (3.7 g, 12.8 mmol) was dissolved in dichloromethane (50 mL), and Dess-Martin periodinane (6 g, 14.2 mmol) was added. The mixture was allowed to react at room temperature for 1 h. The reaction mixture was filtered through celite. The filtrate was washed with saturated sodium bicarbonate (10 mL $\times$ 3). The organic phase was collected, dried over anhydrous sodium sulfate and filtered. The filtrate was concentrated under reduced pressure to remove the solvent, giving the title compound **3e** (2.1 g, crude). The product was directly used in the next step without purification.

MS m/z (ESI): 286.1[M+1].

Step 5

1-(4-Bromo-2-fluorophenyl)-4-(dimethoxymethyl)piperidine **3f**

Compound **3e** (2.1 g, 7.3 mmol) was dissolved in 20 mL of a mixed solvent of methanol and trimethyl orthoformate (V/V = 1/1). p-Toluenesulfonic acid monohydrate (70 mg, 0.37 mmol) was added. The mixture was allowed to react at room temperature for 16 h. The reaction mixture was concentrated under reduced pressure to remove the solvent. The resulting residue was purified by silica gel column chromatography using eluent system B to give the title compound **3f** (1.7 g, 70% yield).

MS m/z (ESI): 332.1[M+1].

Step 6

4-(Dimethoxymethyl)-1-(2-fluoro-4-(4,4,5,5-tetramethyl-1,3,2-dioxaborolan-2-yl)pheny l)piperidine **3g**

Compound **3f** (1.7 g, 5.1 mmol) was dissolved in 1,4-dioxane (15 mL), and bis(pinacolato)diboron (2 g, 7.9 mmol), [1,1'-bis(diphenylphosphino)ferrocene]palladium(II) dichloride (205 mg, 0.28 mmol) and potassium acetate (1.1 g, 11.2 mmol) were added. The reaction was heated to 80 °C and stirred for 16 h in a nitrogen atmosphere. The reaction mixture was filtered through celite. The filtrate was concentrated under reduced pressure to remove the solvent. The resulting residue was purified by silica gel column chromatography using eluent system B to give the title compound **3g** (1.4 g, 72% yield).

MS m/z (ESI): 380.1[M+1].

Step 7

6-(Benzyloxy)-3,4-dihydronaphthalen-1(2H)-one **3i** 6-Hydroxy-3,4-dihydronaphthalen-1(2*H*)-one **3h** (8 g, 49.3 mmol, Shanghai Bide Pharmatech Ltd.) and potassium carbonate (10 g, 72.4 mmol) were added to acetonitrile (60 mL), and benzyl bromide (10 g, 58.5 mmol, 7 mL) was added dropwise. The reaction was heated at reflux for 3 h. The reaction mixture was filtered through celite. The filtrate was concentrated under reduced pressure to remove the solvent. The residue was dissolved in ethyl acetate (100 mL), and the solution was washed with a saturated solution of sodium chloride (20 mL × 3). The organic phase was collected, dried over anhydrous sodium sulfate and filtered. The filtrate was concentrated under reduced pressure to remove the solvent, giving the title compound **3i** (12 g, crude). The product was directly used in the next step without purification.
MS m/z (ESI): 253.1[M+1].

Step 8

6-(Benzyloxy)-3,4-dihydronaphthalen-1-yl trifluoromethanesulfonate **3j** Compound **3i** (8 g, 31.7 mmol) was dissolved in dry tetrahydrofuran (100 mL). The reaction was cooled to -78 °C in an argon atmosphere. [Bis(trimethylsilyl)amino]lithium (1 M, 50.8 mL, 50.8 mmol) was added dropwise. After the dropwise addition, the reaction was stirred at -78 °C for 30 min. 1,1, 1- Trifluoro-N-phenyl-N-(trifluoromethylsulfonyl)methanesulfonamide (17 g, 47.6 mmol) was slowly added. The reaction was naturally warmed to room temperature and stirred for 2 h. The reaction mixture was quenched with water (100 mL) and extracted with dichloromethane (50 mL ×3). The organic phases were combined, dried over anhydrous sodium sulfate and filtered. The filtrate was concentrated under reduced pressure to remove the solvent. The resulting residue was purified by silica gel column chromatography using eluent system B to give the title compound **3j** (10.1 g, 83% yield).
MS m/z (ESI): 385.2[M+1].

Step 9

1-(4-(6-(Benzyloxy)-3,4-dihydronaphthalen-1-yl)-2-fluorophenyl)-4-(dimethoxymethyl) piperidine **3k**

Compound **3j** (800 mg, 2.1 mmol), compound **3g** (790 mg, 2.1 mmol), [1,1'-bis(diphenylphosphino)ferrocene]palladium(II) dichloride (77 mg, 0.1 mmol) and potassium carbonate (432 mg, 3.1 mmol) were dissolved in 35 mL of a mixed solvent of 1,4-dioxane and water (V/V = 6/1). The reaction was heated to 100 °C and stirred for 2 h in a nitrogen atmosphere. The reaction mixture was filtered through celite. The filtrate was concentrated under reduced pressure to remove the solvent. The resulting residue was purified by silica gel column chromatography using eluent system B to give the title compound **3k** (570 mg, 56% yield).
MS m/z (ESI): 488.3[M+1].

Step 10

1-(4-(6-(Benzyloxy)-2-bromo-3,4-dihydronaphthalen-1-yl)-2-fluorophenyl)-4-(dimetho xymethyl)piperidine **31**

Compound **3k** (560 mg, 1.2 mmol) and pyridinium tribromide (441 mg, 1.4 mmol) were dissolved in dichloromethane (30 mL), and triethylamine (235 mg, 2.3 mmol) was added. The mixture was allowed to react at room temperature for 2 h. A saturated solution of sodium bicarbonate (50 mL) was added, and the mixture was extracted with dichloromethane (10 mL × 3). The organic phases were combined, dried over anhydrous sodium sulfate and filtered. The filtrate was concentrated under reduced pressure to remove the solvent. The resulting residue was purified by silica gel column chromatography using eluent system B to give the title compound **3l** (520 mg, 80% yield).
MS m/z (ESI): 568.1[M+1].

Step 11

1-(4-(6-(Benzyloxy)-2-(3,6-dihydro-2*H*-pyran-4-yl)-3,4-dihydronaphthalen-1-yl)-2-fluo rophenyl)-4-(dimethoxymethyl)piperidine **3m**

Compound **3l** (150 mg, 0.26 mmol), 3,6-dihydro-2*H*-pyran-4-boronic acid pinacol ester (91 mg, 0.43 mmol, Shanghai Bide Pharmatech Ltd.), tetrakis(triphenylphosphine)palladium(0) (35 mg, 0.03 mol) and sodium carbonate (61 mg, 0.58 mmol) were added to 21 mL of a mixed solvent of 1,4-dioxane and water (V/V = 6/1). The reaction was heated to 80 °C and stirred for 2 h in a nitrogen atmosphere. The reaction mixture was concentrated under reduced pressure

to remove the solvent. The resulting residue was purified by silica gel column chromatography using eluent system B to give the title compound **3m** (113 mg, 75% yield).
MS m/z (ESI): 570.3[M+1].

Step 12

(5,6)-*cis*-5-(4-(4-(Dimethoxymethyl)piperidin-1-yl)-3-fluorophenyl)-6-(tetrahydro-2*H*-p yran-4-yl)-7,8-dihydronaphthalen-2-ol **3n**

Compound **3m** (113 mg, 0.2 mmol) was dissolved in methanol (15 mL), and palladium on carbon (20 mg, 10 wt%) was added. The mixture was purged with hydrogen three times and was allowed to react in a hydrogen atmosphere for 16 h. The reaction mixture was filtered through celite. The filtrate was concentrated under reduced pressure to remove the solvent, giving the title compound **3n** (80 mg, crude). The product was directly used in the next step without purification.
MS m/z (ESI): 484.2[M+1].

Step 13

1-(2-Fluoro-4-((1,2)-*cis*-6-hydroxy-2-(tetrahydro-2*H*-pyran-4-yl)-1,2,3,4-dihydronaphth alen-1-yl)phenyl)piperidine-4-carbaldehyde **30**

Compound **3n** (80 mg, 0.16 mmol) was dissolved in tetrahydrofuran (5 mL), and dilute sulfuric acid (2 M, 1 mL, 2 mmol) was added. The reaction was heated to 70 °C and stirred for 2 h. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **3o** (81 mg). The product was directly used in the next step without purification.
MS m/z (ESI): 438.1[M+1].

Step 14

3-(5-(4-((4-(2-Fluoro-4-((1,2)-*cis*-6-hydroxy-2-(tetrahydro-2*H*-pyran-4-yl)-1,2,3,4-tetra hydronaphthalen-1-yl)phenyl)piperidin-4-yl)methyl)piperazin-1-yl)-1-oxoisoindolin-2-y l)piperidine-2,6-dione **3**

Compound **1g** (102 mg, 0.28 mmol) was dissolved in 9 mL of a mixed solution of dichloromethane and methanol (V/V = 2/1), and sodium acetate (122 mg, 1.5 mmol) was added. After 10 min of reaction, compound **3o** (81 mg, 0.18 mmol) was added. After 15 min of reaction, sodium triacetoxyborohydride (79 mg, 0.37 mmol) was added. The mixture was allowed to react for 1 h. Dichloromethane (20 mL) was added, and the mixture was washed with a saturated solution of sodium chloride (10 mL × 3). The organic phase was collected, dried over anhydrous sodium sulfate, filtered, and concentrated under reduced pressure to remove the solvent. The resulting residue was purified by preparative high performance liquid chromatography (Waters 2767-SQ Detecor2; elution system: 10 mM ammonium bicarbonate, 60% water, 40% acetonitrile) to give the title compound **3** (50 mg, 36% yield).
MS m/z (ESI): 750.3[M+1].
$^1$H NMR (500 MHz, DMSO-$d_6$) $\delta$ 9.08 (s, 1H), 7.53 (d, 1H), 7.08-7.06 (m, 2H), 6.91-6.81(m, 1H), 6.76-6.74 (m, 1H), 6.70-6.63 (m, 2H), 6.58 (s, 1H), 6.46-6.43 (m, 1H), 5.07-5.01 (m, 1H), 4.32, 4.22 (dd, 2H), 4.13 (d, 1H), 3.85 (d, 1H), 3.74 (d, 1H), 3.29-3.20 (m, 5H), 3.17 (t, 1H), 3.07 (t, 1H), 2.93-2.88 (m, 2H), 2.75-2.71 (m, 3H), 2.65-2.58 (m, 4H), 2.43-2.35 (m, 2H), 2.23 (d, 2H), 2.03-1.95 (m, 3H), 1.82-1.79 (m, 3H), 1.68-1.58 (m, 2H), 1.49-1.42 (m, 2H), 1.27-1.10 (m, 5H).

Example 4-1 and Example 4-2

3-(5-(4-((1-(2-Fluoro-4-((1*R*,2*R*)-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione **4-1** (a 1:1 mixture of diastereomers)
3-(5-(4-((1-(2-Fluoro-4-((1*S*,2*S*)-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione **4-2** (a 1:1 mixture of diastereomers)

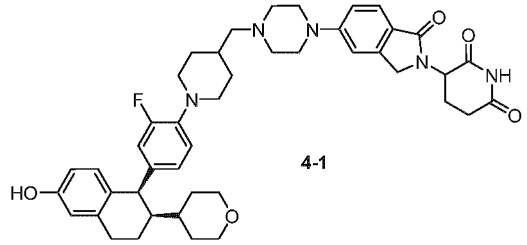

4-1

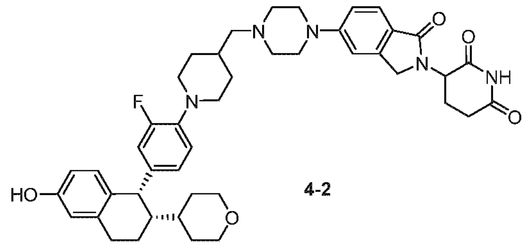

4-2

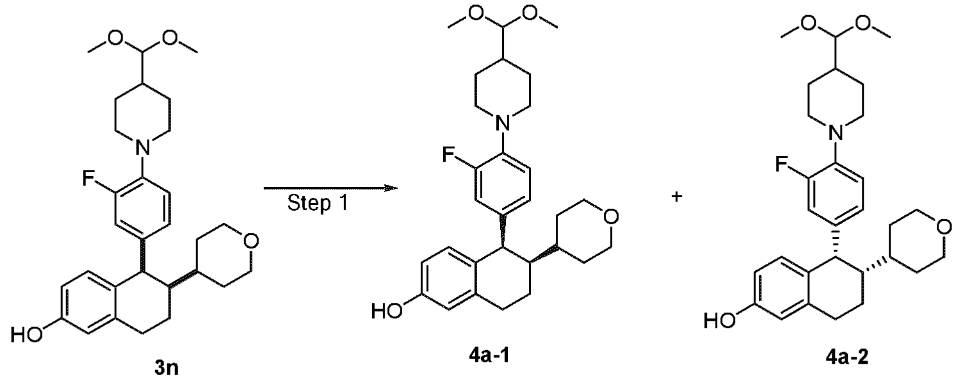


3n

4a-1

4a-2

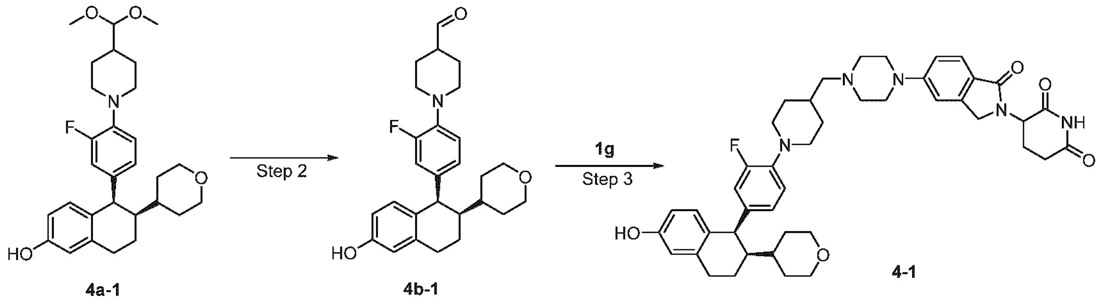


4a-1

4b-1

4-1

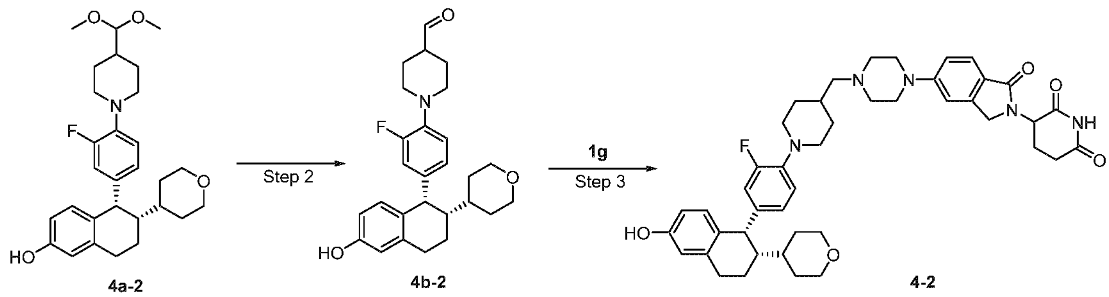


4a-2

4b-2

4-2

Step 1

(5*R*,6*R*)-5-(4-(4-(Dimethoxymethyl)piperidin-1-yl)-3-fluorophenyl)-6-(tetrahydro-2*H*-p yran-4-yl)-5,6,7,8-tetrahydronaphthalen-2-ol **4a-1** (5*S*,6*S*)-5-(4-(4-(Dimethoxymethyl)piperidin-1-yl)-3-fluorophenyl)-6-(tetrahydro-2*H*-py ran-4-yl)-5,6,7,8-tetrahydronaphthalen-2-ol **4a-2**

Compound **3n** (200 mg, 0.41 mmol) was resolved by preparative chiral chromatography (resolution conditions: column: CHIRALPAK IE, 250 mm × 21.2 mm, 5 μm; mobile phases: A: n-hexane, B: ethanol (+ 20 mmol $NH_3$), A: 70%, B: 30%) into the title compound **4a-1** (81 mg) and **4a-2** (80 mg).

**4a-1**

MS m/z (ESI): 484.3[M+1].
Chiral HPLC analysis method: retention time 9.29 min, chiral purity: 100% (column: CHIRALPAK IE A: n-hexane, B: EtOH (+ 0.1% DEA), A: 70%, B: 30%).

**4a-2**

MS m/z (ESI): 484.3[M+1].
Chiral HPLC analysis method: retention time 7.97 min, chiral purity: 100% (column: CHIRALPAK IE A: n-hexane, B: EtOH (+ 0.1% DEA), A: 70%, B: 30%).

Step 2 1-(2-Fluoro-4-((1*R*,2*R*)-6-hydroxy-2-(tetrahydro-2*H*-pyran-4-yl)-1,2,3,4-tetrahydronaph thalen-1-yl)phenyl)piperidine-4-carbaldehyde **4b-1** 1-(2-Fluoro-4-((1*S*,2*S*)-6-hydroxy-2-(tetrahydro-2*H*-pyran-4-yl)-1,2,3,4-tetrahydronapht halen-1-yl)phenyl)piperidine-4-carbaldehyde **4b-2**

Compound **4a-1** (81 mg, 0.17 mol) was dissolved in tetrahydrofuran (5 mL), and dilute sulfuric acid (2 M, 0.9 mL, 1.8 mmol) was added. The mixture was allowed to react at room temperature for 16 h. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to give the title compound **4b-1** (61 mg, 83% yield).
MS m/z (ESI): 438.3[M+1].
Compound **4a-2** (80 mg, 0.16 mol) was dissolved in tetrahydrofuran (5 mL), and dilute sulfuric acid (2 M, 0.9 mL, 1.8 mmol) was added. The mixture was allowed to react at room temperature for 16 h. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to give the title compound **4b-2** (63 mg, 87% yield).
MS m/z (ESI): 438.3[M+1].

Step 3

3-(5-(4-((1-(2-Fluoro-4-((1*R*,2*R*)-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione **4-1** (a 1:1 mixture of diastereomers)
3-(5-(4-((1-(2-Fluoro-4-((1*S*,2*S*)-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione **4-2** (a 1:1 mixture of diastereomers)

Compound **1g** (55 mg, 0.17 mmol) was added to a mixed solvent of dichloromethane and methanol (V/V = 4/1, 5 mL), and sodium acetate (92 mg, 1.1 mmol) was added. After 10 min of reaction, compound **4b-1** (61 mg, 0.14 mmol) was added. After 15 min of reaction, sodium triacetoxyborohydride (60 mg, 0.28 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **4-1** (40 mg, a 1:1 mixture of diastereomers, 38% yield).
MS m/z (ESI): 750.4[M+1].
$^1$H NMR (500 MHz, DMSO-$d_6$) δ 10.95 (s, 1H), 9.08 (s, 1H), 7.53 (d, 1H), 7.07-7.05 (m, 2H), 6.89 (t, 1H), 6.76-6.74 (m, 1H), 6.69-6.64 (m, 2H), 6.53 (s, 1H), 6.45-6.43 (m, 1H), 5.07-5.03 (m, 1H), 4.32, 4.22 (dd, 2H), 4.12 (brs, 1H), 3.87-3.85 (m, 1H), 3.76-3.74 (m, 1H), 3.32-3.25 (m, 8H), 3.20 (t, 1H), 3.09 (t, 1H), 2.94-2.87 (m, 2H), 2.77-2.70 (m, 2H), 2.64-2.57 (m, 3H), 2.53-2.49 (m, 2H), 2.38-2.36 (m, 1H), 2.24-2.22 (m, 2H), 2.03-1.95 (m, 2H), 1.82-1.74 (m, 3H),

1.68-1.61 (m, 2H), 1.49-1.45 (m, 1H), 1.27-1.07 (m, 5H).

Compound **1g** (55 mg, 0.15 mmol) was added to a mixed solvent of dichloromethane and methanol (V/V = 4/1, 5 mL), and sodium acetate (95 mg, 1.2 mmol) was added. After 10 min of reaction, compound **4b-2** (63 mg, 0.14 mmol) was added. After 15 min of reaction, sodium triacetoxyborohydride (63 mg, 0.21 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **4-2** (28 mg, a 1:1 mixture of diastereomers, 25% yield).

MS m/z (ESI): 750.4[M+1].

$^1$H NMR (500 MHz, DMSO-$d_6$) $\delta$ 10.95 (s, 1H), 9.08 (s, 1H), 7.53 (d, 1H), 7.07-7.05 (m, 2H), 6.89 (t, 1H), 6.76-6.74 (m, 1H), 6.69-6.64 (m, 2H), 6.53 (s, 1H), 6.45-6.43 (m, 1H), 5.07-5.03 (m, 1H), 4.32, 4.22 (dd, 2H), 4.12 (brs, 1H), 3.87-3.85 (m, 1H), 3.76-3.74 (m, 1H), 3.32-3.25 (m, 8H), 3.20 (t, 1H), 3.09 (t, 1H), 2.94-2.87 (m, 2H), 2.77-2.70 (m, 2H), 2.64-2.57 (m, 3H), 2.53-2.49 (m, 2H), 2.38-2.36 (m, 1H), 2.24-2.22 (m, 2H), 2.03-1.95 (m, 2H), 1.82-1.74 (m, 3H), 1.68-1.61 (m, 2H), 1.49-1.45 (m, 1H), 1.27-1.07 (m, 5H).

Example 5

(*S*)-3-(5-(4-((1-(2-Fluoro-4-((1*R*,2*R*)-6-hydroxy-2-(tetrahydro-2*H*-pyran-4-yl)-1,2,3,4-te trahydronaphthalen-1 -yl)phenyl)piperidin-4-yl)methyl)piperazin-1 -yl)-1 -oxoisoindolin-2-yl)piperidine-2,6-dione 5

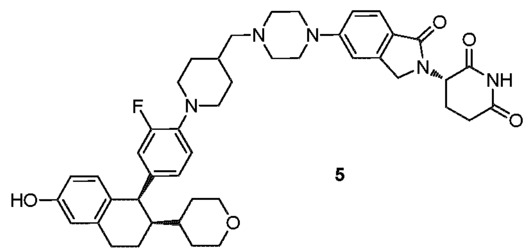

5

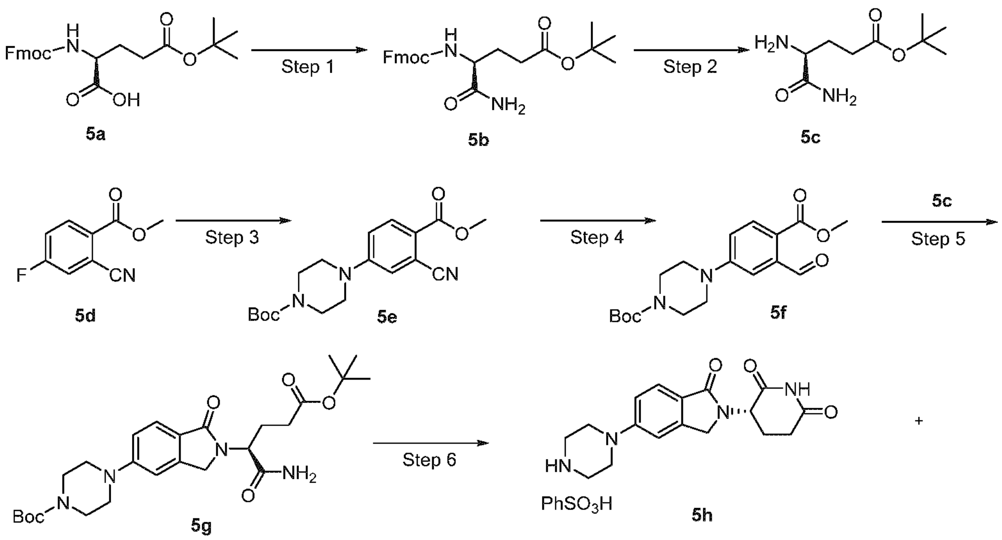


5a 5b 5c 5d 5e 5f 5g 5h

+

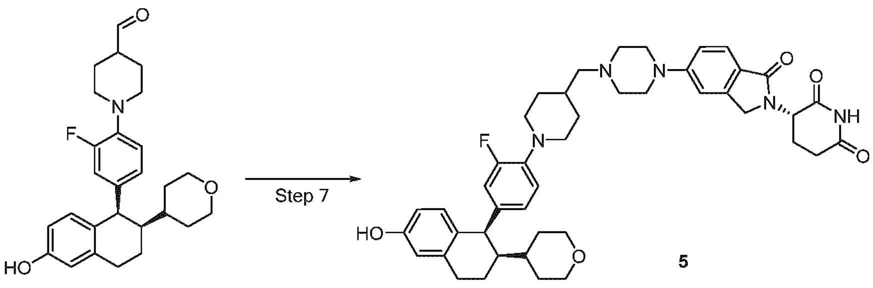


4b-1

5

Step 1

tert-Butyl

(*S*)-4-((((9*H*-fluoren-9-yl)methoxy)carbonyl)amino)-5-amino-5-oxopentanoate **5b** (*S*)-2-((((9*H*-Fluoren-9-yl)methoxy)carbonyl)amino)-5-(tert-butoxy)-5-oxopentanoic acid **5a** (40 g, 94 mmol, Shanghai Hanhong Scientific Co., Ltd.) and di-tert-butyl dicarbonate (32.83 g, 150 mmol) were added to 1,4-dioxane (300 mL). The internal temperature was controlled at below 5 °C using an ice-water bath in a nitrogen atmosphere. Pyridine (15 mL, 188 mmol) was added dropwise. After the dropwise addition, the mixture was allowed to react in an ice-water bath for 0.5 h. Ammonium bicarbonate (66.89 g, 282 mmol) was added. The mixture was warmed to room temperature and was allowed to react for 12 h. The reaction mixture was concentrated under reduced pressure to remove the solvent, and ethyl acetate (500 mL) was added. The mixture was washed with dilute hydrochloric acid (500 mL × 3) and filtered. The filtrate was concentrated under reduced pressure to remove the solvent to give the title compound **5b** (45.3 g, crude). The product was directly used in the next step without purification.

MS m/z (ESI): 369.1[M-55].

Step 2

tert-Butyl (*S*)-4,5-diamino-5-oxopentanoate **5c**

Compound **5b** (45.3 g, 94 mmol) and diethylamine (50 mL) were added to dichloromethane (500 mL). The mixture was allowed to react at room temperature for 12 h. The reaction mixture was concentrated under reduced pressure to remove the solvent. The residue was dissolved in methanol (150 mL), and water (5 mL) was added. The mixture was washed with n-heptane (150 mL × 3). The methanol layer was concentrated under reduced pressure to remove the solvent to give the title compound **5c** (21.5 g, crude). The product was directly used in the next step without purification.

MS m/z (ESI): 203.1[M+1].

Step 3

tert-Butyl 4-(3-cyano-4-(methoxycarbonyl)phenyl)piperazine-1-carboxylate **5e**

Methyl 2-cyano-4-fluorobenzoate **5d** (50 g, 0.28 mol, Jiangsu Aikon Biopharmaceutical R&D Co., Ltd.), tert-butyl piperazine-1-carboxylate acetate (62.3 g, 0.34 mol) and isopropylethylamine (250 mL, 1.39 mol) were added to tetrahydrofuran (1 L). The mixture was allowed to react at 120 °C for 12 h. The reaction mixture was added to water (1 L) and extracted with ethyl acetate (1 L × 3). The organic phases were combined and washed with a saturated solution of sodium chloride (1 L × 2). The organic phase was dried over anhydrous sodium sulfate and filtered. The filtrate was concentrated under reduced pressure to remove the solvent, giving the title compound **5e** (89 g, crude). The product was directly used in the next step without purification.

MS m/z (ESI): 290.1[M-55].

Step 4

tert-Butyl 4-(3-formyl-4-(methoxycarbonyl)phenyl)piperazine-1-carboxylate **5f**

Compound **5e** (5 g, 14.5 mmol), pyridine (10.5 mL), acetic acid (6.6 mL) and Raney nickel (2.5 g) were added to water (5 mL), and the temperature was raised to 70 °C. Sodium hypophosphite (7.5 g) was dissolved in water (15

mL), and the solution was added dropwise to the reaction mixture. After the dropwise addition, the mixture was allowed to react at 70 °C for 12 h. The reaction mixture was cooled to room temperature, and ethyl acetate (50 mL) and water (50 mL) were added. The organic phase was separated, washed with dilute hydrochloric acid (1 M, 50 mL × 3), washed with a saturated solution of sodium chloride (50 mL × 3), dried over anhydrous sodium sulfate and filtered. The filtrate was concentrated under reduced pressure to remove the solvent. The residue was purified by silica gel column chromatography using eluent system A to give the title compound **5f** (3 g, 59% yield).

MS m/z (ESI): 293.1[M-55].

Step 5

tert-Butyl

(*S*)-4-(2-(1-amino-5-(tert-butoxy)-1,5-dioxopentan-2-yl)-1-oxoisoindolin-5-yl)piperazin e-1-carboxylate **5g**

Compound **5f** (1.3 g, 3.7 mmol) and compound **5c** (0.89 g, 4.5 mmol) were added to methanol (10 mL), and the internal temperature was controlled at below 5 °C using an ice-water bath. Acetic acid (0.3 mL, 5.6 mmol) and sodium cyanoborohydride (0.46 g, 7.46 mmol) were added dropwise. The mixture was allowed to react at room temperature for 12 h. The reaction mixture was concentrated under reduced pressure to remove the solvent. Ethyl acetate (50 mL) and water (50 mL) were added to the residue. The organic phase was separated, washed with a saturated solution of citric acid (50 mL × 3), dried over anhydrous sodium sulfate and filtered. The filtrate was concentrated under reduced pressure to remove the solvent. The residue was purified by silica gel column chromatography using eluent system A to give the title product **5g** (0.71 g, 38% yield).

MS m/z (ESI): 503.2[M+1].

Step 6

(*S*)-3-(1-Oxo-5-(piperazin-1-yl)isoindolin-2-yl)piperidine-2,6-dionebenzenesulfonate **5h**

Compound **5g** (5.7 g, 11.4 mmol) and benzenesulfonic acid (3.59 g, 22.7 mmol) were added to acetonitrile (15 mL), and the mixture was stirred at 90 °C for 12 h. The reaction mixture was concentrated under reduced pressure to remove the solvent. The residue was washed with ethyl acetate (100 mL × 3) and dried to give the title compound **5h** (5.7 g, 100% yield).

MS m/z (ESI): 329.1[M+1].

$^1$H NMR (400 MHz, DMSO-$d_6$): δ 10.97 (s, 1H), 8.72 (br, 2H), 7.60-7.58 (m, 3H), 7.34-7.30 (m, 3H), 7.17-7.12 (m, 2H), 5.09-5.05 (m, 1H), 4.38-4.21 (m, 2H), 3.52-3.49 (m, 4H), 3.26 (s, 4H), 2.95-2.87 (m, 1H), 2.62-2.58 (m, 1H), 2.44-2.33 (m, 1H), 1.99-1.96 (m, 1H).

Chiral HPLC analysis method: retention time 16.66 min, chiral purity: 96.7% e.e. (column: CHIRALPAK OJ-H: A: n-hexane (+ 0.1% DEA), B: EtOH; A: 50%, B: 50%).

Step 7

(*S*)-3-(5-(4-((1-(2-Fluoro-4-((1*R*,2*R*)-6-hydroxy-2-(tetrahydro-2*H*-pyran-4-yl)-1,2,3,4-te trahydronaphthalen-1-yl)phenyl)piperidin-4-yl)methyl)piperazin-1-yl)-1-oxoisoindolin-2-yl)piperidine-2,6-dione **5**

Compound **5h** (476 mg, 0.98 mmol) was added to a mixed solvent of dichloromethane and methanol (V/V = 4/1, 20 mL), and sodium acetate (258 mg, 2.7 mmol) was added. After 10 min of reaction, compound **4b-1** (390 mg, 0.89 mmol) was added. After 15 min of reaction, sodium cyanoborohydride (108 mg, 1.8 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **5** (210 mg, 31% yield).

MS m/z (ESI): 750.4[M+1].

$^1$H NMR (500 MHz, DMSO-$d_6$) δ 10.95 (s, 1H), 9.08 (s, 1H), 7.53 (d, 1H), 7.07-7.05 (m, 2H), 6.89 (t, 1H), 6.76-6.74 (m, 1H), 6.69-6.64 (m, 2H), 6.53 (s, 1H), 6.45-6.43 (m, 1H), 5.07-5.03 (m, 1H), 4.32, 4.22 (dd, 2H), 4.12 (brs, 1H), 3.87-3.85 (m, 1H), 3.76-3.74 (m, 1H), 3.32-3.25 (m, 8H), 3.20 (t, 1H), 3.09 (t, 1H), 2.94-2.87 (m, 2H), 2.77-2.70 (m, 2H), 2.64-2.57 (m, 3H), 2.53-2.49 (m, 2H), 2.38-2.36 (m, 1H), 2.24-2.22 (m, 2H), 2.03-1.95 (m, 2H), 1.82-1.74 (m, 3H), 1.68-1.61 (m, 2H), 1.49-1.45 (m, 1H), 1.27-1.07 (m, 5H).

Example 6

3-(2-(4-((1-(4-((*R*)-6'-Hydroxy-3',4'-dihydro-1'*H*-spiro[cyclopentane-1,2'-naphthalen]-1' -yl)phenyl)piperidin-4-yl)methyl)piperazin-1-yl)-5-oxo-5,7-dihydro-6*H*-pyrrolo[3,4-*b*]p yridin-6-yl)piperidine-2,6-dione **6** (a 1:1 mixture of diastereomers)

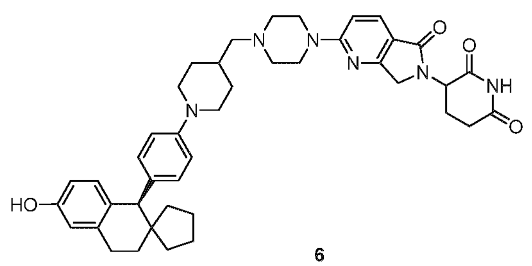

**6**

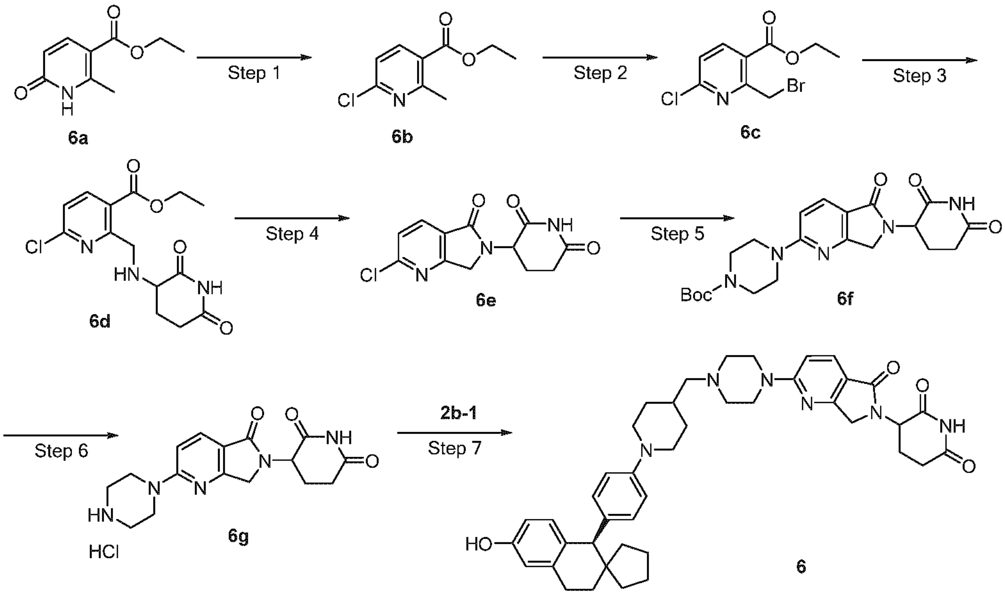


Step 1

Ethyl 6-chloro-2-methylnicotinate **6b**

Ethyl 2-methyl-6-oxo-1,6-dihydropyridine-3-carboxylate **6a** (10 g, 55.2 mmol, Jiangsu Aikon Biopharmaceutical R&D Co., Ltd.) was dissolved in phosphorus oxychloride (80 mL). The reaction was heated at 90 °C for 2 h. The reaction mixture was cooled to room temperature, concentrated under reduced pressure to remove the solvent, and quenched with iced water. The reaction mixture was made neutral with ammonia water and filtered. The filter cake was dried *in vacuo* to give the title compound **6b** (10 g, 97% yield).
MS m/z (ESI): 200.2[M+1].

Step 2

Ethyl 2-(bromomethyl)-6-chloronicotinate **6c**

Compound **6b** (5 g, 25.1 mmol), N-bromosuccinimide (2.9 g, 16.3 mmol) and azobisisobutyronitrile (0.41 g, 2.5 mmol) were added to carbon tetrachloride (125 mL). The mixture was allowed to react at 80 °C for 16 h in a nitrogen atmosphere. The reaction mixture was cooled to room temperature and concentrated under reduced pressure to remove the solvent. The residue was purified by silica gel column chromatography using eluent system B to give the title product **6c** (6.8 g, 97% yield).

MS m/z (ESI): 278.2[M+1].

Step 3

Ethyl 6-chloro-2-(((2,6-dioxopiperidin-3-yl)amino)methyl)nicotinate **6d** Compound **6c** (6.80 g, 24.4 mmol) was dissolved in N,N-dimethylformamide (120 mL), and 3-amino-2,6-piperidinedione hydrochloride (2.61 g, 15.9 mmol) and N,N-diisopropylethylamine (8.8 mL, 48.8 mmol) were added. The reaction was heated at 40 °C for 16 h. The reaction mixture was added to water (100 mL) and extracted with ethyl acetate (100 mL × 3). The organic phases were combined, washed with a saturated solution of sodium chloride (100 mL × 3), dried over anhydrous sodium sulfate and filtered. The filtrate was concentrated under reduced pressure to remove the solvent. The residue was purified by silica gel column chromatography using eluent system B to give the title product **6d** (3 g, 38% yield).
MS m/z (ESI): 326.2[M+1].

Step 4

3-(2-Chloro-5-oxo-5,7-dihydro-6*H*-pyrrolo[3,4-*b*]pyridin-6-yl)piperidine-2,6-dione **6e** Compound **6d** (3 g, 9.2 mmol) was dissolved in N,N-dimethylformamide (55 mL), and N,N-diisopropylethylamine (3.3 mL, 18.5 mmol) was added. The reaction was heated at 110 °C for 16 h. The reaction mixture was cooled to room temperature. Water (50 mL) was added, and the mixture was extracted with ethyl acetate (50 mL × 3). The organic phases were combined, washed with a saturated solution of sodium chloride (50 mL × 3), dried over anhydrous sodium sulfate and filtered. The filtrate was concentrated under reduced pressure to remove the solvent. The residue was purified by silica gel column chromatography using eluent system B to give the title product **6e** (1.4 g, 54% yield).
MS m/z (ESI): 280.0[M+1].

Step 5

tert-Butyl 4-(6-(2,6-dioxopiperidin-3-yl)-5-oxo-6,7-dihydro-5*H*-pyrrolo[3,4-*b*]pyridin-2-yl)pipera zine-1-carboxylate **6f**

Compound **6e** (1.4 g, 5 mmol) was dissolved in dimethyl sulfoxide (50 mL), and tert-butyl piperazine-1-carboxylate hydrochloride (1.87 g, 10 mmol) and N,N-diisopropylethylamine (4.53 mL, 25.2 mmol) were added. The reaction was heated at 110 °C for 16 h. The reaction mixture was cooled to room temperature. Water (20 mL) was added, and the mixture was extracted with ethyl acetate (50 mL × 3). The organic phases were combined, dried over anhydrous sodium sulfate and filtered. The filtrate was concentrated under reduced pressure to remove the solvent. The residue was washed with methyl tert-butyl ether to give the title compound **6f** (2 g, 93% yield).
MS m/z (ESI): 374.1[M-55].

Step 6

3-(5-Oxo-2-(piperazin-1-yl)-5,7-dihydro-6*H*-pyrrolo[3,4-*b*]pyridin-6-yl)piperidine-2,6-dione hydrochloride **6g**

Compound **6f** (2 g, 4.7 mmol) was dissolved in dichloromethane (50 mL), and a 4 M solution of hydrogen chloride in 1,4-dioxane (11.7 mL, 46.8 mmol) was added. The mixture was allowed to react at room temperature for 16 h. The reaction mixture was concentrated under reduced pressure to remove the solvent. The residue was washed with ethyl acetate to give the title compound **6g** (2 g).
MS m/z (ESI): 330.2[M+1].
$^1$H NMR (400 MHz, DMSO-$d_6$): δ 10.94 (s, 1H), 9.67 (br, 2H), 7.85 (d, 1H), 6.99 (d, 1H), 5.08-5.05 (m, 1H), 4.32, 4.11 (dd, 2H), 3.93-3.90 (m, 4H), 3.15 (m, 4H), 3.93-2.85 (m, 1H), 2.53-2.51 (m, 1H), 2.43-2.48 (m, 1H), 1.97-1.95 (m, 1H).

Step 7

3-(2-(4-((1-(4-((*R*)-6'-Hydroxy-3',4'-dihydro-1'*H*-spiro[cyclopentane-1,2'-naphthalen]-1' -yl)phenyl)piperidin-4-yl)methyl)piperazin-1-yl)-5-oxo-5,7-dihydro-6*H*-pyrrolo[3,4-*b*]p yridin-6-yl)piperidine-2,6-dione **6** (a 1:1 mixture of diastereomers)

Compound **6g** (61 mg, 0.17 mmol) was added to a mixed solvent of dichloromethane and methanol (V/V = 4/1, 5 mL), and sodium acetate (72 mg, 0.88 mmol) was added. After 10 min of reaction, compound **2b-1** (45 mg, 0.11 mmol) was added. After 15 min of reaction, sodium triacetoxyborohydride (47 mg, 0.22 mmol) was added. The mixture was

allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **6** (50 mg, a 1:1 mixture of diastereomers, 63% yield).

MS m/z (ESI): 703.2[M+1].

$^1$H NMR (500 MHz, DMSO-$d_6$) $\delta$ 10.95 (s, 1H), 9.10 (s, 1H), 7.77 (d, 1H), 6.91 (d, 1H), 6.79, 6.78 (dd, 4H), 6.59 (d, 1H), 6.53 (s, 1H), 6.43-6.41 (m, 1H), 5.09-5.06 (m, 1H), 4.26, 4.12 (dd, 2H), 3.65-3.56 (m, 4H), 3.50-3.42 (m, 6H), 2.94-2.87 (m, 1H), 2.80-2.77 (m, 2H), 2.65-2.59 (m, 3H), 2.45-2.41 (m, 3H), 2.38-2.33 (m, 1H), 2.22-2.20 (m, 2H), 1.98-1.96 (m, 1H), 1.82-1.76 (m, 2H), 1.64-1.60 (m, 3H), 1.54-1.42 (m, 3H), 1.35-1.28 (m, 2H), 1.25-1.19 (m, 2H), 0.84-0.79 (m, 1H).

Example 7

3-(5-(4-((1-(4-((1,2)-*cis*-6-Hydroxy-2-(tetrahydro-2*H*-pyran-4-yl)-1,2,3,4-tetrahydronap hthalen-1-yl)phenyl)piperidin-4-yl)methyl)piperazin-1-yl)-1-oxoisoindolin-2-yl)piperidi ne-2,6-dione **7**

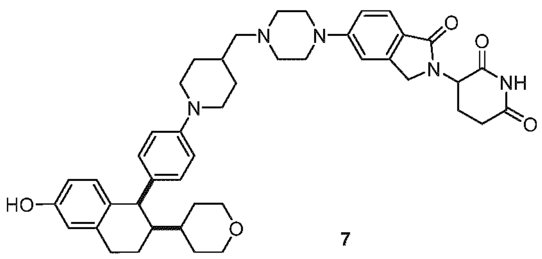

**7**

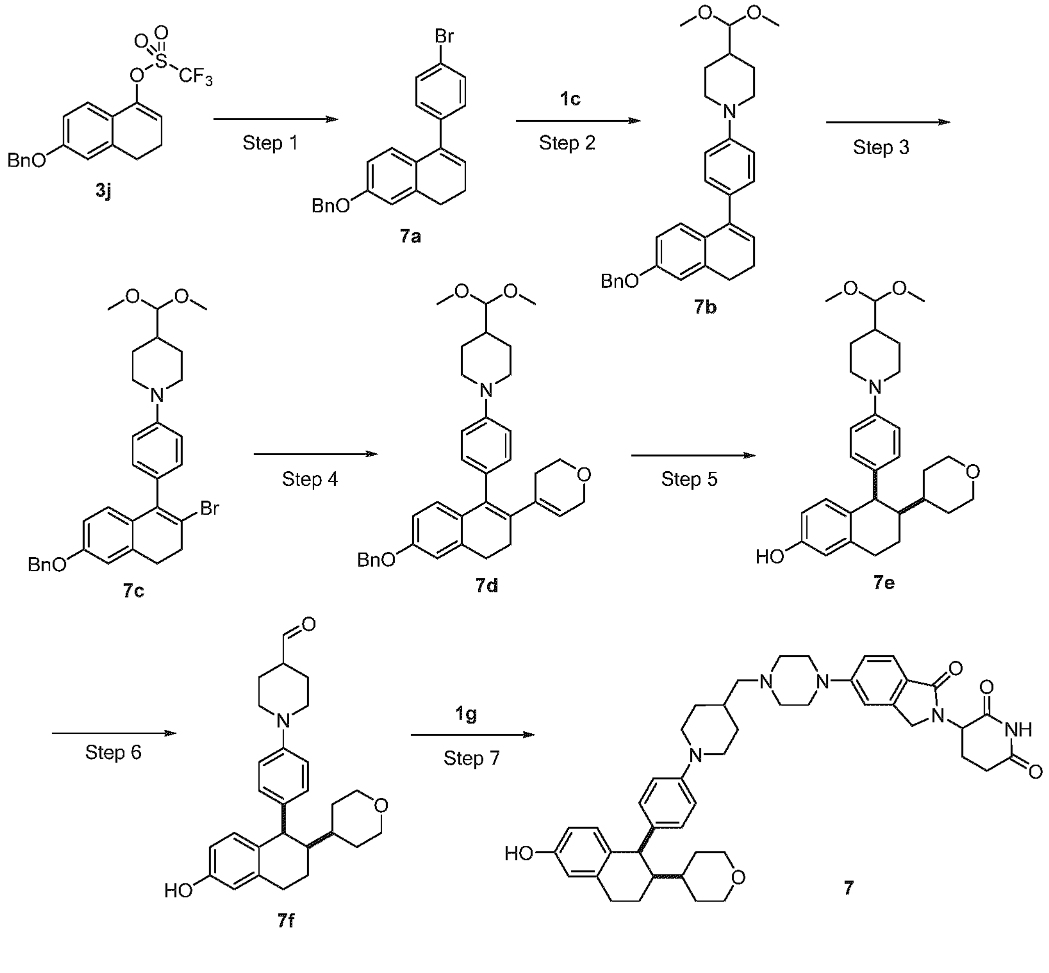

Step 1

7-(Benzyloxy)-4-(4-bromophenyl)-1,2-dihydronaphthalene **7a**

Compound **3j** (32 g, 83.3 mmol), 4-bromobenzeneboronic acid (20 g, 100 mmol, Shanghai Meryer Biochemical Technology Co., Ltd), tetrakis(triphenylphosphine)palladium(0) (9.62 g, 8.3 mmol) and sodium carbonate (26.47 g, 250 mmol) were sequentially added to 360 mL of a mixed solvent of 1,4-dioxane and water (V/V = 5/1). The mixture was allowed to react at 80 °C for 2 h in a nitrogen atmosphere. The reaction mixture was cooled to room temperature. Water (200 mL) was added, and the mixture was extracted with dichloromethane (200 mL × 3). The organic phases were combined, washed with a saturated solution of sodium chloride (200 mL × 2), dried over anhydrous sodium sulfate and filtered. The filtrate was concentrated under reduced pressure to remove the solvent. The residue was purified by silica gel column chromatography using eluent system B to give the title compound **7a** (14 g, 43% yield).

Step 2

1-(4-(6-(Benzyloxy)-3,4-dihydronaphthalen-1-yl)phenyl)-4-(dimethoxymethyl)piperidin **e 7b**

Compound **7a** (16 g, 40.9 mmol), compound **1c** (7.81 g, 49.1 mmol), palladium acetate (1.38 g, 6.1 mmol), 2-dicyclohexylphosphino-2',4',6'-triisopropylbiphenyl (3.9 g, 8.2 mmol) and sodium tert-butoxide (11.79 g, 123 mmol) were added to toluene (350 mL). The mixture was allowed to react at 90 °C for 2 h in a nitrogen atmosphere. The reaction mixture was cooled to room temperature. Water (100 mL) was added, and the mixture was extracted with dichloromethane (200 mL × 3). The organic phases were combined, washed with a saturated solution of sodium chloride (100 mL × 2), dried over anhydrous sodium sulfate and filtered. The filtrate was concentrated under reduced pressure to remove the solvent. The residue was purified by silica gel column chromatography using eluent system A to give the title compound **7b** (10.5 g, 55% yield).

MS m/z (ESI): 470.2[M+1].

Step 3

1-(4-(6-(Benzyloxy)-2-bromo-3,4-dihydronaphthalen-1-yl)phenyl)-4-(dimethoxymethyl )piperidine **7c**

Compound **7b** (10.5 g, 22.4 mmol) was dissolved in dichloromethane (350 mL), and the solution was cooled to -5 °C in an ice-salt bath. Pyridinium tribromide (8.58 g, 26.8 mmol) and triethylamine (4.52 g, 44.7 mmol) were added portionwise. The mixture was allowed to react at -5 °C for 30 min. A saturated solution of sodium bicarbonate (100 mL) was added to the reaction mixture. The organic phase was separated, washed with a saturated solution of sodium chloride (100 mL × 3), dried over anhydrous sodium sulfate and filtered. The filtrate was concentrated under reduced pressure to remove the solvent. The residue was purified by silica gel column chromatography using eluent system B to give the title product **7c** (5.1 g, 42% yield).

MS m/z (ESI): 550.2[M+1].

$^1H$ NMR (400 MHz, DMSO-$d_6$): δ 7.45-7.35 (m, 4H), 7.34 (m, 1H), 6.99 (s, 4H), 6.90 (d, 1H), 6.71 (dd, 1H), 6.47 (d, 1H), 5.07 (s, 2H), 4.11 (d, 1H), 3.75 (d, 2H), 3.29 (s, 6H), 2.98-2.91 (m, 2H), 2.87 (t, 2H), 2.66 (m, 2H), 1.75 (d, 3H), 1.35 (m, 2H).

Step 4

1-(4-(6-(Benzyloxy)-2-(3,6-dihydro-2*H*-pyran-4-yl)-3,4-dihydronaphthalen-1-yl)phenyl )-4-(dimethoxymethyl)piperidine **7d**

Compound **7c** (640 mg, 1.2 mmol) and 3,6-dihydro-2*H*-pyran-4-boronic acid pinacol ester (370 mg, 1.8 mmol) were added to 12.5 mL of a mixed solvent of 1,4-dioxane and water (V/V = 4/1), and tetrakis(triphenylphosphine)palladium(0) (135 mg, 0.12 mmol) and sodium carbonate (250 mg, 2.36 mmol) were then added. The reaction was heated at 80 °C for 2 h in a nitrogen atmosphere. The reaction mixture was cooled to room temperature and concentrated under reduced pressure to remove the solvent. The residue was dissolved in ethyl acetate (30 mL), and the solution was washed with a saturated solution of sodium bicarbonate (10 mL × 3). The organic phase was dried over anhydrous sodium sulfate and filtered. The filtrate was concentrated under reduced pressure to remove the solvent. The residue was purified by silica gel column chromatography using eluent system A to give the title compound **7d** (215 mg, 33% yield).

MS m/z (ESI): 552.3[M+1].

Step 5

(5,6)-*cis*-5-(4-(4-(Dimethoxymethyl)piperidin-1-yl)phenyl)-6-(tetrahydro-2*H*-pyran-4-y l)-5,6,7,8-tetrahydronaphthalen-2-ol **7e**

Compound **7d** (215 mg, 0.39 mmol) was added to methanol (15 mL), and palladium hydroxide on carbon (100 mg, 20 wt%) was added. The mixture was allowed to react in a hydrogen atmosphere for 16 h. The reaction mixture was filtered. The filtrate was concentrated under reduced pressure to remove the solvent, giving the title compound **7e** (120 mg, 66% yield).

MS m/z (ESI): 466.3[M+1].

Step 6

1-(4-((1,2)-*cis*-6-Hydroxy-2-(tetrahydro-2*H*-pyran-4-yl)-1,2,3,4-tetrahydronaphthalen-1 -yl)phenyl)piperidine-4-carbaldehyde **7f**

Compound **7e** (25 mg, 0.05 mmol) was added to tetrahydrofuran (2 mL), and dilute sulfuric acid (2 M, 0.1 mL, 0.2 mmol) was added. The reaction was heated at 50 °C for 1 h. The reaction mixture was cooled to room temperature, made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **7f (22** mg, 100% yield).

Step 7

3-(5-(4-((1-(4-((1,2)-*cis*-6-Hydroxy-2-(tetrahydro-2*H*-pyran-4-yl)-1,2,3,4-tetrahydronap hthalen-1-yl)phenyl)piperidin-4-yl)methyl)piperazin-1-yl)-1-oxoisoindolin-2-yl)piperidi ne-2,6-dione **7**

Compound **1g** (25 mg, 0.076 mmol) was added to 5 mL of a mixed solvent of dichloromethane and methanol (V/V = 4/1), and sodium acetate (100 mg, 1.2 mmol) was added. After 10 min of reaction, compound **7f** (22 mg, 0.05 mmol) was added. After 15 min of reaction, sodium triacetoxyborohydride (23 mg, 0.11 mmol) was added. The mixture was allowed to react at room temperature for 16 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **7** (9 mg, 23% yield).

MS m/z (ESI): 732.4[M+1].

$^1$H NMR (500 MHz, DMSO-$d_6$) $\delta$ 10.95 (bs, 1H), 9.02 (s, 1H), 7.53 (d, 1H), 7.11-7.01 (m, 2H), 6.82, 6.78 (dd, 4H), 6.62 (d, 1H), 6.51 (d, 1H), 6.42-6.40 (m, 1H), 5.07-5.03 (m, 1H), 4.32, 4.22 (dd, 2H), 4.05 (d, 1H), 3.86 (d, 1H), 3.74 (d, 1H), 3.61 (t, 2H), 3.37-3.22 (m, 8H), 3.18 (t, 1H), 3.08 (t, 1H), 2.96-2.85 (m, 2H), 2.79-2.68 (m, 1H), 2.67-2.54 (m, 3H), 2.44-2.33 (m, 1H), 2.22 (d, 2H), 2.06-1.92 (m, 2H), 1.88-1.44 (m, 6H), 1.31-1.02 (m, 6H).

Examples 8-1 and 8-2 3-(5-(4-((1-(4-((1*R*,2*R*)-6-Hydroxy-2-(tetrahydro-2*H*-pyran-4-yl)-1,2,3,4-tetrahydronap hthalen-1-yl)phenyl)piperidin-4-yl)methyl)piperazin-1-yl)-1-oxoisoindolin-2-yl)piperidi ne-2,6-dione **8-1** (a 1:1 mixture of diastereomers) 3-(5-(4-((1-(4-((1*S*,2*S*)-6-Hydroxy-2-(tetrahydro-2*H*-pyran-4-yl)-1,2,3,4-tetrahydronaph thalen-1-yl)phenyl)piperidin-4-yl)methyl)piperazin-1-yl)-1-oxoisoindolin-2-yl)piperidin e-2,6-dione **8-2** (a 1:1 mixture of diastereomers)

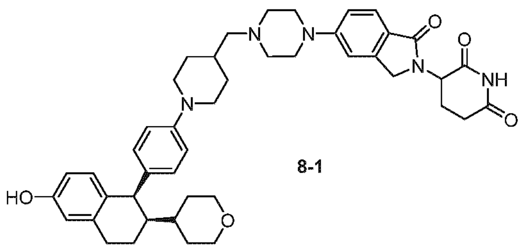

8-1

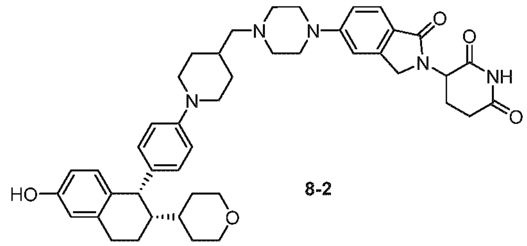

8-2

By using the synthesis schemes of Examples 2-1 and 2-2 in which **7e** was used in place of the starting material **1m** of step **1,** the title compounds **8-1** (19 mg, a 1:1 mixture of diastereomers) and **8-2** (24 mg, a 1:1 mixture of diastereomers) were prepared.

**8-1**

MS m/z (ESI): 732.4[M+1].

$^1$H NMR (500 MHz, DMSO-$d_6$) $\delta$ 10.95 (bs, 1H), 9.02 (s, 1H), 7.53 (d, 1H), 7.11-7.01 (m, 2H), 6.82, 6.78 (dd, 4H), 6.62 (d, 1H), 6.51 (d, 1H), 6.42-6.40 (m, 1H), 5.07-5.03 (m, 1H), 4.32, 4.22 (dd, 2H), 4.05 (d, 1H), 3.86 (d, 1H), 3.74 (d, 1H), 3.61 (t, 2H), 3.37-3.22 (m, 8H), 3.18 (t, 1H), 3.08 (t, 1H), 2.96-2.85 (m, 2H), 2.79-2.68 (m, 1H), 2.67-2.54 (m, 3H), 2.44-2.33 (m, 1H), 2.22 (d, 2H), 2.06-1.92 (m, 2H), 1.88-1.44 (m, 6H), 1.31-1.02 (m, 6H)

**8-2**

MS m/z (ESI): 732.4[M+1].

$^1$H NMR (500 MHz, DMSO-$d_6$) $\delta$ 10.95 (bs, 1H), 9.02 (s, 1H), 7.53 (d, 1H), 7.11-7.01 (m, 2H), 6.82, 6.78 (dd, 4H), 6.62 (d, 1H), 6.51 (d, 1H), 6.42-6.40 (m, 1H), 5.07-5.03 (m, 1H), 4.32, 4.22 (dd, 2H), 4.05 (d, 1H), 3.86 (d, 1H), 3.74 (d, 1H), 3.61 (t, 2H), 3.37-3.22 (m, 8H), 3.18 (t, 1H), 3.08 (t, 1H), 2.96-2.85 (m, 2H), 2.79-2.68 (m, 1H), 2.67-2.54 (m, 3H), 2.44-2.33 (m, 1H), 2.22 (d, 2H), 2.06-1.92 (m, 2H), 1.88-1.44 (m, 6H), 1.31-1.02 (m, 6H).

Example 9

3-(5-(4-((1-(4-((1,2)-cis-2-(4,4-Difluorocyclohexyl)-6-hydroxy-1,2,3,4-tetrahydronapht halen-1 -yl)phenyl)piperidin-4-yl)methyl)piperazin-1 -yl)-1 -oxoisoindolin-2-yl)piperidin e-2,6-dione **9**

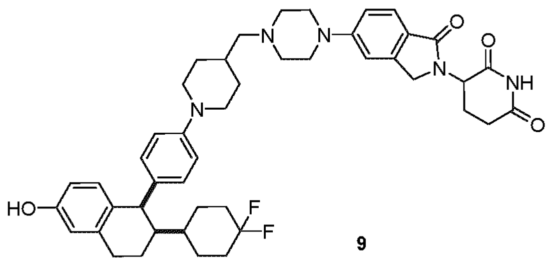

9

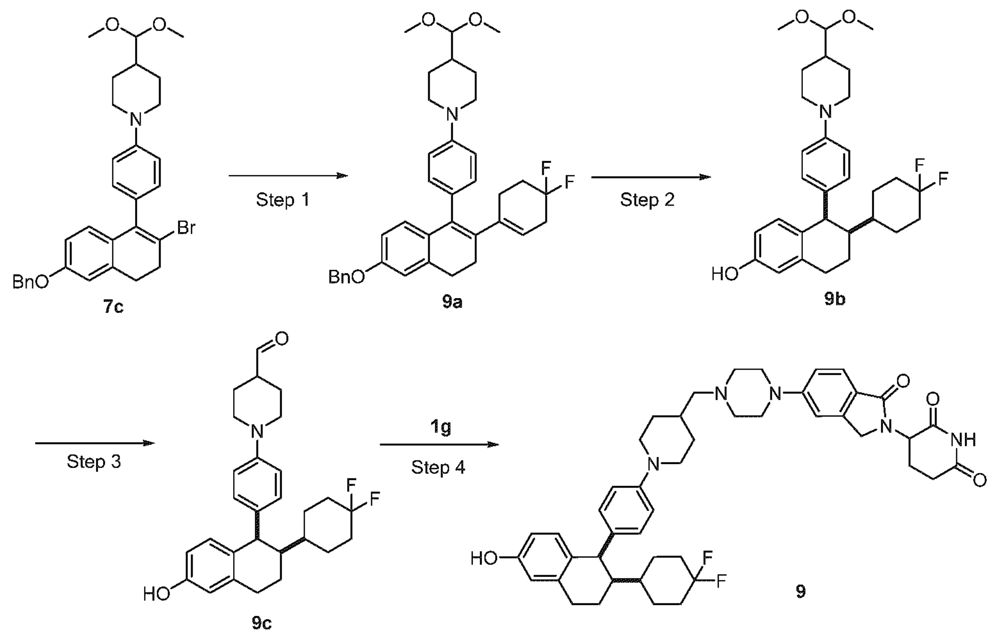


7c 9a 9b 9c 9

Step 1

1-(4-(6-(Benzyloxy)-2-(4,4-difluorocyclohex-1-en-1-yl)-3,4-dihydronaphthalen-1-yl)ph enyl)-4-(dimethoxymethyl)piperidine **9a**

Compound **7c** (200 mg, 0.36 mmol) and 4,4-difluorocyclohexene-1-boronic acid pinacol ester (135 mg, 0.55 mmol) were added to 12 mL of a mixed solvent of 1,4-dioxane and water (V/V = 5/1), and tetrakis(triphenylphosphine)palladium(0) (135 mg, 0.12 mmol) and sodium carbonate (250 mg, 2.4 mmol) were added. The mixture was allowed to react at 80 °C for 2 h in a nitrogen atmosphere. The reaction mixture was cooled to room temperature and concentrated under reduced pressure to remove the solvent. The residue was dissolved in ethyl acetate (30 mL), and the solution was washed with a saturated solution of sodium bicarbonate (10 mL × 3). The organic phase was dried over anhydrous sodium sulfate and filtered. The filtrate was concentrated under reduced pressure to remove the solvent. The residue was purified by silica gel column chromatography using eluent system A to give the title compound **9a** (150 mg, 70% yield).
MS m/z (ESI): 586.2[M+1].

Step 2

(5,6)-*cis*-6-(4,4-Difluorocyclohexyl)-5-(4-(4-(dimethoxymethyl)piperidin-1-yl)phenyl)-5,6,7,8-tetrahydronaphthalen-2-ol **9b**

Compound **9a** (150 mg, 0.26 mmol) was added to methanol (10 mL), and palladium hydroxide on carbon (100 mg, 20 wt%) was added. The mixture was allowed to react in a hydrogen atmosphere for 16 h. The reaction mixture was filtered. The filtrate was concentrated under reduced pressure to remove the solvent, giving the title compound **9b** (120 mg, 94% yield).
MS m/z (ESI): 500.2[M+1].

Step 3

1-(4-((1,2)-*cis*-2-(4,4-Difluorocyclohexyl)-6-hydroxy-1,2,3,4-tetrahydronaphthalen-1-yl )phenyl)piperidine-4-carbaldehyde **9c**

Compound **9b** (120 mg, 0.24 mol) was dissolved in tetrahydrofuran (2 mL), and dilute sulfuric acid (2 M, 0.5 mL, 1 mmol) was added. The mixture was allowed to react at 50 °C for 1 h. The reaction mixture was made neutral with a saturated solution of sodium bicarbonate and extracted with ethyl acetate (8 mL × 3). The organic phases were combined, dried over anhydrous sodium sulfate and filtered. The filtrate was concentrated under reduced pressure to remove the solvent, giving the title compound **9c** (108 mg, 100% yield).

Step 4

3-(5-(4-((1-(4-((1,2)-*cis*-2-(4,4-Difluorocyclohexyl)-6-hydroxy-1,2,3,4-tetrahydronapht halen-1 -yl)phenyl)piperidin-4-yl)methyl)piperazin-1 -yl)-1 -oxoisoindolin-2-yl)piperidin e-2,6-dione **9**

Compound **1g** (25 mg, 0.076 mmol) was added to 5 mL of a mixed solvent of dichloromethane and methanol (V/V = 4/1), and sodium acetate (100 mg, 1.2 mmol) was added. After 10 min of reaction, compound **9c** (30 mg, 0.066 mmol) was added. After 15 min of reaction, sodium triacetoxyborohydride (23 mg, 0.11 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **9** (10 mg, 20% yield).

MS m/z (ESI): 766.5[M+1].

$^1$H NMR (500 MHz, DMSO-$d_6$) $\delta$ 10.95 (s, 1H), 9.02 (s, 1H), 7.53 (d, 1H), 7.11-7.01 (m, 2H), 6.82, 6.79 (dd, 4H), 6.62 (d, 1H), 6.51 (d, 1H), 6.43-6.40 (m, 1H), 5.07-5.03 (m, 1H), 4.32, 4.22 (dd, 2H), 4.05 (brs, 1H), 3.59-3.56 (m, 2H), 3.35-3.22 (m, 10H), 2.96-2.85 (m, 2H), 2.79-2.68 (m, 3H), 2.67-2.54 (m, 5H), 2.44-2.33 (m, 1H), 2.22 (d, 2H), 2.06-1.93 (m, 2H), 1.84-1.45 (m, 4H), 1.31-1.02 (m, 6H).

Example 10

3-(5-(4-((1-(4-((1*R*,2*R*)-2-(4,4-Difluorocyclohexyl)-6-hydroxy-1,2,3,4-tetrahydronaphth alen-1 -yl)phenyl)piperidin-4-yl)methyl)piperazin-1 -yl)-1 -oxoisoindolin-2-yl)piperidine-2,6-dione **10** a 1:1 mixture of diastereomers)

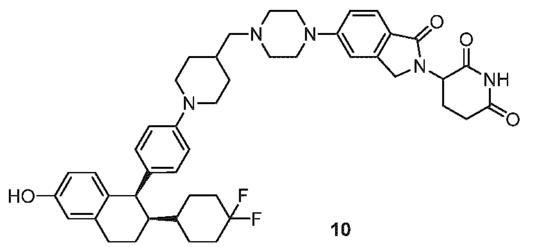

10

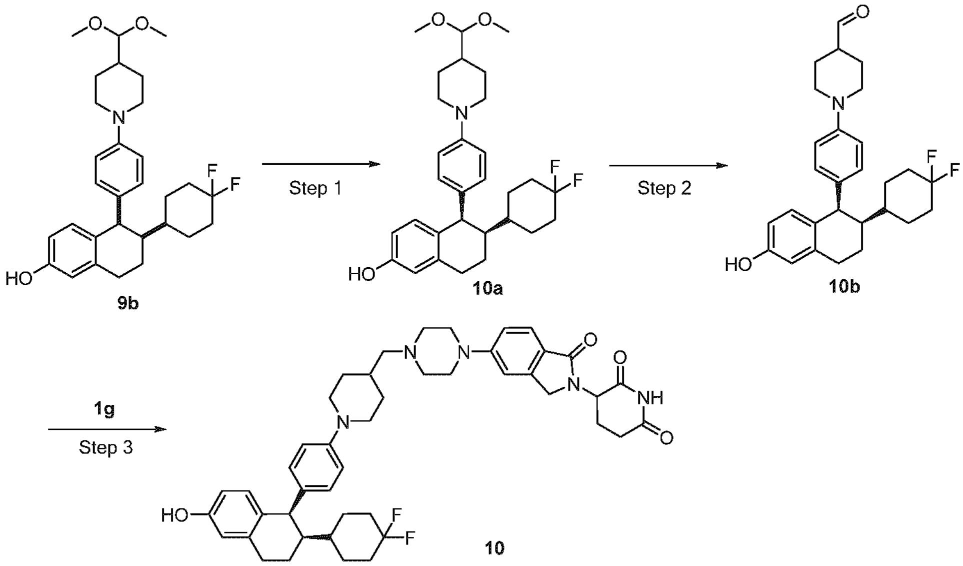


9b

10a

10b

10

Step 1

(SR,6R)-6-(4,4-Difluorocyclohexyl)-5-(4-(4-(dimethoxymethyl)piperidin-1-yl)phenyl)-5 ,6,7,8-tetrahydronaphthalen-2-ol **10a**

Compound **9b** (400 mg, 0.8 mmol) was resolved by preparative chiral chromatography (resolution conditions: column: CHIRALPAK IE, 20 mm × 250 mm, 5 μm; mobile phases: A: n-hexane, B: ethanol (+ 20 mmol $NH_3$), A: 70%, B: 30%) to give the title compound **10a** (120 mg).

MS m/z (ESI): 500.2[M+1].

Chiral HPLC analysis method: retention time 15.86 min, chiral purity: 100% (column: CHIRALPAK IE A: n-hexane, B: EtOH (+ 0.1% DEA), A: 85%, B: 15%).

Step 2

1-(4-((1*R*,2*R*)-2-(4,4-Difluorocyclohexyl)-6-hydroxy-1,2,3,4-tetrahydronaphthalen-1-yl) phenyl)piperidine-4-carbaldehyde **10b**

Compound **10a** (120 mg, 0.24 mmol) was dissolved in tetrahydrofuran (2 mL), and dilute sulfuric acid (2 M, 0.5 mL, 1 mmol) was added. The mixture was allowed to react at 50 °C for 1 h. The reaction mixture was made neutral with a saturated solution of sodium bicarbonate and extracted with ethyl acetate (8 mL × 3). The organic phases were combined, dried over anhydrous sodium sulfate and filtered. The filtrate was concentrated under reduced pressure to remove the solvent, giving the title compound **10b** (100 mg, 92% yield).

Step 3

3-(5-(4-((1-(4-((1*R*,2*R*)-2-(4,4-Difluorocyclohexyl)-6-hydroxy-1,2,3,4-tetrahydronaphth alen-1 -yl)phenyl)piperidin-4-yl)methyl)piperazin-1 -yl)-1 -oxoisoindolin-2-yl)piperidine-2,6-dione **10** (a 1:1 mixture of diastereomers)

Compound **1g** (37 mg, 0.11 mmol) was added to 5 mL of a mixed solvent of dichloromethane and methanol (V/V = 4/1), and sodium acetate (46 mg, 0.56 mmol) was added. After 10 min of reaction, compound **10b** (50 mg, 0.11 mmol) was added. After 15 min of reaction, sodium triacetoxyborohydride (47 mg, 0.22 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **10** (20 mg, a 1:1 mixture of diastereomers, 23% yield).

MS m/z (ESI): 766.5[M+1].

$^1$H NMR (500 MHz, DMSO-$d_6$) $\delta$ 10.95 (s, 1H), 9.02 (s, 1H), 7.53 (d, 1H), 7.11-7.01 (m, 2H), 6.82, 6.79 (dd, 4H), 6.62 (d, 1H), 6.51 (d, 1H), 6.43-6.40 (m, 1H), 5.07-5.03 (m, 1H), 4.32, 4.22 (dd, 2H), 4.05 (brs, 1H), 3.59-3.56 (m, 2H), 3.35-3.22 (m, 10H), 2.96-2.85 (m, 2H), 2.79-2.68 (m, 3H), 2.67-2.54 (m, 5H), 2.44-2.33 (m, 1H), 2.22 (d, 2H), 2.06-1.93 (m, 2H), 1.84-1.45 (m, 4H), 1.31-1.02 (m, 6H).

Example 11

3-(5-(4-((1-(4-((1,2)-*cis*-6-Hydroxy-2-(1-methylpiperidin-4-yl)-1,2,3,4-tetrahydronapht halen-1 -yl)phenyl)piperidin-4-yl)methyl)piperazin-1 -yl)-1 -oxoisoindolin-2-yl)piperidin e-2,6-dione **11**

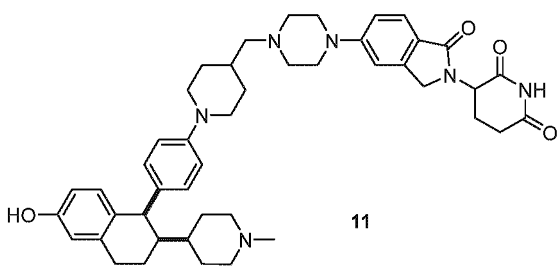

11

By using the synthesis scheme of Example 7 in which 1-methyl-1,2,3,6-tetrahydropyridine-4-boronic acid was used in place of the starting material 3,6-dihydro-2*H*-pyran-4-boronic acid of step 4, the title compound **11** (14 mg) was prepared.

MS m/z (ESI): 743.4[M-1].

$^{1}$H NMR (500 MHz, DMSO-$d_6$) $\delta$ 10.95 (bs, 1H), 9.02 (s, 1H), 7.53 (d, 1H), 7.18-7.02 (m, 2H), 6.80, 6.78 (dd, 4H), 6.62 (d, 1H), 6.50 (d, 1H), 6.42-6.40 (m, 1H), 5.07-5.03 (m, 1H), 4.32, 4.22 (dd, 2H), 4.05 (d, 1H), 3.66-3.55 (m, 2H), 3.40-3.22 (m, 8H), 2.96-2.54 (m, 7H), 2.42-2.31 (m, 1H), 2.22 (d, 2H), 2.14-2.02 (m, 4H), 2.01-1.93 (m, 1H), 1.85-1.43 (m, 8H), 1.31-1.14 (m, 4H), 1.09-0.98 (m, 1H), 0.95-0.80 (m, 2H).

Examples 12-1 and 12-2

3-(5-(4-((1-(3-Fluoro-4-((1*S*,2*R*)-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione **12-1** (a 1:1 mixture of diastereomers)
3-(5-(4-((1-(3-Fluoro-4-((1*R*,2*S*)-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione **12-2** (a 1:1 mixture of diastereomers)

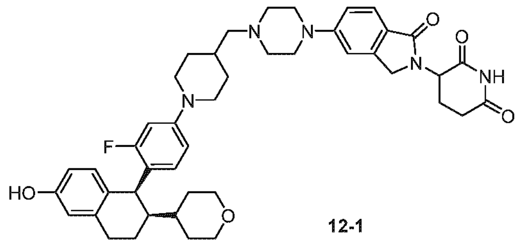

**12-1**

+

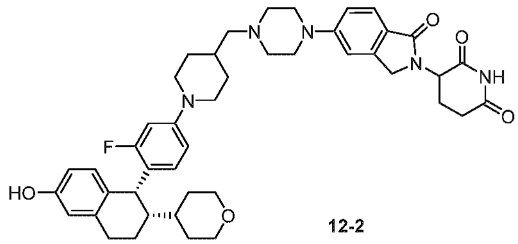

**12-2**

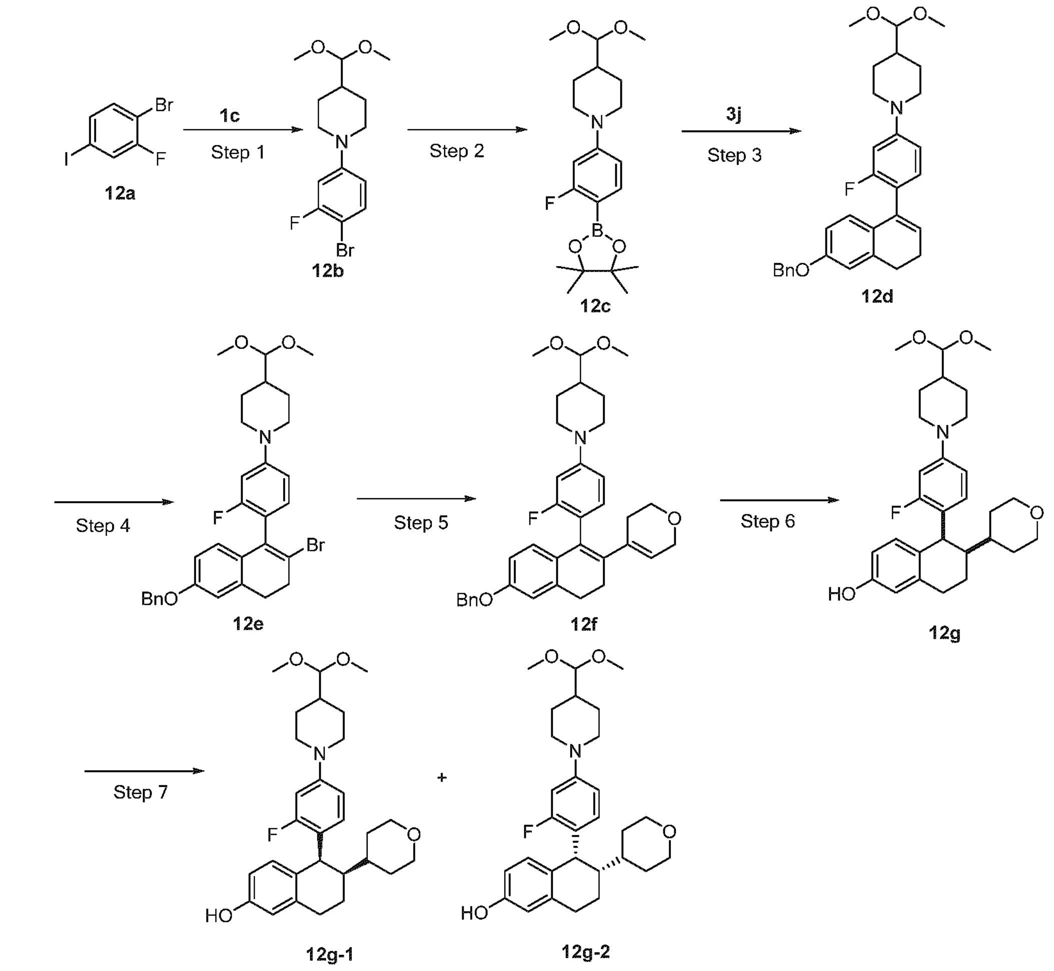


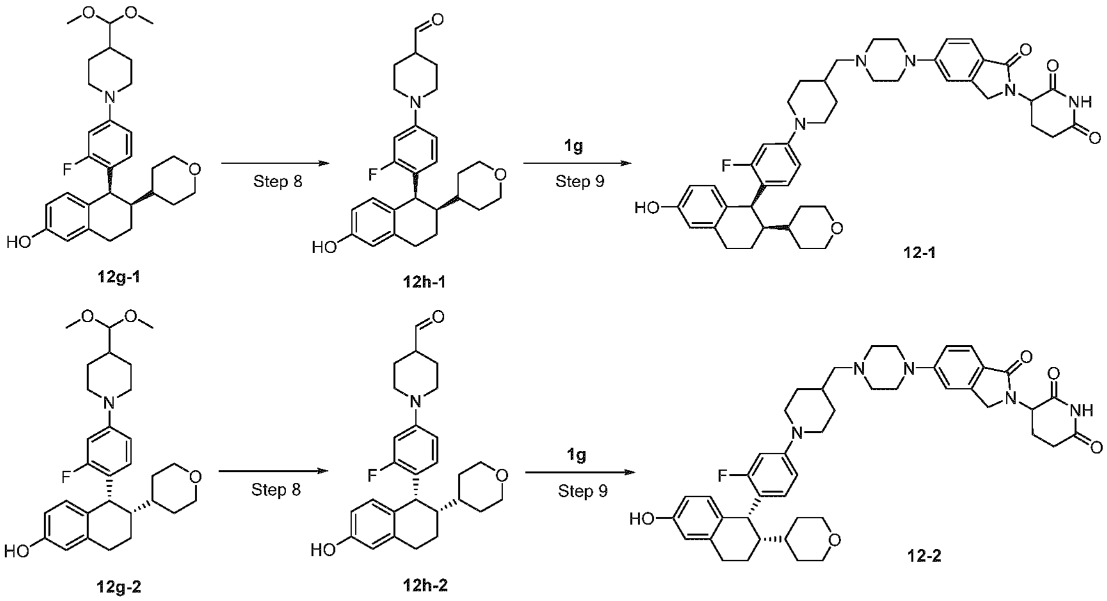


Step 1

1-(4-Bromo-3-fluorophenyl)-4-(dimethoxymethyl)piperidine **12b**

1-Bromo-2-fluoro-4-iodobenzene **12a** (2 g, 6.7 mmol), compound **1c** (1.1 g, 6.9 mmol), cuprous iodide (254

mg, 1.3 mmol), L-proline (306 mg, 2.7 mmol) and anhydrous potassium carbonate (1.84 g, 13.3 mmol) were added to *N,N*-dimethylformamide (20 mL). The reaction was heated at 90 °C for 16 h in a nitrogen atmosphere. The reaction mixture was cooled to room temperature and filtered through celite. The filtrate was concentrated under reduced pressure to remove the solvent. The residue was purified by silica gel column chromatography using eluent system B to give the title compound **12b** (1.35 g, 61% yield).

MS m/z (ESI): 332.1[M+1].

Step 2

4-(Dimethoxymethyl)-1-(3-fluoro-4-(4,4,5,5-tetramethyl-1,3,2-dioxaborolan-2-yl)pheny l)piperidine **12c**

Compound **12b** (2 g, 6 mmol), bis(pinacolato)diboron (2.3 g, 9.1 mmol), potassium acetate (1.2 g, 12.2 mmol), 2-dicyclohexylphosphino-2',4',6'-triisopropylbiphenyl (X-phos, 575 mg, 1.2 mmol) and palladium acetate (136 mg, 0.61 mmol) were added to 1,4-dioxane (40 mL). The reaction was heated at 100 °C for 16 h in a nitrogen atmosphere. The reaction mixture was cooled to room temperature and filtered through celite. The filtrate was concentrated under reduced pressure to remove the solvent. The residue was purified by silica gel column chromatography using eluent system B to give the title compound **12c** (1.7 g, 74% yield).

MS m/z (ESI): 380.3[M+1].

Step 3

1-(4-(6-(Benzyloxy)-3,4-dihydronaphthalen-1-yl)-3-fluorophenyl)-4-(dimethoxymethyl) piperidine **12d**

Compound **3j** (1.7 g, 4.4 mmol), compound **12c** (1.7 g, 4.5 mmol), [1,1'-bis(diphenylphosphino)ferrocene]palladium(II) dichloride (325 mg, 0.44 mmol) and anhydrous potassium carbonate (1.3 g, 9.4 mmol) were added to 20 mL of a mixed solvent of 1,4-dioxane and water (V/V = 3/1). The reaction was heated at 100 °C for 3 h in a nitrogen atmosphere. The reaction mixture was cooled to room temperature and filtered through celite. The filtrate was concentrated under reduced pressure to remove the solvent. The residue was purified by silica gel column chromatography using eluent system B to give the title compound **12d** (1.7 g, 79% yield).

MS m/z (ESI): 488.2[M+1].

Step 4

1-(4-(6-(Benzyloxy)-2-bromo-3,4-dihydronaphthalen-1-yl)-3-fluorophenyl)-4-(dimetho xymethyl)piperidine **12e**

Compound **12d** (276 mg, 0.57 mmol) was dissolved in dichloromethane (15 mL), and the solution was cooled to -5 °C in an ice-salt bath. Pyridinium tribromide (218 mg, 0.68 mmol) and triethylamine (115 mg, 1.1 mmol) were added. The mixture was allowed to react at -5 °C for 30 min. A saturated solution of sodium bicarbonate (20 mL) was added to the reaction mixture. The aqueous phase was extracted with dichloromethane (10 mL × 3). The organic phases were combined, dried over anhydrous sodium sulfate and filtered. The filtrate was concentrated under reduced pressure to remove the solvent. The residue was purified by silica gel column chromatography using eluent system B to give the title product **12e** (150 mg, 47% yield).

MS m/z (ESI): 568.1[M+1].

Step 5

1-(4-(6-(Benzyloxy)-2-(3,6-dihydro-2*H*-pyran-4-yl)-3,4-dihydronaphthalen-1-yl)-3-fluo rophenyl)-4-(dimethoxymethyl)piperidine **12f**

Compound **12e** (330 mg, 0.58 mmol) and 3,6-dihydro-2*H*-pyran-4-boronic acid pinacol ester (150 mg, 0.71 mmol) were added to 12.5 mL of a mixed solvent of 1,4-dioxane and water (V/V = 4/1), and tetrakis(triphenylphosphine)palladium(0) (68 mg, 0.06 mmol) and sodium carbonate (125 mg, 1.2 mmol) were then added. The reaction was heated at 80 °C for 2 h in a nitrogen atmosphere. The reaction mixture was cooled to room temperature and concentrated under reduced pressure to remove the solvent. The residue was dissolved in ethyl acetate (30 mL), and the solution was washed with a saturated solution of sodium bicarbonate (10 mL × 3). The organic phase was dried over anhydrous sodium sulfate and filtered. The filtrate was concentrated under reduced pressure to remove the solvent. The residue was purified by silica gel column chromatography using eluent system B to give the title compound **12f** (166 mg, 50% yield).

MS m/z (ESI): 570.1[M+1].

Step 6

(5,6)-*cis*-5-(4-(4-(Dimethoxymethyl)piperidin-1-yl)-2-fluorophenyl)-6-(tetrahydro-2*H*-p yran-4-yl)-5,6,7,8-tetrahydronaphthalen-2-ol **12g**

Compound **12f** (166 mg, 0.29 mmol) was added to 15 mL of a mixed solvent of methanol and tetrahydrofuran (V/V = 2/1), and palladium hydroxide on carbon (60 mg, 20 wt%) was added. The mixture was allowed to react in a hydrogen atmosphere for 16 h. The reaction mixture was filtered. The filtrate was concentrated under reduced pressure to remove the solvent, giving the title compound **12g** (136 mg, 95% yield).
MS m/z (ESI): 484.3[M+1].

Step 7

(5*S*,6*R*)-5-(4-(4-(Dimethoxymethyl)piperidin-1-yl)-2-fluorophenyl)-6-(tetrahydro-2*H*-p yran-4-yl)-5,6,7,8-tetrahydronaphthalen-2-ol **12g-1** (5*R*,6*S*)-5-(4-(4-(Dimethoxymethyl)piperidin-1-yl)-2-fluorophenyl)-6-(tetrahydro-2*H*-p yran-4-yl)-5,6,7,8-tetrahydronaphthalen-2-ol **12g-2**

Compound **12g** (136 mg, 0.093 mmol) was resolved by preparative chiral chromatography (resolution conditions: column: CHIRALPAK IE, 250 mm × 21.2 mm, 5 μm; mobile phases: A: n-hexane, B: ethanol (+ 20 mmol $NH_3$), A: 70%, B: 30%) into the title compound **12g-1** (45 mg) and **12g-2** (45 mg).

**12g-1**

MS m/z (ESI): 484.3[M+1].
Chiral HPLC analysis method: retention time 10.91 min, chiral purity: 100% (column: CHIRALPAK IE A: n-hexane, B: EtOH (+ 0.1% DEA), A: 70%, B: 30%).

**12g-2**

MS m/z (ESI): 484.3[M+1].
Chiral HPLC analysis method: retention time 6.47 min, chiral purity: 100% (column: CHIRALPAK IE A: n-hexane, B: EtOH (+ 0.1% DEA), A: 70%, B: 30%).

Step 8

1-(3-Fluoro-4-((1*S*,2*R*)-6-hydroxy-2-(tetrahydro-2*H*-pyran-4-yl)-1,2,3,4-tetrahydronaph thalen-1-yl)phenyl)piperidine-4-carbaldehyde **12h-1** 1-(3-Fluoro-4-((1*R*,2*S*)-6-hydroxy-2-(tetrahydro-2*H*-pyran-4-yl)-1,2,3,4-tetrahydronaph thalen-1-yl)phenyl)piperidine-4-carbaldehyde **12h-2**

Compound **12g-1** (45 mg, 0.093 mol) was dissolved in tetrahydrofuran (5 mL), and dilute sulfuric acid (2 M, 0.3 mL, 0.6 mmol) was added. The mixture was allowed to react at room temperature for 16 h. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **12h-1** (25 mg, 61% yield).
MS m/z (ESI): 438.3[M+1].
Compound **12g-2** (45 mg, 0.093 mol) was dissolved in tetrahydrofuran (5 mL), and dilute sulfuric acid (2 M, 0.3 mL, 0.6 mmol) was added. The mixture was allowed to react at room temperature for 16 h. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **12h-2** (35 mg, 86% yield).
MS m/z (ESI): 438.3[M+1].

Step 9

3-(5-(4-((1-(3-Fluoro-4-((1*S*,2*R*)-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione **12-1** (a 1:1 mixture of diastereomers)
3-(5-(4-((1-(3-Fluoro-4-((1*R*,2*S*)-6-hydroxy-2-(tetrahydro-2*H*-pyran-4-yl)-1,2,3,4-tetrah ydronaphthalen-1-yl)phenyl)piperidin-4-yl)methyl)piperazin-1-yl)-1-oxoisoindolin-2-yl )piperidine-2,6-dione **12-2** (a 1:1 mixture of diastereomers)

Compound **1g** (32 mg, 0.088 mmol) was added to 5 mL of a mixed solvent of dichloromethane and methanol (V/V = 4/1), and sodium acetate (38 mg, 0.46 mmol) was added. After 10 min of reaction, compound **12h-1** (25 mg, 0.057 mmol) was added. After 15 min of reaction, sodium triacetoxyborohydride (25 mg, 0.12 mmol) was added. The mixture was allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **12-1** (25 mg, a 1:1 mixture of diastereomers, 58% yield).
MS m/z (ESI): 750.4[M+1].
$^1$H NMR (500 MHz, DMSO-$d_6$) $\delta$ 10.93 (s, 1H), 9.06 (s, 1H), 7.52 (d, 1H), 7.07-7.05 (m, 1H), 6.67-6.52 (m, 4H), 6.43 (d, 1H), 5.07-5.04 (m, 1H), 4.32, 4.23 (dd, 2H), 3.85 (d, 1H), 3.76 (d, 1H), 3.66 (brs, 2H), 3.32-3.25 (m, 8H), 3.28 (t, 1H), 3.18 (t, 1H), 2.91-2.88 (m, 2H), 2.75-2.65 (m, 1H), 2.60-2.51 (m, 3H), 2.50-2.46 (m, 3H), 2.39-2.35 (m, 2H), 2.22-2.20 (m, 2H), 1.98-1.91 (m, 2H), 1.80-1.70 (m, 4H), 1.56-1.52 (m, 2H), 1.26-1.09 (m, 5H).
Compound **1g** (45 mg, 0.12 mmol) was added to 5 mL of a mixed solvent of dichloromethane and methanol (V/V = 4/1), and sodium acetate (70 mg, 0.73 mmol) was added. After 10 min of reaction, compound **12h-2** (40 mg, 0.091 mmol) was added. After 15 min of reaction, sodium triacetoxyborohydride (40 mg, 0.19 mmol) was added. The mixture was allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **12-2** (35 mg, a 1:1 mixture of diastereomers, 51% yield).
MS m/z (ESI): 750.4[M+1].
$^1$H NMR (500 MHz, DMSO-$d_6$) $\delta$ 10.93 (s, 1H), 9.06 (s, 1H), 7.52 (d, 1H), 7.07-7.05 (m, 1H), 6.67-6.52 (m, 4H), 6.43 (d, 1H), 5.07-5.04 (m, 1H), 4.32, 4.23 (dd, 2H), 3.85 (d, 1H), 3.76 (d, 1H), 3.66 (brs, 2H), 3.32-3.25 (m, 8H), 3.28 (t, 1H), 3.18 (t, 1H), 2.91-2.88 (m, 2H), 2.75-2.65 (m, 1H), 2.60-2.51 (m, 3H), 2.50-2.46 (m, 3H), 2.39-2.35 (m, 2H), 2.22-2.20 (m, 2H), 1.98-1.91 (m, 2H), 1.80-1.70 (m, 4H), 1.56-1.52 (m, 2H), 1.26-1.09 (m, 5H).

Examples 13-1 and 13-2

(*S*)-3-(5-(4-((1-(4-((1*R*,2*R*)-2-Cyclobutyl-6-hydroxy-1,2,3,4-tetrahydronaphthalen-1-yl) phenyl)piperidin-4-yl)methyl)piperazin-1-yl)-1-oxoisoindolin-2-yl)piperidine-2,6-dione **13-1** 3-(5-(4-((1-(4-((1*S*,2*S*)-2-Cyclobutyl-6-hydroxy-1,2,3,4-tetrahydronaphthalen-1-yl)phen yl)piperidin-4-yl)methyl)piperazin-1-yl)-1-oxoisoindolin-2-yl)piperidine-2,6-dione **13-2** (a 1:1 mixture of diastereomers)

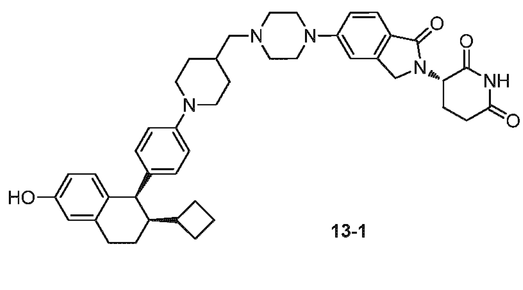

**13-1**

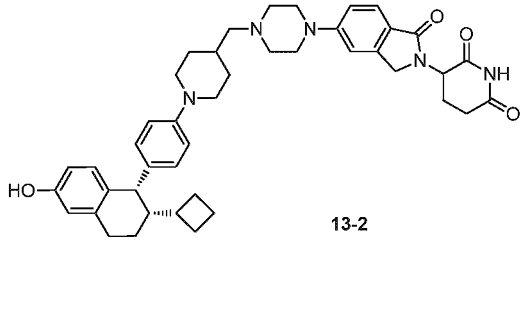

**13-2**

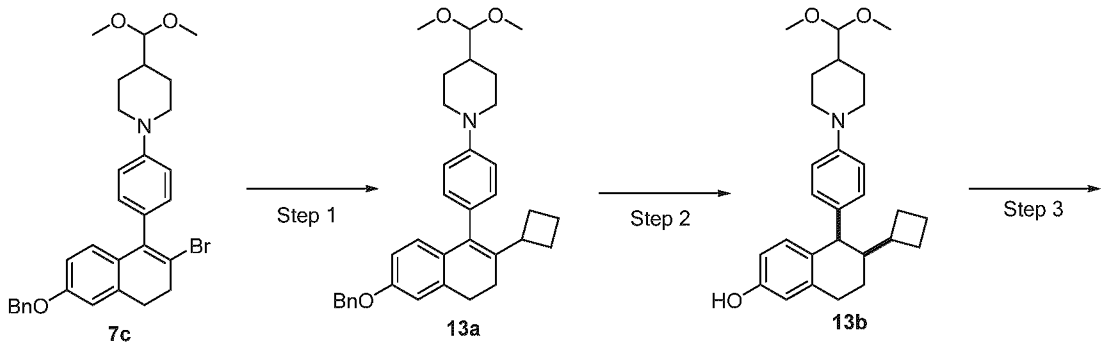


7c 13a 13b

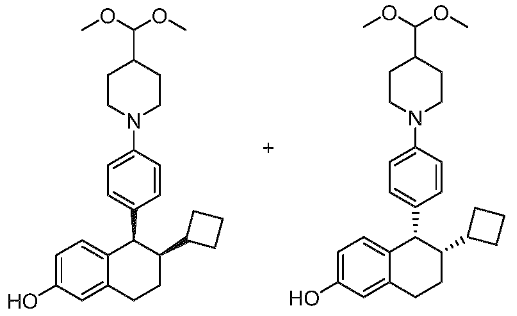

13b-1 13b-2

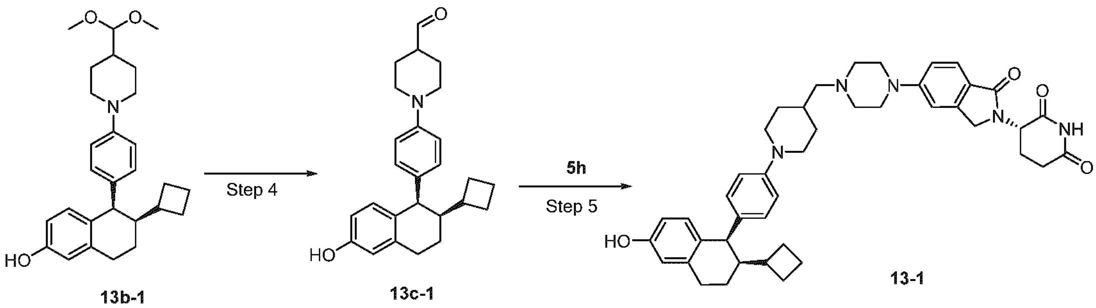


13b-1 13c-1 13-1

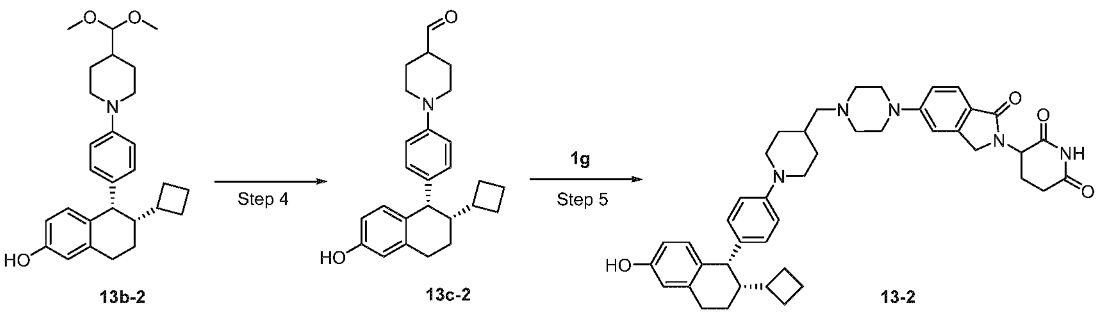


13b-2 13c-2 13-2

Step 1

1-(4-(6-(Benzyloxy)-2-cyclobutyl-3,4-dihydronaphthalen-1-yl)phenyl)-4-(dimethoxyme thyl)piperidine **13a**

Smoothed magnesium ribbon (200 mg, 8.2 mmol) was cut into pieces, and tetrahydrofuran (5 mL) was added. In another reaction flask, bromocyclobutane (800 mg, 5.9 mmol, Shanghai Accela ChemBio Co., Ltd.) was dissolved in tetrahydrofuran (5 mL). In a nitrogen atmosphere, the solution of bromocyclobutane (0.5 mL) was added to the solution containing magnesium ribbon, and 1,2-dibromoethane (5 drops) was added to initiate a reaction. The solution of bromocyclobutane (4.5 mL) was slowly added dropwise. After the dropwise addition, the mixture was allowed to react at room temperature for 2 h. The reaction mixture was cooled to 0 °C in an ice bath, and a solution of zinc chloride in tetrahydrofuran (1 M, 6.5 mL, 6.5 mmol) was added dropwise. After the dropwise addition, the mixture was allowed to react at room temperature for 2 h. Compound **7c** (300 mg, 0.55 mmol) and [1,1'-bis(diphenylphosphino)ferrocene]palladium(II) dichloride (200 mg, 0.27 mmol) were added, and the mixture was allowed to react at room temperature for 16 h. A saturated solution of ammonium chloride (15 mL) was added, and the mixture was extracted with dichloromethane (15 mL × 3). The organic phases were combined, dried over anhydrous sodium sulfate and filtered. The filtrate was concentrated under reduced pressure to remove the solvent. The residue was purified by silica gel column chromatography using eluent system B to give the title compound **13a** (150 mg, 52% yield).
MS m/z (ESI): 524.3[M+1].

Step 2

(5,6)-*cis*-6-Cyclobutyl-5-(4-(4-(dimethoxymethyl)piperidin-1-yl)phenyl)-5,6,7,8-tetrahy dronaphthalen-2-ol **13b**

Compound **13a** (150 mg, 0.29 mmol) was added to methanol (10 mL), and palladium hydroxide on carbon (150 mg, 20 wt%) was added. The mixture was allowed to react in a hydrogen atmosphere for 16 h. The reaction mixture was filtered. The filtrate was concentrated under reduced pressure to remove the solvent, giving the title compound **13b** (100 mg, 80% yield).
MS m/z (ESI): 436.3[M+1].

Step 3

(5*R*,6*R*)-6-Cyclobutyl-5-(4-(4-(dimethoxymethyl)piperidin-1-yl)phenyl)-5,6,7,8-tetrahy dronaphthalen-2-ol **13b-1**
(5*S*,6*S*)-Cyclobutyl-5-(4-(4-(dimethoxymethyl)piperidin-1-yl)phenyl)-5,6,7,8-tetrahyd ronaphthalen-2-ol **13b-2**

Compound **13b** (100 mg, 0.23 mmol) was resolved by preparative chiral chromatography (resolution conditions: column: CHIRALPAK IE, 20 mm × 250 mm, 5 μm; mobile phases: A: n-hexane, B: ethanol (+ 20 mmol $NH_3$), A: 70%, B: 30%, flow rate: 20 mL/min) into the title compounds **13b-1** (40 mg) and **13b-2** (50 mg).

**13b-1**

MS m/z (ESI): 436.3[M+1].
Chiral HPLC analysis method: retention time 7.43 min, chiral purity: 100% (column: CHIRALPAK IE A: n-hexane, B: EtOH (+ 0.1% DEA), A: 70%, B: 30%).

**13b-2**

MS m/z (ESI): 436.3[M+1].
Chiral HPLC analysis method: retention time 3.88 min, chiral purity: 100% (column: CHIRALPAK IE A: n-hexane, B: EtOH (+ 0.1% DEA), A: 70%, B: 30%).

Step 4

1-(4-((1*R*,2*R*)-2-Cyclobutyl-6-hydroxy-1,2,3,4-tetrahydronaphthalen-1-yl)phenyl)piperi dine-4-carbaldehyde **13c-1** 1 -(4-((1*S*,2*S*)-2-Cyclobutyl-6-hydroxy-1,2,3,4-tetrahydronaphthalen-1-yl)phenyl)piperi dine-4-carbaldehyde **13c-2**

Compound **13b-1** (40 mg, 0.092 mol) was dissolved in tetrahydrofuran (5 mL), and dilute sulfuric acid (2 M, 0.2 mL, 0.4 mmol) was added. The mixture was allowed to react at 60 °C for 1 h. The reaction mixture was cooled to room temperature, made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **13c-1** (35 mg, 97% yield).
MS m/z (ESI): 390.3[M+1].
Compound **13b-2** (50 mg, 0.12 mol) was dissolved in tetrahydrofuran (5 mL), and dilute sulfuric acid (2 M, 0.2 mL, 0.4 mmol) was added. The mixture was allowed to react at 60 °C for 1 h. The reaction mixture was cooled to room temperature, made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **13c-2** (44 mg, 98% yield).
MS m/z (ESI): 390.3[M+1].

Step 5

(*S*)-3-(5-(4-((1-(4-((1*R*,2*R*)-2-Cyclobutyl-6-hydroxy-1,2,3,4-tetrahydronaphthalen-1-yl) phenyl)piperidin-4-yl)methyl)piperazin-1-yl)-1-oxoisoindolin-2-yl)piperidine-2,6-dione **13-1** 3-(5-(4-((1-(4-((1*S*,2*S*)-2-Cyclobutyl-6-hydroxy-1,2,3,4-tetrahydronaphthalen-1-yl)phen yl)piperidin-4-yl)methyl)piperazin-1-yl)-1-oxoisoindolin-2-yl)piperidine-2,6-dione **13-2** (a 1:1 mixture of diastereomers)

Compound **5h** (48 mg, 0.15 mmol) was added to 5 mL of a mixed solvent of dichloromethane and methanol (V/V = 4/1), and sodium acetate (98 mg, 1.2 mmol) was added. After 10 min of reaction, compound **13c-1** (35 mg, 0.09 mmol) was added. After 15 min of reaction, sodium triacetoxyborohydride (48 mg, 0.23 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **13-1** (23 mg, 29% yield).

MS m/z (ESI): 702.4[M+1].

$^1$H NMR (500 MHz, DMSO-$d_6$) $\delta$ 10.95 (bs, 1H), 9.02 (s, 1H), 7.53 (d, 1H), 7.12-7.01 (m, 2H), 6.76, 6.69 (dd, 4H), 6.62 (d, 1H), 6.52 (d, 1H), 6.43-6.41 (m, 1H), 5.07-5.03(m, 1H), 4.32, 4.22 (dd, 2H), 3.89 (d, 1H), 3.66-3.55 (m, 2H), 3.38-3.21 (m, 8H), 2.96-2.67 (m, 4H), 2.67-2.55 (m, 3H), 2.42-2.31 (m, 1H), 2.22 (d, 2H), 2.15-2.05 (m, 1H), 2.01-1.92 (m, 1H), 1.90-1.57 (m, 8H), 1.52-1.44 (m, 1H), 1.38-1.14 (m, 4H).

Compound **1g** (45 mg, 0.12 mmol) was added to 5 mL of a mixed solvent of dichloromethane and methanol (V/V = 4/1), and sodium acetate (98 mg, 1.2 mmol) was added. After 10 min of reaction, compound **13c-2** (44 mg, 0.11 mmol) was added. After 15 min of reaction, sodium triacetoxyborohydride (48 mg, 0.23 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **13-2** (23 mg, a 1:1 mixture of diastereomers, 36% yield).

MS m/z (ESI): 702.5[M+1].

$^1$H NMR (500 MHz, DMSO-$d_6$) $\delta$ 10.95 (bs, 1H), 9.03 (s, 1H), 7.53 (d, 1H), 7.12-7.01 (m, 2H), 6.76, 6.69 (dd, 4H), 6.62 (d, 1H), 6.52 (d, 1H), 6.43-6.41 (m, 1H), 5.07-5.03(m, 1H), 4.32, 4.22 (dd, 2H), 3.89 (d, 1H), 3.66-3.55 (m, 2H), 3.38-3.21 (m, 8H), 2.96-2.67 (m, 4H), 2.67-2.55 (m, 3H), 2.42-2.31 (m, 1H), 2.22 (d, 2H), 2.15-2.05 (m, 1H), 2.01-1.92 (m, 1H), 1.90-1.57 (m, 8H), 1.52-1.44 (m, 1H), 1.38-1.14 (m, 4H).

Examples 14-1 and 14-2

3-(5-(4-((1-(4-((1*R*,2*R*)-2-Cyclopentyl-6-hydroxy-1,2,3,4-tetrahydronaphthalen-1-yl)ph enyl)piperidin-4-yl)methyl)piperazin-1-yl)-1-oxoisoindolin-2-yl)piperidine-2,6-dione **14-1** (a 1:1 mixture of diastereomers) 3-(5-(4-((1-(4-((1*S*,2*S*)-2-Cyclopentyl-6-hydroxy-1,2,3,4-tetrahydronaphthalen-1-yl)phe nyl)piperidin-4-yl)methyl)piperazin-1-yl)-1-oxoisoindolin-2-yl)piperidine-2,6-dione **14-2** (a 1:1 mixture of diastereomers)

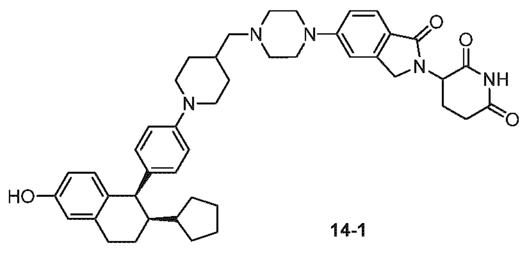

**14-1**

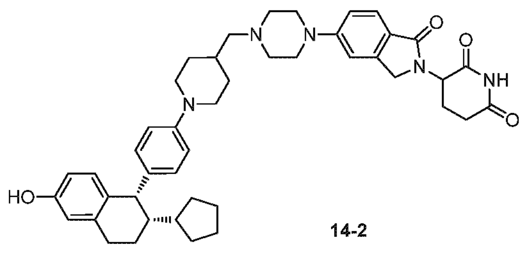

**14-2**

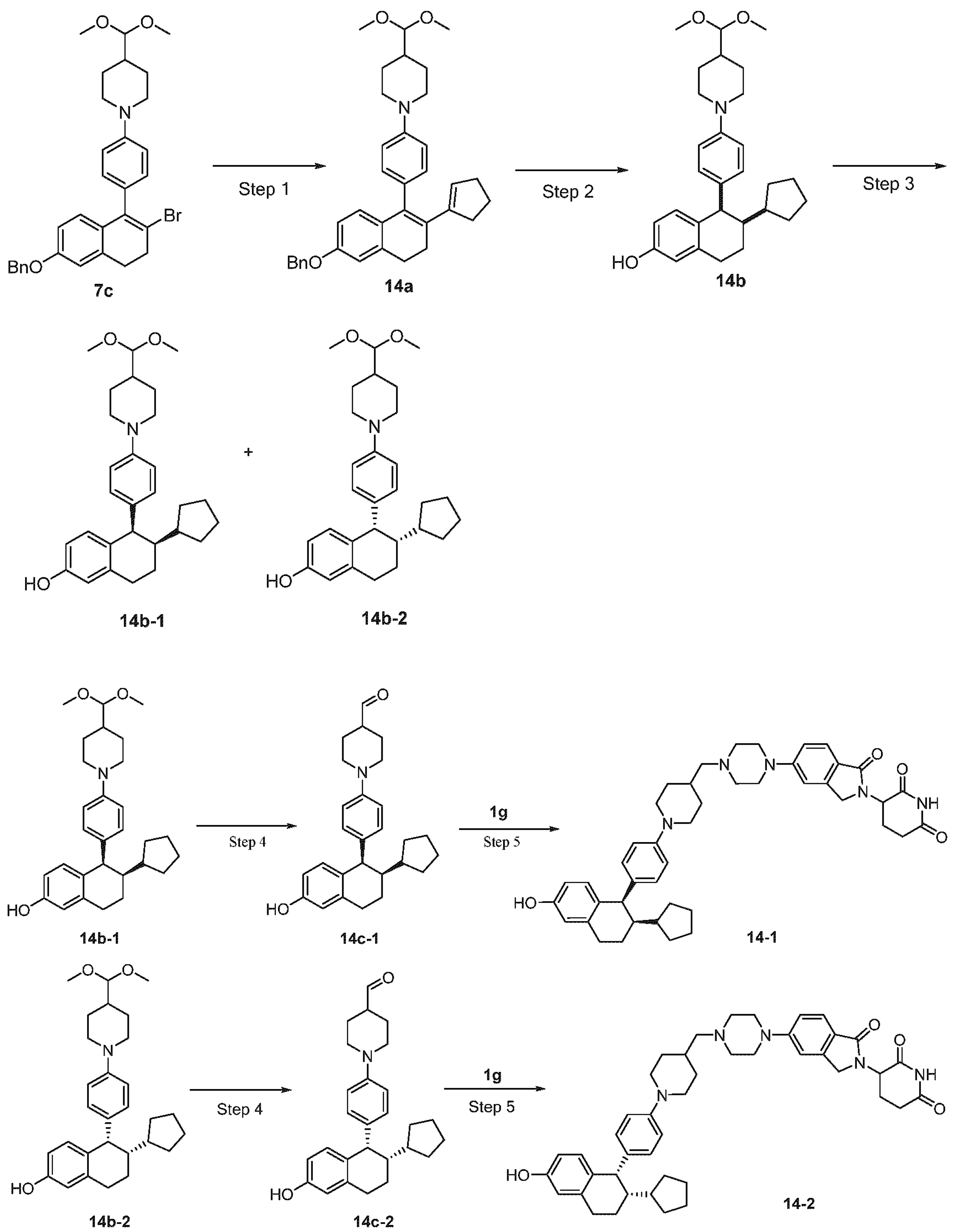


Step 1

1-(4-(6-(Benzyloxy)-2-(cyclopent-1-en-1-yl)-3,4-dihydronaphthalen-1-yl)phenyl)-4-(di methoxymethyl)piperidine **14a**

Compound **7c** (250 mg, 0.46 mmol) and cyclopentene-1-boronic acid pinacol ester (135 mg, 0.56 mmol) were added to 6 mL of a mixed solvent of 1,4-dioxane and water (V/V = 5/1), and tetrakis(triphenylphosphine)palladium(0) (53 mg, 0.046 mmol) and sodium carbonate (100 mg, 0.94 mmol) were added. The mixture was allowed to react at 80 °C for 2 h in a nitrogen atmosphere. The reaction mixture was cooled to room temperature and concentrated under reduced pressure to remove the solvent. The residue was dissolved in ethyl acetate (30 mL), and the solution was washed with a saturated solution of sodium bicarbonate (10 mL × 3). The organic phase was dried over anhydrous sodium sulfate and filtered. The filtrate was concentrated under reduced pressure to remove the solvent. The residue

was purified by silica gel column chromatography using eluent system A to give the title compound **14a** (110 mg, 45% yield).

MS m/z (ESI): 536.3[M+1].

Step 2

(5,6)-*cis*-6-Cyclopentyl-5-(4-(4-(dimethoxymethyl)piperidin-1-yl)phenyl)-5,6,7,8-tetrah ydronaphthalen-2-ol **14b**

Compound **14a** (110 mg, 0.21 mmol) was added to methanol (10 mL), and palladium hydroxide on carbon (100 mg, 20 wt%) was added. The mixture was allowed to react in a hydrogen atmosphere for 16 h. The reaction mixture was filtered. The filtrate was concentrated under reduced pressure to remove the solvent, giving the title compound **14b** (80 mg, 85% yield).

MS m/z (ESI): 450.2[M+1].

Step 3

(S*R*,6*R*)-6-Cyclopentyl-5-(4-(4-(dimethoxymethyl)piperidin-1-yl)phenyl)-5,6,7,8-tetrah ydronaphthalen-2-ol **14b-1**
(5*S*,6*S*)-6-Cyclopentyl-5-(4-(4-(dimethoxymethyl)piperidin-1-yl)phenyl)-5,6,7,8-tetrahy dronaphthalen-2-ol **14b-2**

Compound **14b** (80 mg, 0.18 mmol) was resolved by preparative chiral chromatography (resolution conditions: column: CHIRALPAK IE, 20 mm × 250 mm, 5 μm; mobile phases: A: n-hexane, B: ethanol (+ 20 mmol $NH_3$), A: 80%, B: 20%) into the title compound **14b-1** (20 mg) and **14b-2** (20 mg).

**14b-1**

MS m/z (ESI): 450.2[M+1].
Chiral HPLC analysis method: retention time 8.12 min, chiral purity: 100% (column: CHIRALPAK IE A: n-hexane, B: EtOH (+ 0.1% DEA), A: 80%, B: 20%).

**14b-2**

MS m/z (ESI): 450.2[M+1].
Chiral HPLC analysis method: retention time 6.61 min, chiral purity: 100% (column: CHIRALPAK IE A: n-hexane, B: EtOH (+ 0.1% DEA), A: 80%, B: 20%).

Step 4

1-(4-((1*R*,2*R*)-2-Cyclopentyl-6-hydroxy-1,2,3,4-tetrahydronaphthalen-1-yl)phenyl)piper idine-4-carbaldehyde **14c-1**
1-(4-((1*S*,2*S*)-2-Cyclopentyl-6-hydroxy-1,2,3,4-tetrahydronaphthalen-1-yl)phenyl)piperi dine-4-carbaldehyde **14c-2**

Compound **14b-1** (20 mg, 0.044 mol) was dissolved in tetrahydrofuran (1.5 mL), and dilute sulfuric acid (2 M, 0.06 mL, 0.12 mmol) was added. The mixture was allowed to react at 60 °C for 1 h. The reaction mixture was made neutral with a saturated solution of sodium bicarbonate and extracted with ethyl acetate (8 mL × 3). The organic phases were combined, dried over anhydrous sodium sulfate and filtered. The filtrate was concentrated under reduced pressure to remove the solvent, giving the title compound **14c-1** (18 mg, 100% yield).

MS m/z (ESI): 404.3[M+1].

Compound **14b-2** (20 mg, 0.044 mmol) was dissolved in tetrahydrofuran (1.5 mL), and dilute sulfuric acid (2 M, 0.06 mL, 0.12 mmol) was added. The mixture was allowed to react at 60 °C for 1 h. The reaction mixture was made neutral with a saturated solution of sodium bicarbonate and extracted with ethyl acetate (8 mL × 3). The organic phases were combined, dried over anhydrous sodium sulfate and filtered. The filtrate was concentrated under reduced pressure to remove the solvent, giving the title compound **14c-2** (18 mg, 100% yield).

MS m/z (ESI): 404.3[M+1].

Step 5

3-(5-(4-((1-(4-((1*R*,2*R*)-2-Cyclopentyl-6-hydroxy-1,2,3,4-tetrahydronaphthalen-1-yl)ph enyl)piperidin-4-yl)methyl)piperazin-1-yl)-1-oxoisoindolin-2-yl)piperidine-2,6-dione **14-1** (a 1:1 mixture of diastereomers) 3-(5-(4-((1-(4-((1*S*,2*S*)-2-Cyclopentyl-6-hydroxy-1,2,3,4-tetrahydronaphthalen-1-yl)phe nyl)piperidin-4-yl)methyl)piperazin-1-yl)-1-oxoisoindolin-2-yl)piperidine-2,6-dione **14-2** (a 1:1 mixture of diastereomers)

Compound **1g** (30 mg, 0.082 mmol) was added to 5 mL of a mixed solvent of dichloromethane and methanol (V/V = 4/1), and sodium acetate (100 mg, 1.22 mmol) was added. After 10 min of reaction, compound **14c-1** (18 mg, 0.044 mmol) was added. After 15 min of reaction, sodium triacetoxyborohydride (20 mg, 0.094 mmol) was added. The mixture was allowed to react at room temperature for 1 h. The reaction mixture was concentrated under reduced pressure to remove the solvent. The residue was purified by preparative high performance liquid chromatography (Gilson GX281; column: SharpSil-T; elution system: 10 mM ammonium bicarbonate, 60% water, 40% acetonitrile) to give the title compound **14-1** (23 mg, a 1:1 mixture of diastereomers, 47% yield).

MS m/z (ESI): 716.5[M+1].

$^1$H NMR (500 MHz, DMSO-$d_6$) $\delta$ 10.95 (bs, 1H), 9.02 (s, 1H), 7.53 (d, 1H), 7.12-7.02 (m, 2H), 6.82, 6.77 (dd, 4H), 6.62 (d, 1H), 6.51 (d, 1H), 6.42-6.40 (m, 1H), 5.07-5.03 (m, 1H), 4.32, 4.22 (dd, 2H), 3.94 (d, 1H), 3.66-3.55 (m, 2H), 3.39-3.21 (m, 8H), 2.96-2.90 (m, 3H), 2.63-2.54 (m, 4H), 2.41-2.33 (m, 1H), 2.22 (d, 2H), 2.07-1.92 (m, 2H), 1.85-1.77 (m, 2H), 1.73-1.44 (m, 7H), 1.33-1.15 (m, 5H), 1.10-1.00 (m, 1H).

Compound **1g** (30 mg, 0.082 mmol) was added to 5 mL of a mixed solvent of dichloromethane and methanol (V/V = 4/1), and sodium acetate (100 mg, 1.22 mmol) was added. After 10 min of reaction, compound **14c-2** (18 mg, 0.044 mmol) was added. After 15 min of reaction, sodium triacetoxyborohydride (20 mg, 0.094 mmol) was added. The mixture was allowed to react at room temperature for 1 h. The reaction mixture was concentrated under reduced pressure to remove the solvent. The residue was purified by preparative high performance liquid chromatography (Gilson GX281; column: SharpSil-T; elution system: 10 mM ammonium bicarbonate, 60% water, 40% acetonitrile) to give the title compound **14-2** (2 mg, a 1:1 mixture of diastereomers, 6% yield).

MS m/z (ESI): 716.4[M+1].

$^1$H NMR (500 MHz, DMSO-$d_6$) $\delta$ 10.95 (bs, 1H), 9.02 (s, 1H), 7.53 (d, 1H), 7.12-7.02 (m, 2H), 6.82, 6.77 (dd, 4H), 6.62 (d, 1H), 6.51 (d, 1H), 6.42-6.40 (m, 1H), 5.07-5.03 (m, 1H), 4.32, 4.22 (dd, 2H), 3.94 (d, 1H), 3.66-3.55 (m, 2H), 3.39-3.21 (m, 8H), 2.96-2.90 (m, 3H), 2.63-2.54 (m, 4H), 2.41-2.33 (m, 1H), 2.22 (d, 2H), 2.07-1.92 (m, 2H), 1.85-1.77 (m, 2H), 1.73-1.44 (m, 7H), 1.33-1.15 (m, 5H), 1.10-1.00 (m, 1H).

Examples 15-1 and 15-2

3-(5-(4-((1-(4-((1*R*,2*R*)-2-Cyclohexyl-6-hydroxy-1,2,3,4-tetrahydronaphthalen-1-yl)phe nyl)piperidin-4-yl)methyl)piperazin-1-yl)-1-oxoisoindolin-2-yl)piperidine-2,6-dione **15-1** (a 1:1 mixture of diastereomers) 3-(5-(4-((1-(4-((1*S*,2*S*)-2-Cyclohexyl-6-hydroxy-1,2,3,4-tetrahydronaphthalen-1-yl)phe nyl)piperidin-4-yl)methyl)piperazin-1-yl)-1-oxoisoindolin-2-yl)piperidine-2,6-dione **15-2** (a 1:1 mixture of diastereomers)

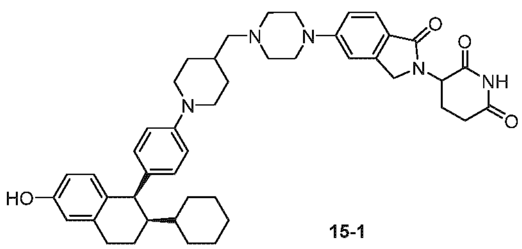

**15-1**

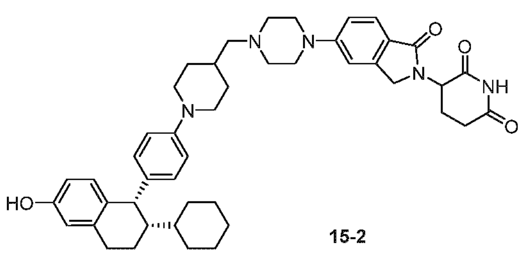

**15-2**

By using the synthesis schemes of Examples 14-1 and 14-2 in which cyclohexene-1-boronic acid pinacol ester was used in place of the starting material cyclopentene-1-boronic acid pinacol ester of step 1, the title compounds **15-1** (30 mg, a 1:1 mixture of diastereomers) and **15-2** (30 mg, a 1:1 mixture of diastereomers) were prepared.

**15-1**

MS m/z (ESI): 730.4[M+1].

$^1$H NMR (500 MHz, DMSO-$d_6$) $\delta$ 10.94 (bs, 1H), 9.02 (s, 1H), 7.52 (d, 1H), 7.11-7.01 (m, 2H), 6.87-6.73 (m, 4H), 6.60 (d, 1H), 6.50 (d, 1H), 6.41-6.39 (m, 1H), 5.07-5.03 (m, 1H), 4.32, 4.22 (dd, 2H), 4.05 (d, 1H), 3.63 (t, 2H), 3.37-3.22 (m, 6H), 2.94-2.81 (m, 2H), 2.75-2.65 (m, 1H), 2.62-2.53 (m, 3H), 2.42-2.29 (m, 1H), 2.27-2.15 (m, 2H), 2.12-2.03 (m, 1H), 2.02-1.87 (m, 1H), 1.85-1.75 (m, 2H), 1.74-1.61 (m, 3H), 1.60-1.41 (m, 5H), 1.30-0.70 (m, 10H).

**15-2**

MS m/z (ESI): 730.4[M+1].

$^1$H NMR (500 MHz, DMSO-$d_6$) $\delta$ 10.94 (bs, 1H), 9.02 (s, 1H), 7.52 (d, 1H), 7.11-7.01 (m, 2H), 6.87-6.73 (m, 4H), 6.60 (d, 1H), 6.50 (d, 1H), 6.41-6.39 (m, 1H), 5.07-5.03 (m, 1H), 4.32, 4.22 (dd, 2H), 4.05 (d, 1H), 3.63 (t, 2H), 3.37-3.22 (m, 6H), 2.94-2.81 (m, 2H), 2.75-2.65 (m, 1H), 2.62-2.53 (m, 3H), 2.42-2.29 (m, 1H), 2.27-2.15 (m, 2H), 2.12-2.03 (m, 1H), 2.02-1.87 (m, 1H), 1.85-1.75 (m, 2H), 1.75-1.61 (m, 3H), 1.61-1.41 (m, 5H), 1.30-0.70 (m, 10H).

Example 16

3-(5-(4-((1-(4-((1,2)-*cis*-2-Cyclopropyl-6-hydroxy-1,2,3,4-tetrahydronaphthalen-1-yl)ph enyl)piperidin-4-yl)methyl)piperazin-1-yl)-1-oxoisoindolin-2-yl)piperidine-2,6-dione **16**

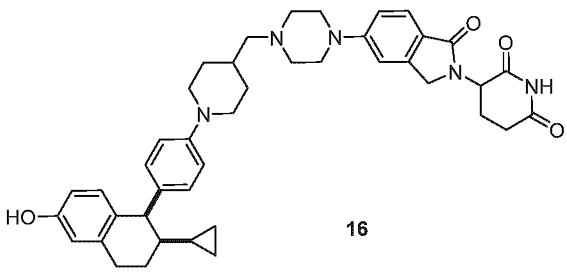

**16**

By using the synthesis scheme of Example **7** in which cyclopropylboronic acid was used in place of the starting material 3,6-dihydro-2*H*-pyran-4-boronic acid of step 4, the title compound **16** (13 mg) was prepared.

MS m/z (ESI): 688.3[M+1].

$^1$H NMR (500 MHz, DMSO-$d_6$) $\delta$ 10.94 (bs, 1H), 9.02 (s, 1H), 7.51 (d, 1H), 7.08-7.03 (m, 2H), 6.84-6.73 (m, 4H), 6.62 (d, 1H), 6.50 (d, 1H), 6.43-6.01 (m, 1H), 5.07-5.03 (m, 1H), 4.32, 4.22 (dd, 2H), 3.96 (d, 1H), 3.63-3.56 (m, 2H), 3.37-3.22 (m, 6H), 2.94-2.77 (m, 2H), 2.75-2.65 (m, 1H), 2.64-2.53 (m, 3H), 2.50-2.45 (m, 2H), 2.42-2.29 (m, 1H), 2.27-2.15 (m, 2H), 2.04-1.91 (m, 1H), 1.85-1.75 (m, 2H), 1.71-1.57 (m, 3H), 1.33-1.13 (m, 2H), 1.01-0.91 (m, 1H), 0.44-0.37 (m, 1H), 0.35-0.29 (m, 1H), 0.27-0.19 (m, 1H), 0.10-0.01 (m, 2H).

Examples 17-1 and 17-2

(*S*)-3-(5-(4-((1-(4-(((1*R*,2*R*)-2-Cyclopropyl-6-hydroxy-1,2,3,4-tetrahydronaphthalen-1-y l)phenyl))piperidin-4-yl)methyl)piperazin-1-yl)-1-oxoisoindolin-2-yl)piperidine-2,6-dio ne **17-1** (*S*)-3-(5-(4-((1-(4-(((1*S*,2*S*)-2-Cyclopropyl-6-hydroxy-1,2,3,4-tetrahydronaphthalen-1-yl )phenyl))piperidin-4-yl)methyl)piperazin-1-yl)-1-oxoisoindolin-2-yl)piperidine-2,6-dion e **17-2**

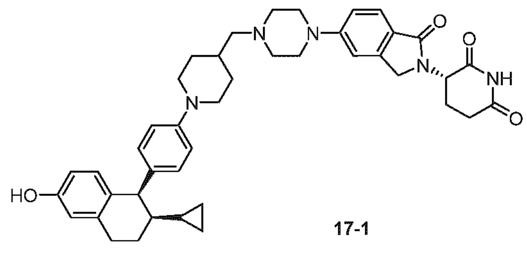

17-1

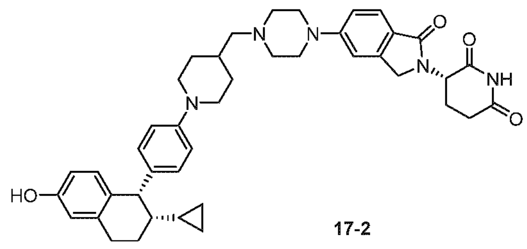

17-2

By using the synthesis schemes of Examples 14-1 and 14-2 in which cyclopropylboronic acid was used in place of the starting material cyclopentene-1-boronic acid pinacol ester of step 1 and **5h** in place of the starting material **1g** of step 5, the title compounds **17-1** (70 mg) and **17-2** (80 mg) were prepared.

**17-1**

MS m/z (ESI): 688.3[M+1].
$^1$H NMR (500 MHz, DMSO-$d_6$) $\delta$ 10.94 (bs, 1H), 9.02 (s, 1H), 7.51 (d, 1H), 7.08-7.03 (m, 2H), 6.84-6.73 (m, 4H), 6.62 (d, 1H), 6.50 (d, 1H), 6.43-6.01 (m, 1H), 5.07-5.03 (m, 1H), 4.32, 4.22 (dd, 2H), 3.96 (d, 1H), 3.63-3.56 (m, 2H), 3.37-3.22 (m, 6H), 2.94-2.77 (m, 2H), 2.75-2.65 (m, 1H), 2.64-2.53 (m, 3H), 2.50-2.45 (m, 2H), 2.42-2.29 (m, 1H), 2.27-2.15 (m, 2H), 2.04-1.91 (m, 1H), 1.85-1.75 (m, 2H), 1.71-1.57 (m, 3H), 1.33-1.13 (m, 2H), 1.01-0.91 (m, 1H), 0.44-0.37 (m, 1H), 0.37-0.29 (m, 1H), 0.27-0.19 (m, 1H), 0.10-0.01 (m, 2H).

**17-2**

MS m/z (ESI): 688.3[M+1].
$^1$H NMR (500 MHz, DMSO-$d_6$) $\delta$ 10.94 (bs, 1H), 9.02 (s, 1H), 7.51 (d, 1H), 7.08-7.03 (m, 2H), 6.84-6.73 (m, 4H), 6.62 (d, 1H), 6.50 (d, 1H), 6.43-6.01 (m, 1H), 5.07-5.03 (m, 1H), 4.32, 4.22 (dd, 2H), 3.96 (d, 1H), 3.63-3.56 (m, 2H), 3.37-3.22 (m, 6H), 2.94-2.77 (m, 2H), 2.75-2.65 (m, 1H), 2.64-2.53 (m, 3H), 2.50-2.45 (m, 2H), 2.42-2.29 (m, 1H), 2.27-2.15 (m, 2H), 2.04-1.91 (m, 1H), 1.85-1.75 (m, 2H), 1.71-1.57 (m, 3H), 1.33-1.13 (m, 2H), 1.01-0.91 (m, 1H), 0.44-0.37 (m, 1H), 0.35-0.29 (m, 1H), 0.27-0.19 (m, 1H), 0.10-0.01 (m, 2H).

Examples 18-1 and 18-2

(*S*)-3-(5-(4-((1-(4-((1*R*,2*R*)-2-Cyclobutyl-6-hydroxy-1,2,3,4-tetrahydronaphthalen-1-yl)-2-fluorophenyl)piperidin-4-yl)methyl)piperazin-1 -yl)-1 -oxoisoindolin-2-yl)piperidine-2, 6-dione **18-1** (*S*)-3-(5-(4-((1-(4-((1*S*,2*S*)-2-Cyclobutyl-6-hydroxy-1,2,3,4-tetrahydronaphthalen-1-yl)-2-fluorophenyl)piperidin-4-yl)methyl)piperazin-1 -yl)-1 -oxoisoindolin-2-yl)piperidine-2, 6-dione **18-2**

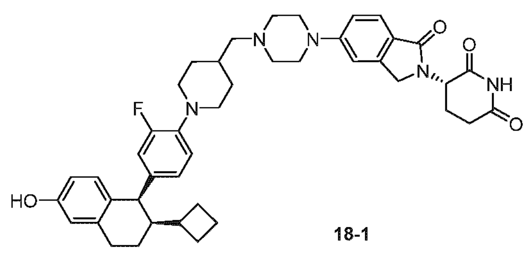

18-1

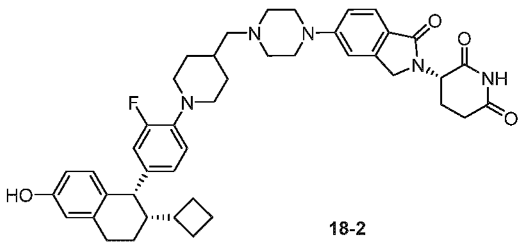

18-2

By using the synthesis scheme of Example 13-1 in which **31** was used in place of the starting material **7c** of step 1, the title compound **18-1** (13 mg) was prepared.

By using the synthesis scheme of Example 13-2 in which **31** was used in place of the starting material **7c** of step 1 and 5h in place of the starting material of step 5, the title compound **18-2** (10 mg) was prepared.

**18-1**

MS m/z (ESI): 720.4[M+1].

$^1$H NMR (500 MHz, DMSO-$d_6$) $\delta$ 10.95 (bs, 1H), 9.09 (s, 1H), 7.53 (d, 1H), 7.12-7.01 (m, 2H), 6.89 (t, 1H), 6.63 (d, 2H), 6.57-6.41 (m, 3H), 5.07-5.03 (m, 1H), 4.32, 4.22 (dd, 2H), 3.95 (d, 1H), 3.39-3.24 (m, 10H), 2.96-2.69 (m, 3H), 2.67-2.56 (m, 3H), 2.41-2.32 (m, 1H), 2.24 (bs, 2H), 2.13-2.05 (m, 1H), 2.02-1.93 (m, 1H), 1.91-1.57 (m, 9H), 1.56-1.46(m, 1H), 1.35-1.17 (m, 4H).

**18-2**

MS m/z (ESI): 720.4[M+1].

$^1$H NMR (500 MHz, DMSO-$d_6$) $\delta$10.95 (bs, 1H), 9.09 (s, 1H), 7.53 (d, 1H), 7.12-7.01 (m, 2H), 6.89 (t, 1H), 6.63 (d, 2H), 6.57-6.41 (m, 3H), 5.07-5.03 (m, 1H), 4.32, 4.22 (dd, 2H), 3.95 (d, 1H), 3.39-3.24 (m, 10H), 2.96-2.69 (m, 3H), 2.67-2.56 (m, 3H), 2.41-2.32 (m, 1H), 2.24 (bs, 2H), 2.13-2.05 (m, 1H), 2.02-1.93 (m, 1H), 1.91-1.57 (m, 9H), 1.56-1.46(m, 1H), 1.35-1.17 (m, 4H).

Example 19

(*S*)-3-(5-(4-((1-(4-((1*R*,2*R*)-2-cyclopentyl-6-hydroxy-1,2,3,4-tetrahydronaphthalen-1-yl) -2-fluorophenyl)piperidin-4-yl)methyl)piperazin-1-yl)-1-oxoisoindolin-2-yl)piperidine-2,6-dione **19**

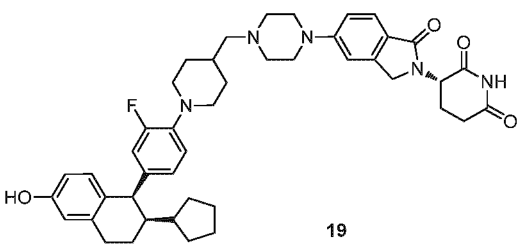

19

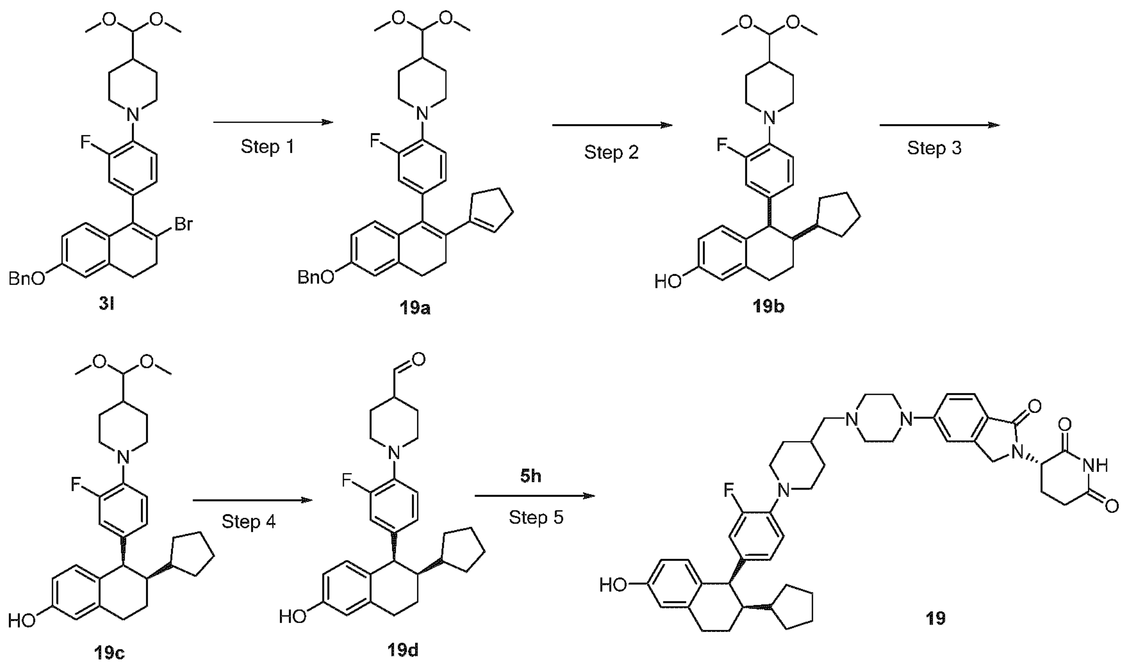


Step 1

1-(4-(6-(Benzyloxy)-2-(cyclopent-1-en-1-yl)-3,4-dihydronaphthalen-1-yl)-2-fluorophen yl)-4-(dimethoxymethyl)piperidine **19a**

Compound **3l** (300 mg, 0.53 mmol), cyclopentene-1-boronic acid pinacol ester (309 mg, 1.3 mmol, Shanghai Bide Pharmatech Ltd.), tetrakis(triphenylphosphine)palladium(0) (62 mg, 0.05 mol) and sodium carbonate (169 mg, 1.6 mmol) were added to 6 mL of a mixed solvent of 1,4-dioxane and water (V/V = 5/1). The reaction was heated at 80 °C for 4 h in a nitrogen atmosphere. The reaction mixture was cooled to room temperature, diluted with water (25 mL) and extracted with ethyl acetate (25 mL × 3). The organic phases were combined, dried over anhydrous sodium sulfate and filtered. The filtrate was concentrated under reduced pressure to remove the solvent. The residue was purified by silica gel column chromatography using eluent system B to give the title compound **19a** (220 mg, 75% yield).

MS m/z (ESI): 554.3[M+1].

Step 2

(5,6)-*cis*-6-Cyclopentyl-5-(4-(4-(dimethoxymethyl)piperidin-1-yl)-3-fluorophenyl)-5,6, 7,8-tetrahydronaphthalen-2-ol **19b**

Compound **19a** (220 mg, 0.40 mmol) was dissolved in 12 mL of a mixed solvent of methanol and tetrahydrofuran (V/V = 5/1), and palladium on carbon (97 mg, 10 wt%) was added. The mixture was allowed to react at 50 °C for 24 h and at 80 °C for 2 h in a hydrogen atmosphere. The reaction mixture was filtered through celite. The filtrate was concentrated under reduced pressure to remove the solvent, giving the title compound **19b** (180 mg, crude). The product was directly used in the next step without purification.

MS m/z (ESI): 468.2[M+1].

Step 3

(5*R*,6*R*)-6-Cyclopentyl-5-(4-(4-(dimethoxymethyl)piperidin-1-yl)-3-fluorophenyl)-5,6,7 ,8-tetrahydronaphthalen-2-ol **19c**

Compound **19b** (180 mg, 0.34 mmol) was resolved by preparative chiral chromatography (resolution conditions: column: CHIRALPAK IE, 20 mm × 250 mm, 5 μm; mobile phases: A: n-hexane, B: ethanol (+ 20 mmol $NH_3$); A: 80%, B: 20%) to give the title compound **19c** (70 mg).

MS m/z (ESI): 468.2[M+1].

Chiral HPLC analysis method: retention time 16.12 min, chiral purity: 100% (column: CHIRALPAK IE A: n-hexane, B: EtOH (+ 0.1% DEA), A: 80%, B: 20%).

Step 4

1-(4-((1*R*,2*R*)-2-Cyclopentyl-6-hydroxy-1,2,3,4-tetrahydronaphthalen-1-yl)-2-fluorophe nyl)piperidine-4-carbaldehyde **19d**

Compound **19c** (70 mg, 0.15 mmol) was dissolved in tetrahydrofuran (1 mL), and dilute sulfuric acid (2 M, 1 mL, 2 mmol) was added. The reaction was heated at 70 °C for 30 min. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **19d** (63 mg, 100% yield).
MS m/z (ESI): 422.2[M+1].

Step 5

(*S*)-3-(5-(4-((1-(4-((1*R*,2*R*)-2-cyclopentyl-6-hydroxy-1,2,3,4-tetrahydronaphthalen-1-yl) -2-fluorophenyl)piperidin-4-yl)methyl)piperazin-1 -yl)-1 -oxoisoindolin-2-yl)piperidine-2,6-dione **19**

Compound **5h** (112 mg, 0.22 mmol) was dissolved in 2.5 mL of a mixed solvent of dichloromethane and methanol (V/V = 1/4), and sodium acetate (56 mg, 0.68 mmol) was added. After 10 min of reaction, compound **19d** (63 mg, 0.15 mmol) was added. After 15 min of reaction, sodium triacetoxyborohydride (96 mg, 0.45 mmol) was added. The mixture was allowed to react for 1 h. The reaction mixture was filtered. The filtrate was concentrated under reduced pressure to remove the solvent. The residue was purified by preparative high performance liquid chromatography (instrument: Waters 2545; column: Sharpsil-T; elution system: 10 mM ammonium bicarbonate, 60% water, 40% acetonitrile) to give the title compound **19** (50 mg, 45% yield).
MS m/z (ESI): 734.3[M+1].
$^1$H NMR (500 MHz, DMSO-$d_6$) $\delta$ 10.94 (bs, 1H), 9.06 (s, 1H), 7.52 (d, 1H), 7.11-7.01 (m, 2H), 6.88 (t, 1H), 6.78-6.72 (m, 1H), 6.66-6.58 (m, 2H), 6.51 (d, 1H), 6.42-6.40 (m, 1H), 5.07-5.03(m, 1H), 4.32, 4.22 (dd, 2H), 4.00 (d, 1H), 3.37-3.22 (m, 10H), 2.95-2.83 (m, 2H), 2.79-2.69 (m, 1H), 2.64-2.54 (m, 3H), 2.41-2.31 (m, 1H), 2.26-2.16 (m, 2H), 2.02-1.92 (m, 2H), 1.83-1.76 (m, 2H), 1.71-1.62 (m, 2H), 1.61-1.43 (m, 5H), 1.37-1.16 (m, 6H), 1.08-0.98 (m, 1H).

Example 20

(*S*)-3-(5-(4-((1-(4-((1*R*,2*R*)-2-Cyclohexyl-6-hydroxy-1,2,3,4-tetrahydronaphthalen-1-yl) -2-fluorophenyl)piperidin-4-yl)methyl)piperazin-1-yl)-1-oxoisoindolin-2-yl)piperidine-2,6-dione **20**

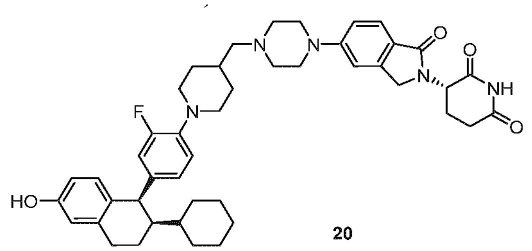

**20**

By using the synthesis scheme of Example **19** in which cyclohexene-1-boronic acid pinacol ester was used in place of the starting material cyclopentene-1-boronic acid pinacol ester of step 1, the title compound **20** (50 mg) was prepared.
MS m/z (ESI): 748.4[M+1].
$^1$H NMR (500 MHz, DMSO-$d_6$) $\delta$ 10.94 (bs, 1H), 9.06 (s, 1H), 7.52 (d, 1H), 7.11-7.01 (m, 2H), 6.88 (t, 1H), 6.72-6.68 (m, 1H), 6.66-6.60 (m, 2H), 6.51 (d, 1H), 6.43-6.40 (m, 1H), 5.07-5.03 (m, 1H), 4.32, 4.22 (dd, 2H), 4.11 (d, 1H), 3.37-3.22 (m, 8H), 2.95-2.83 (m, 2H), 2.75-2.65 (m, 1H), 2.64-2.54 (m, 3H), 2.41-2.31 (m, 2H), 2.26-2.16 (m, 2H), 2.12-2.03 (m, 1H), 2.01-1.91 (m, 2H), 1.83-1.76 (m, 2H), 1.76-1.63 (m, 3H), 1.49-1.38 (m, 2H), 1.61-1.41 (m, 4H), 1.18-1.05 (m, 2H), 1.05-0.92 (m, 3H), 0.83-0.70 (m, 2H).

Example 21

(*S*)-3-(5-(4-((1-(4-((1*R*,2*R*)-2-Cyclopropyl-6-hydroxy-1,2,3,4-tetrahydronaphthalen-1-yl )-2-fluorophenyl)piperidin-4-yl)methyl)piperazin-1-yl)-1-oxoisoindolin-2-yl)piperidine-2,6-dione **21**

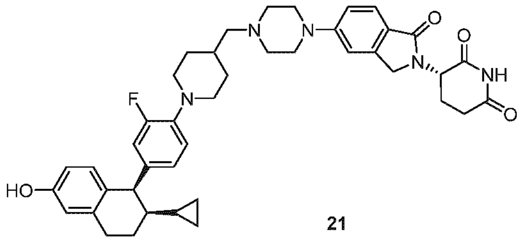

**21**

By using the synthesis scheme of Example **19** in which cyclopropylboronic acid was used in place of the starting material cyclopentene-1-boronic acid pinacol ester of step 1, the title compound **21** (50 mg) was prepared.

MS m/z (ESI): 706.3[M+1].

$^1$H NMR (500 MHz, DMSO-$d_6$) $\delta$ 10.94 (bs, 1H), 9.09 (s, 1H), 7.51 (d, 1H), 7.08-7.03 (m, 2H), 6.89 (t, 1H), 6.74-6.69 (m, 1H), 6.66-6.60 (m, 2H), 6.51 (d, 1H), 6.43-6.41 (m, 1H), 5.07-5.03 (m, 1H), 4.32, 4.22 (dd, 2H), 4.02 (d, 1H), 3.37-3.22 (m, 8H), 2.94-2.77 (m, 2H), 2.75-2.65 (m, 1H), 2.64-2.53 (m, 3H), 2.42-2.29 (m, 1H), 2.27-2.15 (m, 2H), 2.04-1.91 (m, 1H), 1.85-1.75 (m, 2H), 1.71-1.54 (m, 3H), 1.31-1.18 (m, 4H), 1.05-0.95 (m, 1H), 0.45-0.31 (m, 2H), 0.28-0.21 (m, 1H), 0.11-0.00 (m, 2H).

Example 22

(*S*)-3-(5-(4-((1-(4-((*R*)-6-Hydroxy-2',3,3',4,5',6'-hexahydro-1*H*-spiro[naphthalene-2,4'-p yran]-1-yl)phenyl)piperidin-4-yl)methyl)piperazin-1-yl)-1-oxoisoindolin-2-yl)piperidin e-2,6-dione **22**

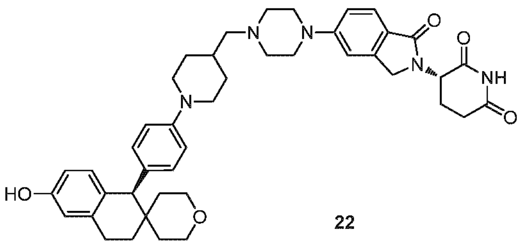

**22**

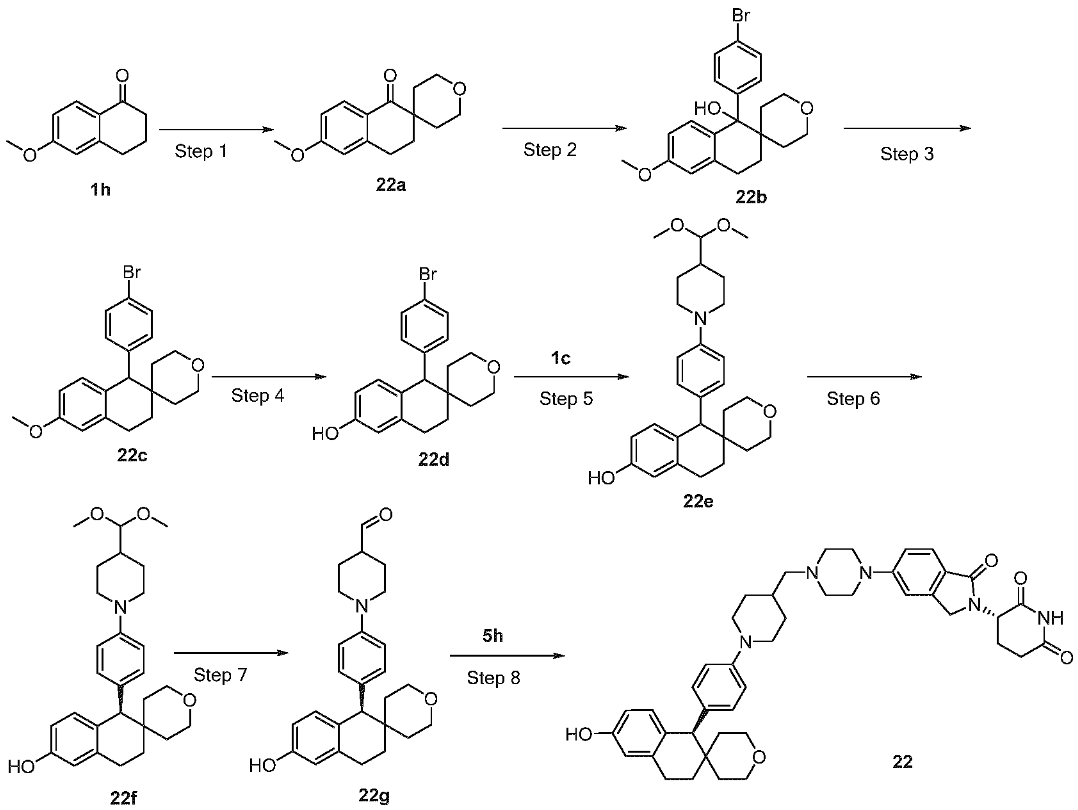


Step 1

6-Methoxy-2',3,3',4,5',6'-hexahydro-1*H*-spiro[naphthalene-2,4'-pyran]-1-one **22a**

Compound **1h** (5 g, 28.4 mmol), 1-bromo-2-(2-bromoethoxy)ethane (7.24 g, 31.2 mmol, Accela ChemBio (Shanghai) Co., Ltd.) and potassium tert-butoxide (9.55 g, 85.1 mmol) were added to toluene (100 mL). The reaction was heated at 130 °C for 4 h. The reaction mixture was cooled to room temperature and filtered. The filtrate was concentrated under reduced pressure to remove the solvent. The residue was purified by silica gel column chromatography using eluent system A to give the title compound **22a** (2.68 g, 38% yield).

MS m/z (ESI): 247.2[M+1].

Step 2

1-(4-Bromophenyl)-6-methoxy-2',3,3',4,5',6'-hexahydro-1*H*-spiro[naphthalene-2,4'-pyra n]-1-ol **22b**

1,4-Dibromobenzene (3.40 g, 14.4 mmol) was dissolved in tetrahydrofuran (40 mL), and the solution was cooled to -78 °C in a nitrogen atmosphere. n-Butyllithium (2.5 M, 5.7 mL, 14.3 mmol) was added dropwise. After the dropwise addition, the mixture was allowed to react at -78 °C for 30 min. A solution of compound **22a** (2.68 g, 10.9 mmol) in tetrahydrofuran (10 mL) was added dropwise, and the mixture was allowed to react at -78 °C for 2 h. The reaction mixture was warmed to room temperature, quenched by dropwise addition of a saturated aqueous solution of ammonium chloride (10 mL), and extracted with ethyl acetate (30 mL × 3). The organic phases were combined, washed with water (30 mL), dried over anhydrous sodium sulfate and filtered. The filtrate was concentrated under reduced pressure to remove the solvent. The residue was purified by silica gel column chromatography using eluent system A to give the title compound **22b** (2.3 g, 52% yield).

Step 3

1-(4-Bromophenyl)-6-methoxy-2',3,3',4,5',6'-hexahydro-1*H*-spiro[naphthalene-2,4'-pyra n] **22c**

Compound **22b** (2.3 g, 5.7 mmol) was dissolved in dichloromethane (40 mL), and triethylsilane (1.32 g, 11.4 mmol) was added. The mixture was cooled in an ice bath, and trifluoroacetic acid (650 mg, 5.7 mmol) was added

dropwise. After 1 h of reaction in the ice bath, additional trifluoroacetic acid (460 mg, 4 mmol) was added. The mixture was allowed to react at room temperature for 6 h. The reaction was quenched with saturated sodium bicarbonate (20 mL), and the aqueous phase was extracted with dichloromethane (20 mL × 3). The organic phases were combined, dried over anhydrous sodium sulfate and filtered. The filtrate was concentrated under reduced pressure to remove the solvent. The residue was purified by silica gel column chromatography using eluent system A to give the title compound **22c** (2 g, 90% yield).

MS m/z (ESI): 389.1[M+1].

Step 4

1-(4-Bromophenyl)-2',3,3',4,5',6'-hexahydro-1*H*-spiro[naphthalene-2,4'-pyran]-6-ol **22d**

Compound **22c** (1.8 g, 4.6 mmol) was dissolved in dichloromethane (40 mL), and the solution was cooled in an ice bath. Boron tribromide (1 M, 7 mL, 7 mmol) was added dropwise. The mixture was allowed to react at room temperature for 2 h. Additional boron tribromide (3 mL, 3 mmol) was added, and the reaction was continued at room temperature for 2.5 h. The reaction was quenched by dropwise addition of a saturated solution of sodium bicarbonate (20 mL), and the aqueous phase was extracted with dichloromethane (20 mL × 3). The organic phases were combined, dried over anhydrous sodium sulfate and filtered. The filtrate was concentrated under reduced pressure to remove the solvent. The residue was purified by silica gel column chromatography using eluent system B to give the title compound **22d** (1.3 g, 75% yield).

Step 5

1-(4-(4-(Dimethoxymethyl)piperidin-1-yl)phenyl)-2',3,3',4,5',6'-hexahydro-1*H*-spiro[na phthalene-2,4'-pyran]-6-ol **22e**

Compound **22d** (500 mg, 1.3 mmol) and compound **1c** (320 mg, 2 mmol) were dissolved in 1,4-dioxane (3 mL), and sodium tert-butoxide (258 mg, 2.7 mmol) and methanesulfonato(2-dicyclohexylphosphino)-3,6-dimethoxy-2',4',6'-triisopropoxy-1,1'-b iphenyl)(2'-amino-1,1'-biphenyl-2-yl)palladium(II) (BrettPhos Pd G3, 121 mg, 0.13 mmol) were added. The reaction was heated at 100 °C for 16 h in a nitrogen atmosphere. The reaction mixture was cooled to room temperature and filtered through celite. The filtrate was concentrated under reduced pressure. The residue was purified by silica gel column chromatography using eluent system B to give the title compound **22e** (277 mg, 46% yield).

MS m/z (ESI): 452.3[M+1].

Step 6

(*R*)-1-(4-(4-(Dimethoxymethyl)piperidin-1-yl)phenyl)-2',3,3',4, 5',6'-hexahydro-1*H*-spiro [naphthalene-2,4'-pyran]-6-ol **22f**

Compound **22e** (277 mg, 0.61 mmol) was resolved by preparative chiral chromatography (resolution conditions: column: CHIRALPAK IG, 250 mm × 20 mm, 5 μm; mobile phases: A: n-hexane, B: ethanol (+ 20 mmol $NH_3$), A: 70%, B: 30%) to give the title compound **22f** (80 mg).

MS m/z (ESI): 452.3[M+1].

Chiral HPLC analysis method: retention time 8.11 min, chiral purity: 100% (column: CHIRALPAK IG A: n-hexane, B: ethanol (+ 0.1% diethylamine), A: 60%, B: 40%).

Step 7

(*R*)-1-(4-(6-Hydroxy-2',3,3',4,5',6'-hexahydro-1*H*-spiro[naphthalene-2,4'-pyran]-1-yl)ph enyl)piperidine-4-carbaldehyde **22g**

Compound **22f** (80 mg, 0.18 mmol) was dissolved in tetrahydrofuran (5 mL), and dilute sulfuric acid (2 M, 0.25 mL, 0.5 mmol) was added. The mixture was allowed to react at 70 °C for 1 h. The reaction mixture was cooled to room temperature, made neutral with saturated sodium bicarbonate (5 mL) and extracted with ethyl acetate (10 mL × 3). The organic phases were combined, dried over anhydrous sodium sulfate and filtered. The filtrate was concentrated under reduced pressure to remove the solvent, giving the title compound **22g** (70 mg, 70% yield).

MS m/z (ESI): 406.2[M+1].

Step 8

(*S*)-3-(5-(4-((1-(4-((*R*)-6-Hydroxy-2',3,3',4,5',6'-hexahydro-1*H*-spiro[naphthalene-2,4'-p yran]-1-yl)phenyl)piperidin-4-yl)methyl)piperazin-1-yl)-1-oxoisoindolin-2-yl)piperidin e-2,6-dione **22**

Compound **5h** (63 mg, 0.13 mmol) was added to 5 mL of a mixed solvent of dichloromethane and methanol (V/V = 4/1), and sodium acetate (71 mg, 0.87 mmol) was added. After 10 min of reaction, compound **22g** (70 mg, 0.17 mmol) was added. After 15 min of reaction, sodium triacetoxyborohydride (73 mg, 0.34 mmol) was added. The mixture was allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **22** (30 mg, 24% yield).

MS m/z (ESI): 718.4[M+1].

$^1$H NMR (500 MHz, DMSO-$d_6$) $\delta$ 10.95 (bs, 1H), 9.04 (s, 1H), 7.53 (d, 1H), 7.06 (brs, 2H), 6.79 (brs, 4H), 6.62 (d, 1H), 6.53 (s, 1H), 6.44 (d, 1H), 5.07-5.3 (m, 1H), 4.32, 4.22 (dd, 2H), 3.67-3.35 (m, 10H), 3.29-3.27 (m, 2H), 2.94-2.87 (m, 1H), 2.82-2.68 (m, 2H), 2.61-2.52 (m, 5H), 2.41-2.33 (m, 2H), 2.22-2.21 (m, 2H), 1.98-1.95 (m, 1H), 1.71-1.66 (m, 5H), 1.39-1.33 (m, 3H), 1.25-1.16 (m, 3H).

Examples 23-1 and 23-2

3-(5-(4-((1-(4-((*R*)-6'-Hydroxy-3',4'-dihydro-1'*H*-spiro[cyclobutane-1,2'-naphthalen]-1'-yl)phenyl)piperidin-4-yl)methyl)piperazin-1-yl)-1-oxoisoindolin-2-yl)piperidine-2,6-dio ne **23-1** (a 1:1 mixture of diastereomers) 3-(5-(4-((1-(4-((*S*)-6'-Hydroxy-3',4'-dihydro-1'*H*-spiro[cyclobutane-1,2'-naphthalen]-1'-yl)phenyl)piperidin-4-yl)methyl)piperazin-1-yl)-1-oxoisoindolin-2-yl)piperidine-2,6-dio ne **23-2** (a 1:1 mixture of diastereomers)

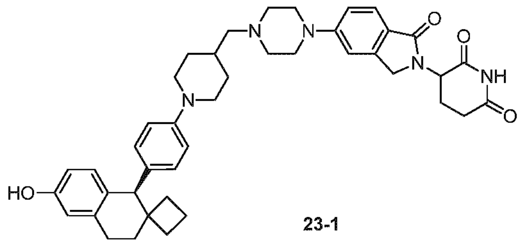

**23-1**

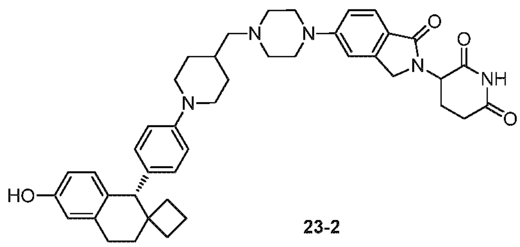

**23-2**

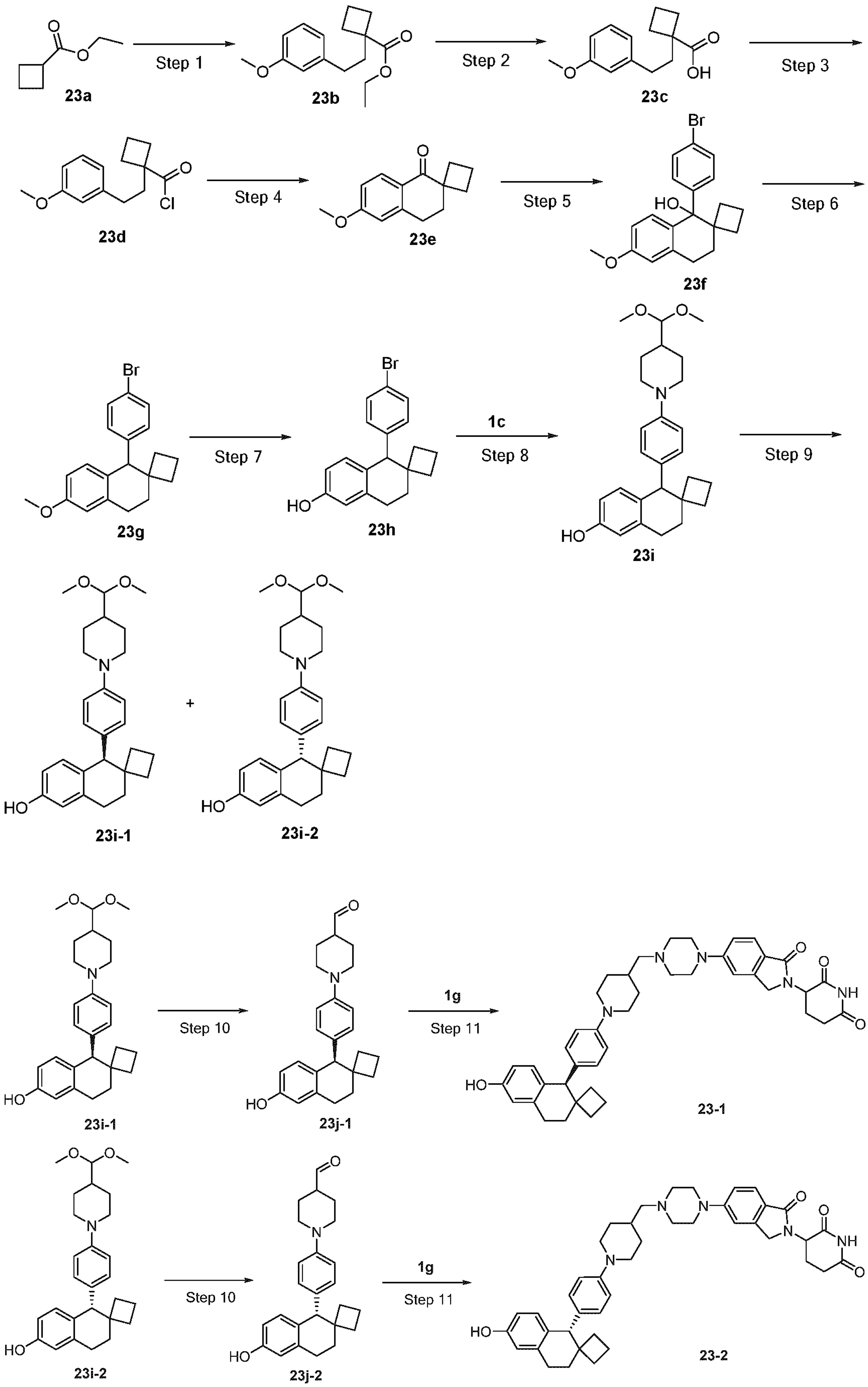


23a 23b 23c 23d 23e 23f 23g 23h 23i 23i-1 23i-2 23i-1 23j-1 23-1 23i-2 23j-2 23-2

Step 1

Ethyl 1-(3-methoxyphenethyl)cyclobutane-1-carboxylate **23b**

Lithium diisopropylamide (2 M in tetrahydrofuran, 5.1 mL, 10.2 mmol) was added to tetrahydrofuran (20 mL), and the solution was cooled to -78 °C in a nitrogen atmosphere. A solution of ethyl cyclobutanecarboxylate **23a** (1 g, 7.8 mmol) in tetrahydrofuran (5 mL) was added dropwise. After the dropwise addition, the mixture was warmed to -40 °C. After 15 min of reaction, the mixture was cooled again to -78 °C, and a solution of 1-(2-bromoethyl)-3-methoxybenzene (2.5 g, 11.6 mmol, Shanghai Titan Scientific Co., Ltd.) in tetrahydrofuran (5 mL) was slowly added dropwise. After the dropwise addition, the mixture was slowly warmed to room temperature and was allowed to react for 16 h. The reaction mixture was quenched with a saturated solution of ammonium chloride (50 mL) and extracted with dichloromethane (30 mL × 3). The organic phases were combined, dried over anhydrous sodium sulfate and filtered. The filtrate was concentrated under reduced pressure to remove the solvent. The residue was purified by silica gel column chromatography using eluent system B to give the title compound **23b** (215 mg, 63% yield).

$^1$H NMR (500 MHz, $CDCl_3$) $\delta$ 7.25-7.18 (m, 1H), 6.82-6.72 (m, 3H), 4.19 (q, 2H), 3.83 (m, 3H), 2.55-2.42 (m, 4H), 2.14-2.07 (m, 2H), 2.00-1.88 (m, 4H), 1.31 (t, 3H).

Step 2

1-(3-Methoxyphenethyl)cyclobutane-1-carboxylic acid **23c**

Compound **23b** (1.3 g, 5 mmol) was added to 12 mL of a mixed solvent of tetrahydrofuran and water (V/V = 5/1), and lithium hydroxide monohydrate (600 mg, 14.3 mmol) was added. The reaction was heated at 60 °C for 16 h. The reaction mixture was cooled to room temperature and concentrated under reduced pressure to remove the solvent. The residue was added to water (10 mL), and the solution was adjusted to pH 4 with dilute hydrochloric acid (1 M) and extracted with ethyl acetate (10 mL × 3). The organic phases were combined, dried over anhydrous sodium sulfate and filtered. The filtrate was concentrated under reduced pressure to remove the solvent, giving the title compound **23c** (900 mg, 77% yield).

MS m/z (ESI): 233.1[M-1].

Step 3

1-(3-Methoxyphenethyl)cyclobutane carbonyl chloride **23d**

Compound **23c** (900 mg, 3.8 mmol) was added to dichloromethane (35 mL), and oxalyl chloride (975 mg, 7.7 mmol) was added. The mixture was allowed to react at room temperature for 16 h. The filtrate was concentrated under reduced pressure to remove the solvent, giving the title compound **23d** (970 mg, 100% yield). The product was directly used in the next step without purification.

Step 4

6'-Methoxy-3',4'-dihydro-1'*H*-spiro[cyclobutane-1,2'-naphthalen]-1'-one **23e**

Compound **23d** (800 mg, 3.2 mmol) was added to 1,2-dichloroethane (20 mL), and aluminum trichloride (60 mg, 0.45 mmol) was added. The mixture was allowed to react at room temperature for 16 h. The reaction was quenched with iced water. The organic phase was separated, dried over anhydrous sodium sulfate and filtered. The filtrate was concentrated under reduced pressure to remove the solvent. The residue was purified by silica gel column chromatography using eluent system B to give the title compound **23e** (570 mg, 83% yield).

MS m/z (ESI): 217.1[M+1].

Step 5

1'-(4-Bromophenyl)-6'-methoxy-3',4'-dihydro-1'*H*-spiro[cyclobutane-1,2'-naphthalen]-1' -ol **23f**

1,4-Dibromobenzene (565 mg, 2.4 mmol, Shanghai Titan Scientific Co., Ltd.) was added to tetrahydrofuran (7.5 mL), and the solution was cooled to -78 °C in a nitrogen atmosphere. n-Butyllithium (2.5 M, 1.0 mL, 2.5 mmol) was added dropwise. After the dropwise addition, the mixture was allowed to react at -78 °C for 30 min. A solution of compound **23e** (470 mg, 2.2 mmol) in tetrahydrofuran (5 mL) was added dropwise. After the dropwise addition, the mixture was

allowed to react at -78 °C for 1 h. The reaction mixture was quenched with a saturated solution of ammonium chloride and extracted with ethyl acetate (10 mL × 3). The organic phases were combined, dried over anhydrous sodium sulfate and filtered. The filtrate was concentrated under reduced pressure to remove the solvent. The residue was purified by silica gel column chromatography using eluent system B to give the title compound **23f** (730 mg, 89% yield).

MS m/z (ESI): 354.0[M-18].

Step 6

1'-(4-Bromophenyl)-6'-methoxy-3',4'-dihydro-1'*H*-spiro[cyclobutane-1,2'-naphthalen] **23g**

Compound **23f** (730 mg, 2 mmol) was dissolved in dichloromethane (5 mL), and triethylsilane (455 mg, 3.9 mmol) was added. The mixture was cooled in an ice bath, and trifluoroacetic acid (245 mg, 2.2 mmol) was added dropwise. The reaction was cooled in the ice bath and stirred for 10 min. The reaction was quenched with saturated sodium bicarbonate (10 mL). The aqueous phase was extracted with dichloromethane (10 mL × 3). The organic phases were combined, dried over anhydrous sodium sulfate and filtered. The filtrate was concentrated under reduced pressure to remove the solvent, giving the title compound **23g** (600 mg, crude). The product was directly used in the next step without purification.

Step 7

1'-(4-Bromophenyl)-3',4'-dihydro-1'*H*-spiro[cyclobutane-1,2'-naphthalen]-6'-ol **23h**

Compound **23g** (600 mg, 1.7 mmol) was dissolved in dichloromethane (5 mL), and boron tribromide (1 M, 1.7 mL, 1.7 mmol) was added dropwise under an ice bath condition. The mixture was allowed to react at room temperature for 16 h. The reaction was quenched by slow addition of a saturated solution of sodium bicarbonate (10 mL). The aqueous phase was extracted with dichloromethane (10 mL × 3). The organic phases were combined, dried over anhydrous sodium sulfate and filtered. The filtrate was concentrated under reduced pressure to remove the solvent. The residue was purified by silica gel column chromatography using eluent system B to give the title compound **23h** (180 mg, 31% yield).

MS m/z (ESI): 341.1[M-1].

Step 8

1'-(4-(4-(Dimethoxymethyl)piperidin-1-yl)phenyl)-3',4'-dihydro-1'*H*-spiro[cyclobutane-1,2'-naphthalen]-6'-ol **23i**

Compound **23h** (180 mg, 0.52 mmol), compound **1c** (130 mg, 0.82 mmol), methanesulfonato(2-dicyclohexylphosphino)-3,6-dimethoxy-2',4',6'-triisopropoxy-1,1'-b iphenyl)(2'-amino-1,1'-biphenyl-2-yl)palladium(II) (BrettPhos Pd G3, 48 mg, 0.05 mmol) and sodium tert-butoxide (101 mg, 1.1 mmol) were added to 1,4-dioxane (7 mL). The reaction was heated at 85 °C for 16 h in a nitrogen atmosphere. The reaction mixture was cooled to room temperature and filtered through celite. The filtrate was concentrated under reduced pressure to remove the solvent. The residue was purified by silica gel column chromatography using eluent system A to give the title compound **23i** (180 mg, 80% yield).

MS m/z (ESI): 422.1[M+1].

Step 9

(*R*)-1'-(4-(4-(Dimethoxymethyl)piperidin-1-yl)phenyl)-3',4'-dihydro-1'*H*-spiro[cyclobut ane-1,2'-naphthalen]-6'-ol **23i-1**
(*S*)-1'-(4-(4-(Dimethoxymethyl)piperidin-1-yl)phenyl)-3',4'-dihydro-1'*H*-spiro[cyclobuta ne-1,2'-naphthalen]-6'-ol **23i-2**

Compound **23i** (180 mg, 0.23 mmol) was resolved by preparative chiral chromatography (resolution conditions: column: CHIRALPAK IG, 20 mm × 250 mm, 5 μm; mobile phases: A: n-hexane, B: ethanol (+ 20 mmol $NH_3$), A: 70%, B: 30%) into the title compound **23i-1** (47 mg) and **23i-2** (60 mg).

**23i-1:**

MS m/z (ESI): 422.3[M+1].
Chiral HPLC analysis method: retention time 18.66 min, chiral purity: 100% (column: CHIRALPAK IG A: n-hexane, B: EtOH (+ 0.1% DEA), A: 30%, B: 70%).

**23i-2:**

MS m/z (ESI): 422.3[M+1].
Chiral HPLC analysis method: retention time 6.53 min, chiral purity: 100% (column: CHIRALPAK IG A: n-hexane, B: EtOH (+ 0.1% DEA), A: 70%, B: 30%).

Step 10

(*R*)-1-(4-(6'-Hydroxy-3',4'-dihydro-1'*H*-spiro[cyclobutane-1,2'-naphthalen]-1'-yl)phenyl )piperidine-4-carbaldehyde **23j-1** (*S*)-1-(4-(6'-Hydroxy-3',4'-dihydro-1'*H*-spiro[cyclobutane-1,2'-naphthalen]-1'-yl)phenyl) piperidine-4-carbaldehyde **23j-2**

Compound **23i-1** (47 mg, 0.11 mol) was dissolved in tetrahydrofuran (5 mL), and dilute sulfuric acid (2 M, 0.15 mL, 0.3 mmol) was added. The mixture was allowed to react at 60 °C for 1 h. The reaction mixture was made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **23j-1** (41 mg, 100% yield).
MS m/z (ESI): 376.2[M+1].
Compound **23i-2** (60 mg, 0.14 mol) was dissolved in tetrahydrofuran (5 mL), and dilute sulfuric acid (2 M, 0.25 mL, 0.5 mmol) was added. The mixture was allowed to react at 60 °C for 1 h. The reaction mixture was cooled, made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **23j-2** (53 mg, 99% yield).
MS m/z (ESI): 376.3[M+1].

Step 11

3-(5-(4-((1-(4-((*R*)-6'-Hydroxy-3',4'-dihydro-1'*H*-spiro[cyclobutane-1,2'-naphthalen]-1'-yl)phenyl)piperidin-4-yl)methyl)piperazin-1-yl)-1-oxoisoindolin-2-yl)piperidine-2,6-dio ne **23-1** (a 1:1 mixture of diastereomers) 3-(5-(4-((1-(4-((*S*)-6'-Hydroxy-3',4'-dihydro-1'*H*-spiro[cyclobutane-1,2'-naphthalen]-1'-yl)phenyl)piperidin-4-yl)methyl)piperazin-1-yl)-1-oxoisoindolin-2-yl)piperidine-2,6-dio ne **23-2** (a 1:1 mixture of diastereomers)

Compound **1g** (60 mg, 0.16 mmol) was added to 5 mL of a mixed solution of dichloromethane and methanol (V/V = 4/1), and sodium acetate (98 mg, 1.2 mmol) was added. After 10 min of reaction, compound **23j-1** (41 mg, 0.11 mmol) was added. After 15 min of reaction, sodium triacetoxyborohydride (43 mg, 0.20 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **23-1** (35 mg, a 1:1 mixture of diastereomers, 46% yield).
MS m/z (ESI): 688.4[M+1].
$^1$H NMR (500 MHz, DMSO-$d_6$) $\delta$ 10.95 (bs, 1H), 9.03 (s, 1H), 7.53 (d, 1H), 7.13-7.01 (m, 2H), 6.80, 6.79 (dd, 4H), 6.62 (d, 2H), 6.51 (d, 3H), 6.43-6.41 (m, 1H), 5.07-5.03 (m, 1H), 4.32, 4.22 (dd, 2H), 3.81 (s, 1H), 3.64-3.55 (m, 2H), 3.39-3.24 (m, 8H), 2.96-2.69 (m, 3H), 2.42-2.32 (m, 1H), 2.22 (d, 2H), 2.08-1.93 (m, 2H), 1.91-1.73 (m, 6H), 1.72-1.56 (m, 3H), 1.30-1.13 (m, 3H).
Compound **1g** (69 mg, 0.19 mmol) was added to 5 mL of a mixed solution of dichloromethane and methanol (V/V = 4/1), and sodium acetate (98 mg, 1.2 mmol) was added. After 10 min of reaction, compound **23j-2** (53 mg, 0.14 mmol) was added. After 15 min of reaction, sodium triacetoxyborohydride (53 mg, 0.25 mmol) was added. The mixture was allowed to react at room temperature for 1 h. The reaction mixture was concentrated under reduced pressure to remove the solvent. The residue was purified by preparative high performance liquid chromatography (Waters 2545; column: SharpSil-T; elution system: 10 mM ammonium bicarbonate, 60% water, 40% acetonitrile) to give the title compound **23-2** (43 mg, a 1:1 mixture of diastereomers, 44% yield).
MS m/z (ESI): 688.4[M+1].
$^1$H NMR (500 MHz, DMSO-$d_6$) $\delta$ 10.95 (bs, 1H), 9.03 (s, 1H), 7.53 (d, 1H), 7.13-7.01 (m, 2H), 6.80, 6.79 (dd, 4H), 6.62 (d, 2H), 6.51 (d, 3H), 6.43-6.41 (m, 1H), 5.07-5.03 (m, 1H), 4.32, 4.22 (dd, 2H), 3.81 (s, 1H), 3.64-3.55 (m, 2H), 3.39-3.24 (m, 8H), 2.96-2.69 (m, 3H), 2.42-2.32 (m, 1H), 2.22 (d, 2H), 2.08-1.93 (m, 2H), 1.91-1.73 (m, 6H), 1.72-1.56 (m, 3H), 1.30-1.13 (m, 3H).

Example 24

(*S*)-3-(5-(4-((1-(4-((1*R*,2*R*)-6-Hydroxy-2-isopropyl-1,2,3,4-tetrahydronaphthalen-1-yl)p henyl)piperidin-4-yl)methyl)piperazin-1-yl)-1-oxoisoindolin-2-yl)piperidine-2,6-dione **24**

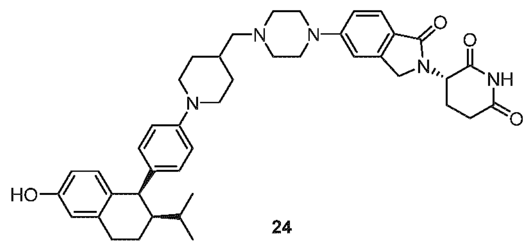

**24**

By using the synthesis scheme of Example 14-1 in which isopropenylboronic acid pinacol ester was used in place of the cyclopentene-1-boronic acid pinacol ester of step 1 and **5h** in place of the starting material **1g** of step 5, the title compound **24** was prepared.

MS m/z (ESI): 690.4[M+1].

$^1$H NMR (500 MHz, DMSO-$d_6$) $\delta$ 10.95 (s, 1H), 9.01 (s, 1H), 7.53 (d, 1H), 7.07-7.05 (m, 2H), 6.83, 6.77 (dd, 4H), 6.32 (d, 1H), 6.51 (d, 1H), 6.43-6.41 (m, 1H), 5.07-5.03 (m, 1H), 4.32, 4.22 (dd, 2H), 4.04 (d, 1H), 3.61-3.58 (m, 2H), 3.30-3.27 (m, 5H), 2.94-2.86 (m, 3H), 2.75-2.69 (m, 2H), 2.62-2.55 (m, 3H), 2.49-2.35 (m, 1H), 2.22 (d, 2H), 2.03-1.94 (m, 2H), 1.80 (d, 2H), 1.76-1.67 (m, 2H), 1.54-1.47 (m, 2H), 1.21-1.16 (m, 2H), 1.03 (d, 3H), 0.86 (t, 1H), 0.63 (d, 3H).

Examples 25-1 and 25-2

3-(5-(4-((1-(4-((*R*)-6-Hydroxy-2,2-dimethyl-1,2,3,4-tetrahydronaphthalen-1-yl)phenyl)p iperidin-4-yl)methyl)piperazin-1-yl)-1-oxoisoindolin-2-yl)piperidine-2,6-dione **25-1** (a 1:1 mixture of diastereomers) 3-(5-(4-((1-(4-((*S*)-6-Hydroxy-2,2-dimethyl-1,2,3,4-tetrahydronaphthalen-1-yl)phenyl)p iperidin-4-yl)methyl)piperazin-1-yl)-1-oxoisoindolin-2-yl)piperidine-2,6-dione **25-2** (a 1:1 mixture of diastereomers)

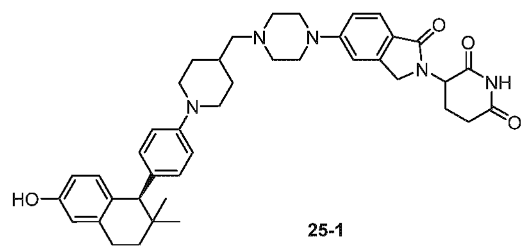

**25-1**

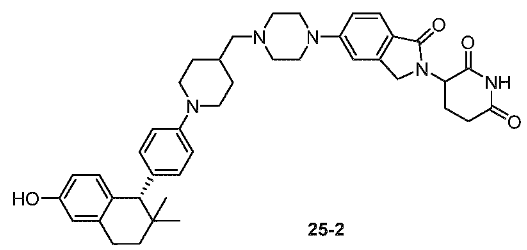

**25-2**

By using the synthesis scheme of Example 22 in which iodomethane was used in place of the starting material 1-bromo-2-(2-bromoethoxy)ethane of step 1, preparative chiral chromatography resolution was performed in step 6 to obtain two enantiomers, the two enantiomers were individually subjected to the next step, and **1g** was used in place of the starting material **5h** of step 8, the title compounds **25-1** (20 mg, a 1:1 mixture of diastereomers) and **25-2** (20 mg, a 1:1 mixture of diastereomers) were prepared.

**25-1**

MS m/z (ESI): 676.4[M+1].
$^1$H NMR (500 MHz, DMSO-$d_6$) $\delta$ 10.95 (s, 1H), 9.02 (s, 1H), 7.53 (d, 1H), 7.11-7.01 (m, 2H), 6.80 (brs, 4H), 6.62-6.51 (m, 2H), 6.43-6.41 (m, 1H), 5.07-5.03 (m, 1H), 4.32, 4.22 (dd, 2H), 3.60-3.57 (m, 2H), 3.50 (s,1H), 3.35-3.22 (m, 8H), 2.98-2.54 (m, 6H), 2.45-2.43 (m, 1H), 2.23-2.20 (m, 2H), 2.03-2.00 (m, 1H), 1.95-1.75 (m, 2H), 1.74-1.52 (m, 2H), 1.45-1.30 (m, 1H), 1.25-1.10 (m, 2H), 0.91 (s, 3H), 0.66 (s, 3H).

**25-2**

MS m/z (ESI): 676.4[M+1].
$^1$H NMR (500 MHz, DMSO-$d_6$) $\delta$ 10.95 (s, 1H), 9.02 (s, 1H), 7.53 (d, 1H), 7.11-7.01 (m, 2H), 6.80 (brs, 4H), 6.62-6.51 (m, 2H), 6.43-6.41 (m, 1H), 5.07-5.03 (m, 1H), 4.32, 4.22 (dd, 2H), 3.60-3.57 (m, 2H), 3.50 (s,1H), 3.35-3.22 (m, 8H), 2.98-2.54 (m, 6H), 2.45-2.43 (m, 1H), 2.23-2.20 (m, 2H), 2.03-2.00 (m, 1H), 1.95-1.75 (m, 2H), 1.74-1.52 (m, 2H), 1.45-1.30 (m, 1H), 1.25-1.10 (m, 2H), 0.91 (s, 3H), 0.66 (s, 3H).

Examples 26-1 and 26-2

(*S*)-3-(5-(4-((1-(4-((1*R*,2*R*)-6-Hydroxy-2-isobutyl-1,2,3,4-tetrahydronaphthalen-1-yl)ph enyl)piperidin-4-yl)methyl)piperazin-1-yl)-1-oxoisoindolin-2-yl)piperidine-2,6-dione **26-1** (*S*)-3-(5-(4-((1-(4-((1*S*,2*S*)-6-Hydroxy-2-isobutyl-1,2,3,4-tetrahydronaphthalen-1-yl)phe nyl)piperidin-4-yl)methyl)piperazin-1-yl)-1-oxoisoindolin-2-yl)piperidine-2,6-dione **26-2**

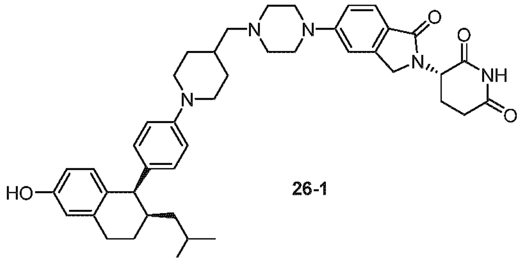

26-1

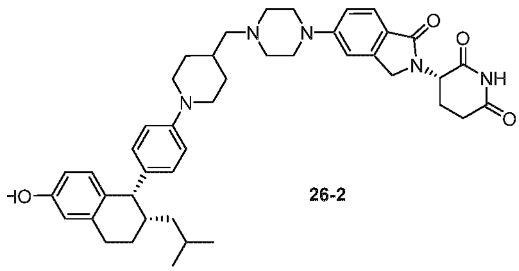

26-2

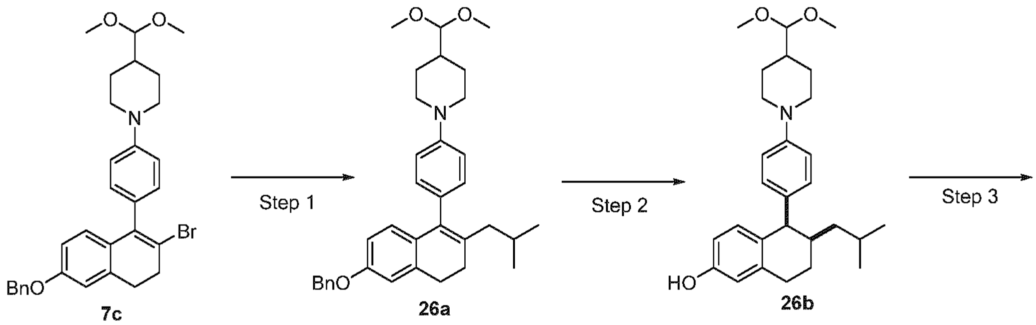


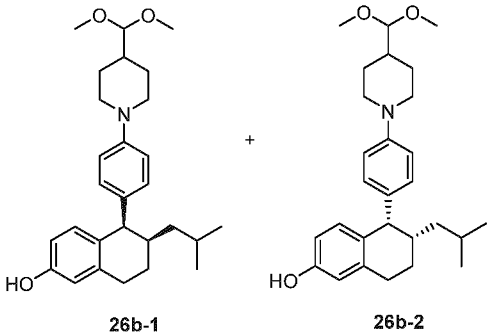


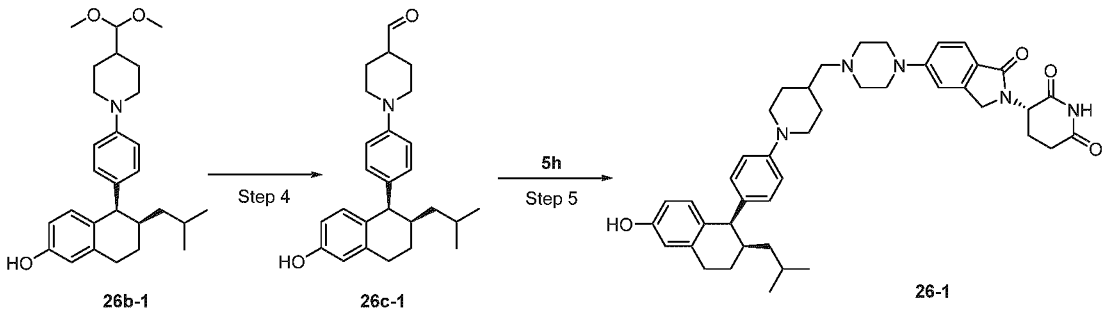


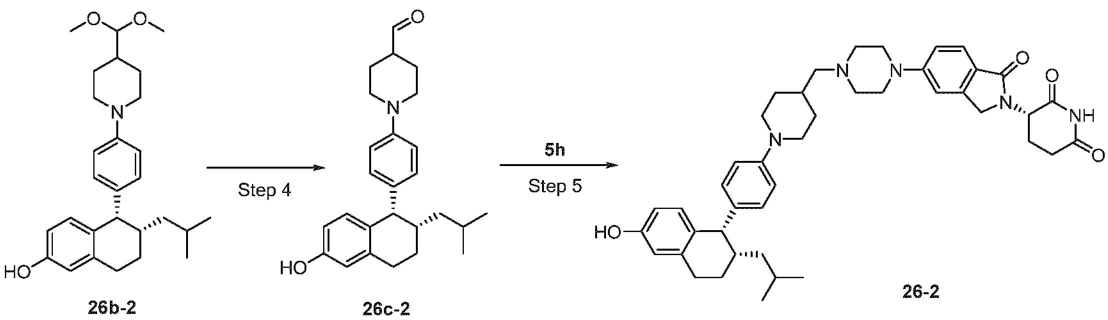


Step 1

1-(4-(6-(Benzyloxy)-2-isobutyl-3,4-dihydronaphthalen-1-yl)phenyl)-4-(dimethoxymeth yl)piperidine **26a**

In a nitrogen atmosphere, a solution of zinc chloride in tetrahydrofuran (1 M, 8.2 mL) was slowly added dropwise to an ice-bath-cooled solution of tert-butylmagnesium chloride in tetrahydrofuran (1 M, 7.5 mL, Shanghai Adamas Co., Ltd.). After the dropwise addition, the mixture was allowed to react at room temperature for 3 h. A solution of compound **7c** (400 mg, 0.73 mmol) and methanesulfonato(2-dicyclohexylphosphino-2',6'-diisopropoxy-1,1'-biphenyl)(2-amino-1,1'-biphenyl-2-yl)palladium(II) (Ruphos-Pd G3, 90 mg, 0.11 mmol, Jiangsu Aikon Biopharmaceutical R&D Co., Ltd.) in tetrahydrofuran (2 mL) was added. After the dropwise addition, the mixture was allowed to react at room temperature for 16 h. A saturated solution of ammonium chloride (10 mL) was added, and the organic phase was separated. The aqueous phase was extracted with dichloromethane (15 mL × 2). The organic phases were combined, dried over

anhydrous sodium sulfate, filtered and concentrated under reduced pressure to remove the solvent. The residue was purified by silica gel column chromatography using eluent system B to give the title compound **26a** (300 mg, 75% yield).
MS m/z (ESI): 526.3[M+1].

Step 2

(5,6)-*cis*-5-(4-(4-(Dimethoxymethyl)piperidin-1-yl)phenyl)-6-isobutyl-5,6,7,8-tetrahydr onaphthalen-2-ol **26b**

Compound **26a** (130 mg, 0.25 mmol) was dissolved in methanol (10 mL), and palladium hydroxide on carbon (100 mg, 20 wt%) was added. The mixture was allowed to react at room temperature for 16 h in a hydrogen atmosphere. The reaction mixture was filtered. The filtrate was concentrated under reduced pressure to remove the solvent, giving the title compound **26b** (90 mg, 83% yield).
MS m/z (ESI): 438.3[M+1].

Step 3

(S*R*,6*R*)-5-(4-(4-(Dimethoxymethyl)piperidin-1-yl)phenyl)-6-isobutyl-5,6,7,8-tetrahydr onaphthalen-2-ol **26b-1** (5*S*,6*S*)-5-(4-(4-(Dimethoxymethyl)piperidin-1-yl)phenyl)-6-isobutyl-5,6,7,8-tetrahydro naphthalen-2-ol **26b-2**

Compound **26b** (90 mg, 0.21 mmol) was resolved by preparative chiral chromatography (resolution conditions: column: CHIRALPAK IE, 20 mm × 250 mm, 5 μm; mobile phases: A: n-hexane, B: ethanol (+ 20 mmol $NH_3$), A: 85%, B: 15%, flow rate: 20 mL/min) into the title compounds **26b-1** (31 mg) and **26b-2** (35 mg).

**26b-1:**

MS m/z (ESI): 438.3[M+1].
Chiral HPLC analysis method: retention time 8.29 min, chiral purity: 100% (column: CHIRALPAK IE A: n-hexane, B: EtOH (+ 0.1% DEA), A: 85%, B: 15%).

**26b-2:**

MS m/z (ESI): 438.3[M+1].
Chiral HPLC analysis method: retention time 4.93 min, chiral purity: 100% (column: CHIRALPAK IE A: n-hexane, B: EtOH (+ 0.1% DEA), A: 85%, B: 15%).

Step 4

1-(4-((1*R*,2*R*)-6-Hydroxy-2-isobutyl-1,2,3,4-tetrahydronaphthalen-1-yl)phenyl)piperidi ne-4-carbaldehyde **26c-1**
1-(4-((1*S*,2*S*)-6-Hydroxy-2-isobutyl-1,2,3,4-tetrahydronaphthalen-1-yl)phenyl)piperidin e-4-carbaldehyde **26c-2**

Compound **26b-1** (35 mg, 0.08 mmol) was dissolved in tetrahydrofuran (2.5 mL), and dilute sulfuric acid (2 M, 0.15 mL, 0.3 mmol) was added. The reaction was heated at 55 °C for 1 h. The reaction mixture was cooled to room temperature, made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **26c-1** (31 mg, 99% yield).
MS m/z (ESI): 392.2[M+1].
Compound **26b-2** (31 mg, 0.07 mmol) was dissolved in tetrahydrofuran (2.5 mL), and dilute sulfuric acid (2 M, 0.15 mL, 0.3 mmol) was added. The reaction was heated at 55 °C for 1 h. The reaction mixture was cooled to room temperature, made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **26c-2** (27 mg, 98% yield).
MS m/z (ESI): 392.2[M+1].

Step 5

(*S*)-3-(5-(4-((1-(4-((1*R*,2*R*)-6-Hydroxy-2-isobutyl-1,2,3,4-tetrahydronaphthalen-1-yl)ph enyl)piperidin-4-yl)methyl)piperazin-1-yl)-1-oxoisoindolin-2-yl)piperidine-2,6-dione **26-1** (*S*)-3-(5-(4-((1-(4-((1*S*,2*S*)-6-Hydroxy-2-isobutyl-1,2,3,4-tetrahydronaphthalen-1-yl)phe nyl)piperidin-4-yl)methyl)piperazin-1-yl)-1-oxoisoindolin-2-yl)piperidine-2,6-dione **26-2**

Compound **5h** (50 mg, 0.1 mmol) was added to a mixed solvent of dichloromethane and methanol (V/V = 4/1, 5 mL), and sodium acetate (130 mg, 1.58 mmol) was added. After 10 min of reaction, compound **26c-1** (31 mg, 0.08 mmol) was added. After 15 min of reaction, sodium triacetoxyborohydride (34 mg, 0.16 mmol) was added. The mixture was allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **26-1** (25 mg, 45% yield).

MS m/z (ESI): 704.4[M+1].

$^1$H NMR (500 MHz, DMSO-$d_6$) $\delta$ 10.95 (bs, 1H), 9.02 (s, 1H), 7.52 (d, 1H), 7.10-7.04 (m, 2H), 6.80-6.72 (m, 4H), 6.62 (d, 1H), 6.52 (d, 1H), 6.43-6.40 (m, 1H), 5.05-5.02 (m, 1H), 4.32, 4.22 (dd, 2H), 3.86 (d, 1H), 3.65-3.56 (m, 2H), 3.38-3.34 (m, 4H), 3.32-3.25 (m, 4H), 2.95-2.73 (m, 3H), 2.65-2.54 (m, 3H), 2.41-2.32 (m, 1H), 2.25-2.18 (m, 2H), 2.02-1.87 (m, 2H), 1.84-1.61 (m, 4H), 1.55-1.44 (m, 2H), 1.29-1.14 (m, 2H), 1.07-0.98 (m, 1H), 0.84 (t, 6H), 0.72-0.64 (m, 1H).

Compound **5h** (45 mg, 0.09 mmol) was added to a mixed solvent of dichloromethane and methanol (V/V = 4/1, 5 mL), and sodium acetate (130 mg, 1.58 mmol) was added. After 10 min of reaction, compound **26c-2** (27 mg, 0.07 mmol) was added. After 15 min of reaction, sodium triacetoxyborohydride (34 mg, 0.16 mmol) was added. The mixture was allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **26-2** (23 mg, 36% yield).

MS m/z (ESI): 704.4[M+1].

$^1$H NMR (500 MHz, DMSO-$d_6$) $\delta$ 10.95 (bs, 1H), 9.02 (s, 1H), 7.52 (d, 1H), 7.10-7.04 (m, 2H), 6.80-6.72 (m, 4H), 6.62 (d, 1H), 6.52 (d, 1H), 6.43-6.40 (m, 1H), 5.05-5.02 (m, 1H), 4.32, 4.22 (dd, 2H), 3.86 (d, 1H), 3.65-3.56 (m, 2H), 3.38-3.34 (m, 4H), 3.32-3.25 (m, 4H), 2.95-2.73 (m, 3H), 2.65-2.54 (m, 3H), 2.41-2.32 (m, 1H), 2.25-2.18 (m, 2H), 2.02-1.87 (m, 2H), 1.84-1.61 (m, 4H), 1.55-1.44 (m, 2H), 1.29-1.14 (m, 2H), 1.07-0.98 (m, 1H), 0.84 (t, 6H), 0.72-0.64 (m, 1H).

Example 27

(*S*)-3-(5-(4-((1-(4-((1*R*,2*S*)-6-Hydroxy-2-methyl-1,2,3,4-tetrahydronaphthalen-1-yl)phe nyl)piperidin-4-yl)methyl)piperazin-1-yl)-1-oxoisoindolin-2-yl)piperidine-2,6-dione **27**

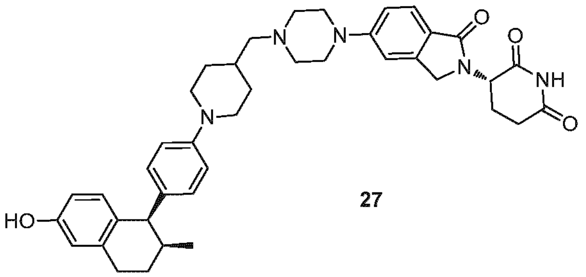

**27**

By using the synthesis scheme of Example 14-1 in which trimethylboroxine was used in place of the cyclopentene-1-boronic acid pinacol ester of step 1 and **5h** in place of the starting material **1g** of step 5, the title compound **27** (60 mg) was prepared.

MS m/z (ESI): 662.3[M+1].

$^1$H NMR (500 MHz, DMSO-$d_6$) $\delta$ 10.95 (s, 1H), 9.05 (s, 1H), 7.54-7.52 (m, 1H), 7.07-7.05 (m, 2H), 6.80-6.74 (m, 3H), 6.60-6.62 (m, 1H), 6.52 (s, 1H), 6.45-6.43 (m, 1H), 5.07-5.03 (m, 1H), 4.35, 4.20 (dd, 2H), 3.85-3.84 (m, 1H), 3.61-3.59 (m, 2H), 3.30-3.28 (m, 8H), 2.94-2.88 (m, 1H), 2.79-2.75 (m, 2H), 2.61-2.51 (m, 3H), 2.41-2.33 (m, 1H), 2.23-2.21 (m, 1H), 2.03-1.95 (m, 2H), 1.82-1.76 (m, 2H), 1.69-1.67 (m, 1H), 1.51-1.47 (m, 2H), 1.24-1.17 (m, 4H),

0.71-0.70 (m, 3H).

Example 28

(*S*)-3-(5-(4-((1-(4-((1*R*,2*S*)-6-Hydroxy-2-ethyl-1,2,3,4-tetrahydronaphthalen-1-yl)pheny 1)piperidin-4-yl)methyl)piperazin-1-yl)-1-oxoisoindolin-2-yl)piperidine-2,6-dione **28**

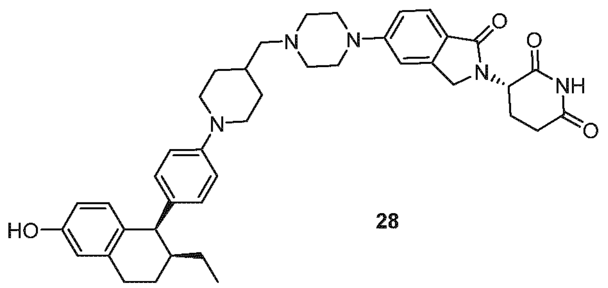

**28**

By using the synthesis scheme of Example 14-1 in which vinylboronic acid pinacol ester was used in place of the cyclopentene-1-boronic acid pinacol ester of step 1 and **5h** in place of the starting material **1g** of step 5, the title compound **28** (20 mg) was prepared.

MS m/z (ESI): 676.3[M+1].

$^1$H NMR (500 MHz, DMSO-$d_6$) δ 10.95 (bs, 1H), 9.02 (s, 1H), 7.52 (d, 1H), 7.10-7.04 (m, 2H), 6.80-6.72 (m, 4H), 6.62 (d, 1H), 6.52 (d, 1H), 6.43 (dd, 1H), 5.07-5.03 (m, 1H), 4.32, 4.22 (dd, 2H), 3.86 (d, 1H), 3.65-3.56 (m, 2H), 3.38-3.34 (m, 4H), 3.32-3.25 (m, 4H), 2.95-2.73 (m, 3H), 2.65-2.54 (m, 3H), 2.41-2.32 (m, 1H), 2.25-2.18 (m, 2H), 2.02-1.87 (m, 1H), 1.84-1.61 (m, 4H), 1.55-1.44 (m, 2H), 1.29-1.14 (m, 2H), 1.07-0.98 (m, 1H), 0.84 (t, 2H), 0.72-0.64 (m, 2H).

Example 29

(*S*)-3-(5-(4-((1-(4-((1*R*,2*S*)-6-Hydroxy-2-propyl-1,2,3,4-tetrahydronaphthalen-1-yl)phen yl)piperidin-4-yl)methyl)piperazin-1-yl)-1-oxoisoindolin-2-yl)piperidine-2,6-dione **29**

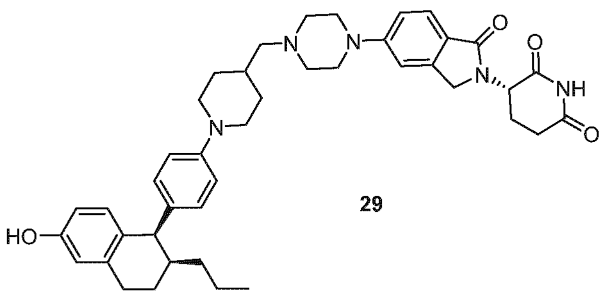

**29**

By using the synthesis scheme of Example 14-1 in which isopropenylboronic acid pinacol ester was used in place of the cyclopentene-1-boronic acid pinacol ester of step 1 and **5h** in place of the starting material **1g** of step 5, the title compound **29** (30 mg) was prepared.

MS m/z (ESI): 690.4[M+1].

$^1$H NMR (500 MHz, DMSO-$d_6$) δ 10.95 (s, 1H), 9.02 (s, 1H), 7.53 (d, 1H), 7.07-7.05 (m, 2H), 6.79-6.75 (m, 4H), 6.62 (d, 1H), 6.52 (d, 1H), 6.44-6.42 (m, 1H), 5.07-5.03 (m, 1H), 4.32, 4.22 (dd, 2H), 3.90 (d, 1H), 3.61-3.59 (m, 2H), 3.34-3.25 (m, 8H), 2.94-2.74 (m, 3H), 2.65-2.59 (m, 2H), 2.42-2.35 (m, 1H), 1.82-1.79 (m, 2H), 2.03-1.95 (m, 2H), 1.82-1.79 (m, 2H), 1.69-1.66 (m, 1H), 1.56-1.45 (m, 3H), 1.39-1.33 (m, 2H), 1.25-1.15 (m, 3H), 0.87-0.82 (m, 3H), 0.77-0.73 (m, 1H).

Example 30

(*S*)-3-(5-(4-((1-(4-((1*R*,2*R*)-2-Benzyl-6-hydroxy-1,2,3,4-tetrahydronaphthalen-1-yl)phen yl)piperidin-4-yl)methyl)piperazin-1-yl)-1-oxoisoindolin-2-yl)piperidine-2,6-dione **30**

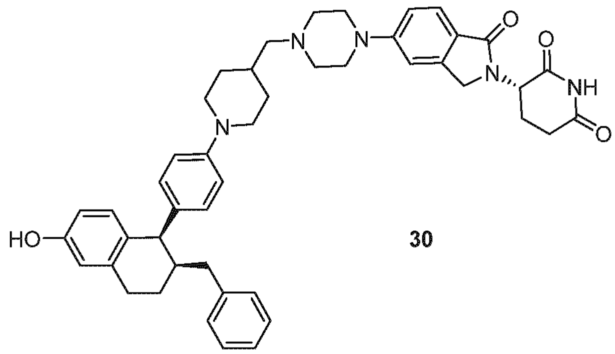

**30**

By using the synthesis scheme of Example 13-1 in which benzyl bromide was used in place of the bromocyclobutane of step 1, the title compound **30** (18 mg) was prepared. MS m/z (ESI): 738.4[M+1].

$^1$H NMR (500 MHz, DMSO-$d_6$) $\delta$ 10.98 (s, 1H), 9.06 (s, 1H), 7.54 (d, 1H), 7.29-7.22 (m, 2H), 7.19-7.02 (m, 5H), 6.80 (brs, 4H), 6.62 (d, 1H), 6.51 (d, 1H), 6.45 (d, 1H), 5.06-5.03 (m, 1H), 4.32, 4.22 (dd, 2H), 4.00 (d, 1H), 3.66-3.57 (m, 2H), 3.38-3.34 (m, 4H), 3.32-3.22 (m, 4H), 2.95-2.65 (m, 3H), 2.65-2.55 (m, 4H), 2.41-2.30 (m, 1H), 2.26-2.13 (m, 3H), 2.00-1.93 (m, 1H), 1.89-1.76 (m, 3H), 1.71-1.61 (m, 1H), 1.55-1.37 (m, 2H), 1.28-1.15 (m, 2H).

Example 31

(*S*)-3-(5-(4-((1-(2-Fluoro-4-((3*R*,4*R*)-7-hydroxy-3-(tetrahydro-2*H*-pyran-4-yl)chroman-4-yl)phenyl)piperidin-4-yl)methyl)piperazin-1-yl)-1-oxoisoindolin-2-yl)piperidine-2,6-d ione **31**

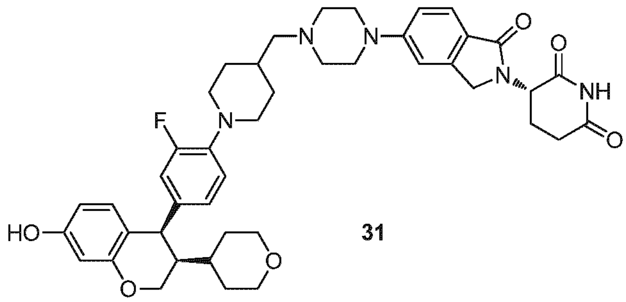

**31**

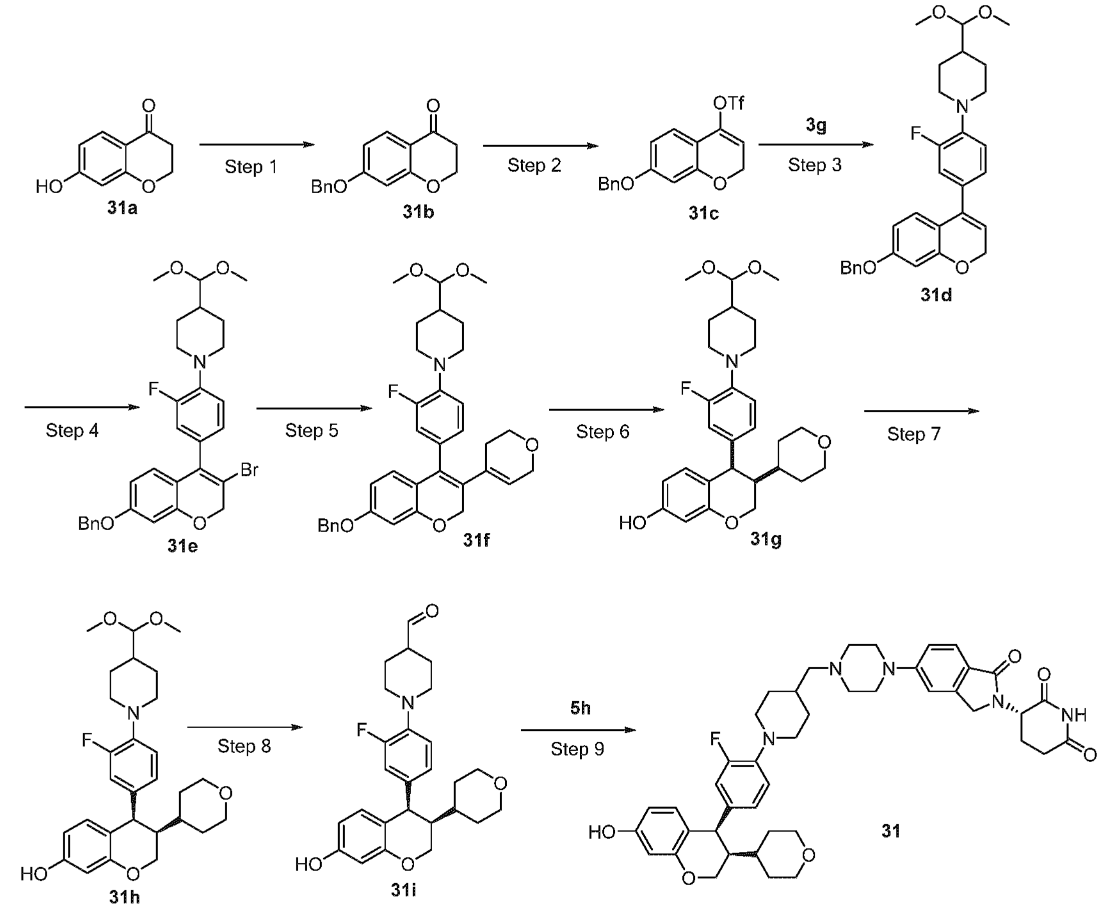


Step 1

7-(Benzyloxy)chroman-4-one **31b**

7-Hydroxychroman-4-one **31a** (5 g, 30.5 mmol, Shanghai Bide Pharmatech Ltd.) was dissolved in *N*,*N*-dimethylformamide (25 mL), and potassium carbonate (8.4 g, 60.9 mmol) was added. Benzyl bromide (5.7 g, 33.5 mmol) was added dropwise at 0 °C. The mixture was naturally warmed to room temperature and was allowed to react for 16 h. Water (150 mL) was added, and the mixture was extracted with ethyl acetate (150 mL × 3). The organic phases were combined, dried over anhydrous sodium sulfate and filtered. The filtrate was concentrated under reduced pressure to remove the solvent. The residue was purified by silica gel column chromatography using eluent system A to give the title compound **31b** (7.74 g, 100% yield).

$^1$H NMR (500 MHz, $CDCl_3$) $\delta$7.88-7.86 (m, 1H), 7.45-7.35 (m, 5H), 6.69-6.67 (m, 1H), 6.51-6.50 (m, 1H), 5.11 (s, 2H), 4.53 (t, 2H), 2.78 (t, 2H).

Step 2

6-(Benzyloxy)-3,4-2*H*-chromen-1-yl trifluoromethanesulfonate **31c**

Compound **31b** (5.00 g, 19.7 mmol) was dissolved in dry tetrahydrofuran (60 mL). The reaction mixture was cooled to -78 °C in an argon atmosphere. [Bis(trimethylsilyl)amino]lithium (1 M, 23.6 mL, 23.6 mmol) was added dropwise. After the dropwise addition, the mixture was allowed to react at -78 °C for 1 h. 1,1,1-Trifluoro-*N*-phenyl-*N*-(trifluoromethylsulfonyl)methanesulfonamide (8.43 g, 23.6 mmol) was slowly added. The mixture was naturally warmed to room temperature and was allowed to react for 1 h. The reaction mixture was concentrated under reduced pressure to remove the solvent. The residue was purified by silica gel column chromatography using eluent system B to give the title compound **31c** (5 g, 65% yield). MS m/z (ESI): 387.0[M+1].

Step 3

1-(4-(7-(Benzyloxy)-2*H*-chromen-4-yl)-2-fluorophenyl)-4-(dimethoxymethyl)piperidine **31d**

Compound **3g** (1.22 g, 3.2 mmol), compound **31c** (2.49 g, 6.4 mmol), tetrakis(triphenylphosphine)palladium(0) (186 mg, 0.16 mmol) and sodium carbonate (853 mg, 8 mmol) were dissolved in 12 mL of a mixed solvent of 1,4-dioxane and water (V/V = 5/1). The reaction was heated at 80 °C for 4 h in a nitrogen atmosphere. The reaction mixture was cooled to room temperature, and ethyl acetate (100 mL) was added. The mixture was washed with a saturated solution of sodium chloride (50 mL × 3). The organic phase was dried over anhydrous sodium sulfate and filtered. The filtrate was concentrated under reduced pressure to remove the solvent. The residue was purified by silica gel column chromatography using eluent system B to give the title compound **31d** (1.05 g, 66% yield).

MS m/z (ESI): 490.2[M+1].

Step 4

1-(4-(7-(Benzyloxy)-3-bromo-2*H*-chromen-4-yl)-2-fluorophenyl)-4-(dimethoxymethyl) piperidine **31e**

Compound **31d** (1.05 g, 2.1 mmol) and *N*,*N*-diisopropylethylamine (832 mg, 6.4 mmol) were dissolved in *N*,*N*-dimethylformamide (10 mL). The solution was cooled to 0 °C, and pyridinium tribromide (1.14g, 2.37 mmol) was added. The mixture was allowed to react at 0 °C for 1 h and at room temperature for 1 h. The reaction mixture was added to ethyl acetate (100 mL), washed with a saturated solution of sodium chloride (50 mL × 3), dried over anhydrous sodium sulfate and filtered. The filtrate was concentrated under reduced pressure to remove the solvent. The residue was purified by silica gel column chromatography using eluent system B to give the title compound **31e** (700 mg, 57% yield).

MS m/z (ESI): 570.2[M+1].

Step 5

1-(4-(7-(Benzyloxy)-3-(3,6-dihydro-2*H*-pyran-4-yl)-2*H*-chromen-4-yl)-2-fluorophenyl) -4-(dimethoxymethyl)piperidine **31f**

Compound **31e** (300 mg, 0.5 mmol), 3,6-dihydro-2*H*-pyran-4-boronic acid pinacol ester (333 mg, 1.6 mmol), tetrakis(triphenylphosphine)palladium(0) (61 mg, 0.05 mol) and sodium carbonate (168 mg, 1.6 mmol) were added to 6 mL of a mixed solvent of 1,4-dioxane and water (V/V = 5/1). The reaction was heated at 80 °C for 4 h in a nitrogen atmosphere. The reaction mixture was cooled to room temperature, and water (25 mL) was added. The mixture was extracted with ethyl acetate (25 mL × 3). The organic phases were combined, dried over anhydrous sodium sulfate and filtered. The filtrate was concentrated under reduced pressure to remove the solvent. The residue was purified by silica gel column chromatography using eluent system B to give the title compound **31f** (301 mg, 99% yield).

MS m/z (ESI): 572.2[M+1].

Step 6

(3,4)-*cis*-4-(4-(4-(Dimethoxymethyl)piperidin-1-yl)-3-fluorophenyl)-3-(tetrahydro-2*H*-p yran-4-yl)chroman-7-ol **31g**

Compound **31f** (301 mg, 0.5 mmol) was dissolved in methanol (5 mL), and palladium on carbon (113 mg, 10 wt%) was added. The mixture was allowed to react in a hydrogen atmosphere for 16 h. The reaction mixture was filtered through celite. The filtrate was concentrated under reduced pressure to remove the solvent, giving the title compound **31g** (255 mg, crude). The product was directly used in the next step without purification.

MS m/z (ESI): 486.3[M+1].

Step 7

(3*R*,4*R*)-4-(4-(4-(Dimethoxymethyl)piperidin-1-yl)-3-fluorophenyl)-3-(tetrahydro-2*H*-p yran-4-yl)chroman-7-ol **31h**

Compound **31g** (255 mg, 0.52 mmol) was resolved by preparative chiral chromatography (resolution conditions: column: CHIRALPAK IE, 250 mm × 21.2 mm, 5 μm; mobile phases: A: n-hexane, B: ethanol (+ 10 nM $NH_3$), A: 70%, B: 30%) to give the title compound **31h** (100 mg).

MS m/z (ESI): 486.3[M+1].

Chiral HPLC analysis method: retention time 15.5 min, chiral purity: 100% (column: CHIRALPAK IE A: n-hexane, B: EtOH (+ 0.1% DEA), A: 30%, B: 70%).

Step 8

1-(2-Fluoro-4-((3*R*,4*R*)-7-hydroxy-3-(tetrahydro-2*H*-pyran-4-yl)chroman-4-yl)phenyl)p iperidine-4-carbaldehyde **31i**

Compound **31h** (100 mg, 0.2 mol) was dissolved in tetrahydrofuran (2 mL), and dilute sulfuric acid (2 M, 1 mL, 2 mmol) was added. The mixture was allowed to react at room temperature for 1 h. The reaction mixture was made neutral with a saturated solution of sodium bicarbonate and extracted with dichloromethane (20 mL × 3). The organic phases were combined, dried over anhydrous sodium sulfate and filtered. The filtrate was concentrated under reduced pressure to remove the solvent, giving the title compound **31i** (90 mg, 99% yield).

MS m/z (ESI): 440.2[M+1].

Step 9

(*S*)-3-(5-(4-((1-(2-Fluoro-4-((3*R*,4*R*)-7-hydroxy-3-(tetrahydro-2*H*-pyran-4-yl)chroman-4-yl)phenyl)piperidin-4-yl)methyl)piperazin-1-yl)-1-oxoisoindolin-2-yl)piperidine-2,6-d ione **31**

Compound **5h** (154 mg, 0.3 mmol) was added to 6 mL of a mixed solvent of dichloromethane and methanol (V/V = 1/1), and sodium acetate (76 mg, 0.92 mmol) was added. After 10 min of reaction, compound **31i** (90 mg, 0.2 mmol) was added. After 10 min of reaction, sodium triacetoxyborohydride (131 mg, 0.6 mmol) was added. The mixture was allowed to react at room temperature for 1 h. The reaction mixture was concentrated under reduced pressure to remove the solvent. The residue was purified by preparative high performance liquid chromatography (instrument model: Waters 2545; elution system: 10 mM ammonium bicarbonate, 60% water, 40% acetonitrile) to give the title compound **31** (65 mg, 39% yield).

MS m/z (ESI): 752.3[M+1].

$^1$H NMR (500 MHz, DMSO-$d_6$) $\delta$ 10.94 (s, 1H), 9.23 (s, 1H), 7.52 (d, 1H), 7.06 (s, 1H), 7.06-7.04 (m, 1H), 6.94 (t, 1H), 6.83-6.76 (m, 2H), 6.64 (d, 1H), 6.23-6.19 (m, 2H), 5.04-5.02 (m, 1H), 4.32, 4.20 (dd, 2H), 4.20 (d, 1H), 4.08 (d, 1H), 3.89 (t, 1H), 3.86-3.81 (m, 1H), 3.74-3.69 (m, 1H), 3.32-3.25 (m, 8H), 3.19-3.12 (m, 1H), 3.08-3.03 (m, 1H), 2.93-2.83 (m, 1H), 2.64-2.56 (m, 3H), 2.40-2.31 (m, 1H), 2.22 (d, 2H), 2.02-1.90 (m, 3H), 1.84-1.76 (m, 2H), 1.72-1.61 (m, 1H), 1.35-1.07 (m, 8H).

Example 32

(*S*)-3-(5-(4-((1-4-((3*R*,4*R*)-3-Cyc1obutyl-7-hydroxychroman-4-yl)phenyl)piperidin-4-yl) methyl)piperazin-1-yl)-1-oxoisoindolin-2-yl)piperidine-2,6-dione **32**

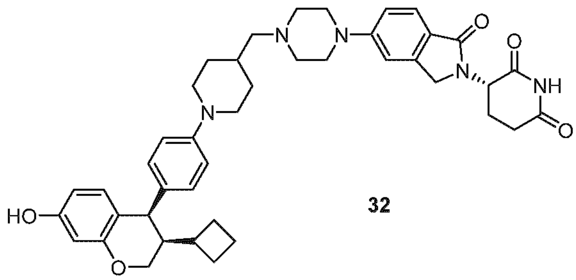

**32**

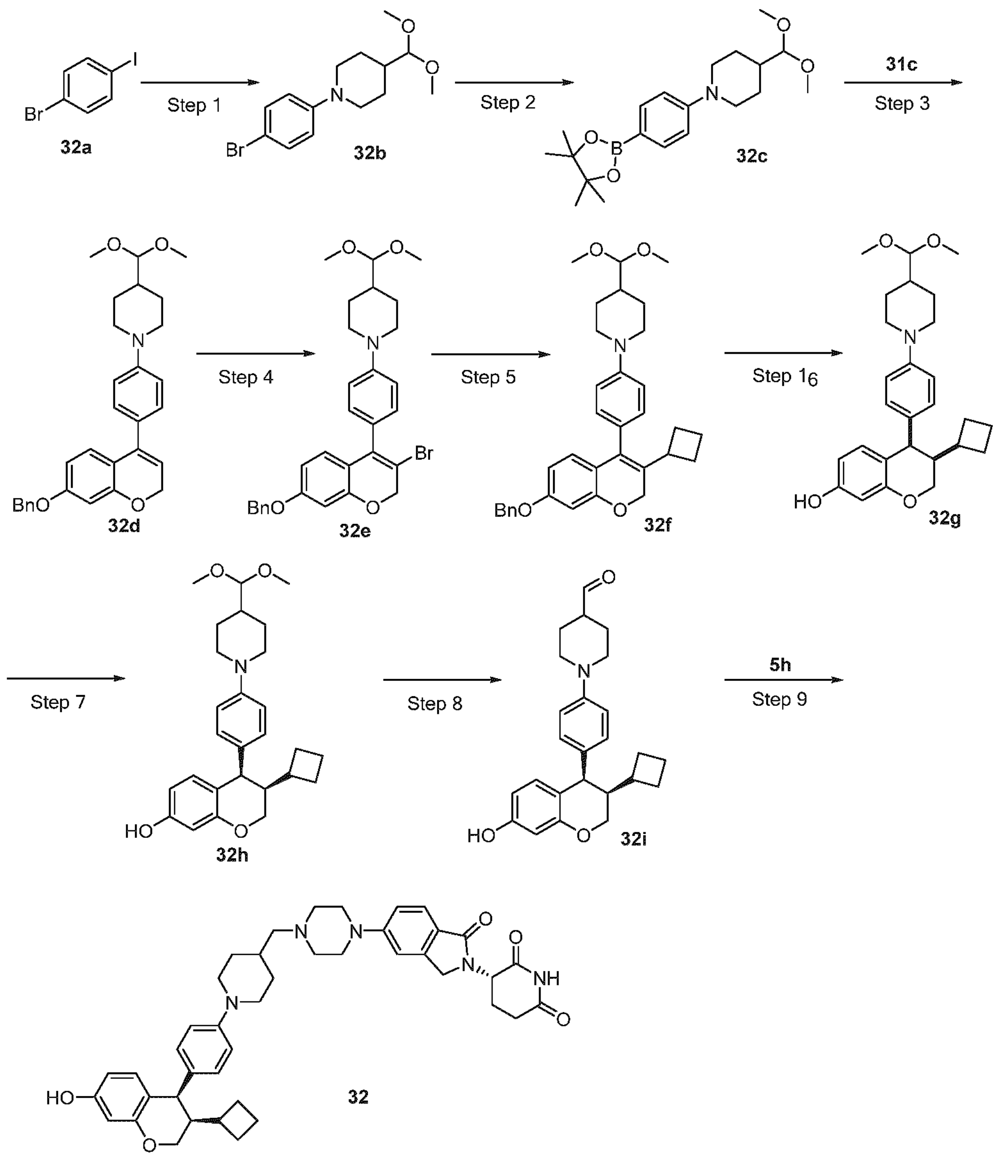


Step 1

1-(4-Bromophenyl)-4-(dimethoxymethyl)piperidine **32b**

1-Bromo-4-iodobenzene **32a** (5.0 g, 17.7 mmol, Accela ChemBio (Shanghai) Co., Ltd.), compound **1c** (2.8 g, 17.6 mmol), tris(dibenzylideneacetone)dipalladium(0) (810 mg, 0.9 mmol), 4,5-bisdiphenylphosphino-9,9-dimethylxanthene (1.03 g, 1.8 mmol) and sodium tert-butoxide (3.40 g, 35.37 mmol) were added to toluene (50 mL). The mixture was allowed to react at 80 °C for 1 h in a nitrogen atmosphere. The reaction mixture was cooled to room temperature and filtered. The filtrate was concentrated under reduced pressure to remove the solvent. The residue was purified by silica gel column chromatography using eluent system B to give the title compound **32b** (2.05 g, 36% yield).
MS m/z (ESI): 314.0[M+1].

Step 2

4-(Dimethoxymethyl)-1-(4-(4,4,5,5-tetramethyl-1,3,2-dioxaborolan-2-yl)phenyl)piperid ine **32c**

Compound **32b** (1.5 g, 4.8 mmol), bis(pinacolato)diboron (1.82 g, 7.16 mmol), [1,1'-bis(diphenylphosphino)ferrocene]palladium(II) dichloride (350 mg, 0.5 mmol) and potassium acetate (938 mg, 9.6 mmol) were added to 1,4-dioxane (20 mL). The mixture was allowed to react at 80 °C overnight in a nitrogen atmosphere. The reaction mixture was cooled to room temperature and filtered. The filtrate was concentrated under reduced pressure to remove the solvent. The

residue was purified by silica gel column chromatography using eluent system B to give the title compound **32c** (1.22 g, 70% yield).

MS m/z (ESI): 362.2[M+1].

Step 3

1-(4-(7-(Benzyloxy)-2*H*-chromen-4-yl)phenyl)-4-(dimethoxymethyl)piperidine **32d**

Compound **32c** (1.22 g, 3.4 mmol), compound **31c** (2.61 g, 6.75 mmol), tetrakis(triphenylphosphine)palladium(0) (196 mg, 0.2 mmol) and sodium carbonate (896 mg, 8.5 mmol) were dissolved in 12 mL of a mixed solvent of 1,4-dioxane and water (V/V = 5/1). The reaction was heated at 80 °C for 4 h in a nitrogen atmosphere. The reaction mixture was cooled to room temperature, and ethyl acetate (100 mL) was added. The mixture was washed with a saturated solution of sodium chloride (50 mL × 3). The organic phase was dried over anhydrous sodium sulfate and filtered. The filtrate was concentrated under reduced pressure to remove the solvent. The residue was purified by silica gel column chromatography using eluent system B to give the title compound **32d** (1.30 g, 81% yield).

MS m/z (ESI): 472.2[M+1].

Step 4

1-(4-(7-(Benzyloxy)-3-bromo-2*H*-chromen-4-yl)phenyl)-4-(dimethoxymethyl)piperidin e **32e**

Compound **32d** (1.3 g, 2.8 mmol) and *N*,*N* diisopropylethylamine (1.07 g, 8.3 mmol) were dissolved in *N*,*N*-dimethylformamide (10 mL). The solution was cooled to 0 °C, and pyridinium tribromide (1.46 g, 3 mmol) was added. The mixture was allowed to react at 0 °C for 1 h and at room temperature for 1 h. The reaction mixture was added to ethyl acetate (100 mL), washed with a saturated solution of sodium chloride (50 mL × 3), dried over anhydrous sodium sulfate and filtered. The filtrate was concentrated under reduced pressure to remove the solvent. The residue was purified by silica gel column chromatography using eluent system B to give the title compound **32e** (1.2 g, 79% yield).

MS m/z (ESI): 550.1[M+1].

Step 5

1-(4-(7-(Benzyloxy)-3-cyclobutyl-2*H*-chromen-4-yl)phenyl)-4-(dimethoxymethyl)piper idine **32f**

Compound **32e** (600 mg, 1.1 mmol), cyclobutylboronic acid (327 mg, 3.3 mmol, Shanghai Bide Pharmatech Ltd.), [1,1'-bis(diphenylphosphino)ferrocene]palladium(II) dichloride (81 mg, 0.1 mol) and cesium carbonate (711 mg, 2.2 mmol) were added to 12.5 mL of a mixed solvent of toluene and water (V/V = 4/1). The mixture was allowed to react in a microwave reactor at 110 °C for 1 h in a nitrogen atmosphere. The reaction mixture was cooled to room temperature, and water (20 mL) was added. The mixture was extracted with dichloromethane (20 mL × 3). The organic phases were combined, dried over anhydrous sodium sulfate and filtered. The filtrate was concentrated under reduced pressure to remove the solvent. The residue was purified by silica gel column chromatography using eluent system B to give the title compound **32f** (40 mg, 7% yield).

MS m/z (ESI): 526.2[M+1].

Step 6

(3,4)-*cis*-3-Cyclobutyl-4-(4-(4-(dimethoxymethyl)piperidin-1-yl)phenyl)chroman-7-ol **32g**

Compound **32f** (40 mg, 0.08 mmol) was dissolved in methanol (3 mL), and palladium on carbon (16 mg, 10 wt%) was added. The mixture was allowed to react in a hydrogen atmosphere for 16 h. The reaction mixture was filtered through celite. The filtrate was concentrated under reduced pressure to remove the solvent, giving the title compound **32g** (33 mg, crude).

MS m/z (ESI): 438.3[M+1].

Step 7

(3*R*,4*R*)-3-Cyclobutyl-4-(4-(4-(dimethoxymethyl)piperidin-1-yl)phenyl)chroman-7-ol **32h**

Compound **32g** (33 mg, 0.08 mmol) was resolved by preparative chiral chromatography (resolution conditions:

column: CHIRALPAK IF, 250 mm × 21.2 mm, 5 μm; mobile phases: A: n-hexane, B: ethanol (0.5% $NH_3$), A: 40%, B: 60%) to give the title compound **32h** (16 mg).

MS m/z (ESI): 438.3[M+1].

Chiral HPLC analysis method: retention time 21.3 min, chiral purity: 100% (column: CHIRALPAK IF A: n-hexane, B: EtOH (+ 0.1% DEA), A: 30%, B: 70%).

Step 8

1-(4-((3*R*,4*R*)-3-Cyclobutyl-7-hydroxychroman-4-yl)phenyl)piperidine-4-carbaldehyde **32i**

Compound **32h** (16 mg, 0.036 mol) was dissolved in tetrahydrofuran (1 mL), and dilute sulfuric acid (2 M, 1 mL, 2 mmol) was added. The mixture was allowed to react at room temperature for 2 h. The reaction mixture was made neutral with a saturated solution of sodium bicarbonate and extracted with dichloromethane (20 mL × 3). The organic phases were combined, dried over anhydrous sodium sulfate and filtered. The filtrate was concentrated under reduced pressure to remove the solvent, giving the title compound **32i** (14 mg, crude).

MS m/z (ESI): 392.2[M+1].

Step 9

(*S*)-3-(5-(4-((1-(4-((3*R*,4*R*)-3-Cyclobutyl-7-hydroxychroman-4-yl)phenyl)piperidin-4-yl )methyl)piperazin-1-yl)-1-oxoisoindolin-2-yl)piperidine-2,6-dione **32**

Compound **5h** (26 mg, 0.05 mmol) was added to 2 mL of a mixed solution of dichloromethane and methanol (V/V = 1/1), and sodium acetate (14 mg, 0.17 mmol) was added. After 10 min of reaction, compound **32i** (14 mg, 0.03 mmol) was added. After 10 min of reaction, sodium triacetoxyborohydride (23 mg, 0.1 mmol) was added. The mixture was allowed to react at room temperature for 1 h. The reaction mixture was concentrated under reduced pressure to remove the solvent. The residue was purified by preparative high performance liquid chromatography (instrument model: Waters 2545; elution system: 10 mM ammonium bicarbonate, 60% water, 40% acetonitrile) to give the title compound **32** (5 mg, 20% yield).

MS m/z (ESI): 704.4[M+1].

$^1H$ NMR (500 MHz, DMSO-$d_6$) $\delta$ 10.94 (s, 1H), 9.19 (s, 1H), 7.52 (d, 1H), 7.08-7.04 (m, 2H), 6.84-6.75 (m, 4H), 6.62 (d, 1H), 6.23-6.19 (m, 2H), 5.04-5.02 (m, 1H), 4.32, 4.20 (dd, 2H), 3.91-3.85 (m, 2H), 3.67-3.57 (m, 3H), 3.32-3.25 (m, 4H), 2.93-2.86 (m, 1H), 2.64-2.54 (m, 4H), 2.40-2.31 (m, 1H), 2.22 (d, 2H), 2.15-1.85 (m, 5H), 1.84-1.62 (m, 7H), 1.58-1.49 (m, 1H), 1.32-1.12 (m, 4H).

Example 33

2-(2,6-Dioxopiperidin-3-yl)-5-(4-((1-(2-fluoro-4-((1*R*,2*R*)-6-hydroxy-2-(tetrahydro-2*H*-pyran-4-yl)-1,2,3,4-tetrahydronaphthalen-1-yl)phenyl)piperidin-4-yl)methyl)piperazin-1 -yl)isoindoline-1,3-dione **33** (a 1:1 mixture of diastereomers)

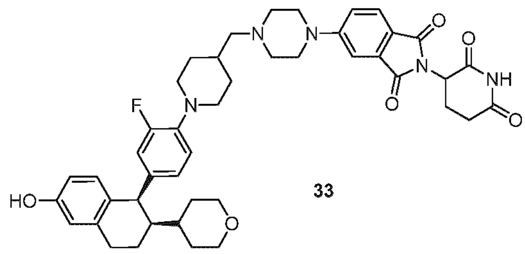

33

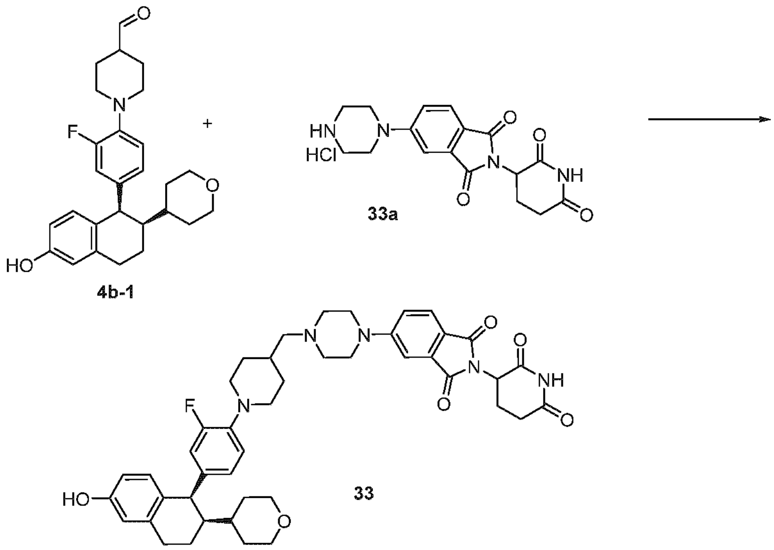

4b-1

33a

33

2-(2,6-Dioxopiperidin-3-yl)-5-(piperazin-1-yl)isoindoline-1,3-dione hydrochloride **33a** (38 mg, 0.1 mmol, prepared using "the synthesis method for Compound 393 on page 523 of the specification in the patent application US20180155322A1") was added to 6 mL of a mixed solution of dichloromethane and methanol (V/V = 1/1), and sodium acetate (68 mg, 0.82 mmol) was added. After 10 min of reaction, compound **4b-1** (35 mg, 0.08 mmol) was added. After another 10 min of reaction, sodium triacetoxyborohydride (35 mg, 0.16 mmol) was added. The mixture was allowed to react at room temperature for 1 h. The reaction mixture was concentrated under reduced pressure to remove the solvent. The residue was purified by preparative high performance liquid chromatography (instrument model: Waters 2545; elution system: 10 mM ammonium bicarbonate, 60% water, 40% acetonitrile) to give the title compound **33** (30 mg, a 1:1 mixture of diastereomers, 49% yield).

MS m/z (ESI): 764.3[M+1].

$^1$H NMR (500 MHz, DMSO-$d_6$) $\delta$ 11.08 (s, 1H), 9.08 (s, 1H), 7.69-7.68 (m, 1H), 7.35 (s, 1H), 7.27-7.25 (m, 1H), 6.91-6.87 (m, 1H), 6.76-6.75 (m, 1H), 6.69-6.64 (m, 2H), 6.53 (s, 1H), 6.45-6.43 (m, 1H), 5.10-5.06 (m, 1H), 4.12 (s, 1H), 3.87-3.85 (m, 1H), 3.76-3.74 (m, 1H), 3.45-3.43 (m, 3H), 3.30-3.25 (m, 8H), 3.22-3.17 (m, 1H), 3.11-3.07 (m, 1H), 2.93-2.89 (m, 2H), 2.76-2.74 (m, 1H), 2.65-2.49 (m, 4H), 2.24-2.23 (m, 1H), 2.03-2.00 (m, 2H), 1.83-1.60 (m, 5H), 1.49-1.46 (m, 2H), 1.27-1.16 (m, 5H).

Example 34

(*S*)-3-(5-(4-((1-(4-((1*R*,2*R*)-2-(Cyclopropylmethyl)-6-hydroxy-1,2,3,4-tetrahydronaphth alen-1-yl)phenyl)piperidin-4-yl)methyl)piperazin-1-yl)-1-oxoisoindolin-2-yl)piperidine-2,6-dione **34**

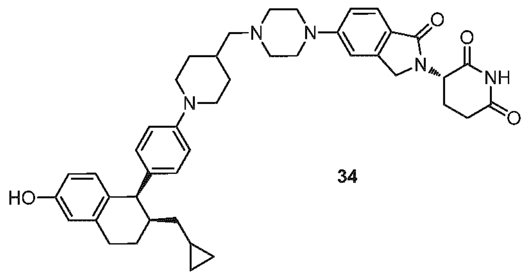

34

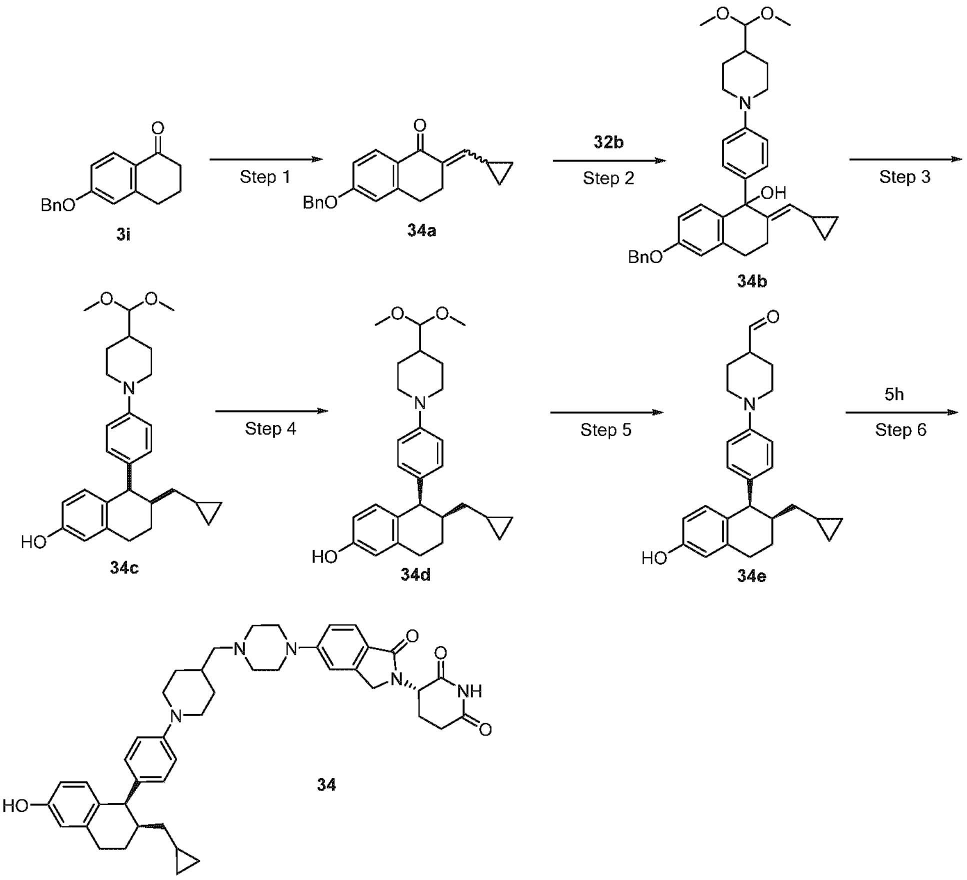


3i, 34a, 34b, 34c, 34d, 34e, 34

Step 1

6-(Benzyloxy)-2-(cyclopropylmethylene)-3,4-dihydronaphthalen-1(2*H*)-one **34a**

Compound **3i** (2.52 g, 10 mmol) was dissolved in ethanol (3.5 mL), and a solution of sodium hydroxide (3 M, 5 mL) and cyclopropylformaldehyde (1.05 g, 15 mmol) were sequentially added. The mixture was allowed to react at 50 °C for 16 h. The reaction mixture was cooled to room temperature, and water (20 mL) was added. The mixture was stirred well and filtered. The filter cake was washed with water (10 mL × 3) and dried to give the title compound **34a** (3 g, 99% yield).

MS m/z (ESI): 305.2[M+1].

Step 2

6-(Benzyloxy)-2-(cyclopropylmethylene)-1-(4-(4-(dimethoxymethyl)piperidin-1-yl)phe nyl)-1,2,3,4-tetrahydronaphthalen-1-ol **34b**

Compound **32b** (1.83 g, 5.8 mmol) was added to 2-methyltetrahydrofuran (10 mL), and the solution was cooled to -78 °C. A solution of n-butyllithium in tetrahydrofuran (2.5 M, 3.5 mL) was added dropwise. The mixture was allowed to react at -78 °C for 1.5 h. A solution of compound **34a** (1.77 g, 5.8 mmol) in 2-methyltetrahydrofuran (5 mL) was added.

The mixture was allowed to react at -78 °C for 1 h, naturally warmed to room temperature, and allowed to react for 1 h. The reaction mixture was quenched by dropwise addition of a saturated solution of ammonium chloride (50 mL) and extracted with ethyl acetate (30 mL × 3). The organic phases were combined, dried over anhydrous sodium sulfate and filtered. The filtrate was concentrated under reduced pressure to remove the solvent, giving the title compound **34b** (3.14 g, crude). The product was directly used in the next step without purification.

MS m/z (ESI): 540.3[M+1].

Step 3

(5,6)-*cis*-6-(Cyclopropylmethyl)-5-(4-(4-(dimethoxymethyl)piperidin-1-yl)phenyl)-5,6, 7,8-tetrahydronaphthalen-2-ol **34c**

Compound **34b** (3.14 g, 5.8 mmol) was dissolved in ethyl acetate (50 mL), and palladium on carbon (1.24 g, 10 wt%) was added. The mixture was allowed to react at room temperature for 16 h and at 50 °C for 3 h in a hydrogen atmosphere. The reaction mixture was filtered through celite. The filtrate was concentrated under reduced pressure to remove the solvent. The resulting residue was purified by silica gel column chromatography using eluent system B to give the title compound **34c** (130 mg, 5% yield).

MS m/z (ESI): 436.2[M+1].

Step 4

(5*R*,6*R*)-6-(Cyclopropylmethyl)-5-(4-(4-(dimethoxymethyl)piperidin-1-yl)phenyl)-5,6,7 ,8-tetrahydronaphthalen-2-ol **34d**

Compound **34c** (130 mg, 0.30 mmol) was resolved by preparative chiral chromatography (resolution conditions: column: CHIRALPAK IE, 20 mm × 250 mm, 5 μm; mobile phases: A: n-hexane, B: ethanol (0.5% $NH_3$), A: 70%, B: 30%, flow rate: 20 mL/min) to give the title compound **34d** (60 mg).

MS m/z (ESI): 436.2[M+1].

Chiral HPLC analysis method: retention time 5.67 min, chiral purity: 100% (column: CHIRALPAK IE A: n-hexane, B: EtOH (+ 0.1% DEA), A: 30%, B: 70%).

Step 5

1-(4-((1*R*,2*R*)-2-(Cyclopropylmethyl)-6-hydroxy-1,2,3,4-tetrahydronaphthalen-1-yl)phe nyl)piperidine-4-carbaldehyde **34e**

Compound **34d** (60 mg, 0.1 mmol) was dissolved in tetrahydrofuran (2 mL), and dilute sulfuric acid (2 M, 1 mL, 2 mmol) was added. The mixture was allowed to react at room temperature for 2 h. The reaction mixture was made neutral with a saturated solution of sodium bicarbonate and extracted with dichloromethane (20 mL × 3). The organic phases were combined, dried over anhydrous sodium sulfate and filtered. The filtrate was concentrated under reduced pressure to remove the solvent, giving the title compound **34e** (53 mg, crude, 98% yield).

MS m/z (ESI): 390.3[M+1].

Step 6

(*S*)-3-(5-(4-((1-(4-((1*R*,2*R*)-2-(Cyclopropylmethyl)-6-hydroxy-1,2,3,4-tetrahydronaphth alen-1-yl)phenyl)piperidin-4-yl)methyl)piperazin-1-yl)-1-oxoisoindolin-2-yl)piperidine-2,6-dione **34**

Compound **5h** (102 mg, 0.2 mmol) was added to 5 mL of a mixed solvent of dichloromethane and methanol (V/V = 1/1), and sodium acetate (50 mg, 0.6 mmol) was added. After 10 min of reaction, compound **34e** (53 mg, 0.1 mmol) was added. After 10 min of reaction, sodium triacetoxyborohydride (86 mg, 0.4 mmol) was added. The mixture was allowed to react at room temperature for 1 h. The reaction mixture was concentrated under reduced pressure to remove the solvent. The residue was purified by preparative high performance liquid chromatography (instrument model: Waters 2545; elution system: 10 mM ammonium bicarbonate, 60% water, 40% acetonitrile) to give the title compound **34** (60 mg, 62% yield).

MS m/z (ESI): 702.3[M+1].

$^1$H NMR (500 MHz, DMSO-$d_6$) $\delta$ 10.95 (s, 1H), 9.02 (s, 1H), 7.52 (d, 1H), 7.09-7.04 (m, 2H), 6.79-6.75 (m, 4H), 6.62 (d, 1H), 6.52 (d, 1H), 6.43 (dd, 1H), 5.05-5.02 (m, 1H), 4.33, 4.21 (dd, 2H), 3.90 (d, 1H), 3.60 (d, 2H), 3.31-3.26 (m, 4H), 2.94-2.73 (m, 3H), 2.64-2.52 (m, 4H), 2.42-2.32 (m, 1H), 2.22 (d, 2H), 2.04-1.91 (m, 4H), 1.80 (d, 2H), 1.76-1.62 (m, 2H), 1.58-1.42 (m, 1H), 1.31-1.11 (m, 6H), 0.54-0.48 (m, 1H), 0.83-0.74 (m, 1H), 0.46-0.33 (m, 2H).

Example 35

(*S*)-3-(5-(4-((1-(4-((1*R*,2*R*)-2-(Cyclobutylmethyl)-6-hydroxy-1,2,3,4-tetrahydronaphthal en-1-yl)phenyl)piperidin-4-yl)methyl)piperazin-1-yl)-1-oxoisoindolin-2-yl)piperidine-2, 6-dione **35**

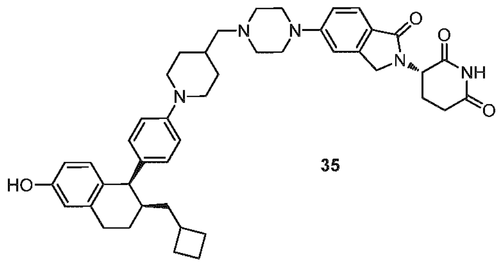

**35**

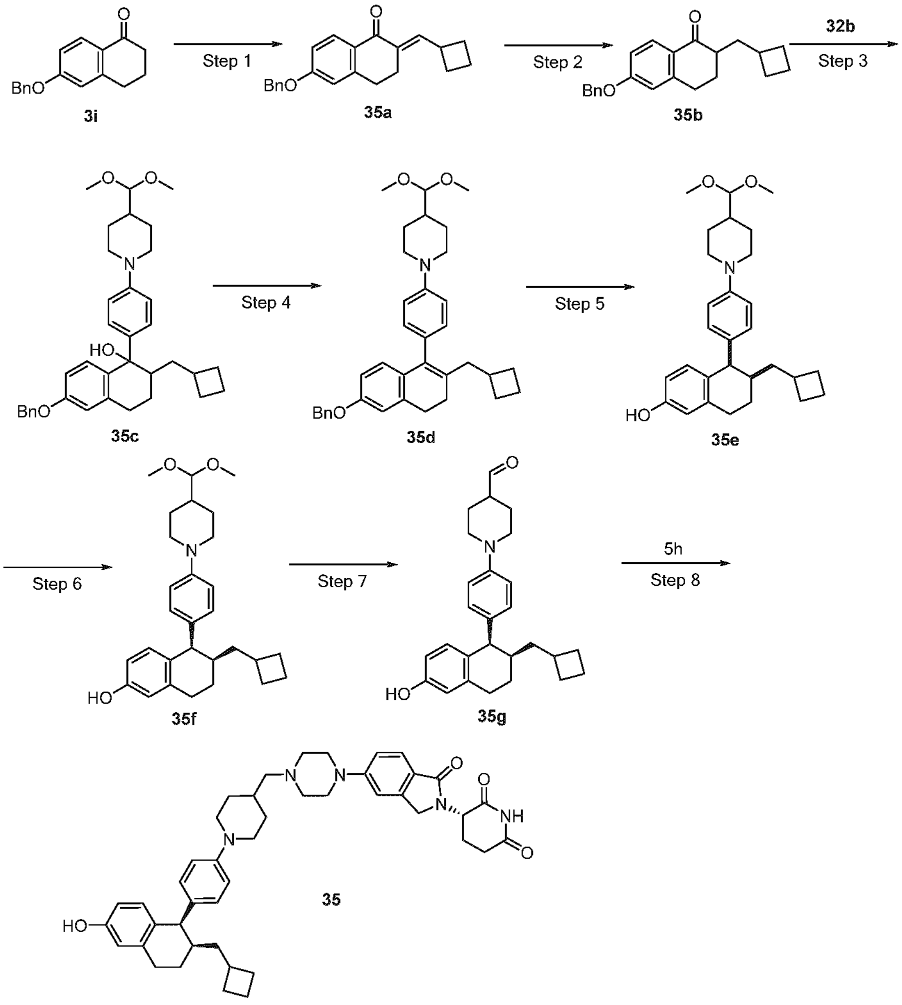


**3i** **35a** **35b** **35c** **35d** **35e** **35f** **35g** **35**

Step 1

6-(Benzyloxy)-2-(cyclobutylmethylene)-3,4-dihydronaphthalen-1(2*H*)-one **35a**

Compound **3i** (5 g, 19.8 mmol) was dissolved in ethanol (25 mL), and potassium hydroxide (611 mg, 10.9 mmol) and cyclobutylformaldehyde (1.84 g, 21.9 mmol) were sequentially added. The mixture was allowed to react at room temperature for 3 h. The reaction mixture was added to ethyl acetate (150 mL), washed with water (50 mL × 3), dried over anhydrous sodium sulfate and filtered. The filtrate was concentrated under reduced pressure to remove the

solvent. The residue was purified by silica gel column chromatography using eluent system B to give the title compound **35a** (1.66 g, 26% yield).

MS m/z (ESI): 319.2[M+1].

Step 2

6-(Benzyloxy)-2-(cyclobutylmethyl)-3,4-dihydronaphthalen-1(2*H*)-one **35b**

Compound **35a** (1.66 g, 5.2 mmol) was dissolved in ethyl acetate (15 mL), and palladium on carbon (555 mg, 10 wt%) was added. The mixture was allowed to react at room temperature for 16 h in a hydrogen atmosphere. The reaction mixture was filtered through celite. The filtrate was concentrated under reduced pressure to remove the solvent. The residue was dissolved in *N*,*N*-dimethylformamide (5 mL), and potassium carbonate (360 mg, 2.6 mmol) and benzyl bromide (267 mg, 1.6 mmol) were sequentially added. The mixture was allowed to react at room temperature for 2 h. The reaction mixture was filtered. The filtrate was concentrated under reduced pressure to remove the solvent. The residue was purified by silica gel column chromatography using eluent system B to give the title compound **35b** (350 mg, 21% yield).

MS m/z (ESI): 321.2[M+1].

Step 3

6-(Benzyloxy)-2-(cyclobutylmethyl)-1 -(4-(4-(dimethoxymethyl)piperidin-1 -yl)phenyl)-1,2,3,4-tetrahydronaphthalen-1-ol **35c**

Compound **32b** (686 mg, 2.2 mmol) was added to 2-methyltetrahydrofuran (10 mL), and the solution was cooled to -78 °C. A solution of n-butyllithium in tetrahydrofuran (2.5 M, 1 mL) was added dropwise. The mixture was allowed to react at -78 °C for 1.5 h. A solution of compound **35b** (350 mg, 1.1 mmol) in 2-methyltetrahydrofuran (3 mL) was added. The mixture was allowed to react at -78 °C for 1 h, naturally warmed to room temperature, and allowed to react for 16 h. The reaction was quenched by dropwise addition of methanol (5 mL). The filtrate was concentrated under reduced pressure to remove the solvent. The residue was purified by silica gel column chromatography using eluent system B to give the title compound **35c** (320 mg, 52% yield).

MS m/z (ESI): 556.3[M+1].

Step 4

1-(4-(6-(Benzyloxy)-2-(cyclobutylmethyl)-3,4-dihydronaphthalen-1-yl)phenyl)-4-(dime thoxymethyl)piperidine **35d**

Compound **35c** (320 mg, 0.57 mmol) was dissolved in methanol (5 mL), and p-toluenesulfonic acid monohydrate (6 mg, 0.03 mmol) was added. The mixture was allowed to react at 80 °C for 30 min. The reaction mixture was cooled to room temperature and filtered. The filter cake was dried to give the title compound **35d** (200 mg, 64% yield).

MS m/z (ESI): 538.2[M+1].

Step 5

(5,6)-*cis*-6-(Cyclobutylmethyl)-5-(4-(4-(dimethoxymethyl)piperidin-1-yl)phenyl)-5,6,7, 8-tetrahydronaphthalen-2-ol **35e**

Compound **35d** (200 mg, 0.4 mmol) was dissolved in 7.5 mL of a mixed solvent of ethyl acetate and methanol (V/V = 2/1), and palladium on carbon (80 mg, 10 wt%) was added. The mixture was allowed to react at room temperature for 16 h in a hydrogen atmosphere. The reaction mixture was filtered. The filtrate was concentrated under reduced pressure to remove the solvent, giving the title compound **35e** (167 mg, 99% yield).

MS m/z (ESI): 450.2[M+1].

Step 6

(5*R*,6*R*)-6-(Cyclobutylmethyl)-5-(4-(4-(dimethoxymethyl)piperidin-1-yl)phenyl)-5,6,7, 8-tetrahydronaphthalen-2-ol **35f**

Compound **35e** (167 mg, 0.4 mmol) was resolved by preparative chiral chromatography (resolution conditions: column: CHIRALPAK IE, 20 mm × 250 mm, 5 μm; mobile phases: A: n-hexane, B: ethanol (0.5% $NH_3$), A: 70%, B: 30%, flow rate: 20 mL/min) to give the title compound **35f** (70 mg).

MS m/z (ESI): 450.2[M+1].

Chiral HPLC analysis method: retention time 4.94 min, chiral purity: 100% (column: CHIRALPAK IE A: n-hexane, B: EtOH (+ 0.1% DEA), A: 30%, B: 70%).

Step 7

1-(4-((1*R*,2*R*)-2-(Cyclobutylmethyl)-6-hydroxy-1,2,3,4-tetrahydronaphthalen-1-yl)phen yl)piperidine-4-carbaldehyde **35g**

Compound **35f** (70 mg, 0.15 mmol) was dissolved in tetrahydrofuran (2 mL), and dilute sulfuric acid (2 M, 1 mL, 2 mmol) was added. The mixture was allowed to react at room temperature for 2 h. The reaction mixture was made neutral with a saturated solution of sodium bicarbonate and extracted with dichloromethane (20 mL × 3). The organic phases were combined, dried over anhydrous sodium sulfate and filtered. The filtrate was concentrated under reduced pressure to remove the solvent, giving the title compound **35g** (62 mg, crude, 98% yield).

MS m/z (ESI): 390.3[M+1].

Step 8

(*S*)-3-(5-(4-((1-(4-((1*R*,2*R*)-2-(Cyclobutylmethyl)-6-hydroxy-1,2,3,4-tetrahydronaphthal en-1-yl)phenyl)piperidin-4-yl)methyl)piperazin-1-yl)-1-oxoisoindolin-2-yl)piperidine-2, 6-dione **35**

Compound **5h** (115 mg, 0.23 mmol) was added to 5 mL of a mixed solvent of dichloromethane and methanol (V/V = 1/1), and sodium acetate (57 mg, 0.69 mmol) was added. After 10 min of reaction, compound **35g** (62 mg, 0.15 mmol) was added. After 10 min of reaction, sodium triacetoxyborohydride (98 mg, 0.46 mmol) was added. The mixture was allowed to react at room temperature for 1 h. The reaction mixture was concentrated under reduced pressure to remove the solvent. The residue was purified by preparative high performance liquid chromatography (instrument model: Waters 2545; elution system: 10 mM ammonium bicarbonate, 60% water, 40% acetonitrile) to give the title compound **35** (60 mg, 54% yield).

MS m/z (ESI): 716.5[M+1].

$^1H$ NMR (500 MHz, DMSO-$d_6$) $\delta$ 10.95 (bs, 1H), 9.01 (s, 1H), 7.52 (d, 1H), 7.10-7.04 (m, 2H), 6.79-6.74 (m, 4H), 6.60 (d, 1H), 6.51 (d, 1H), 6.43 (dd, 1H), 5.05-5.02 (m, 1H), 4.33, 4.21 (dd, 2H), 3.84 (d, 1H), 3.63-3.57 (m, 2H), 3.31-3.26 (m, 4H), 2.94-2.68 (m, 5H), 2.63-2.55 (m, 5H), 2.46-2.32 (m, 2H), 2.22 (d, 2H), 2.04-1.93 (m, 3H), 1.84-1.61 (m, 6H), 1.59-1.41 (m, 4H), 1.34-1.30 (m, 1H), 1.26-1.15 (m, 2H), 0.87-0.81 (m, 1H).

Example 36

(*S*)-3-(5-(4-((1-(2-Fluoro-4-((*R*)-6'-hydroxy-3',4'-dihydro-1'*H*-spiro[cyclopentane-1,2'-n aphthalen]-1'-yl)phenyl)piperidin-4-yl)methyl)piperazin-1-yl)-1-oxoisoindolin-2-yl)pipe ridine-2,6-dione **36**

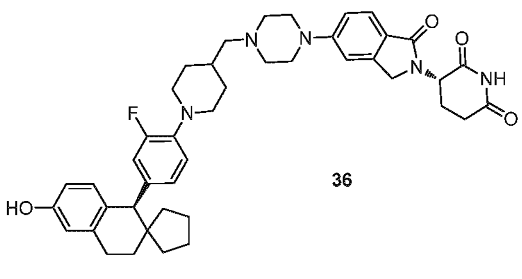

**36**

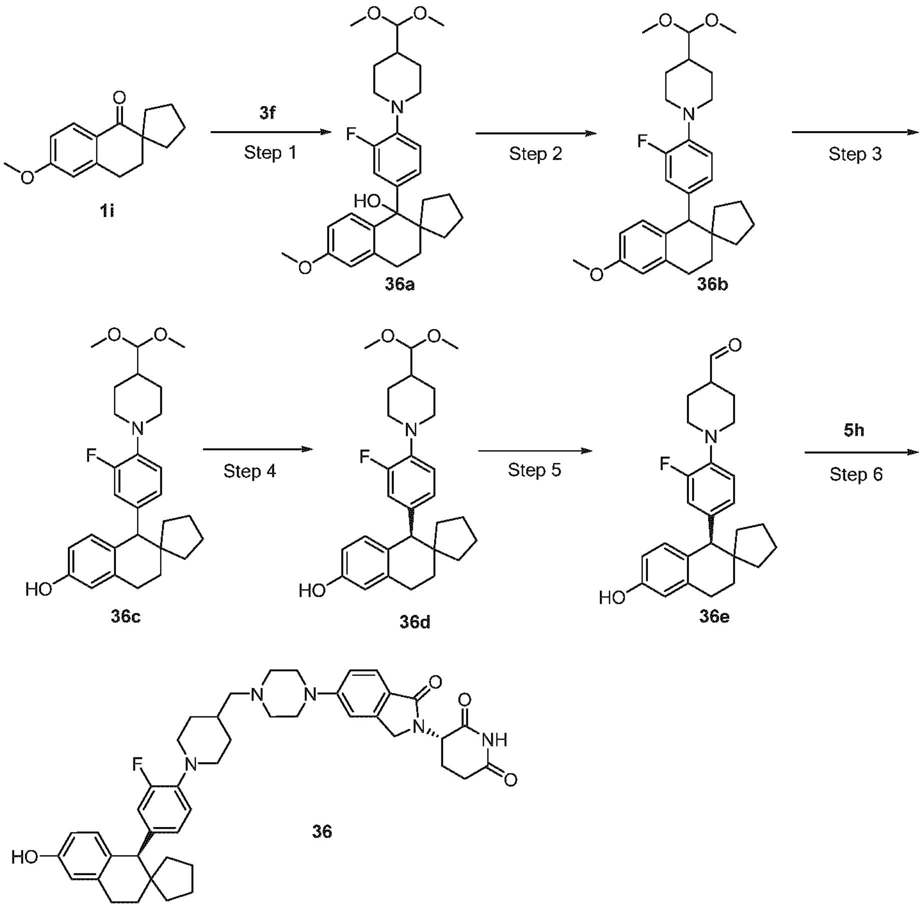


Step 1

1'-(4-(4-(Dimethoxymethyl)piperidin-1-yl)-3-fluorophenyl)-6'-methoxy-3',4'-dihydro-1' *H*-spiro[cyclopentane-1,2'-naphthalen]-1'-ol **36a**

Compound **3f** (1.7 g, 5.1 mmol) was added to dry tetrahydrofuran (30 mL), and n-butyllithium (2.5 M, 2.5 mL, 6.25 mmol) was added dropwise at -78 °C in a nitrogen atmosphere. After the dropwise addition, the mixture was allowed to react at -78 °C for 30 min. A solution of compound **1i** (1.2 g, 5.2 mmol) in tetrahydrofuran was added dropwise. The mixture was allowed to react at -78 °C for 1 h, warmed to room temperature, and allowed to react for 1 h. The reaction mixture was quenched with a saturated solution of ammonium chloride (30 mL) and extracted with ethyl acetate (20 mL × 3). The organic phases were combined, dried over anhydrous sodium sulfate and filtered. The filtrate was concentrated under reduced pressure to remove the solvent. The residue was purified by silica gel column chromatography using eluent system A to give the title compound **36a** (650 mg, 26% yield).

MS m/z (ESI): 484.1[M+1].

Step 2

4-(Dimethoxymethyl)-1-(2-fluoro-4-(6'-methoxy-3',4'-dihydro-1'*H*-spiro[cyclopentane-1,2'-naphthalen]-1'-yl)phenyl)piperidine **36b**

Compound **36a** (600 mg, 1.2 mmol) was dissolved in dichloromethane (20 mL). Under an ice bath condition, triethylsilane (435 mg, 3.7 mmol) was added, and trifluoroacetic acid (285 mg, 2.5 mmol) was added dropwise. After the dropwise addition, the mixture was allowed to react at room temperature for 16 h. The reaction mixture was made neutral with saturated sodium bicarbonate and extracted with dichloromethane (10 mL × 3). The organic phases were combined, dried over anhydrous sodium sulfate and filtered. The filtrate was concentrated under reduced pressure to remove the solvent. The residue was purified by silica gel column chromatography using eluent system A to give the title compound **36b** (640 mg, 100% yield).

MS m/z (ESI): 468.5[M+1].

Step 3

1'-(4-(4-(Dimethoxymethyl)piperidin-1-yl)-3-fluorophenyl)-3',4'-dihydro-1'*H*-spiro[cycl opentane-1,2'-naphthalen]-6'-ol **36c**

Compound **36b** (640 mg, 1.4 mmol) was dissolved in dichloromethane (15 mL), and boron tribromide (1 M, 2.8 mL, 2.8 mmol) was added. The mixture was allowed to react at room temperature for 16 h. The reaction mixture was made neutral with saturated sodium bicarbonate and extracted with dichloromethane (10 mL × 3). The organic phases were combined, dried over anhydrous sodium sulfate and filtered. The filtrate was concentrated under reduced pressure to remove the solvent. The residue was purified by silica gel column chromatography using eluent system A to give the title compound **36c** (220 mg, 35% yield).
MS m/z (ESI): 454.2[M+1].

Step 4

(*R*)-1'-(4-(4-(Dimethoxymethyl)piperidin-1-yl)-3-fluorophenyl)-3',4'-dihydro-1'*H*-spiro[ cyclopentane-1,2'-naphthalen]-6'-ol **36d**

Compound **36c** (220 mg, 0.48 mmol) was resolved by preparative chiral chromatography (resolution conditions: column: CHIRALPAK IE, 250 mm × 21.2 mm, 5 μm; mobile phases: A: n-hexane, B: ethanol (+ 10 nM $NH_3$), A: 70%, B: 30%) to give the title compound **36d** (43 mg).
MS m/z (ESI): 454.3[M+1].
Chiral HPLC analysis method: retention time 5.8 min, chiral purity: 100% (column: CHIRALPAK IE A: n-hexane, B: EtOH (+ 0.1% DEA), A: 30%, B: 70%).

Step 5

(*R*)-1-(2-Fluoro-4-(6'-hydroxy-3',4'-dihydro-1'*H*-spiro[cyclopentane-1,2'-naphthalen]-1'-yl)phenyl)piperidine-4-carbaldehyde **36e**

Compound **36d** (40 mg, 0.09 mmol) was dissolved in tetrahydrofuran (5 mL), and dilute sulfuric acid (2 M, 1 mL, 2 mmol) was added. The mixture was allowed to react at 70 °C for 1 h. The reaction mixture was made neutral with a saturated solution of sodium bicarbonate and extracted with dichloromethane (10 mL × 3). The organic phases were combined, dried over anhydrous sodium sulfate and filtered. The filtrate was concentrated under reduced pressure to remove the solvent, giving the title compound **36e** (36 mg, crude, 100% yield).
MS m/z (ESI): 408.2[M+1].

Step 6

(*S*)-3-(5-(4-((1-(2-Fluoro-4-((*R*)-6'-hydroxy-3',4'-dihydro-1'*H*-spiro[cyclopentane-1,2'-n aphthalen]-1'-yl)phenyl)piperidin-4-yl)methyl)piperazin-1-yl)-1-oxoisoindolin-2-yl)pipe ridine-2,6-dione **36**

Compound **5h** (29 mg, 0.09 mmol) was added to 5 mL of a mixed solvent of dichloromethane and methanol (V/V = 4/1), and sodium acetate (37 mg, 0.17 mmol) was added. After 10 min of reaction, compound **36e** (35 mg, 0.08 mmol) was added. After 10 min of reaction, sodium triacetoxyborohydride (37 mg, 0.17 mmol) was added. The mixture was allowed to react at room temperature for 1 h. The reaction mixture was concentrated under reduced pressure to remove the solvent. The residue was purified by preparative high performance liquid chromatography (instrument model: Waters 2545; elution system: 10 mM ammonium bicarbonate, 60% water, 40% acetonitrile) to give the title compound **36** (35 mg, 57% yield).
MS m/z (ESI): 720.6[M+1].
$^1$H NMR (500 MHz, DMSO-$d_6$) $\delta$ 11.90 (s, 1H), 10.95 (s, 1H), 9.05 (s, 1H), 7.54-7.52 (m, 1H), 7.07-7.05 (m, 2H), 6.90-6.87 (m, 1H), 6.72-6.70 (m, 1H), 6.63-6.60 (m, 2H), 6.54-6.52 (m, 1H), 6.45-6.43 (m, 1H), 5.07-5.04 (m, 1H), 4.32, 4.22 (dd, 2H), 3.63 (s, 1H), 3.34-3.30 (m, 4H), 2.93-2.87 (m, 2H), 2.81-2.77 (m, 2H), 2.61-2.52 (m, 4H), 2.39-2.37 (m, 2H), 2.24-2.22 (m, 2H), 1.97-1.95 (m, 2H), 1.82-1.79 (m, 2H), 1.76-1.74 (m, 2H), 1.65-1.63 (m, 3H), 1.55-1.43 (m, 4H), 1.39-1.36 (m, 5H).

Example 37

(*S*)-3-(5-(4-((1-(2-Fluoro-4-((1*R*,2*R*)-6-hydroxy-2-isobutyl-1,2,3,4-tetrahydronaphthalen -1-yl)phenyl)piperidin-4-yl)methyl)piperazin-1-yl)-1-oxoisoindolin-2-yl)piperidine-2,6-dione **37**

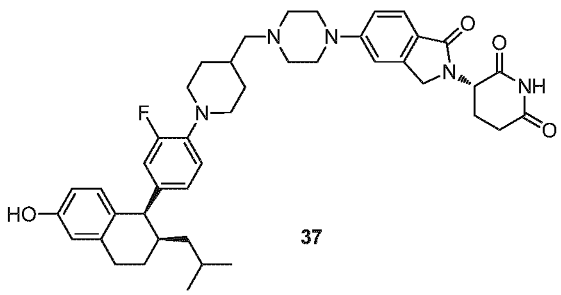

**37**

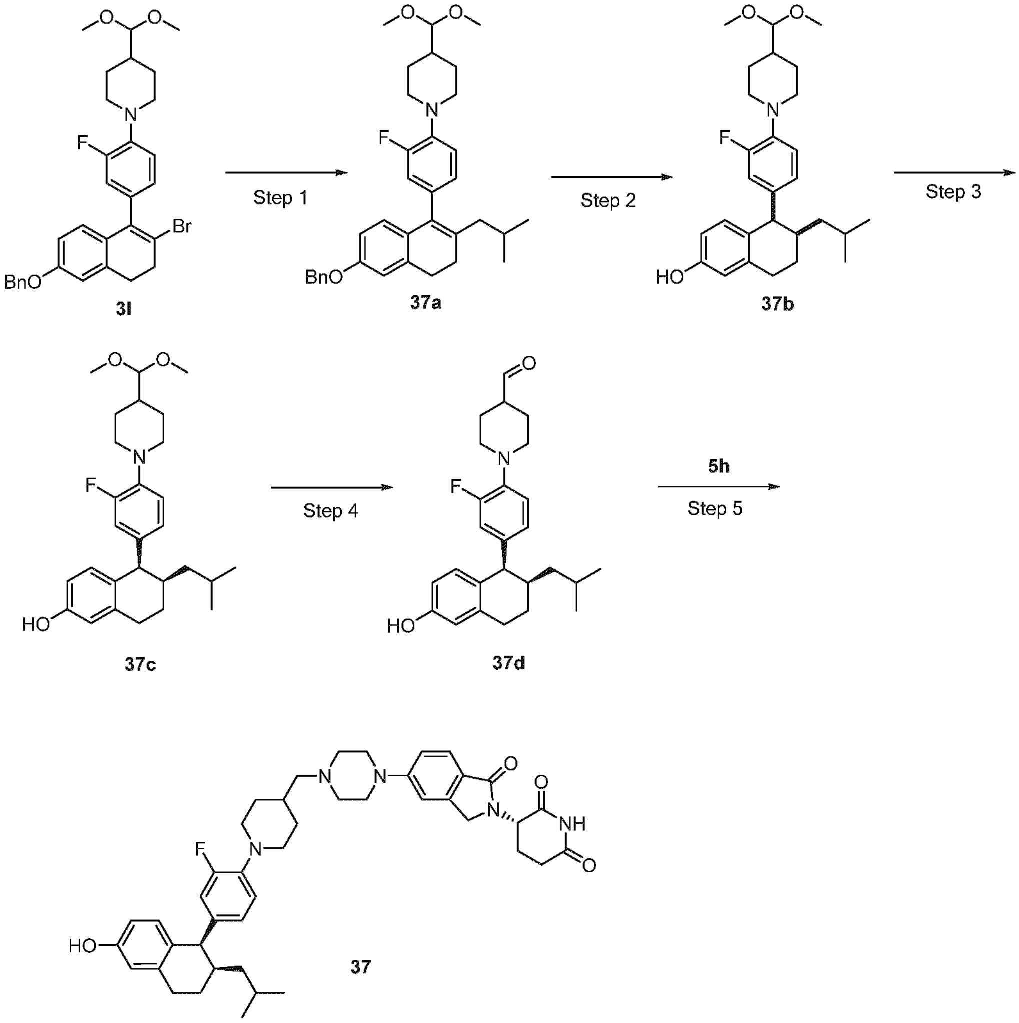


**3l** **37a** **37b** **37c** **37d** **37**

Step 1

1-(4-(6-(Benzyloxy)-2-isobutyl-3,4-dihydronaphthalen-1-yl)-2-fluorophenyl)-4-(dimeth oxymethyl)piperidine **37a**

In a nitrogen atmosphere, a solution of zinc chloride in tetrahydrofuran (1 M, 5.5 mL) was slowly added dropwise to an ice-bath-cooled solution of tert-butylmagnesium chloride in tetrahydrofuran (1 M, 5 mL, Shanghai Adamas Reagent

Co., Ltd.). After the dropwise addition, the mixture was allowed to react at room temperature for 3 h. A solution of compound **31** (300 mg, 0.53 mmol) and methanesulfonato(2-dicyclohexylphosphino-2',6'-diisopropoxy-1,1'-biphenyl)(2-amino-1,1'-biphenyl-2-yl)palladium(II) (Ruphos-Pd G3, 66 mg, 0.08 mmol, Jiangsu Aikon Biopharmaceutical R&D Co., Ltd.) in tetrahydrofuran (2 mL) was added. After the dropwise addition, the mixture was allowed to react at room temperature for 16 h. A saturated solution of ammonium chloride (7 mL) was added, and the organic phase was separated. The aqueous phase was extracted with dichloromethane (15 mL × 2). The organic phases were combined, dried over anhydrous sodium sulfate, filtered and concentrated under reduced pressure to remove the solvent. The residue was purified by silica gel column chromatography using eluent system B to give the title compound **37a** (230 mg, 79% yield).
MS m/z (ESI): 544.3[M+1].

Step 2

(5, 6)-*cis*-5-(4-(4-(Dimethoxymethyl)piperidin-1-yl)-3-fluorophenyl)-6-isobutyl-5, 6,7, 8-tetrahydronaphthalen-2-ol **37b**

Compound **37a** (230 mg, 0.42 mmol) was added to methanol (20 mL), and palladium hydroxide on carbon (150 mg, 20 wt%) was added. The mixture was allowed to react at room temperature for 16 h in a hydrogen atmosphere. The reaction mixture was filtered. The filtrate was concentrated under reduced pressure to remove the solvent, giving the title compound **37b** (80 mg, crude, 93% yield).
MS m/z (ESI): 456.3[M+1].

Step 3

(5*R*,6*R*)-5-(4-(4-(Dimethoxymethyl)piperidin-1-yl)-3-fluorophenyl)-6-isobutyl-5,6,7,8-t etrahydronaphthalen-2-ol **37c**

Compound **37b** (180 mg, 0.40 mmol) was resolved by preparative chiral chromatography (resolution conditions: column: CHIRALPAK IE, 20 mm × 250 mm, 5 μm; mobile phases: A: n-hexane, B: ethanol (+ 20 mmol $NH_3$), A: 85%, B: 15%, flow rate: 20 mL/min) to give the title compound **37c** (47 mg).
MS m/z (ESI): 456.3[M+1].
Chiral HPLC analysis method: retention time 3.98 min, chiral purity: 100% (column: CHIRALPAK IE A: n-hexane, B: EtOH (+ 0.1% DEA), A: 85%, B: 15%).

Step 4

1-(2-Fluoro-4-((1*R*,2*R*)-6-hydroxy-2-isobutyl-1,2,3,4-tetrahydronaphthalen-1-yl)phenyl )piperidine-4-carbaldehyde **37d**

Compound **37c** (47 mg, 0.10 mmol) was dissolved in tetrahydrofuran (2 mL), and dilute sulfuric acid (2 M, 0.15 mL, 0.3 mmol) was added. The reaction was heated at 55 °C for 1 h. The reaction mixture was cooled to room temperature, made neutral with a saturated solution of sodium bicarbonate and extracted with ethyl acetate (10 mL × 3). The organic phases were combined, dried over anhydrous sodium sulfate and filtered. The filtrate was concentrated under reduced pressure to remove the solvent, giving the title compound **37d** (42 mg, 99% yield).
MS m/z (ESI): 410.2[M+1].

Step 5

(*S*)-3-(5-(4-((1-(2-Fluoro-4-((1*R*,2*R*)-6-hydroxy-2-isobutyl-1,2,3,4-tetrahydronaphthalen -1-yl)phenyl)piperidin-4-yl)methyl)piperazin-1-yl)-1-oxoisoindolin-2-yl)piperidine-2,6-dione **37**

Compound **5h** (64 mg, 0.13 mmol) was added to 3.5 mL of a mixed solvent of dichloromethane and methanol (V/V = 6/1), and sodium acetate (160 mg, 1.95 mmol) was added. After 10 min of reaction, compound **37d** (47 mg, 0.10 mmol) was added. After 15 min of reaction, sodium triacetoxyborohydride (45 mg, 0.21 mmol) was added. The mixture was allowed to react at room temperature for 1 h. The reaction mixture was concentrated under reduced pressure to remove the solvent. The residue was purified by preparative high performance liquid chromatography (Waters 2767-SQ Detecor2; elution system: 10 mM ammonium bicarbonate, 60% water, 40% acetonitrile) to give the title compound **37** (26 mg, 35% yield).
MS m/z (ESI): 722.5[M+1].
$^1H$ NMR (500 MHz, DMSO-$d_6$) δ 10.95 (s, 1H), 9.08 (s, 1H), 7.53 (d, 1H), 7.08-7.05 (m, 2H), 6.89(t, 1H), 6.71-6.57 (m, 3H), 6.53 (d, 1H), 6.45 (dd, 1H), 5.05-5.02 (m, 1H), 4.32, 4.22 (dd, 2H), 3.93 (d, 1H), 3.38-3.34 (m, 6H), 3.31-3.26 (m, 4H), 2.96-2.73 (m, 3H), 2.66-2.56 (m, 3H), 2.43-2.32 (m, 1H), 2.23 (d, 2H), 2.01-1.90 (m, 2H), 1.85-1.41

(m, 6H), 1.32-1.21 (m, 2H), 1.09-0.98 (m, 1H), 0.84 (t, 6H), 0.73-0.64 (m, 1H).

Example 38

(*S*)-3-(5-(4-((1-(4-((1*R*,2*R*)-6-Hydroxy-2-(2-ethylbutyl)-1,2,3,4-tetrahydronaphthalen-1-yl)phenyl)piperidin-4-yl)methyl)piperazin-1-yl)-1-oxoisoindolin-2-yl)piperidine-2,6-dio **ne 38**

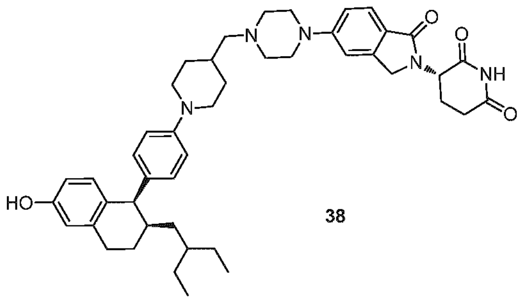

**38**

By using the synthesis scheme of Example 26-1 in which 2-ethylbutylmagnesium bromide was used in place of the starting material tert-butylmagnesium chloride of step 1, the title compound **38** (200 mg) was prepared.

MS m/z (ESI): 732.5[M+1].

$^1$H NMR (500 MHz, DMSO-$d_6$) $\delta$ 10.95 (bs, 1H), 9.02 (s, 1H), 7.52 (d, 1H), 7.10-7.04 (m, 2H), 6.80-6.72 (m, 4H), 6.62 (d, 1H), 6.52 (d, 1H), 6.43 (dd, 1H), 5.05-5.02 (m, 1H), 4.32, 4.22 (dd, 2H), 3.86 (d, 1H), 3.65-3.56 (m, 2H), 3.38-3.34 (m, 4H), 3.32-3.25 (m, 4H), 2.95-2.73 (m, 3H), 2.65-2.54 (m, 3H), 2.41-2.32 (m, 1H), 2.25-2.18 (m, 2H), 2.02-1.87 (m, 2H), 1.84-1.61 (m, 4H), 1.55-1.44 (m, 2H), 1.29-1.14 (m, 2H), 1.07-1.25 (m, 6H), 0.72-0.64 (m, 6H).

Example 39

(*S*)-3-(5-(4-((1-(4-((1*R*,2*R*)-6-Hydroxy-2-neopentyl-1,2,3,4-tetrahydronaphthalen-1-yl)p henyl)piperidin-4-yl)methyl)piperazin-1-yl)-1-oxoisoindolin-2-yl)piperidine-2,6-dione **39**

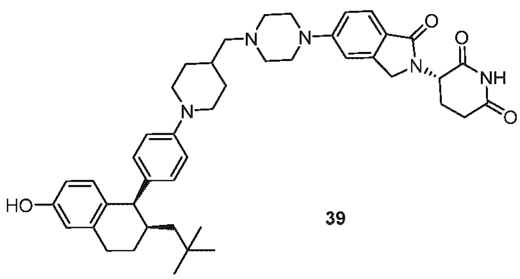

**39**

By using the synthesis scheme of Example 26-1 in which 2,2-dimethylpropylmagnesium bromide was used in place of the starting material tert-butylmagnesium chloride of step 1, the title compound **39** (230 mg) was prepared. MS m/z (ESI): 718.3[M+1].

$^1$H NMR (500 MHz, DMSO-$d_6$) $\delta$ 11.90 (s, 1H), 10.95 (s, 1H), 9.01 (s, 1H), 7.53-7.50 (m, 1H), 7.07-7.05 (m, 2H), 6.81-6.77 (m, 4H), 6.60-6.59 (m, 1H), 6.63-6.51 (m, 1H), 6.43-6.41 (m, 1H), 5.07-5.03 (m, 1H), 4.32, 4.22 (dd, 2H), 3.77-3.76 (m, 1H), 3.61-3.58 (m, 2H), 3.35-3.28 (m, 4H), 2.94-2.81 (m, 1H), 2.83-2.80 (m, 2H), 2.65-2.54 (m, 4H), 2.39-2.45 (m, 1H), 2.23-2.22 (m, 2H), 1.98-1.95 (m, 1H), 1.93-1.90 (m, 4H), 1.82-1.76 (m, 2H), 1.68-1.62 (m, 2H), 1.39-1.36 (m, 1H), 1.24-1.11 (m, 2H), 0.89 (s, 9H), 0.57-0.53 (m, 1H).

Example 40

(*S*)-3-(5-(4-((1-(4-((1*R*,2*S*)-6-Hydroxy-2-isopentyl-1,2,3,4-tetrahydronaphthalen-1-yl)ph enyl)piperidin-4-yl)methyl)piperazin-1-yl)-1-oxoisoindolin-2-yl)piperidine-2,6-dione **40**

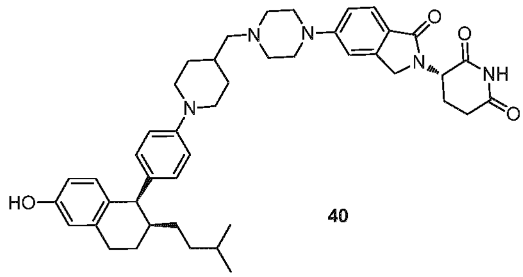

**40**

By using the synthesis scheme of Example 26-1 in which isopentylmagnesium bromide was used in place of the starting material tert-butylmagnesium chloride of step 1, the title compound **40** (180 mg) was prepared.

MS m/z (ESI): 718.4[M+1].

$^1$H NMR (500 MHz, DMSO-$d_6$) $\delta$ 11.90 (s, 1H), 10.95 (s, 1H), 9.02 (s, 1H), 7.54-7.52 (m, 1H), 7.08-7.05 (m, 2H), 6.88-6.77 (m, 4H), 6.63-6.61 (m, 1H), 6.53-6.51 (m, 1H), 6.44-6.42 (m, 1H), 5.07-5.03 (m, 1H), 4.35, 4.23 (dd, 2H), 3.92-3.91 (m, 1H), 3.63-3.58 (m, 2H), 3.29-3.28 (m, 4H), 2.94-2.71 (m, 3H), 2.61-2.55 (m, 4H), 3.41-3.33 (m, 1H), 2.23-2.21 (m, 2H), 2.00-1.95 (m, 1H), 1.94-1.92 (m, 2H), 1.81-1.78 (m, 2H), 1.77-1.73 (m, 1H), 1.69-1.64 (m, 1H), 1.58-1.51 (m, 2H), 1.49-1.41 (m, 1H), 1.34-1.29 (m, 1H), 123-116 (s, 5H), 0.84 (d, 6H).

Example 41

3-(5-(4-((1-(4-((1*R*,2*R*)-6-Hydroxy-2-isobutyl-1,2,3,4-tetrahydronaphthalen-1-yl)phenyl )piperidin-4-yl)methyl)piperazin-1-yl)-1-oxoisoindolin-2-yl)piperidine-2,6-dione **41** (a 1:1 mixture of diastereomers)

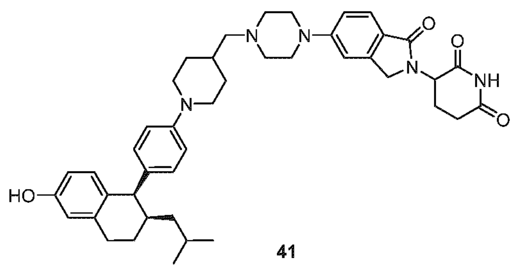

**41**

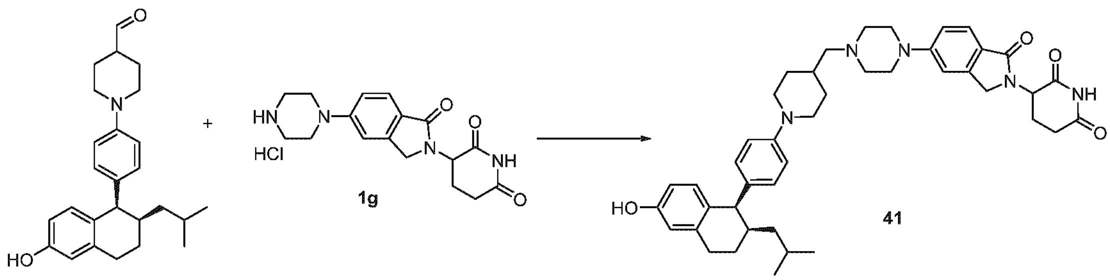

**26c-1**, **1g**, **41**

Compound **1g** (8 mg, 0.02 mmol) was added to 2 mL of a mixed solvent of dichloromethane and methanol (V/V = 4/1), and sodium acetate (10 mg, 0.12 mmol) was added. After 10 min of reaction, compound **26c-1** (6 mg, 0.015 mmol) was added. After 15 min of reaction, sodium triacetoxyborohydride (8 mg, 0.04 mmol) was added. The mixture was allowed to react at room temperature for 1 h. The reaction mixture was concentrated under reduced pressure to

remove the solvent. The residue was purified by silica gel column chromatography using eluent system A to give the title compound **41** (4 mg, a 1:1 mixture of diastereomers, 37% yield).

MS m/z (ESI): 704.4[M+1].

$^1$H NMR (500 MHz, DMSO-$d_6$) $\delta$ 10.95 (bs, 1H), 9.02 (s, 1H), 7.52 (d, 1H), 7.10-7.04 (m, 2H), 6.80-6.72 (m, 4H), 6.62 (d, 1H), 6.52 (d, 1H), 6.43 (dd, 1H), 5.05 (dd, 1H), 4.32, 4.22 (dd, 2H), 3.86 (d, 1H), 3.65-3.56 (m, 2H), 3.38-3.34 (m, 4H), 3.32-3.25 (m, 4H), 2.95-2.73 (m, 3H), 2.65-2.54 (m, 3H), 2.41-2.32 (m, 1H), 2.25-2.18 (m, 2H), 2.02-1.87 (m, 2H), 1.84-1.61 (m, 4H), 1.55-1.44 (m, 2H), 1.29-1.14 (m, 2H), 1.07-0.98 (m, 1H), 0.84 (t, 6H), 0.72-0.64 (m, 1H).

Example 42

(*R*)-3-(5-(4-((1-(4-((1*R*,2*R*)-6-Hydroxy-2-isobutyl-1,2,3,4-tetrahydronaphthalen-1-yl)ph enyl)piperidin-4-yl)methyl)piperazin-1-yl)-1-oxoisoindolin-2-yl)piperidine-2,6-dione **42**

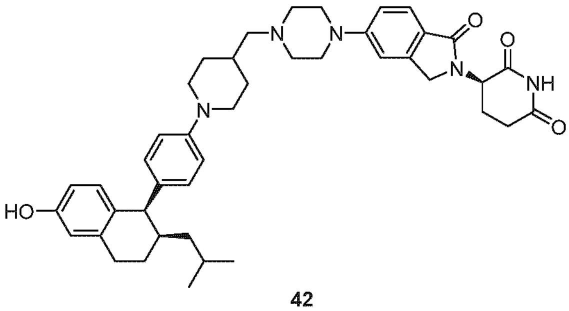

**42**

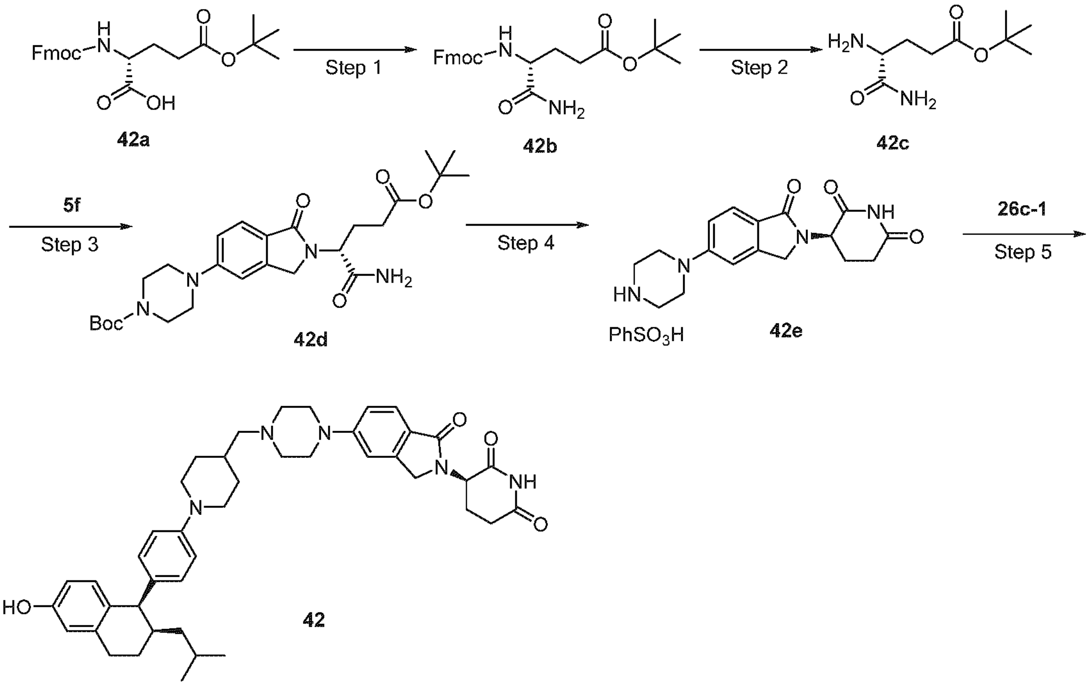


**42a** **42b** **42c** **42d** **42e** **42**

Step 1

tert-Butyl (*R*)-4-((((9*H*-fluoren-9-yl)methoxy)carbonyl)amino)-5-amino-5-oxopentanoate **42b**

(*R*)-2-((((9*H*-Fluoren-9-yl)methoxy)carbonyl)amino)-5-(tert-butoxy)-5-oxopentanoic acid **42a** (20 g, 47 mmol, Shanghai Hanhong Scientific Co., Ltd.) and di-tert-butyl dicarbonate (16.42 g, 75 mmol) were added to 1,4-dioxane (150 mL). The internal temperature was controlled at below 5 °C using an ice-water bath in a nitrogen atmosphere. Pyridine (7.44 g, 94 mmol) was added dropwise. After the dropwise addition, the mixture was allowed to react in an ice-water bath for 30 min. Ammonium bicarbonate (33.45 g, 141 mmol) was added. The mixture was warmed to room temperature

and was allowed to react for 16 h. The reaction mixture was concentrated under reduced pressure to remove the solvent, and ethyl acetate (500 mL) and water (500 mL) were added for extraction. The mixture was washed with dilute hydrochloric acid (500 mL × 3) and filtered. The filtrate was concentrated under reduced pressure to remove the solvent, giving the title compound **42b** (24.13 g, crude). The product was directly used in the next step without purification.
MS m/z (ESI): 425.1[M+1].

Step 2

tert-Butyl (*R*)-4,5-diamino-5-oxopentanoate **42c**

Compound **42b** (20 g, 47.1 mmol) and diethylamine (10 mL) were dissolved in dichloromethane (100 mL). The mixture was allowed to react at room temperature for 16 h. The reaction mixture was concentrated under reduced pressure to remove the solvent. The residue was dissolved in methanol (150 mL), and water (5 mL) was added. The mixture was washed with n-heptane (150 mL × 3). The methanol layer was concentrated under reduced pressure to remove the solvent, giving the title compound **42c** (9.8 g, crude). The product was directly used in the next step without purification.
MS m/z (ESI): 203.1[M+1].

Step 3

tert-Butyl (*R*)-4-(2-(1-amino-5-(tert-butoxy)-1, 5-dioxopentan-2-yl)-1-oxoisoindolin-5-yl)piperazin e-1-carboxylate **42d**

Compound **5f** (3 g, 8.6 mmol) and compound **42c** (2.6 g, 12.9 mmol) were added to methanol (25 mL), and the internal temperature was controlled at below 5 °C using an ice-water bath. Acetic acid (0.77 mL, 12.9 mmol) and sodium cyanoborohydride (1.1 g, 17.2 mmol) were added dropwise. The mixture was allowed to react at room temperature for 16 h. The reaction mixture was concentrated under reduced pressure to remove the solvent. Ethyl acetate (100 mL) and water (100 mL) were added to the residue. The organic phase was separated, washed with a saturated solution of citric acid (100 mL × 3), dried over anhydrous sodium sulfate and filtered. The filtrate was concentrated under reduced pressure to remove the solvent. The residue was purified by silica gel column chromatography using eluent system A to give the title product **42d** (4.03 g, 93% yield).
MS m/z (ESI): 503.2[M+1].

Step 4

(*R*)-3-(1-Oxo-5-(piperazin-1-yl)isoindolin-2-yl)piperidine-2,6-dione benzenesulfonate **42e**

Compound **42d** (4.03 g, 8 mmol) and benzenesulfonic acid (2.54 g, 16.1 mmol) were added to acetonitrile (30 mL), and the reaction was heated at 90 °C for 16 h. The reaction mixture was concentrated under reduced pressure to remove the solvent. The residue was washed with ethyl acetate (100 mL × 3) and dried to give the title compound **42e** (1.84 g, 47% yield).
MS m/z (ESI): 329.1[M+1].
$^1$H NMR (400 MHz, DMSO-$d_6$): δ 10.97 (s, 1H), 8.71 (br, 2H), 7.60-7.58 (m, 3H), 7.32-7.30 (m, 3H), 7.16-7.13 (m, 2H), 5.09-5.04 (m, 1H), 4.38-4.21 (m, 2H), 3.52-3.49 (m, 4H), 3.25 (s, 4H), 2.94-2.87 (m, 1H), 2.62-2.58 (m, 1H), 2.41-2.36 (m, 1H), 2.00-1.96 (m, 1H).
Chiral HPLC analysis method: retention time 11.55 min, chiral purity: 99% ee (column: CHIRALPAK OJ-H: A: n-hexane (+ 0.1% DEA), B: EtOH; A: 50%, B: 50%).

Step 5

(*R*)-3-(5-(4-((1-(4-((1*R*,2*R*)-6-Hydroxy-2-isobutyl-1,2,3,4-tetrahydronaphthalen-1-yl)ph enyl)piperidin-4-yl)methyl)piperazin-1-yl)-1-oxoisoindolin-2-yl)piperidine-2,6-dione **42**

Compound **42e** (18 mg, 0.04 mmol) was added to 2 mL of a mixed solvent of dichloromethane and methanol (V/V = 4/1), and sodium acetate (13 mg, 0.16 mmol) was added. After 10 min of reaction, compound **26c-1** (6 mg, 0.015 mmol) was added. After 15 min of reaction, sodium triacetoxyborohydride (13 mg, 61.3 μmol) was added. The mixture was allowed to react at room temperature for 1 h. The reaction mixture was concentrated under reduced pressure to remove the solvent. The residue was purified by silica gel column chromatography using eluent system A to give the title compound **42** (8 mg, 74% yield).

MS m/z (ESI): 704.4[M+1].

$^1$H NMR (500 MHz, DMSO-$d_6$) $\delta$ 10.95 (bs, 1H), 9.02 (s, 1H), 7.52 (d, 1H), 7.10-7.04 (m, 2H), 6.80-6.72 (m, 4H), 6.62 (d, 1H), 6.52 (d, 1H), 6.43 (dd, 1H), 5.05 (dd, 1H), 4.32, 4.22 (dd, 2H), 3.86 (d, 1H), 3.65-3.56 (m, 2H), 3.38-3.34 (m, 4H), 3.32-3.25 (m, 4H), 2.95-2.73 (m, 3H), 2.65-2.54 (m, 3H), 2.41-2.32 (m, 1H), 2.25-2.18 (m, 2H), 2.02-1.87 (m, 2H), 1.84-1.61 (m, 4H), 1.55-1.44 (m, 2H), 1.29-1.14 (m, 2H), 1.07-0.98 (m, 1H), 0.84 (t, 6H), 0.72-0.64 (m, 1H).

Example 43

(*R*)-3-(5-(4-((1-(2-Fluoro-4-((1*R*,2*R*)-6-hydroxy-2-(tetrahydro-2*H*-pyran-4-yl)-1,2,3,4-te trahydronaphthalen-1-yl)phenyl)piperidin-4-yl)methyl)piperazin-1-yl)-1-oxoisoindolin-2-yl)piperidine-2,6-dione **43**

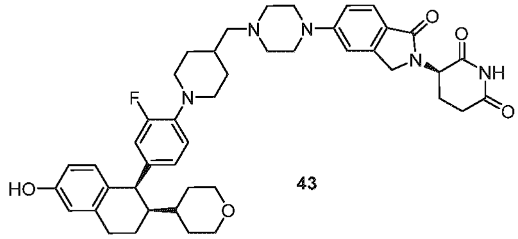

**43**

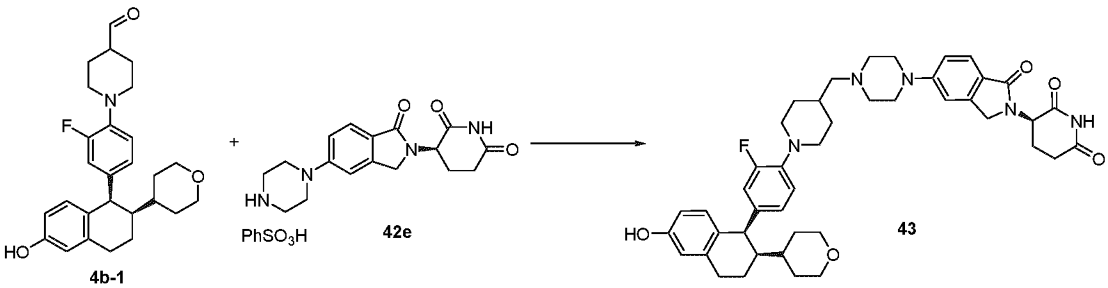

**4b-1** PhSO$_3$H **42e** **43**

Compound **42e** (117 mg, 0.24 mmol) was added to 5 mL of a mixed solvent of dichloromethane and methanol (V/V = 4/1), and sodium acetate (88 mg, 0.92 mmol) was added. After 10 min of reaction, compound **4b-1** (100 mg, 0.23 mmol) was added. After 15 min of reaction, sodium triacetoxyborohydride (98 mg, 0.46 mmol) was added. The mixture was allowed to react at room temperature for 1 h. The reaction mixture was concentrated under reduced pressure to remove the solvent. The residue was purified by silica gel column chromatography using eluent system A to give the title compound **43** (75 mg, 44% yield).

MS m/z (ESI): 750.3[M+1].

$^1$H NMR (500 MHz, DMSO-$d_6$) $\delta$ 10.95 (s, 1H), 9.08 (s, 1H), 7.53 (d, 1H), 7.07-7.05 (m, 2H), 6.89 (t, 1H), 6.76-6.74 (m, 1H), 6.69-6.64 (m, 2H), 6.53 (s, 1H), 6.45-6.43 (m, 1H), 5.07-5.03 (m, 1H), 4.32, 4.22 (dd, 2H), 4.12 (brs, 1H), 3.87-3.85 (m, 1H), 3.76-3.74 (m, 1H), 3.32-3.25 (m, 8H), 3.20 (t, 1H), 3.09 (t, 1H), 2.94-2.87 (m, 2H), 2.77-2.70 (m, 2H), 2.64-2.57 (m, 3H), 2.53-2.49 (m, 2H), 2.38-2.36 (m, 1H), 2.24-2.22 (m, 2H), 2.03-1.95 (m, 2H), 1.82-1.74 (m, 3H), 1.68-1.61 (m, 2H), 1.49-1.45 (m, 1H), 1.27-1.07 (m, 5H).

Biological Evaluation

The present disclosure is further described and explained below with reference to test examples. However, these test examples are not intended to limit the scope of the present disclosure.

Test Example 1: Inhibitory Effects of Compounds of the Present Disclosure on MCF7 Cell Proliferation

MCF7 cells (TCHu74, National Collection of Authenticated Cell Cultures) were cultured in an MEM (GE Healthcare, SH30024.01) complete medium containing 10% fetal bovine serum. On day 1 of the experiment, MCF7 cells were seeded into a 96-well plate at a density of 3,000 cells/well using MEM containing 2% fetal bovine serum, with each well containing 135 μL of cell suspension. The plate was incubated in a 37 °C, 5% $CO_2$ cell incubator overnight. The next day, different concentrations of test compounds prepared in media were added at 15 μL/well. The final concentrations

of the compounds were 9 concentration points obtained by 4-fold serial dilution from 1000 nM. A blank control containing 0.5% DMSO was set up. The plate was incubated in a 37 °C, 5% $CO_2$ cell incubator for 6 days. On day 8, the 96-well cell culture plate was taken out, and CellTiter-Glo® Luminescent Cell Viability Assay (Promega, G7573) was added at 75 μL/well. After the plate was left at room temperature for 10 min, luminescence signal readings were taken on a multilabel microplate reader (PerkinElmer, VICTOR 3). The $IC_{50}$ values for the inhibitory activity of the compounds were calculated from the concentrations of the compounds and the luminescence signal readings using Graphpad Prism software. The results are shown in Table 1.

Table 1. The inhibitory effects of the compounds of the present disclosure on MCF7 cell proliferation

| Example No. | $IC_{50}$ (nM) |
|---|---|
| **1** | 6.15 |
| **2-1** | 1.94 |
| **3** | 1.56 |
| **4-1** | 0.92 |
| **5** | 0.72 |
| **6** | 0.75 |
| **7** | 0.67 |
| **8-1** | 0.46 |
| **8-2** | 36.5 |
| **9** | 2.14 |
| **10** | 0.30 |
| **12-1** | 0.45 |
| **12-2** | 13.73 |
| **13-1** | 0.83 |
| **14-1** | 0.75 |
| **14-2** | 8.17 |
| **15-1** | 0.46 |
| **15-2** | 14.27 |
| **16** | 4.63 |
| **18-1** | 1.35 |
| **19** | 0.87 |
| **20** | 0.73 |
| **21** | 1.72 |
| **22** | 3.13 |
| **23-1** | 2.39 |
| **23-2** | 20.04 |
| **24** | 1.21 |
| **25-1** | 2.46 |
| **26-1** | 0.59 |
| **27** | 13.42 |
| **28** | 1.51 |
| **29** | 0.78 |

(continued)

| Example No. | $IC_{50}$ (nM) |
|---|---|
| **30** | 3.02 |
| **32** | 8.34 |
| **34** | 1.51 |
| **35** | 3.00 |
| **36** | 1.56 |
| **37** | 1.46 |
| **38** | 4.64 |
| **39** | 5.23 |
| **40** | 3.17 |
| **41** | 0.62 |
| **42** | 0.88 |
| **43** | 3.57 |

Conclusion: The compounds claimed by the present disclosure showed significant inhibitory effects on MCF7 cell proliferation.

Test Example 2: Biological Evaluation of Inhibitory Activity of Compounds of the Present Disclosure on Proliferation of ERα Mutant-Expressing MCF7 Cells

1. Objective
The objective of this experiment was to determine the inhibitory activity of the compounds of the present disclosure on the proliferation of ERα mutant-expressing MCF7 cells.
2. Method

MCF7 cells expressing the ERα Y537S mutant were constructed by lentiviral infection. MCF7/ERα Y537S cells were cultured in an MEM (GE Healthcare, SH30024.01) complete medium containing 10% fetal bovine serum and 1 μg/mL puromycin. On day 1 of the experiment, cells were seeded into a 96-well plate at a density of 3,000 cells/well using MEM containing 2% fetal bovine serum, with each well containing 135 μL of cell suspension. The plate was incubated in a 37 °C, 5% $CO_2$ cell incubator overnight. The next day, different concentrations of test compounds prepared in media were added at 15 μL/well. The final concentrations of the compounds were 9 concentration points obtained by 4-fold serial dilution from 1000 nM. A blank control containing 0.5% DMSO was set up. The plate was incubated in a 37 °C, 5% $CO_2$ cell incubator for 6 days. On day 8, the 96-well cell culture plate was taken out, and CellTiter-Glo® Luminescent Cell Viability Assay (Promega, G7573) was added at 75 μL/well. After the plate was left at room temperature for 10 min, luminescence signal readings were taken on a multilabel microplate reader (PerkinElmer, VICTOR 3). The $IC_{50}$ values for the inhibitory activity of the compounds were calculated from the concentrations of the compounds and the luminescence signal readings using Graphpad Prism software. The results are shown in Table 2.

Table 2. The $IC_{50}$ values for the inhibitory effects of the compounds of the present disclosure on the proliferation of ERα mutant-expressing MCF7 cells

| Example No. | $IC_{50}$ (nM) |
|---|---|
| **1** | 14.93 |
| **2-1** | 7.83 |
| **3** | 5.79 |
| **4-1** | 4.71 |
| **5** | 2.29 |
| **6** | 3.19 |

(continued)

| Example No. | $IC_{50}$ (nM) |
|---|---|
| **7** | 3.31 |
| **8-1** | 2.80 |
| **8-2** | 92.20 |
| **9** | 8.73 |
| **10** | 1.38 |
| **12-1** | 1.28 |
| **12-2** | 39.38 |
| **13-1** | 4.79 |
| **14-1** | 2.45 |
| **14-2** | 24.45 |
| **15-1** | 2.18 |
| **15-2** | 31.44 |
| **16** | 14.95 |
| **18-1** | 7.77 |
| **19** | 5.04 |
| **20** | 2.74 |
| **21** | 5.66 |
| **22** | 9.19 |
| **23-1** | 7.53 |
| **23-2** | 72.09 |
| **24** | 5.44 |
| **25-1** | 12.87 |
| **26-1** | 1.67 |
| **27** | 44.65 |
| **28** | 5.21 |
| **29** | 3.62 |
| **30** | 12.72 |
| **32** | 28.84 |
| **34** | 19.17 |
| **35** | 8.48 |
| **36** | 8.35 |
| **37** | 5.27 |
| **38** | 13.84 |
| **39** | 15.82 |
| **40** | 8.89 |
| **41** | 2.89 |
| **42** | 3.09 |
| **43** | 36.29 |

Conclusion: The compounds claimed by the present disclosure showed significant inhibitory effects on the proliferation of ERα mutant-expressing MCF7 cells.

Test Example 3: Degrading Effects of Compounds of the Present Disclosure on ERα The ERα-positive breast cancer cell line MCF7 cells (TCHu74, National Collection of Authenticated Cell Cultures) were cultured in a DMEM/F12 medium (HyClone, SH30023.01) containing 10% fetal bovine serum (Corning, 35-010-CV). On day 1 of the experiment, the cells were digested, washed once with a phenol red-free DMEM/F12 medium (ThermoFisher, 11039-021) containing 5% charcoal stripped fetal bovine serum (BIOSUN, BS-0004-500), resuspended and counted. The cell suspension was added to a 96-well plate (Corning, 3599) at 140 μL/well, and the cells were incubated in a 37 °C, 5% $CO_2$ incubator overnight. The next day, the compounds were serially diluted with DMSO and further diluted with a phenol red-free DMEM/F12 medium containing 5% charcoal stripped fetal bovine serum. The diluted compounds were added to the cells in the 96-well plate at 10 μL/well, with final concentrations being 300, 30, 3, 0.3, 0.03, 0.003, 0.0003 and 0.00003 nM, respectively. The 96-well plate was placed in an incubator for 16 to 18 h. The 96-well plate was coated with the capture antibody from the human ERα/NR3A1 total protein assay kit (R&D, DYC5715-5). 1 μg/mL capture antibody was prepared in PBS and added to the 96-well plate (Corning, 3590) at 100 μL/well. The plate was left in a 26 °C incubator overnight. On day 3 of the experiment, the 96-well plate coated with the antibody was washed once with PBS, and a solution of Blocker casein (Thermo, 37528) was added at 200 μL/well. The plate was incubated in a 37 °C incubator for 1.5 h for blocking. The cell culture medium supernatant was discarded. The cells were washed once with PBS, and a cell lysis buffer was added at 60 μL/well. The cell lysis buffer was PBS containing 6 M urea, 1 mM EDTA, 0.5% TritonX-100, 1 mM PMSF and a protease inhibitor (Roche, 04693159001). The cells were lysed on ice for 15 min, and PBS containing 1 mM EDTA and 0.5% TritonX-100 was added at 300 μL/well to dilute the urea to 1 M. The blocking solution in the blocked 96-well plate was discarded, and the diluted cell lysis buffer was added at 100 μL/well. The plate was incubated in a 37 °C incubator for 2 h and washed five times with PBS. A biotinylated assay antibody was diluted to 0.4 μg/mL with a 10-fold diluted solution of Blocker casein and then added at 100 μL/well. The plate was incubated in a 37 °C incubator for 1 h. Then the plate was washed five more times, and avidin-HRP diluted 200-fold with the 10-fold diluted solution of Blocker casein was added at 100 μL/well. The plate was incubated at 37 °C for 30 min. Then the plate was washed five more times, and the TMB substrate was added at 100 μL/well. The plate was incubated at room temperature until the color blue appeared, and a stop solution was added at 100 μL/well. OD450 signal readings were taken on a PHERAstar multi-mode microplate reader. The $IC_{50}$ values for the degrading activity of the compounds were calculated using Graphpad Prism software.

The calculated $IC_{50}$ values for the degrading effects of the compounds of the present disclosure on ERα are shown in Table 3.

Table 3. The degrading effects of the compounds of the present disclosure on ERα

| Example No. | $IC_{50}$ (nM) |
|---|---|
| **1** | 17.65 |
| **2-1** | 0.72 |
| **3** | 0.69 |
| **4-1** | 0.23 |
| **5** | 0.98 |
| **6** | 0.94 |
| **7** | 0.46 |
| **8-1** | 0.31 |
| **9** | 1.77 |
| **10** | 0.15 |
| **12-1** | 0.38 |
| **12-2** | 3.69 |
| **13-1** | 0.29 |
| **14-1** | 2.49 |
| **15-1** | 0.66 |
| **16** | 2.60 |

(continued)

| Example No. | $IC_{50}$ (nM) |
|---|---|
| **18-1** | 0.53 |
| **19** | 1.12 |
| **20** | 2.93 |
| **21** | 0.84 |
| **22** | 1.79 |
| **23-1** | 2.99 |
| **23-2** | 51.99 |
| **24** | 0.77 |
| **25-1** | 1.02 |
| **26-1** | 0.66 |
| **28** | 1.55 |
| **29** | 1.06 |
| **30** | 15.21 |
| **31** | 2.18 |
| **32** | 6.15 |
| **33** | 2.50 |
| **34** | 3.53 |
| **35** | 4.72 |
| **36** | 1.63 |
| **37** | 5.01 |
| **38** | 3.58 |
| **39** | 4.57 |
| **40** | 6.27 |
| **41** | 1.42 |
| **42** | 4.21 |
| **43** | 1.12 |

Conclusion: The compounds claimed by the present disclosure showed significant degrading effects on ERα.

Test Example 4: Pharmacokinetic Evaluation

I. C57 mouse test

1. Abstract

With C57 mice as test animals, the plasma concentration of the compound of Example 26-1 was determined by LC/MS/MS at different time points after intragastric (i.g.)/intravenous (i.v.) administration to C57 mice. The pharmacokinetic behavior of the compound of the present disclosure in C57 mice was studied and its pharmacokinetic profile was evaluated.

2. Test protocol

2.1 Test compound

The compound of Example 26-1.

2.2 Test animals

Eighteen female C57 mice were evenly divided into 2 groups and fasted overnight, and the 2 groups were

dosed intragastrically and intravenously, respectively. The mice were provided by Zhejiang Vital River Laboratory Animal Technology Co., Ltd.

2.3. Drug preparation

An appropriate amount of the compound of Example 26-1 was weighed out and mixed with 5% DMSO + 5% Tween 80 + 90% normal saline to make a 0.1 mg/mL colorless clear solution (for the intragastric administration group) and a 0.1 mg/mL colorless clear solution (for the intravenous administration group).

2.4. Administration

The intragastric administration group: the dose was 2.0 mg/kg, and the volume was 0.2 mL/10 g.
The intravenous administration group: the dose was 1.0 mg/kg, and the volume was 0.1 mL/10 g.

3. Procedures

The intragastric administration group: Blood (0.1 mL) was collected into EDTA-K2 anticoagulation tubes before administration and 0.25 h, 0.5 h, 1.0 h, 2.0 h, 4.0 h, 6.0 h, 8.0 h, 11.0 h and 24.0 h after administration and centrifuged at 10,000 rpm for 1 min (4 °C). The plasma was separated within 1 h and stored at -20 °C before analysis. The process from blood collection to centrifugation was performed in an ice bath. Two hours after administration, feeding was resumed.

The intravenous administration group: Blood was collected before administration and 5 min, 15 min, 0.5 h, 1.0 h, 2.0 h, 4.0 h, 8.0 h, 11.0 h and 24 h after administration and processed as in the intragastric administration group.

The plasma concentration of the test compound was determined after administration of different concentrations of the test compound to C57 mice: plasma samples (25 μL) were collected from C57 mice at various time points after administration, proteins were precipitated with 200 μL of acetonitrile, and 50 μL of camptothecin (100 ng/mL) was added. The mixtures were vortexed for 5 min and centrifuged at 4000 rpm for 10 min. 1 μL of supernatant was collected for LC-MS/MS analysis.

4. Pharmacokinetic parameters

Table 4. The pharmacokinetic parameters of the compound of the present disclosure

| No. | Route of administration/ dose (mg/kg) | Plasma concentration Cmax (ng/mL) | Area under curve $AUC_{0-t}$ (h*ng/mL) | Half-life T1/2 (h) | Clearance CL/F (mL/min/kg) | Apparent distribution volume Vz/F (mL/kg) |
|---|---|---|---|---|---|---|
| Compound of Example 26-1 | i.g. /2.0 | 59.0 | 517 | 5.3 | 62.1 | 28321 |
| | i.v. 11.0 | 1219 | 1072 | 4.0 | 15.4 | 5376 |

Conclusion: The compound of the present disclosure showed good pharmacokinetic absorption activity in C57 mice: it has pharmacokinetic advantages.

II. SD rat test

1. Abstract

With SD rats as test animals, the plasma concentration of the compound of Example 26-1 was determined by LC/MS/MS at different time points after intragastric (i.g.)/intravenous (i.v.) administration to SD rats. The pharmacokinetic behavior of the compound of the present disclosure in SD rats was studied and its pharmacokinetic profile was evaluated.

2. Test protocol

2.1 Test compound

The compound of Example 26-1.

2.2 Test animals

Eight SD rats, of which half were male and half female, were evenly divided into 2 groups. The rats were provided by Zhejiang Vital River Laboratory Animal Technology Co., Ltd. The 2 groups were fasted overnight and then dosed intragastrically and intravenously, respectively.

2.3. Drug preparation

An appropriate amount of the compound of Example 26-1 was weighed out and mixed with 5% EtOH + 95% (a 15% solution of polyethylene glycol 15-hydroxystearate prepared in a 5% aqueous solution of glucose) to make a 0.2 mg/mL colorless clear solution (for the intragastric administration group) and a 0.2 mg/mL colorless clear solution (for the intravenous administration group).

2.4. Administration

The intragastric administration group: the dose was 2.0 mg/kg, and the volume was 10.0 mL/kg.
The intravenous administration group: the dose was 1.0 mg/kg, and the volume was 5.0 mL/kg.

3. Procedures

The intragastric administration group: Blood (0.2 mL) was collected from the orbit into EDTA-K2 anticoagulation tubes before administration and 0.25 h, 0.5 h, 1.0 h, 2.0 h, 4.0 h, 6.0 h, 8.0 h, 11.0 h and 24.0 h after administration and centrifuged at 10,000 rpm for 1 min (4 °C). The plasma was separated within 1 h and stored at -20 °C before analysis. The process from blood collection to centrifugation was performed in an ice bath. Two hours after administration, feeding was resumed.
The intravenous administration group: Blood was collected before administration and 5 min, 15 min, 0.5 h, 1.0 h, 2.0 h, 4.0 h, 8.0 h, 11.0 h and 24 h after administration and processed as in the intragastric administration group.
The plasma concentration of the test compound was determined after administration of different concentrations of the test compound to SD rats: plasma samples (25 μL) were collected from SD rats at various time points after administration, and 50 μL of camptothecin (100 ng/mL) was added to each of the samples; proteins were precipitated with 200 μL of acetonitrile; the mixtures were vortexed for 5 min and centrifuged at 4000 rpm for 10 min. 1 μL of supernatant was collected for LC/MS/MS analysis.

4. Pharmacokinetic parameters

Table 5. The pharmacokinetic parameters of the compound of the present disclosure

| No. | Route of administration/ dose (mg/kg) | Plasma concentration Cmax (ng/mL) | Area under curve $AUC_{0-t}$ (h*ng/mL) | Half-life T1/2 (h) | Clearance CL/F (mL/min/kg) | Apparent distribution volume Vz/F (mL/kg) |
|---|---|---|---|---|---|---|
| Compound of Example 26-1 | i.g. /2.0 | 32.9 | 302 | 4.4 | 102 | 38512 |
| | i.v. 11.0 | 4045 | 2643 | 4.8 | 6.4 | 2695 |

Conclusion: The compound of the present disclosure showed good pharmacokinetic absorption activity in SD rats: it has pharmacokinetic advantages.

III. Beagle test

1. Abstract

With beagles as test animals, the plasma concentrations of the compound of Example 26-1 and Comparative Example 1 were determined by LC/MS/MS at different time points after intragastric (i.g.)/intravenous (i.v.) administration to beagles. The pharmacokinetic behavior of the compound of the present disclosure in beagles was studied and its

pharmacokinetic profile was evaluated.

2. Test protocol

2.1 Test compounds

The compound of Example 26-1 and Comparative Example 1 (see patent WO2018102725A1, Example 411), whose structure is shown below:

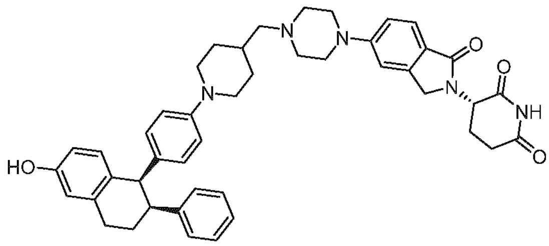

2.2 Test animals

Sixteen beagles, of which half were male and half female, were evenly divided into 4 groups. The beagles were provided by Shanghai Medicilon Inc. The 4 groups were fasted overnight and then dosed intragastrically and intravenously.

2.3. Drug preparation

Certain amounts of the compound of Example 26-1 and the compound of Comparative Example 1 were weighed out and mixed with 5% (v/v) DMSO + 30% (v/v) PG + 30% (v/v) PEG400 + 35% (v/v) normal saline to make 0.4 mg/mL clear solutions (for the intragastric administration groups) and 0.25 mg/mL clear solutions (for the intravenous administration groups).

2.4. Administration

The intragastric administration groups: the dose was 2.0 mg/kg, and the volume was 5.0 mL/kg.
The intravenous administration groups: the dose was 0.5 mg/kg, and the volume was 2.0 mL/kg.

3. Procedures

The intragastric administration group: Blood (1.0 mL) was collected from the jugular vein or the forelimb vein into EDTA-K2 anticoagulation tubes before administration and 0.25 h, 0.5 h, 1.0 h, 2.0 h, 4.0 h, 6.0 h, 8.0 h, 12.0 h and 24.0 h after administration and centrifuged at 10,000 rpm for 5 min (4 °C). The plasma was separated within 1 h and stored at -80 °C before analysis. The process from blood collection to centrifugation was performed in an ice bath. Three hours after administration, feeding was resumed. The intravenous administration groups: Blood was collected before administration and 5 min, 0.25 h, 0.5 h, 1.0 h, 2.0 h, 4.0 h, 8.0 h, 12.0 h and 24 h after administration and processed as in the intragastric administration groups.
The plasma concentrations of the test compounds were determined after administration of different concentrations of the test compounds to beagles: plasma samples (50 μL) were collected from beagles at various time points after administration, and proteins were precipitated with 500 μL of methanol (containing 100 ng/mL internal standard Verapamil) for each of the samples; the mixtures were vortexed for 1 min and centrifuged at 18,000 g for 7 min. 500 μL of supernatant was transferred to a 96-well plate. 1 μL of supernatant was collected for LC/MS/MS analysis.

4. Pharmacokinetic parameters

Table 6. The pharmacokinetic parameters of the compound of the present disclosure

| No. | Route of administration/ dose (mg/kg) | Plasma concentration Cmax (ng/mL) | Area under curve $AUC_{0-t}$ (h*ng/mL) | Half-life T1/2 (h) | Clearance CL/F (mL/min/kg) | Apparent distribution volume Vz/F (mL/kg) |
|---|---|---|---|---|---|---|
| Compound of Example 26-1 | i.g. /2.0 | 436 | 4822 | 10.4 | 6.2 | 5669 |
| | i.v. 10.5 | 1602 | 4073 | 9.7 | 1.7 | 1461 |
| Comparative Example 1 | i.g. /2.0 | 324 | 4050 | 9.0 | 7.3 | 5498 |
| | i.v. 10.5 | 600 | 2682 | 8.1 | 2.8 | 1959 |

Conclusion: The compound of the present disclosure showed low clearance in beagles and demonstrated a good absorption profile: it has pharmacokinetic advantages.

Test Example 5: Pharmacodynamic Test

1. Objective

To evaluate the inhibitory effect of the compound of the present disclosure on the growth of human breast cancer cell MCF-7 (Y537S) xenograft tumors in BEIGE SCID mice.

2. Test compound

The compound of Example 26-1.
A 2% Tween 80 + 98% PEG-400 solution was used.

3. Method and materials

3.1. Animals and housing conditions

Animals: BEIGE SCID female mice, purchased from Beijing Vital River Laboratory Animal Technology Co., Ltd. (certificate No. SCXK (Beijing) 2016-0006), weighing about 19 g on purchase.
Housing conditions: 5 rats/cage, 12/12-hour light/dark cycles, at a constant temperature of 23 ± 1 °C with humidity at 50% to 60%, given *ad libitum* access to food and water.

3.2. Grouping of animals

BEIGE SCID mice were acclimatized and then grouped as follows:

| Group | Number of mice | Route of administration |
|---|---|---|
| Vehicle control | 8 | 2% Tween 80 + 98% PEG-400 solution (i.g./qd) |
| Example 26-1 | 8 | 5 mpk (i.g./qd) |
| Example 26-1 | 8 | 15 mpk (i.g./qd) |
| Example 26-1 | 8 | 45 mpk (i.g./qd) |
| Notes: qd stands for "once a day"; i.g. stands for intragastric administration. | | |

3.3. Method:

MCF-7 (Y537S) cells in the logarithmic growth phase were subcutaneously inoculated at $1.0 \times 10^7$/mouse/200 μL (containing 100 μL of matrigel) into the right flank of female BEIGE SCID mice. After 18 days, when the tumor volume of tumor-bearing mice reached about 170 $mm^3$, the mice were randomly divided into 4 groups of 8 by tumor volume and body weight: a vehicle control group, an Example 26-1 5 mpk group, an Example 26-1 15 mpk group, and an Example

26-1 45 mpk group. The day of grouping was set as D0, and intragastric administration was started and performed once a day for 28 days. The 28th day after administration was set as D28 (Table 7). For the tumor-bearing mice, the tumor volume was measured with a vernier caliper and the body weight was measured with a balance twice a week, and data were recorded.

3.4. Statistics

All data were plotted and statistically analyzed using Excel and GraphPad Prism 8 software.

The tumor volume (V) was calculated as follows: $V = 1/2 \times a \times b^2$, where a and b represent length and width, respectively.

$$\text{The relative tumor proliferation rate T/C (\%)} = (T - T_0)/(C - C_0) \times 100\ (\%),$$

where T

and C are tumor volumes of a treatment group and the control group respectively at the end of the experiment; $T_0$ and $C_0$ are tumor volumes of a treatment group and the control group respectively at the beginning of the experiment.

$$\text{Tumor growth inhibition TGI (\%)} = 1 - \text{T/C (\%)},$$

and if TGI (%) exceeds 100%, it will not be shown as a specific value but >100%.

$$\text{Tumor regression (\%)} = [(T_0 - T)/T_0] \times 100\ (\%).$$

4. Results

Data on the efficacy of the compound of Example 26-1 against MCF-7 (Y537S) xenograft tumors in BEIGE SCID mice are shown in Table 7 and FIG. 1.

The effect of the compound of Example 26-1 on the body weight of BEIGE SCID mice is shown in FIG. 2.

Table 7. The efficacy of the compound of the present disclosure against MCF-7 (Y537S) xenograft tumors in BEIGE SCID mice

| Group | Administration | Route Dose (mpk) | Mean tumor volume ($mm^3$) | | | | TGI (%) D28 | P (vs. vehicle control) | Number of remaining animals/group |
|---|---|---|---|---|---|---|---|---|---|
| | | | D0 | SEM | D28 | SEM | | | |
| Vehicle control | qd/28d | i.g., 0 | 171.7 | 11.8 | 657.3 | 73.4 | / | / | 8/8 |
| Example 26-1 | qd/28d | i.g., 5 | 170.2 | 11.8 | 305.0 | 23.7 | 72 | <0.001 | 8/8 |
| Example 26-1 | qd/28d | i.g., 15 | 168.2 | 12.8 | 219.7 | 15.7 | 89 | <0.001 | 8/8 |
| Example 26-1 | qd/28d | i.g., 45 | 168.6 | 13.0 | 192.0 | 12.3 | 95 | <0.001 | 8/8 |
| Notes: qd stands for "once a day"; d stands for day; i.g. stands for intragastric administration; SEM stands for standard error of measurement. | | | | | | | | | |

5. Conclusion

The administration of the compound of Example 26-1 was started and performed once a day 18 days after tumor cell grafting. 28 days after the administration, the 5 mpk low-dose group showed 72% tumor growth inhibition, the 15 mpk medium-dose group showed 89% tumor growth inhibition, the 45 mpk high-dose group showed 95% tumor

growth inhibition, and the administration did not affect mouse body weight. In addition, the combination of the compound of Example 26-1 and a CDK4/6 inhibitor can further improve efficacy.

Test Example 6: Solubility of Compound of the Present Disclosure

1. Materials

Reagents: dimethyl sulfoxide (chromatographically pure, Sigma-Aldrich, catalog No. 472301-4X4L), ethanol (chromatographically pure, CNW, catalog No. 4.016362.4000), acetonitrile (chromatographically pure, Merck, catalog No. 1.00030.4008), $NaH_2PO_4{\cdot}2H_2O$ (analytically pure, Sinopharm Chemical Reagent Co., Ltd., catalog No. 20040717), ammonium acetate (chromatographically pure, Fluka Honeywell, catalog No. 17836-250G), sodium taurocholate (98%, J&K Scientific, catalog No. 551055-25G), lecithin (≥99%, sigma aldrich, catalog No. P3556-1G), sodium hydroxide (analytically pure, Sinopharm Chemical Reagent Co., Ltd., catalog No. 10019718), sodium chloride (analytically pure, Sinopharm Chemical Reagent Co., Ltd., catalog No. 10019318), hydrochloric acid (analytically pure, Sinopharm Chemical Reagent Co., Ltd., catalog No. 10011018), acetic acid (analytically pure, Sinopharm Chemical Reagent Co., Ltd., catalog No. 10000218) and ultrapure water (prepared in-house by using an ELGA CHORUS laboratory ultrapure water system). Instrument: Agilent 1200 DAD liquid chromatograph (Agilent, U.S.)

2. Material preparation

2.1. FassIF solution preparation

Solution (A): 4.441 g of $NaH_2PO_4{\cdot}2H_2O$, 0.348 g of NaOH granules and 6.186 g of NaCl were added to 900 mL of ultrapure water and well mixed, and the solution was adjusted to pH 6.5±0.05 with 1 M NaOH, brought to volume (1000 mL) with water, and stored at 4 °C for future use.

FaSSIF solution (B): 0.161 g of sodium taurocholate (NaTC) and 59 mg of lecithin were dissolved in 20 mL of solution (A). The resulting solution was vigorously stirred overnight to form a clear micellar solution, and solution (A) was added to make a volume of 100 mL. The resulting solution was stored at 4 °C for future use (not more than 2 weeks).

2.2. FessIF solution preparation

Solution (A): 20.2 g of NaOH granules, 43.25 g of acetic acid and 59.37 g of sodium chloride were precisely weighed out and dissolved in a proper amount of ultrapure water. The solution was brought to volume (5 L), adjusted to pH 5.0 with 1 M NaOH or 1 M HCl, and stored at 4 °C for future use.

FeSSIF solution (B): 0.80652 g of sodium taurocholate (NaTC) and 295.5 mg of lecithin were dissolved in 25 mL of solution (A). The resulting solution was vigorously stirred overnight to form a clear micellar solution, and solution (A) was added to make a volume of 100 mL. The resulting solution was stored at 4 °C for future use (not more than 2 weeks).

3. Procedures

Dissolution tests in FassIF solution and FessIF solution.

3.1. A proper amount of the test compound was weighed out to prepare a 10 mM stock solution in DMSO. 10 μL of the stock solution (concentration 10 mM, dissolved in DMSO) was precisely measured out and well mixed with 990 μL of an organic solvent mixture (usually DMSO:acetonitrile:ethanol = 1:1:1) in a 2 mL sample flask to obtain a 100 μM clear sample solution as a reference solution.

3.2. 1 mg of the test sample was dissolved in 900 μL of the FassIF solution (or the FessIF solution), and the resulting solution was vigorously stirred. The solution was prepared in duplicate. The solutions were shaken in a 37 °C water bath for 24 h and centrifuged at 12,000 rpm for 30 min. The supernatants were used as sample solutions and transferred for liquid chromatography analysis.

4. Results

$$\text{Solubility } (\mu M) = \text{sample peak area/reference peak area} \times \text{reference solution concentration } (\mu M) \times \text{sample solution dilution factor.}$$

The mean value of two measurements was used as the final solubility in the FassIF solution and the FessIF

solution.

Table 8. The solubility of the compound of the present disclosure

| No. | FassIF solution (μM) | FessIF solution (μM) |
|---|---|---|
| Compound of Example 26-1 | 97 | 553 |

Conclusion: The compound of the present disclosure has excellent solubility in both the FassIF solution and the FessIF solution.

## Claims

1. A compound of general formula (I) or a pharmaceutically acceptable salt thereof:

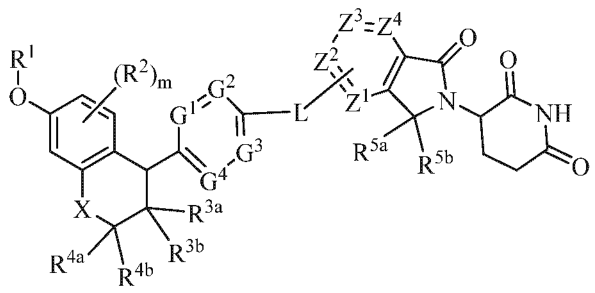

(I)

wherein:

$R^{3a}$ and $R^{3b}$ are identical or different and are each independently selected from the group consisting of hydrogen atom, alkyl, cycloalkyl, heterocyclyl, 8- to 10-membered aryl and heteroaryl, wherein the alkyl, cycloalkyl, heterocyclyl, 8- to 10-membered aryl and heteroaryl are each independently optionally substituted with one or more substituents selected from the group consisting of halogen, oxo, alkyl, alkoxy, haloalkyl, haloalkoxy, cyano, $-NR^9R^{10}$, nitro, hydroxy, hydroxyalkyl, cycloalkyl, heterocyclyl, aryl and heteroaryl;

or $R^{3a}$ and $R^{3b}$, together with the carbon atom to which they are attached, form cycloalkyl or heterocyclyl, wherein the cycloalkyl or heterocyclyl is each independently optionally substituted with one or more substituents selected from the group consisting of halogen, oxo, alkyl, alkoxy, haloalkyl, haloalkoxy, cyano, $-NR^9R^{10}$, nitro, hydroxy, hydroxyalkyl, cycloalkyl, heterocyclyl, aryl and heteroaryl;

X is an oxygen atom or $CH_2$;

$R^1$ is selected from the group consisting of hydrogen atom, alkyl, haloalkyl, hydroxyalkyl and $-C(O)R^6$;

$G^1$, $G^2$, $G^3$ and $G^4$ are identical or different and are each independently a nitrogen atom or $CR^7$;

one of $Z^1$, $Z^2$, $Z^3$ and $Z^4$ is a carbon atom, and the remaining three are identical or different and are each independently a nitrogen atom or $CR^8$;

L is a linker unit;

$R^{4a}$ and $R^{4b}$ are identical or different and are each independently selected from the group consisting of hydrogen atom, halogen, alkyl, alkoxy, haloalkyl, haloalkoxy and hydroxyalkyl;

$R^{5a}$ and $R^{5b}$ are identical or different and are each independently selected from the group consisting of hydrogen atom, halogen, alkyl, alkoxy, haloalkyl, haloalkoxy and hydroxyalkyl, or together $R^{5a}$ and $R^{5b}$ form oxo;

each $R^2$, $R^7$ and $R^8$ is identical or different and is independently selected from the group consisting of hydrogen atom, halogen, alkyl, alkoxy, haloalkyl, haloalkoxy, hydroxyalkyl, cyano, $-NR^{9a}R^{10a}$, hydroxy, $-C(O)R^6$, $-C(O)OR^6$, $-C(O)NR^{9a}R^{10a}$, $-S(O)_tR^{9a}$, $-S(O)_tNR^{9a}R^{10a}$, cycloalkyl, heterocyclyl, aryl and heteroaryl, wherein the alkyl, alkoxy, cycloalkyl, heterocyclyl, aryl and heteroaryl are each independently optionally substituted with one or more substituents selected from the group consisting of halogen, oxo, alkyl, alkoxy, haloalkyl, haloalkoxy, cyano, $-NR^9R^{10}$, nitro, hydroxy, hydroxyalkyl, cycloalkyl, heterocyclyl, aryl and heteroaryl;

$R^6$ is selected from the group consisting of alkyl, haloalkyl, hydroxyalkyl, cycloalkyl, heterocyclyl, aryl and heteroaryl;

$R^9$, $R^{10}$, $R^{9a}$ and $R^{10a}$ are identical or different and are each independently selected from the group consisting

of hydrogen atom, alkyl, hydroxyalkyl, cycloalkyl and heterocyclyl, wherein the alkyl, cycloalkyl and heterocyclyl are each independently optionally substituted with one or more substituents selected from the group consisting of halogen, alkyl, alkoxy, haloalkyl and haloalkoxy;

or $R^9$ and $R^{10}$, together with the nitrogen atom to which they are attached, form heterocyclyl, and the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl and heteroaryl;

or $R^{9a}$ and $R^{10a}$, together with the nitrogen atom to which they are attached, form heterocyclyl, and the heterocyclyl is optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl and heteroaryl;

t is 0, 1 or 2;

m is 0, 1, 2 or 3.

**2.** The compound of general formula (I) or the pharmaceutically acceptable salt thereof according to claim 1, wherein each $R^2$ is identical or different and is independently selected from the group consisting of hydrogen atom, halogen and $C_{1-6}$ alkyl.

**3.** The compound of general formula (I) or the pharmaceutically acceptable salt thereof according to claim 1 or 2, being a compound of general formula (II) or a pharmaceutically acceptable salt thereof:

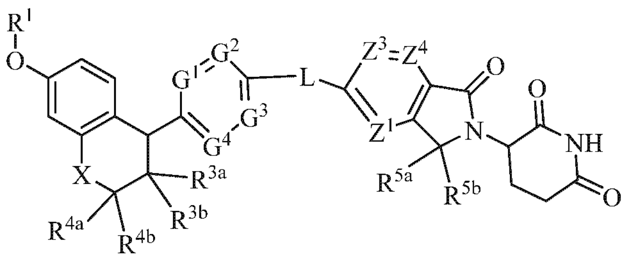

( II )

wherein:

$Z^1$, $Z^3$ and $Z^4$ are identical or different and are each independently a nitrogen atom or $CR^8$;

X, L, $G^1$ to $G^4$, $R^1$, $R^{3a}$, $R^{3b}$, $R^{4a}$, $R^{4b}$, $R^{5a}$, $R^{5b}$ and $R^8$ are as defined in claim 1.

**4.** The compound of general formula (I) or the pharmaceutically acceptable salt thereof according to any one of claims 1 to 3, wherein L is selected from the group consisting of $-L^1-$, $-L^2-$, $-R^{1L}-$, $-R^{2L}-$, $-Q^1-$, $-Q^2-$,

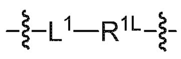 , 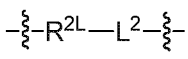 , 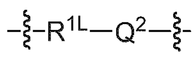 ,

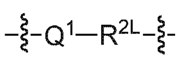 , 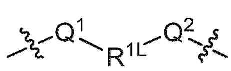 , 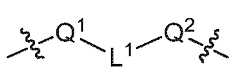 , 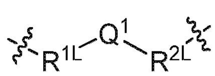 ,

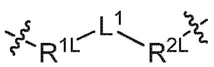 , 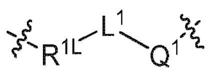 , 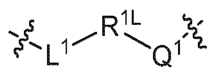 , 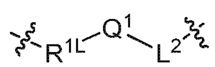 ,

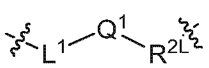 , 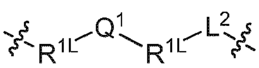 , 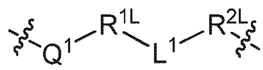 , 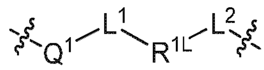 ,

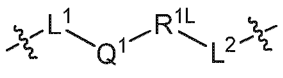 , 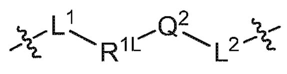 , 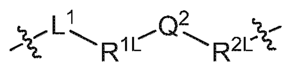 ,

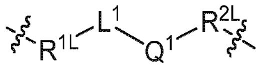 , 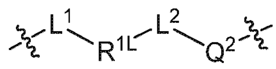 , 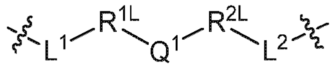 ,

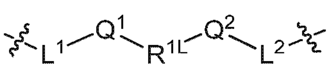 , 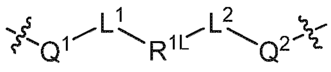 and 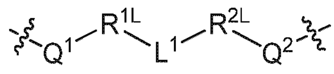 ;

$-L^1-$ and $-L^2-$ are identical or different and are each independently selected from the group consisting of bond, -O-, -S-, $-NR^{11}-$, $-CR^{12a}R^{12b}-$, -C(O)-, -S(O)-, $-S(O)_2-$, -C(S)-, -C(O)O-, $-C(O)NR^{11}-$ and $-NR^{11}C(O)-$;
$R^{1L}$ and $R^{2L}$ are identical or different and are each independently selected from the group consisting of bond, alkylene, heteroalkylene, alkenylene and alkynylene, wherein the alkylene, heteroalkylene, alkenylene and alkynylene are each independently optionally substituted with one or more substituents selected from the group consisting of halogen, alkyl, alkoxy, haloalkyl, hydroxy, hydroxyalkyl, cyano, amino, oxo, cycloalkyl, heterocyclyl, aryl and heteroaryl;
$Q^1$ and $Q^2$ are identical or different and are each independently selected from the group consisting of cycloalkyl, heterocyclyl, aryl and heteroaryl, wherein the cycloalkyl, heterocyclyl, aryl and heteroaryl are each independently optionally substituted with one or more substituents selected from the group consisting of halogen, alkyl, alkoxy, haloalkyl, hydroxy, hydroxyalkyl, cyano, amino, cycloalkyl, heterocyclyl, aryl and heteroaryl;
$R^{11}$ is selected from the group consisting of hydrogen atom, alkyl, heteroalkyl, haloalkyl, cycloalkyl, heterocyclyl, aryl and heteroaryl;
$R^{12a}$ and $R^{12b}$ are identical or different and are each independently selected from the group consisting of hydrogen atom, halogen, alkyl, alkoxy, haloalkyl, hydroxy, hydroxyalkyl, cyano, amino, oxo, cycloalkyl, heterocyclyl, aryl and heteroaryl.

5. The compound of general formula (I) or the pharmaceutically acceptable salt thereof according to any one of claims 1 to 4, wherein L is selected from the group consisting of $-R^{1L}-$,

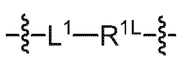 , 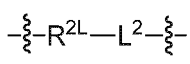 , 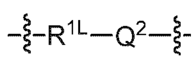 , 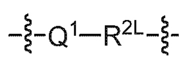 , 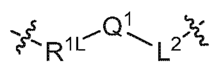 ,

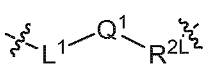 , 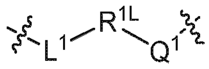 , 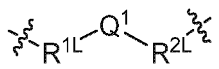 , 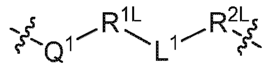 ,

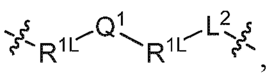 , 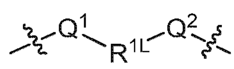 and 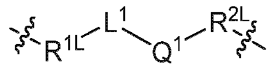 ;

$Q^1$, $Q^2$, $R^{1L}$, $R^{2L}$, $L^1$ and $L^2$ are as defined in claim 4.

6. The compound of general formula (I) or the pharmaceutically acceptable salt thereof according to any one of claims 1 to 5, wherein L is selected from the group consisting of $-(CH_2)_v-$,

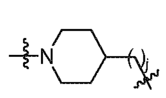 , 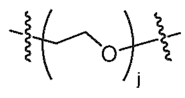 , 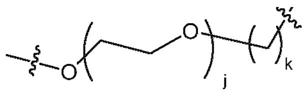 , 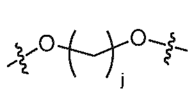 ,

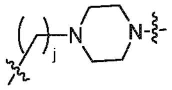, 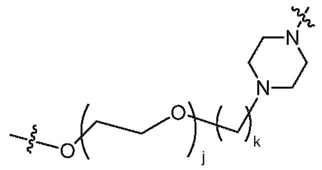, 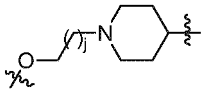, 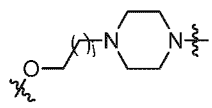,

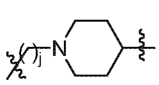, 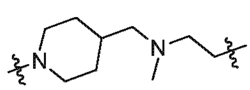, 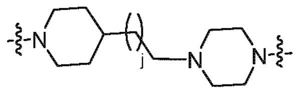, 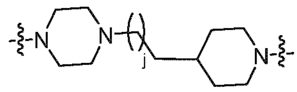,

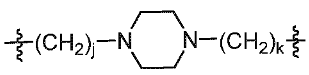, 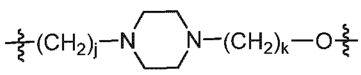, 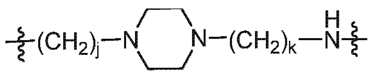

and

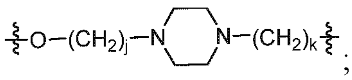;

v is an integer of 1 to 10;
j is an integer of 0 to 10; and
k is an integer of 0 to 10;
preferably, L is

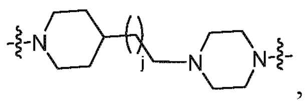,

and j is 0.

7. The compound of general formula (I) or the pharmaceutically acceptable salt thereof according to any one of claims 1 to 6, wherein $R^{5a}$ and $R^{5b}$ are both hydrogen atoms, or together $R^{5a}$ and $R^{5b}$ form oxo; preferably, $R^{5a}$ and $R^{5b}$ are both hydrogen atoms.

8. The compound of general formula (I) or the pharmaceutically acceptable salt thereof according to any one of claims 1 to 7, being a compound of general formula (III) or a pharmaceutically acceptable salt thereof:

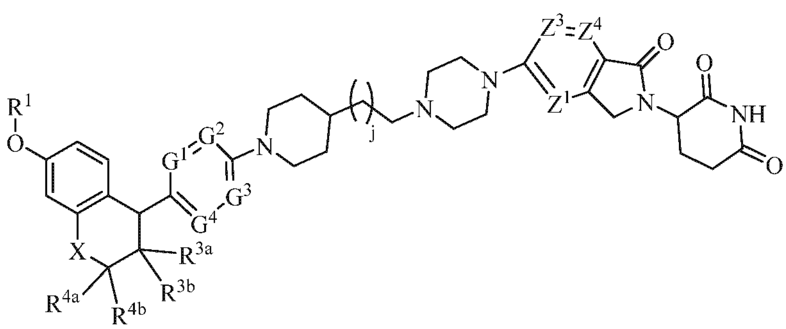

( III )

wherein:

j is an integer of 0 to 10;
X, $G^1$ to $G^4$, $Z^1$, $Z^3$, $Z^4$, $R^1$, $R^{3a}$, $R^{3b}$, $R^{4a}$ and $R^{4b}$ are as defined in claim 3.

**9.** The compound of general formula (I) or the pharmaceutically acceptable salt thereof according to any one of claims 1 to 8, wherein $R^{3a}$ and $R^{3b}$ are identical or different and are each independently selected from the group consisting of hydrogen atom, $C_{1-6}$ alkyl, 3- to 8-membered cycloalkyl, 3- to 8-membered heterocyclyl, 8- to 10-membered aryl and 5- to 10-membered heteroaryl, wherein the $C_{1-6}$ alkyl, 3- to 8-membered cycloalkyl, 3- to 8-membered heterocyclyl, 8- to 10-membered aryl and 5- to 10-membered heteroaryl are each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5-to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl.

**10.** The compound of general formula (I) or the pharmaceutically acceptable salt thereof according to any one of claims 1 to 8, wherein $R^{3a}$ and $R^{3b}$, together with the carbon atom to which they are attached, form 3- to 8-membered cycloalkyl or 3- to 8-membered heterocyclyl, wherein the 3- to 8-membered cycloalkyl or 3- to 8-membered heterocyclyl is each independently optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, cyano, $-NR^9R^{10}$, nitro, hydroxy, $C_{1-6}$ hydroxyalkyl, 3- to 8-membered cycloalkyl, 3- to 8-membered heterocyclyl, 6- to 10-membered aryl and 5-to 10-membered heteroaryl; $R^9$ and $R^{10}$ are identical or different and are each independently a hydrogen atom or $C_{1-6}$ alkyl; or $R^9$ and $R^{10}$, together with the nitrogen atom to which they are attached, form 3- to 6-membered heterocyclyl, and the 3- to 6-membered heterocyclyl formed is optionally substituted with one or more substituents selected from the group consisting of halogen, oxo, $C_{1-6}$ alkyl, $C_{1-6}$ alkoxy, $C_{1-6}$ haloalkyl, $C_{1-6}$ haloalkoxy, hydroxy and $C_{1-6}$ hydroxyalkyl.

**11.** The compound of general formula (I) or the pharmaceutically acceptable salt thereof according to any one of claims 1 to 10, wherein $R^{4a}$ and $R^{4b}$ are both hydrogen atoms.

**12.** The compound of general formula (I) or the pharmaceutically acceptable salt thereof according to any one of claims 1 to 11, wherein $G^1$, $G^2$, $G^3$ and $G^4$ are all $CR^7$; $R^7$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl.

**13.** The compound of general formula (I) or the pharmaceutically acceptable salt thereof according to any one of claims 1 to 12, wherein $Z^1$, $Z^3$ and $Z^4$ are all $CR^8$; or $Z^1$ is a nitrogen atom, and $Z^3$ and $Z^4$ are $CR^8$; $R^8$ are identical or different and are each independently selected from the group consisting of hydrogen atom, halogen, $C_{1-6}$ alkyl, $C_{1-6}$ alkoxy, $C_{1-6}$ haloalkyl, $C_{1-6}$ haloalkoxy and $C_{1-6}$ hydroxyalkyl; preferably, $Z^1$, $Z^3$ and $Z^4$ are all CH, or $Z^1$ is a nitrogen atom, and $Z^3$ and $Z^4$ are CH; more preferably, $Z^1$, $Z^3$ and $Z^4$ are all CH.

**14.** The compound of general formula (I) or the pharmaceutically acceptable salt thereof according to any one of claims 1 to 13, wherein X is $CH_2$.

**15.** The compound of general formula (I) or the pharmaceutically acceptable salt thereof according to any one of claims 1 to 14, wherein $R^1$ is a hydrogen atom.

**16.** The compound of general formula (I) or the pharmaceutically acceptable salt thereof according to any one of claims 1 to 15, being selected from the group consisting of the following compounds:

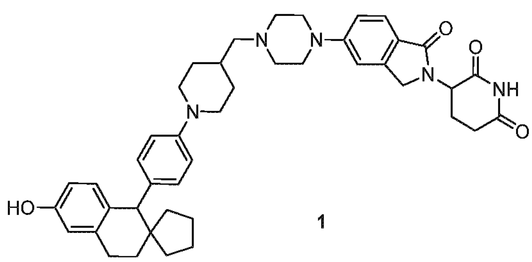

1 ,

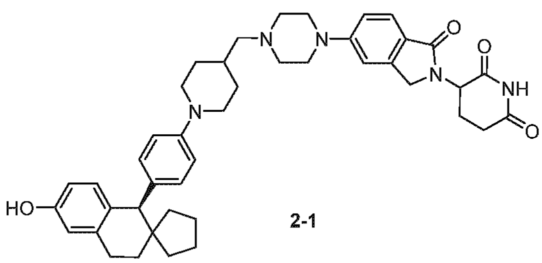

2-1 ,

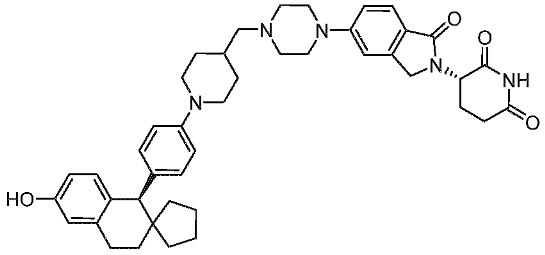

,

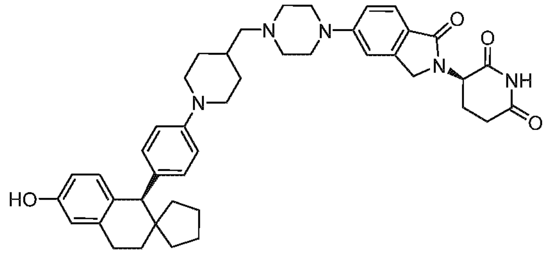

,

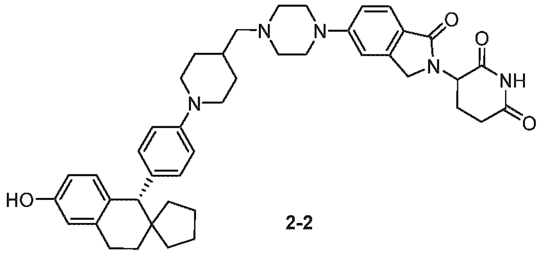

2-2

,

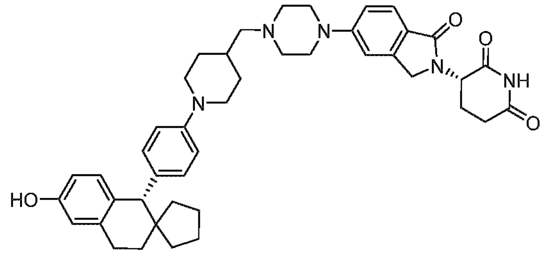

,

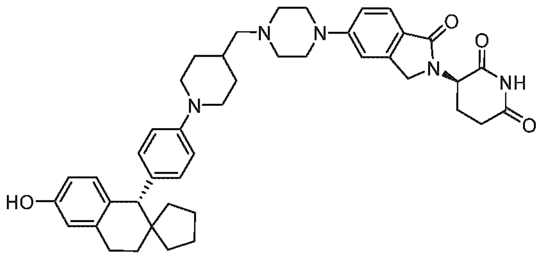

,

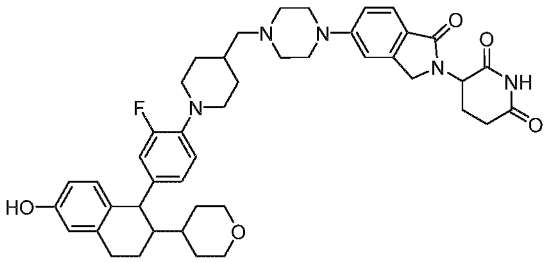

,

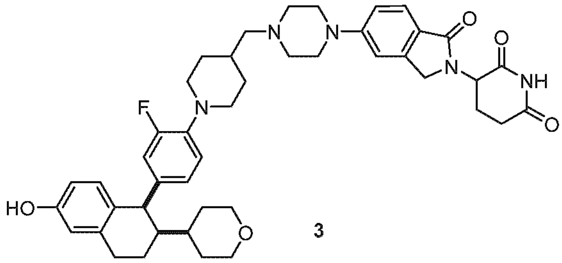

3

,

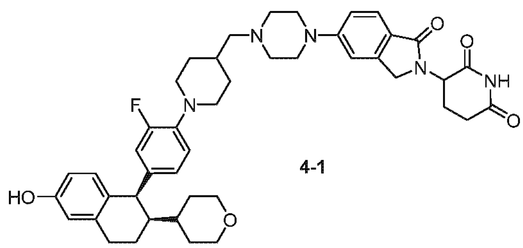

4-1

,

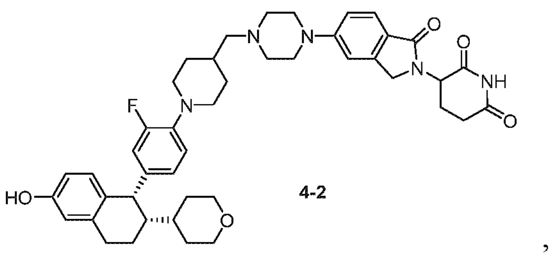

4-2

,

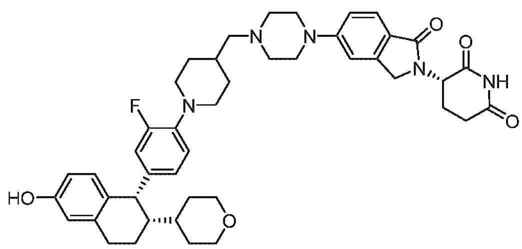

,

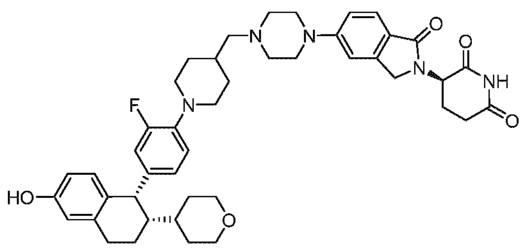
,
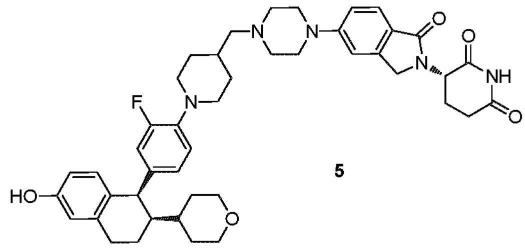
5
,
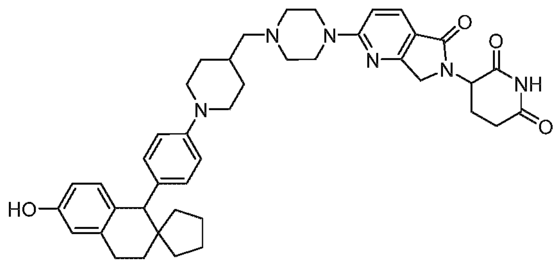
,
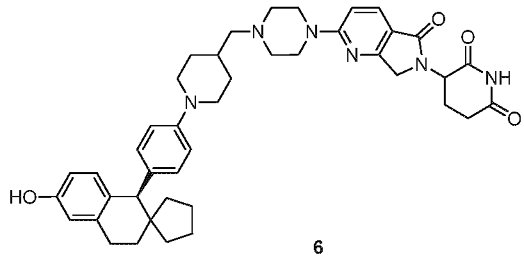
6
,
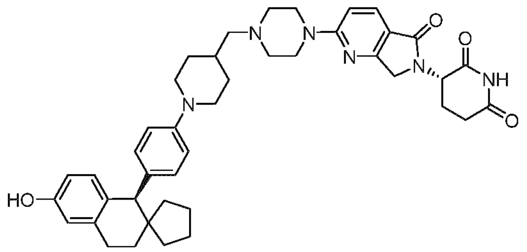
,
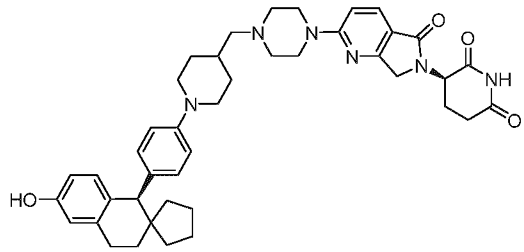
,
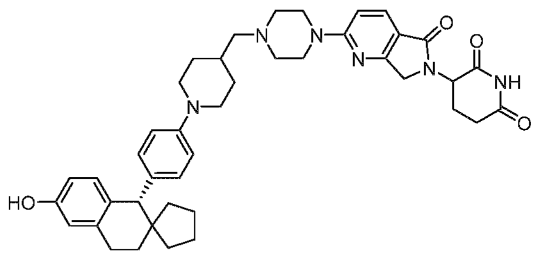
,
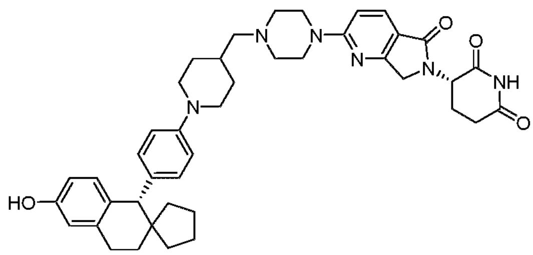
,
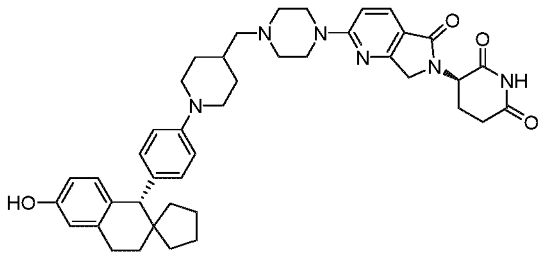
,
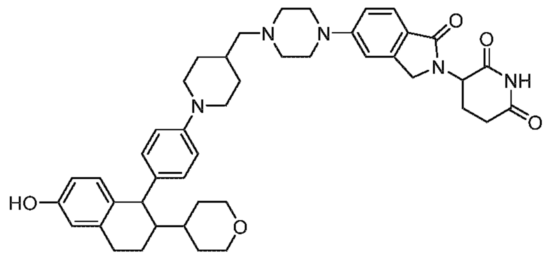
,

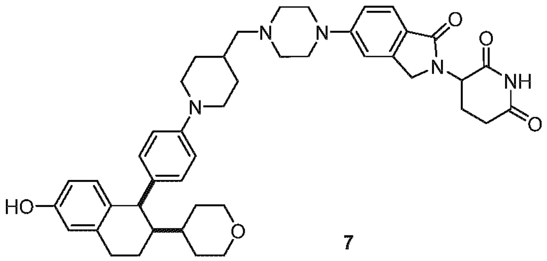

7

,

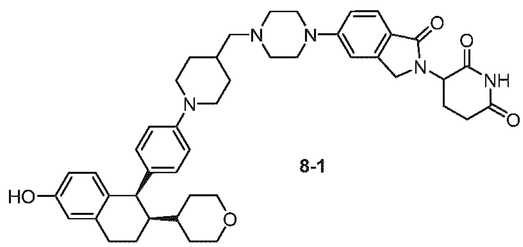

8-1

,

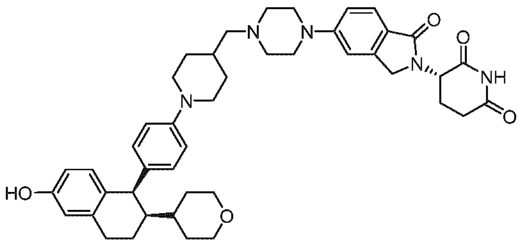

,

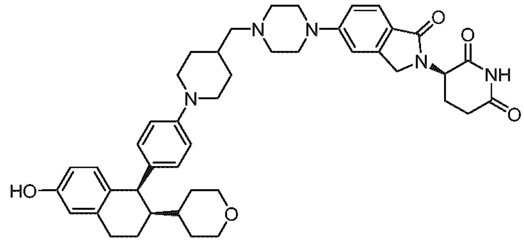

,

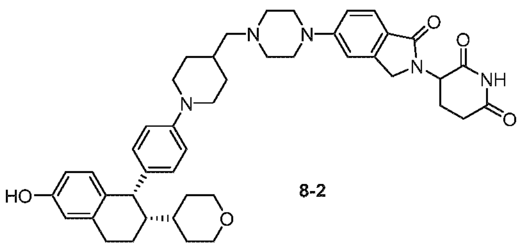

8-2

,

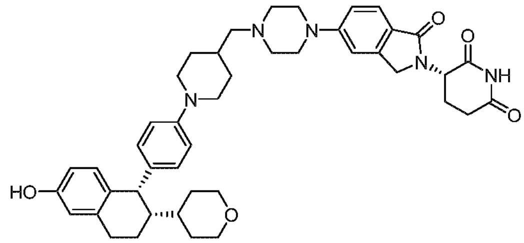

,

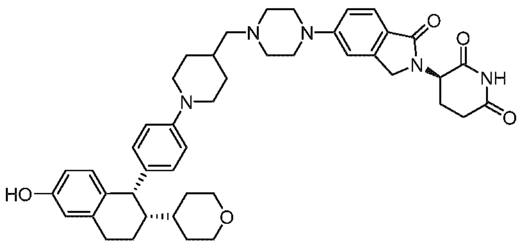

,

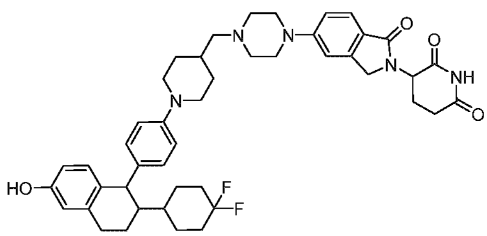

,

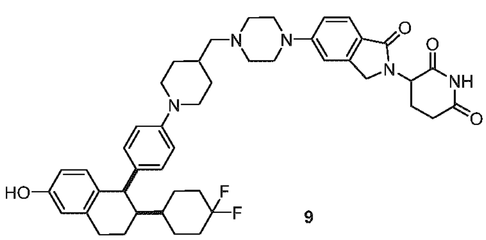

9

,

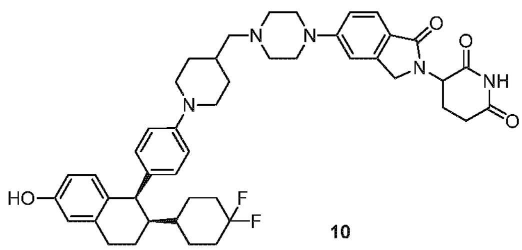

10

,

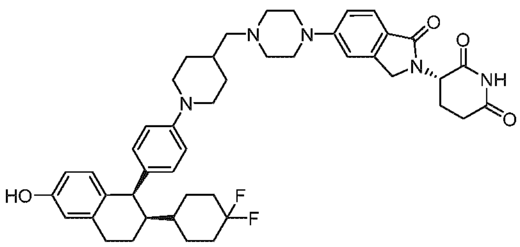

,

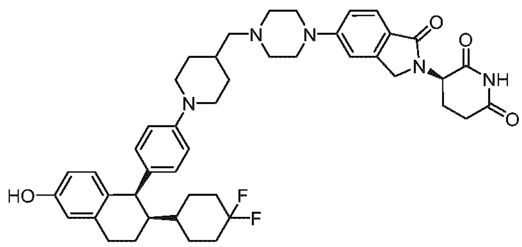

,

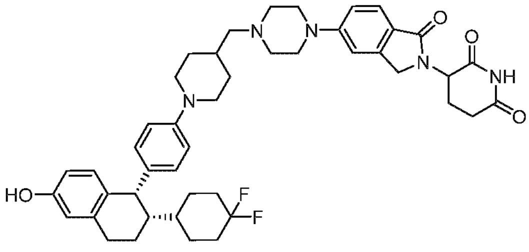

,

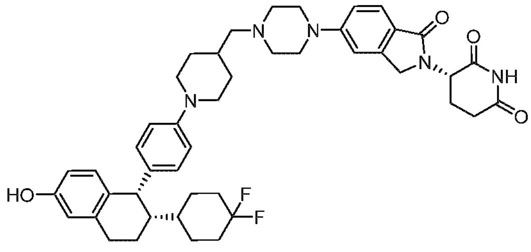

,

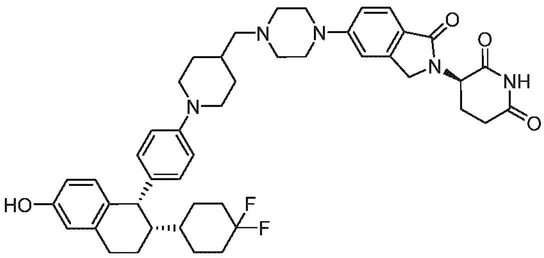

,

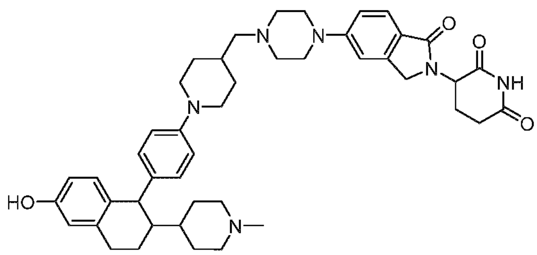

,

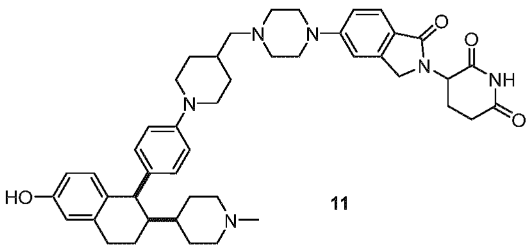

11

,

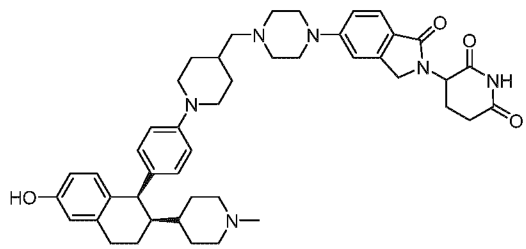

,

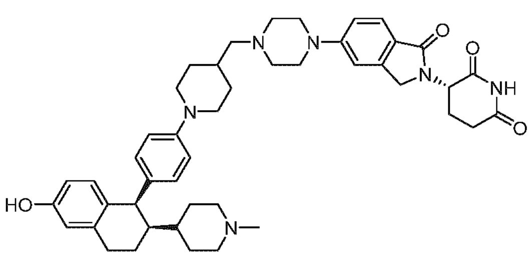

,

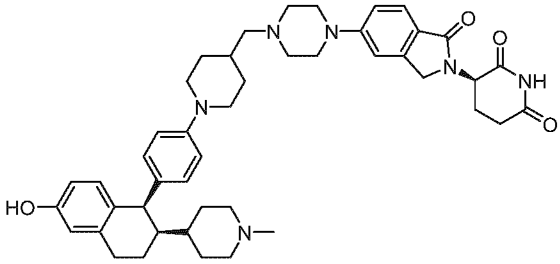

,

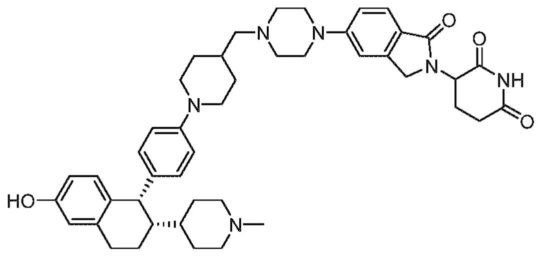,

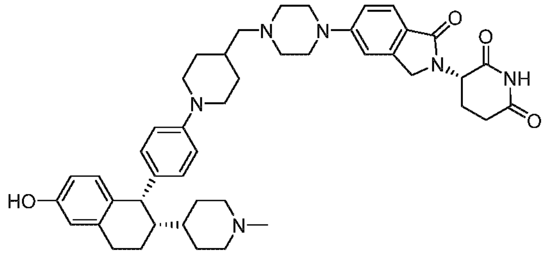,

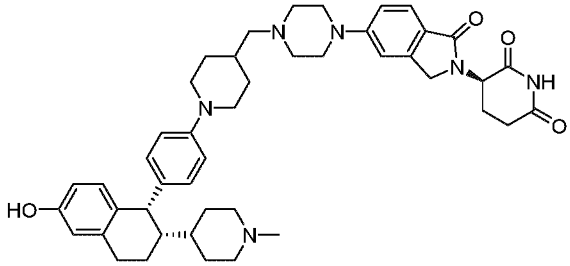,

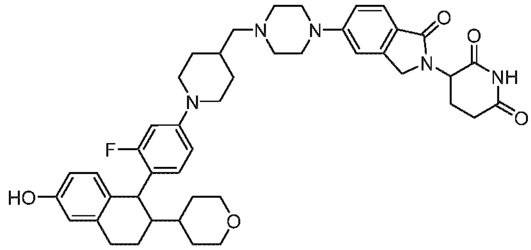,

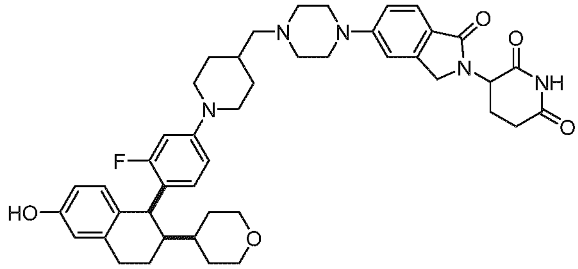,

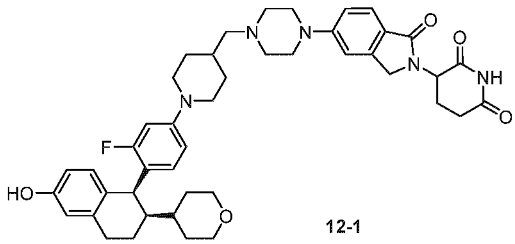

12-1,

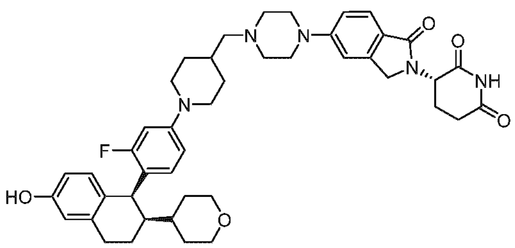,

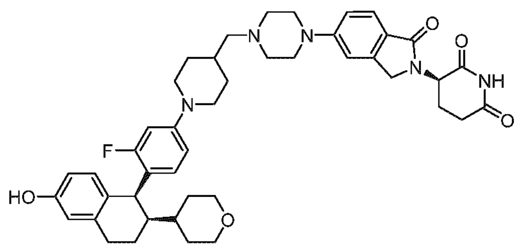,

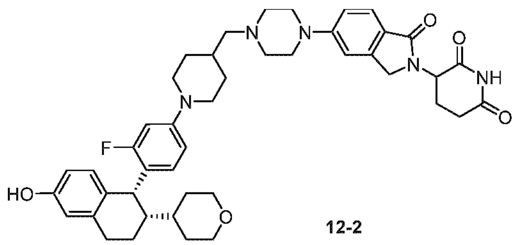

12-2,

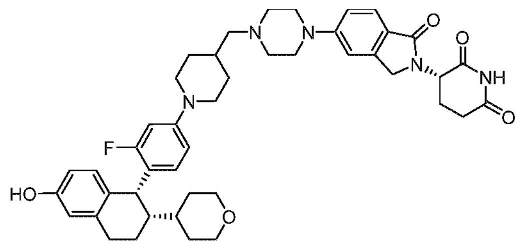,

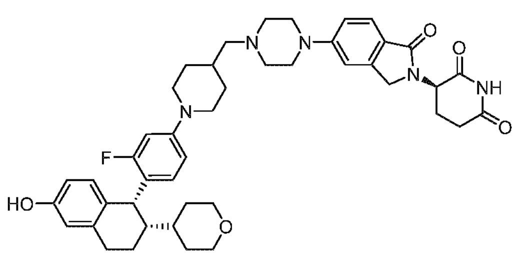,

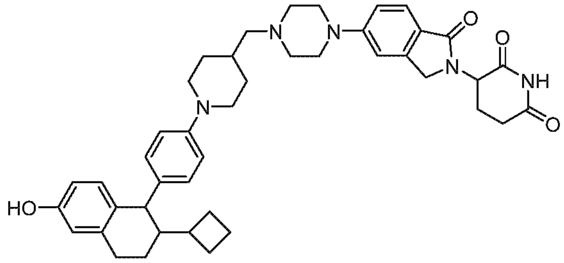,

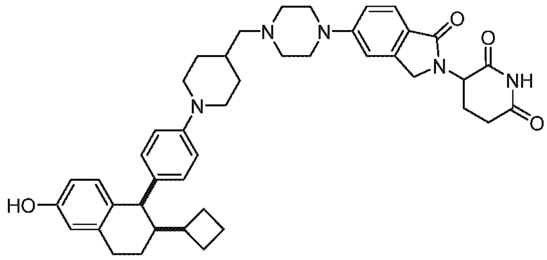

,

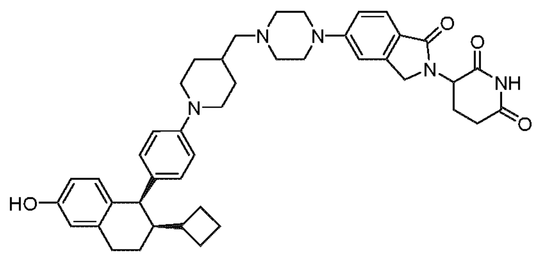

,

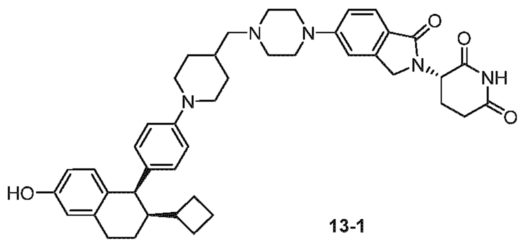

13-1

,

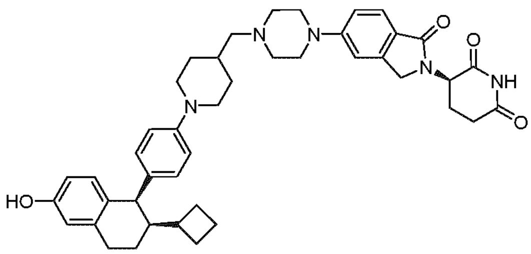

,

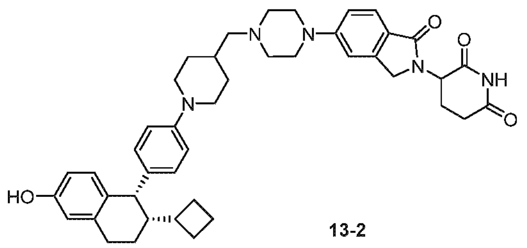

13-2

,

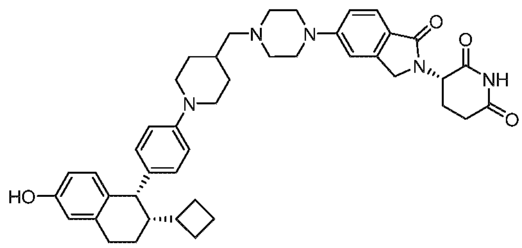

,

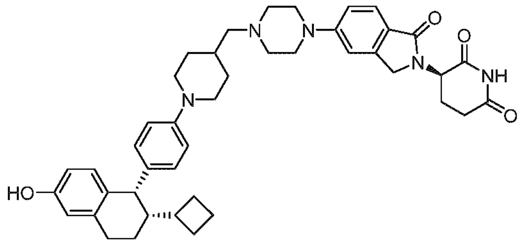

,

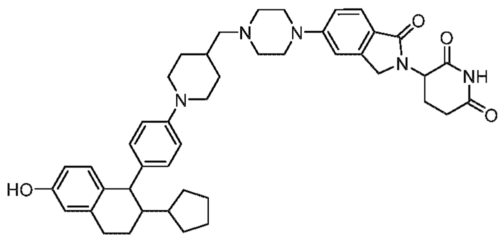

,

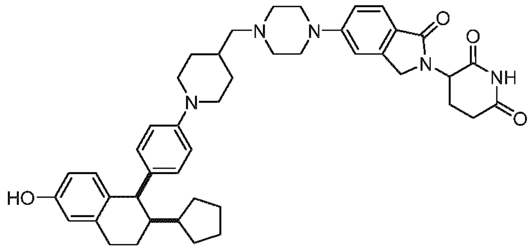

,

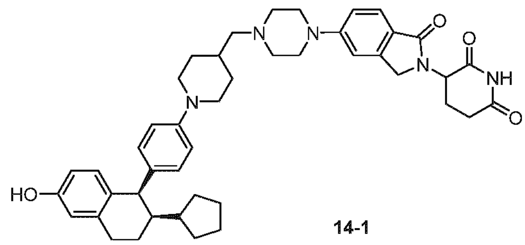

14-1

,

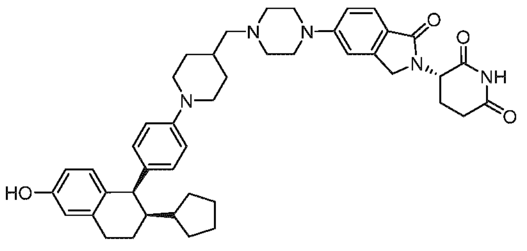
,
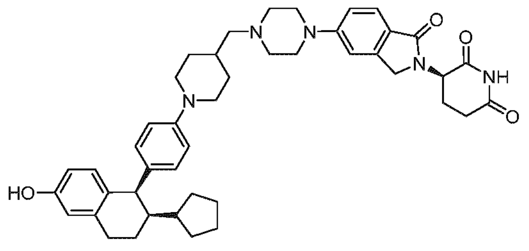
,
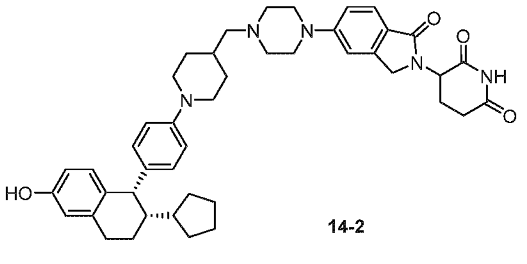
14-2
,
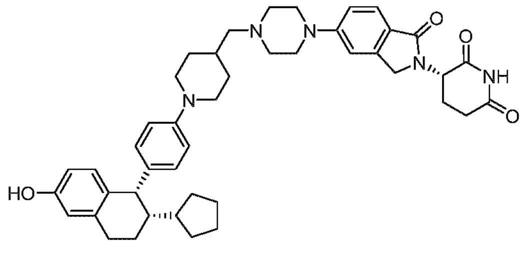
,
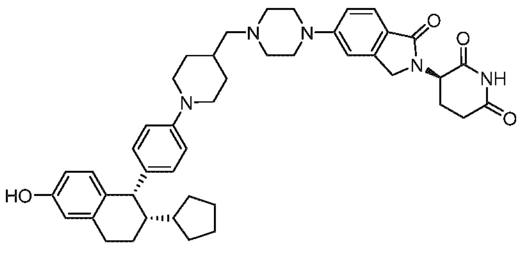
,
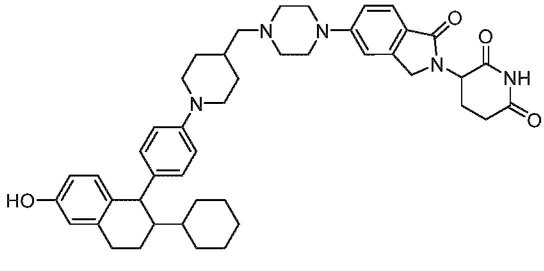
,
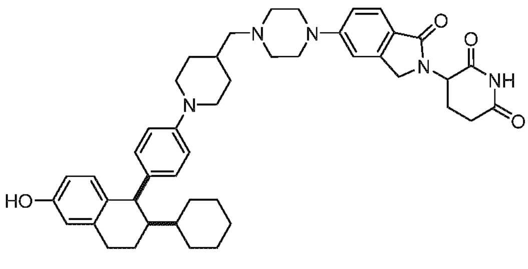
,
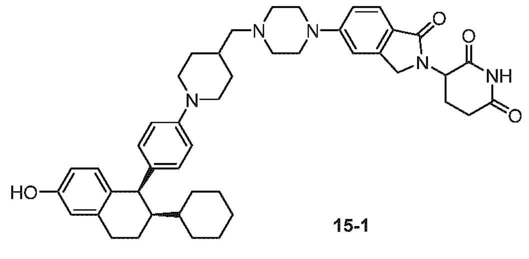
15-1
,
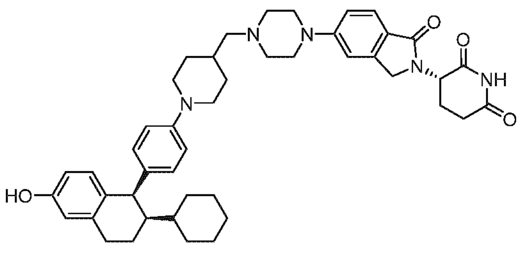
,
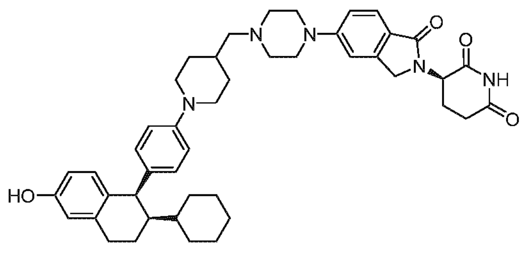
,

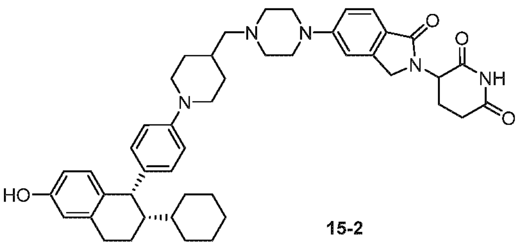

15-2

,

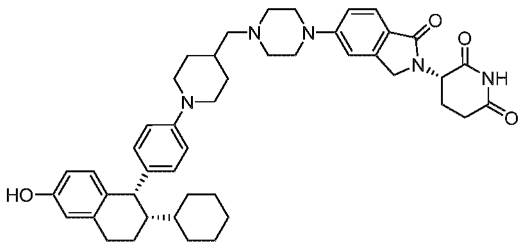

,

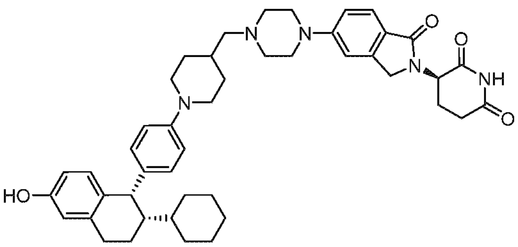

,

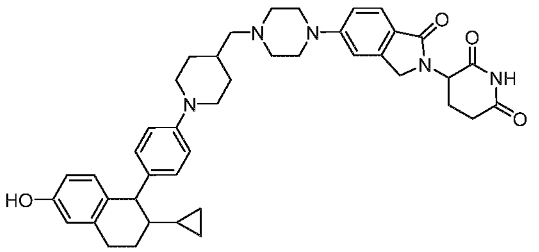

,

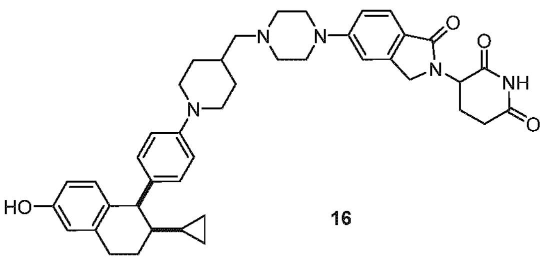

16

,

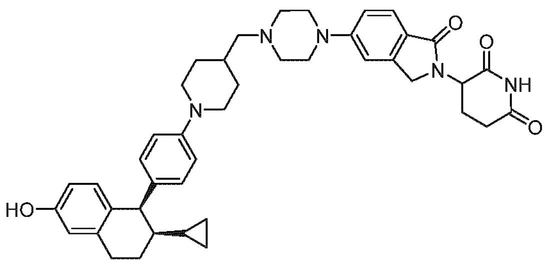

,

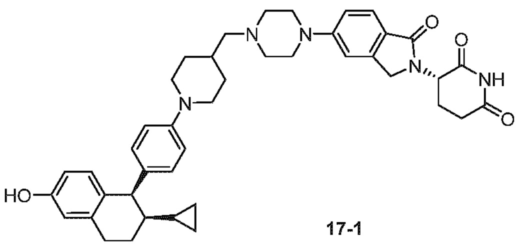

17-1

,

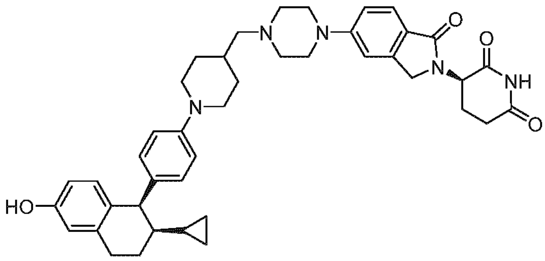

,

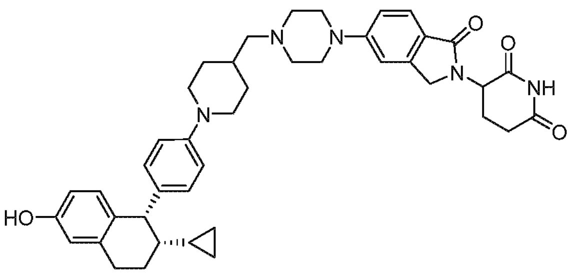

,

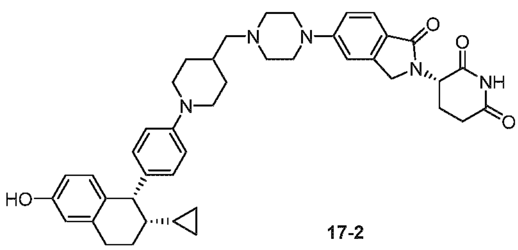

17-2

,

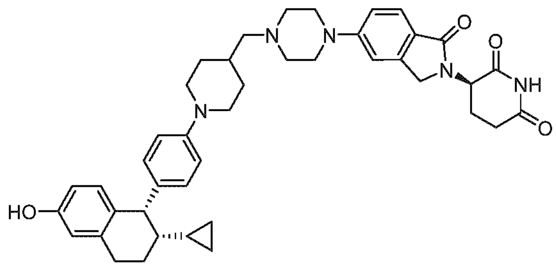,

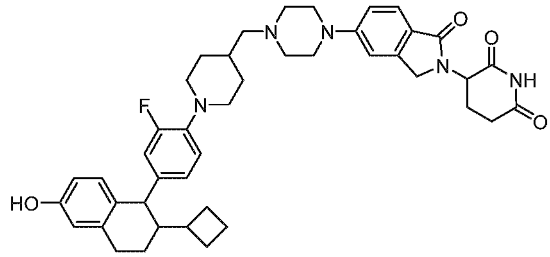,

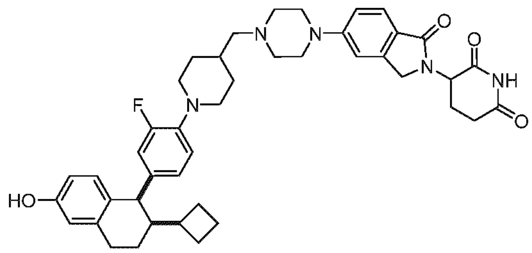,

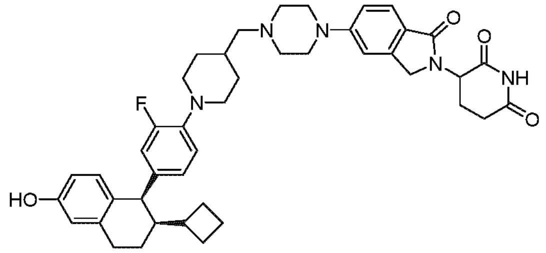,

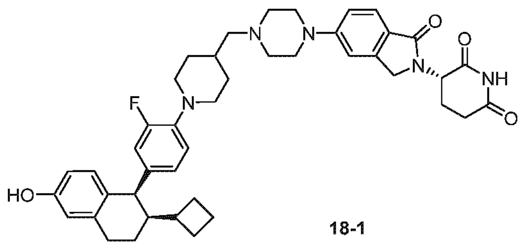

18-1,

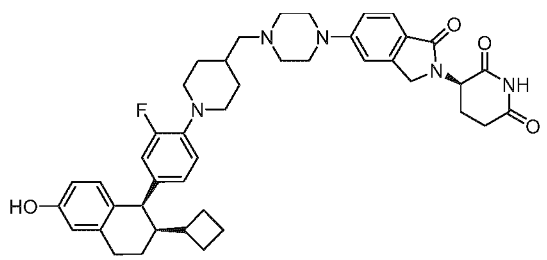,

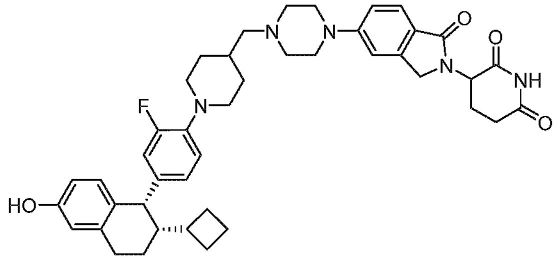,

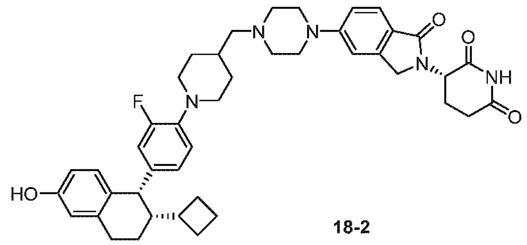

18-2,

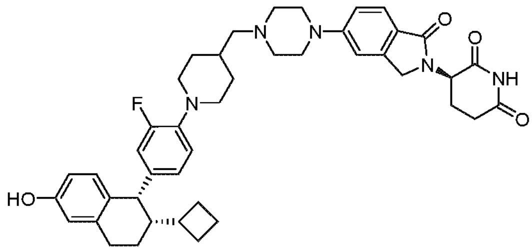,

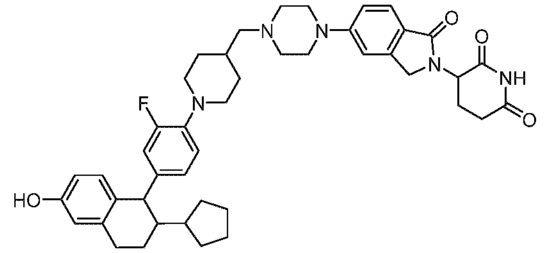,

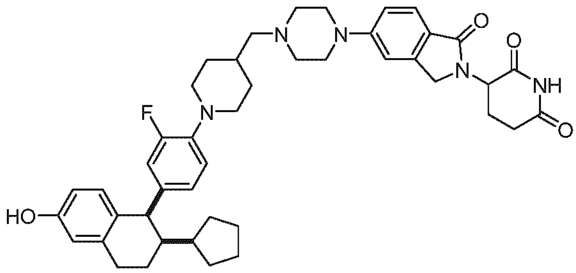,

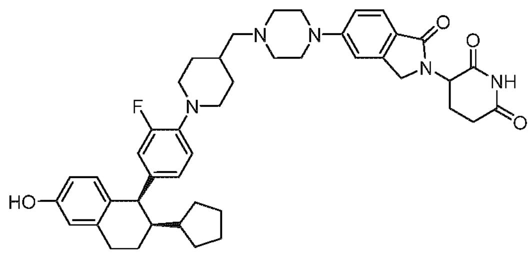,

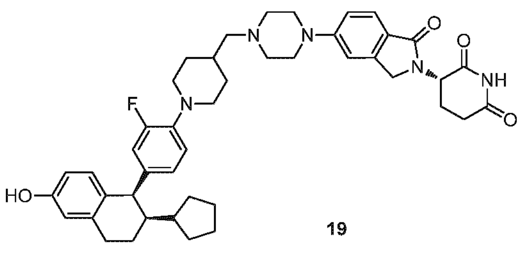

19,

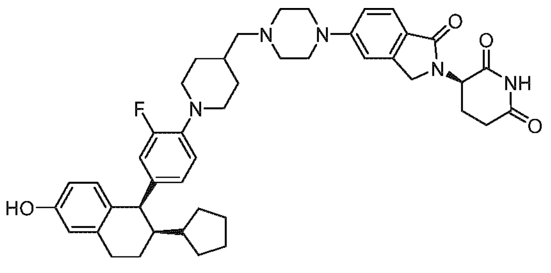,

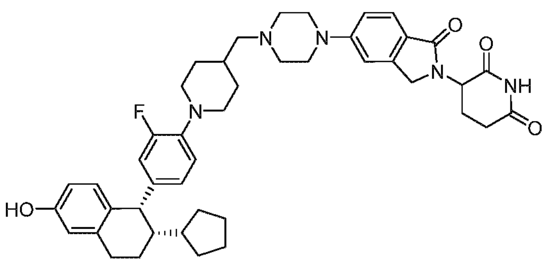,

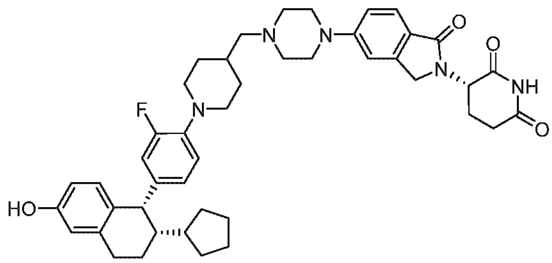,

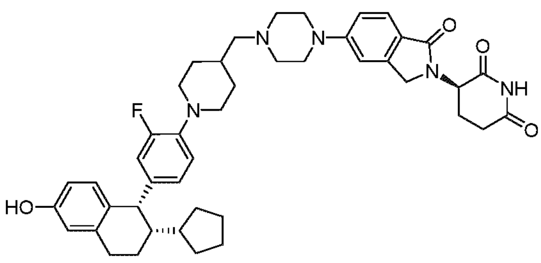,

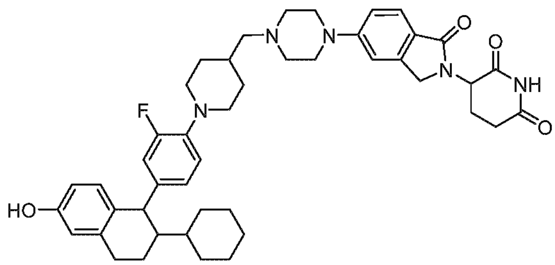,

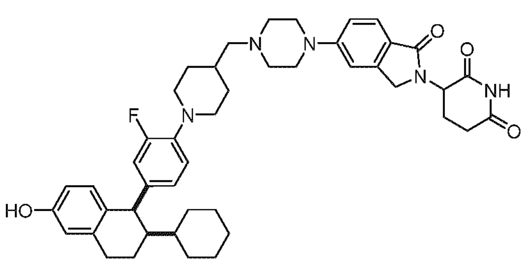,

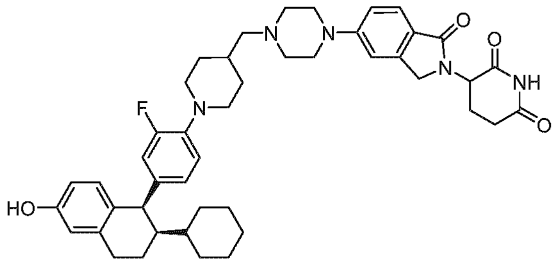,

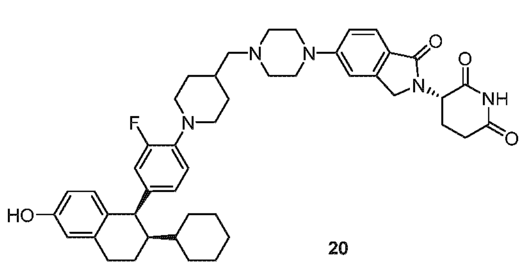

20

,

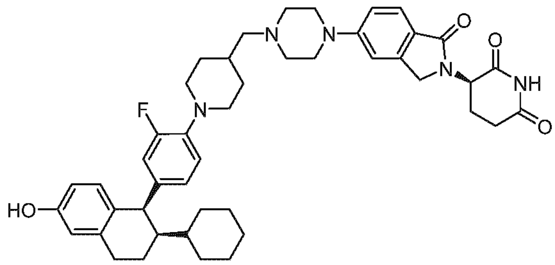

,

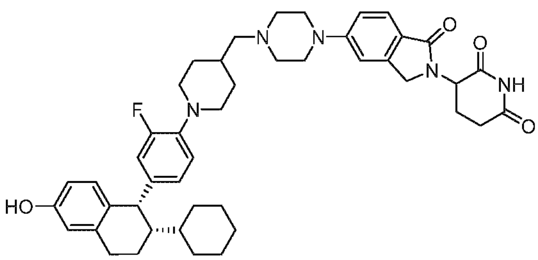

,

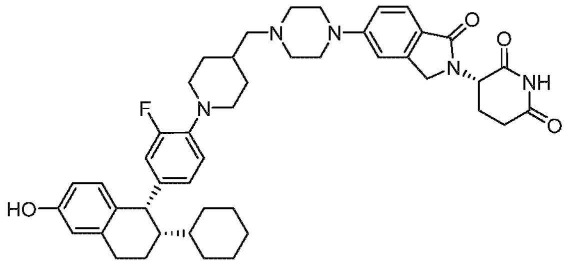

,

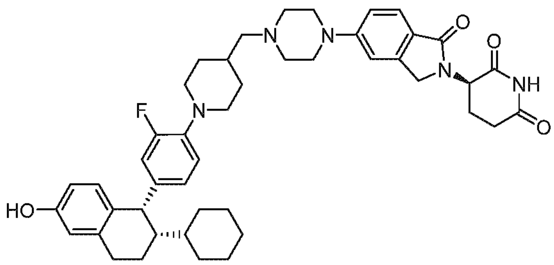

,

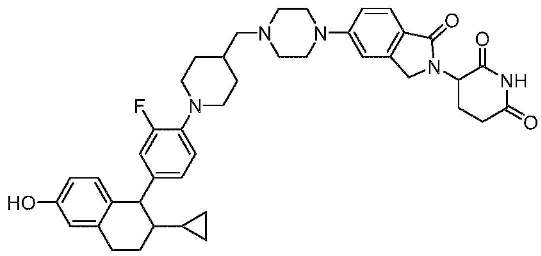

,

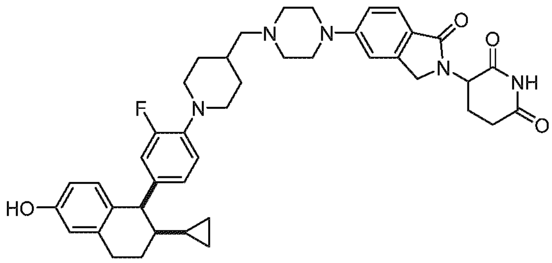

,

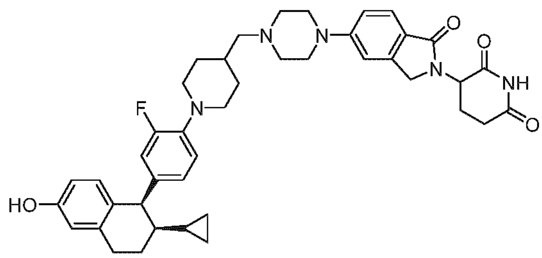

,

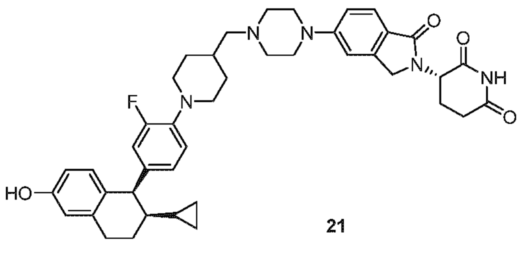

21

,

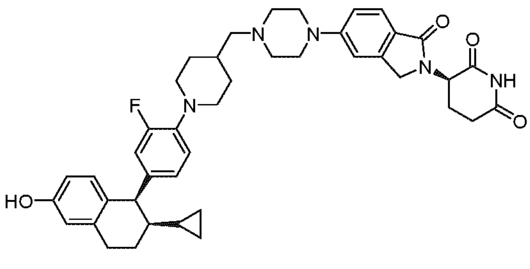

,

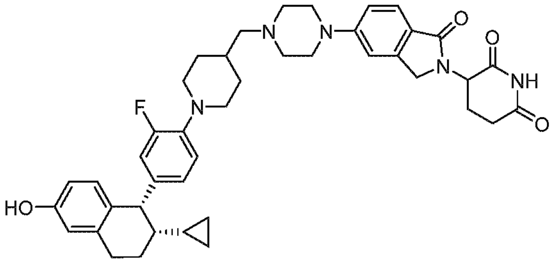,

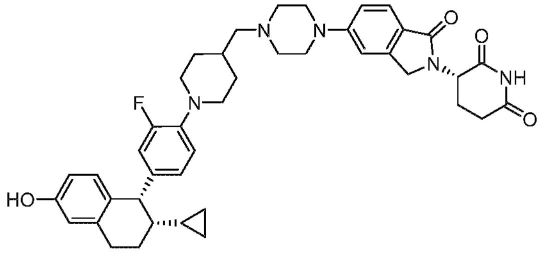,

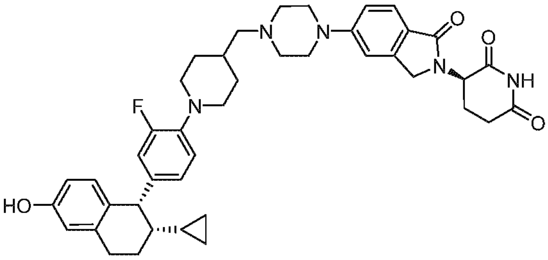,

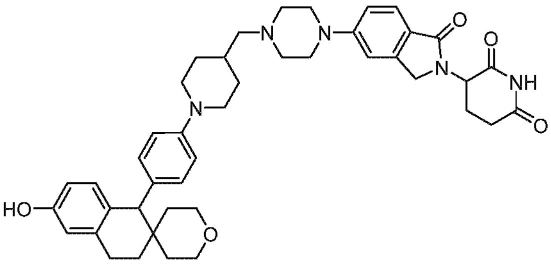,

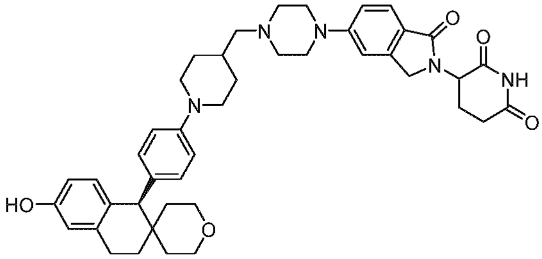,

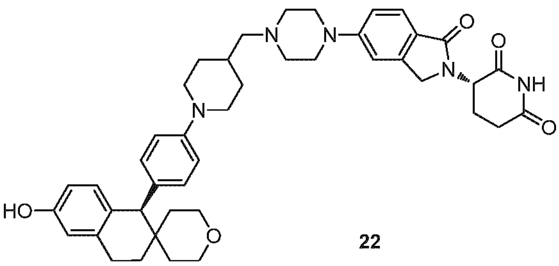

22,

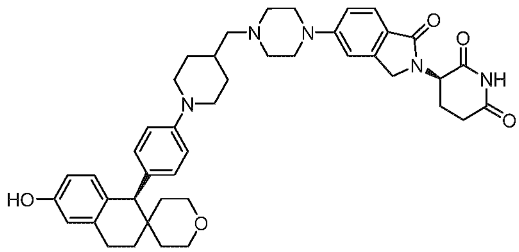,

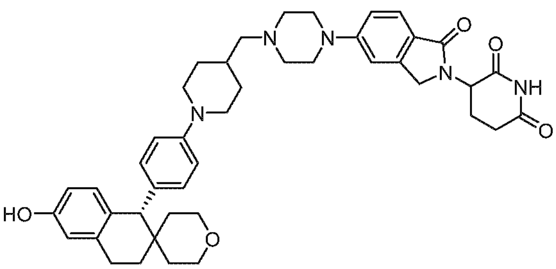,

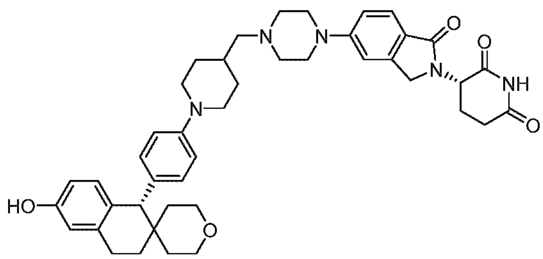,

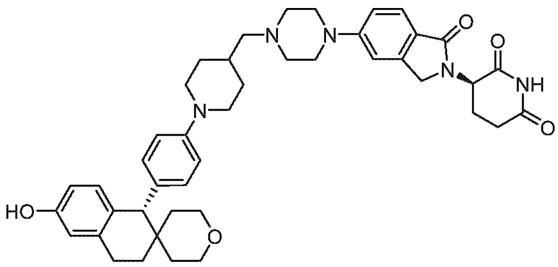

,

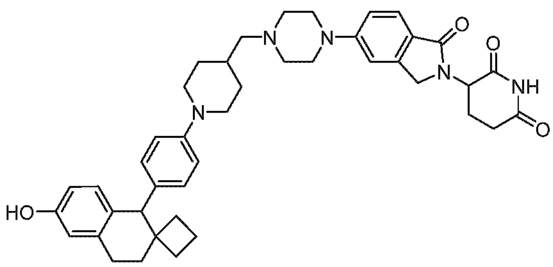

,

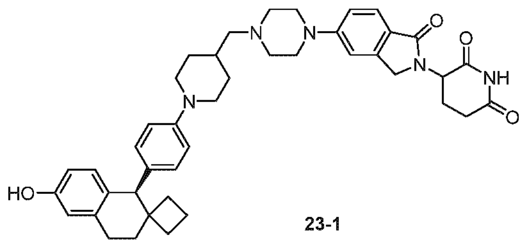

23-1

,

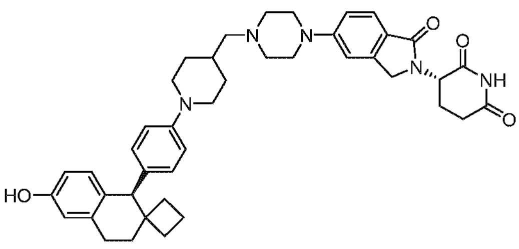

,

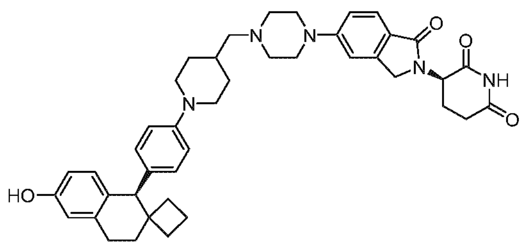

,

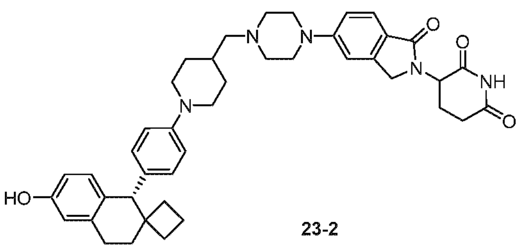

23-2

,

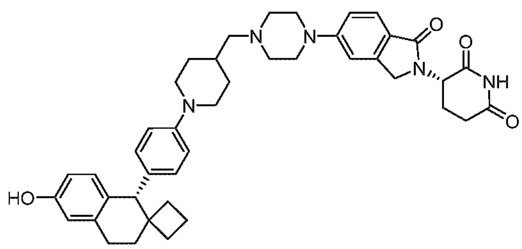

,

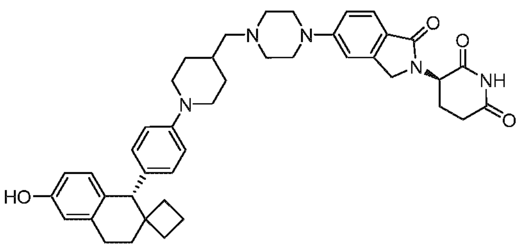

,

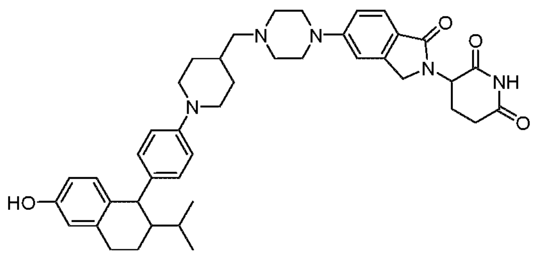

,

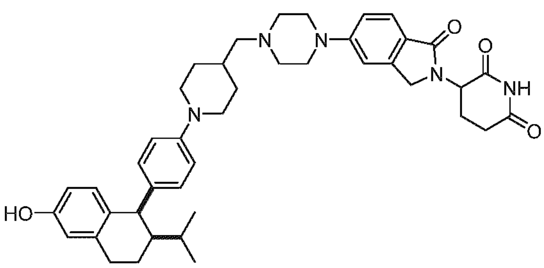

,

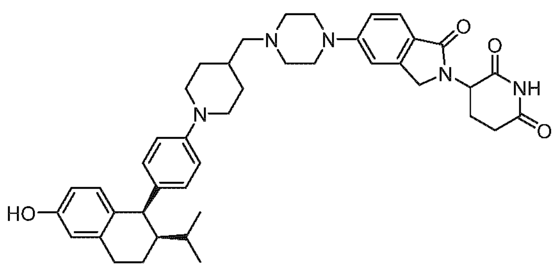

,

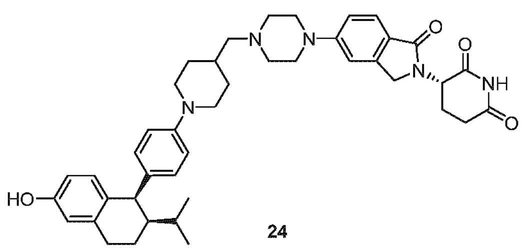
24
,

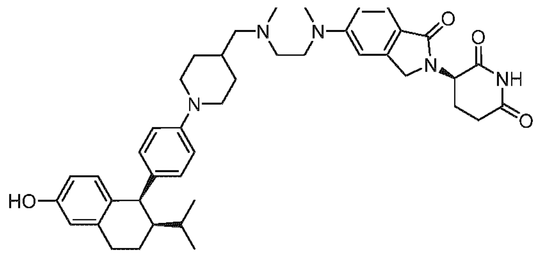
,

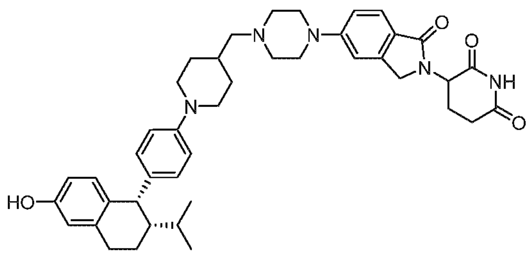
,

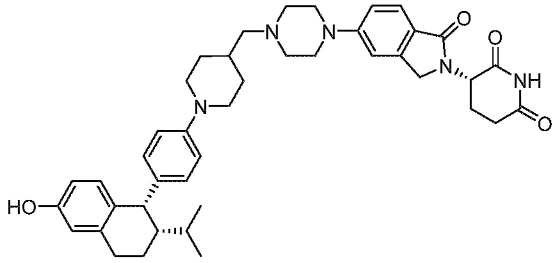
,

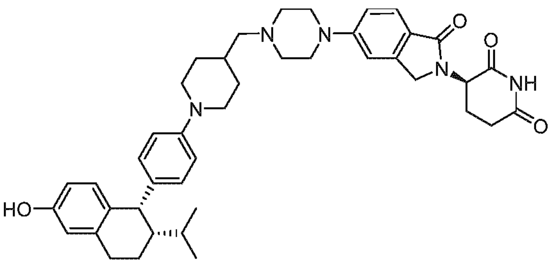
,

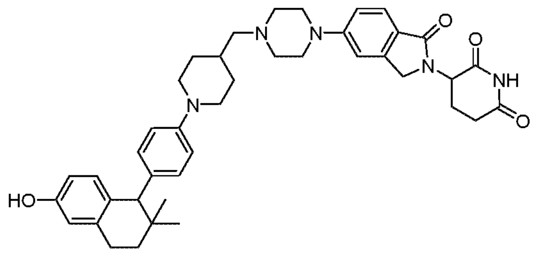
,

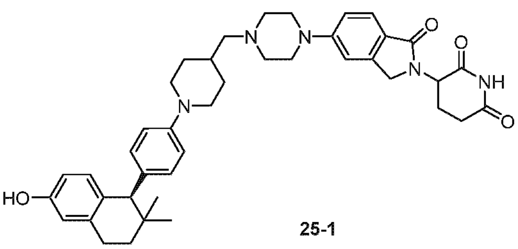
25-1
,

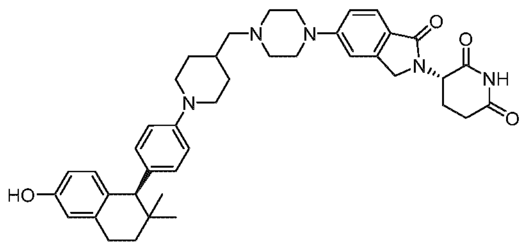
,

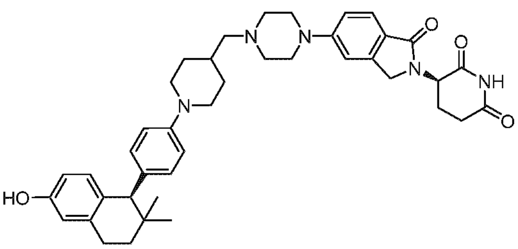
,

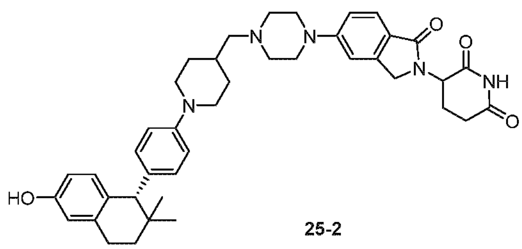
25-2
,

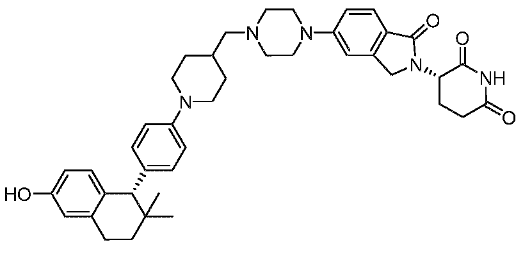
,
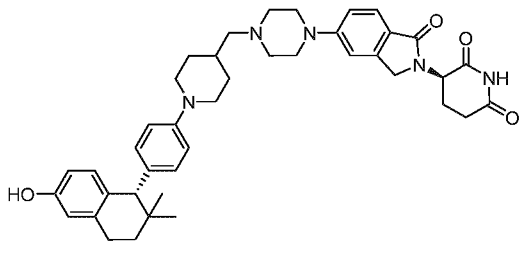
,
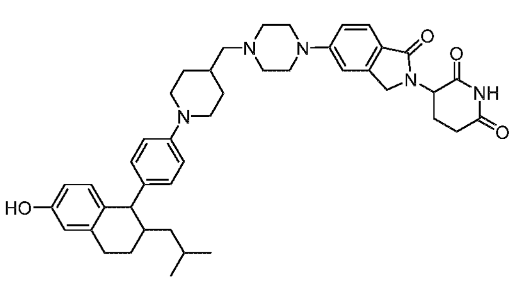
,
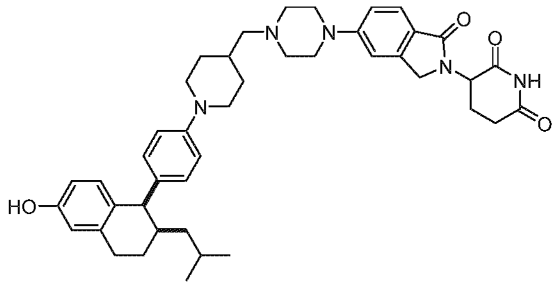
,
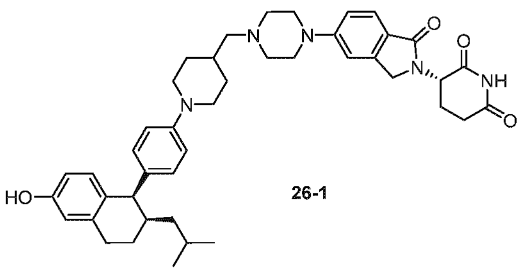
26-1
,
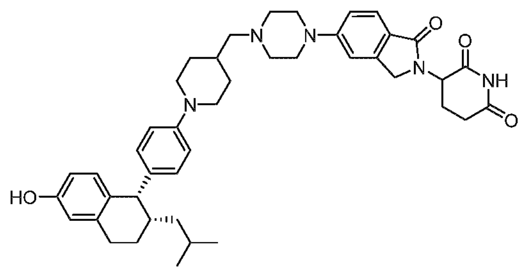
,
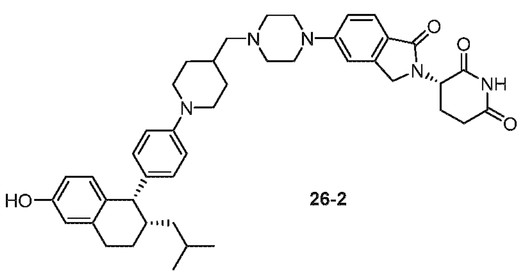
26-2
,
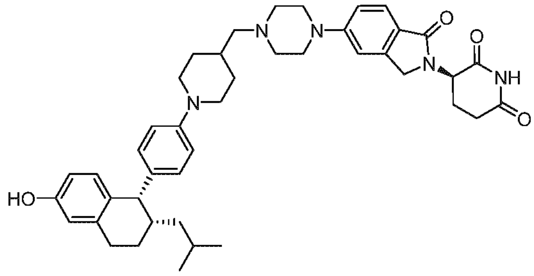
,
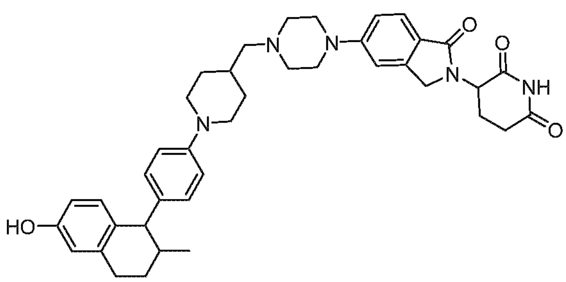
,
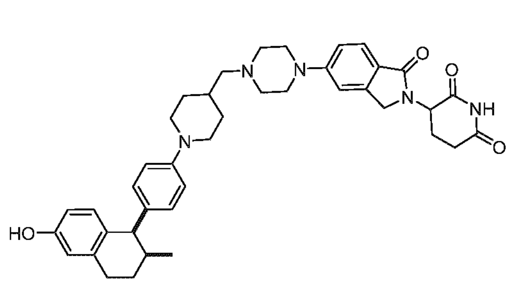
,

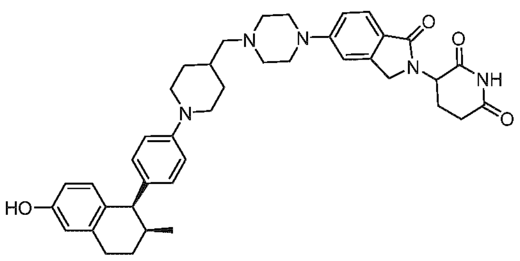

,

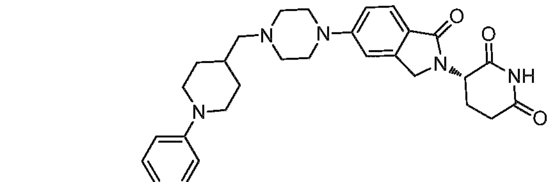

27

,

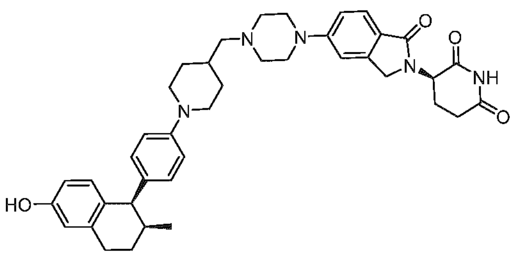

,

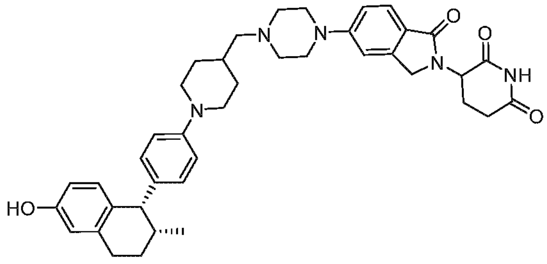

,

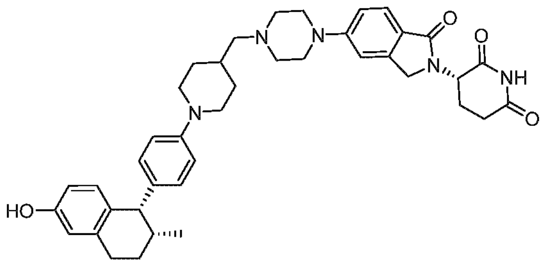

,

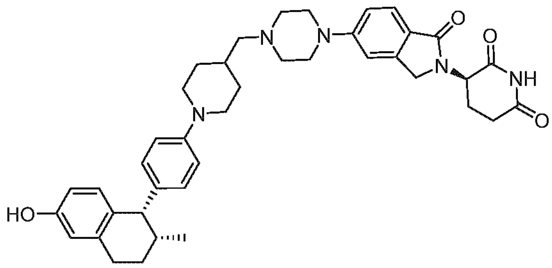

,

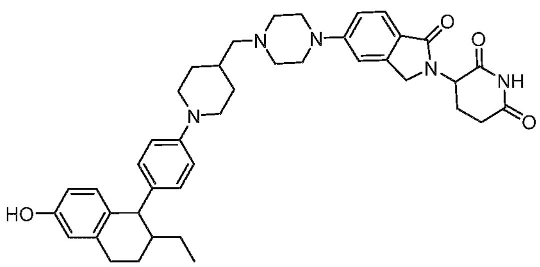

,

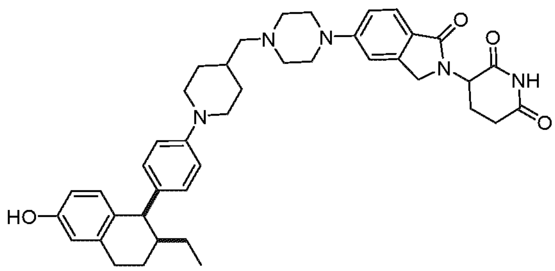

,

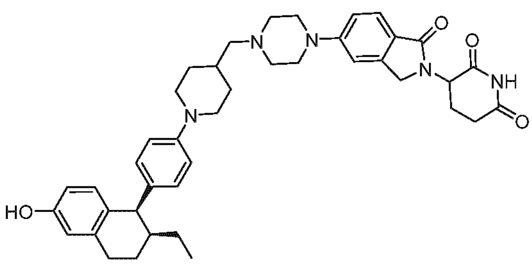

,

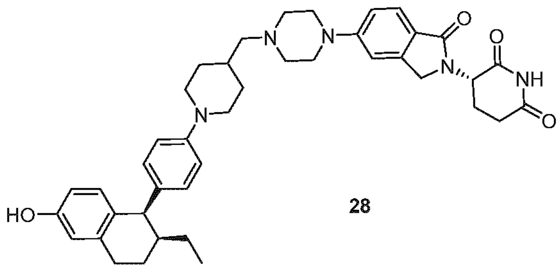

28

,

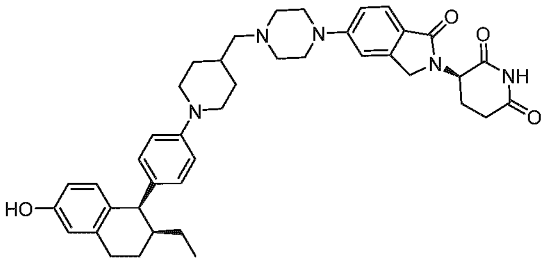
,
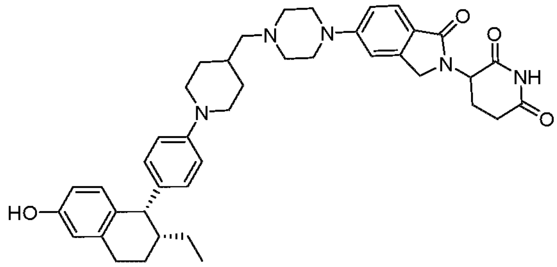
,
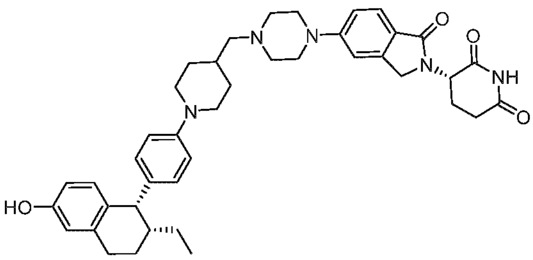
,
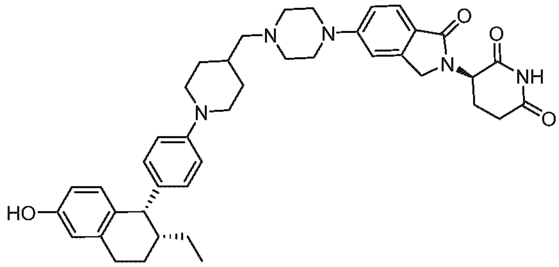
,
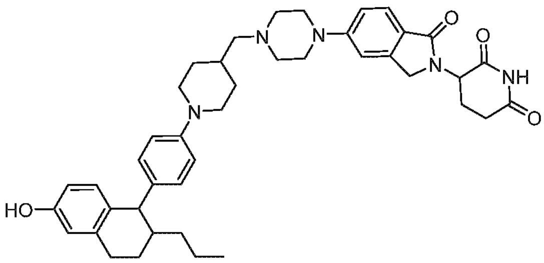
,
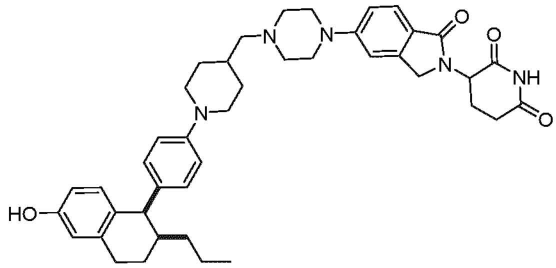
,
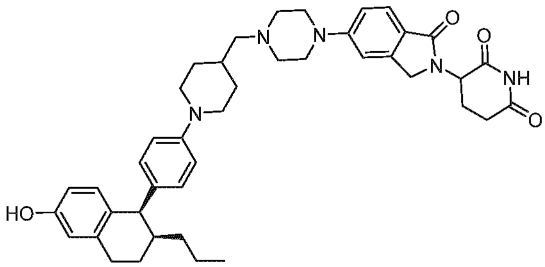
,
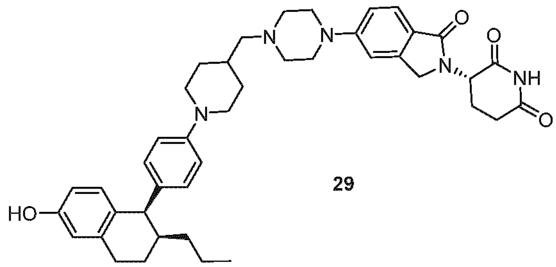

29

,
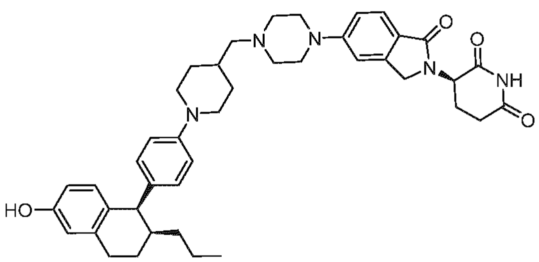
,
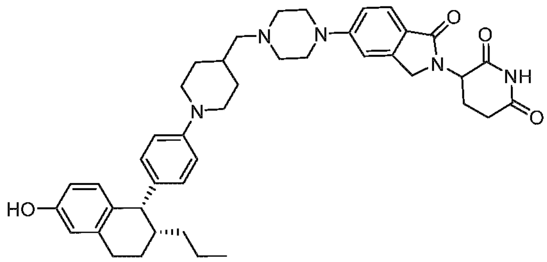
,

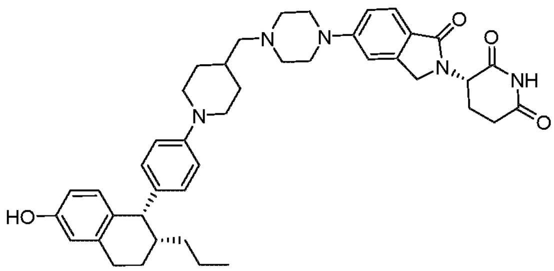,

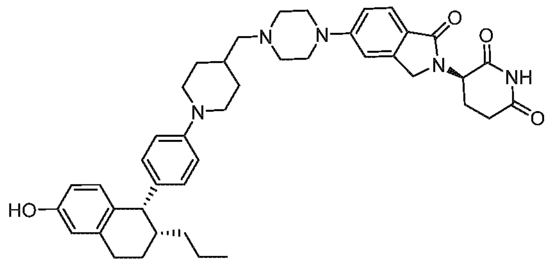,

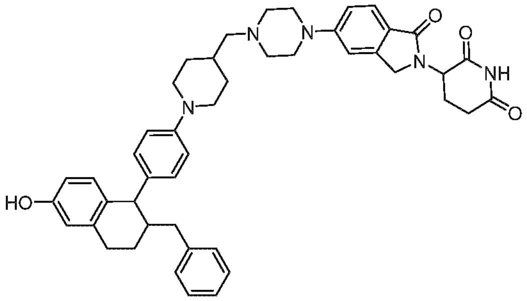,

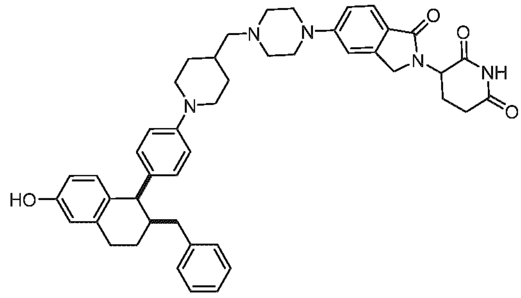,

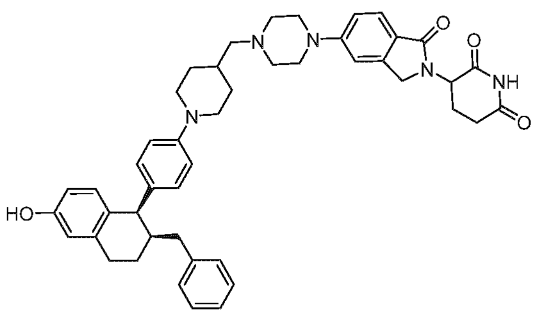,

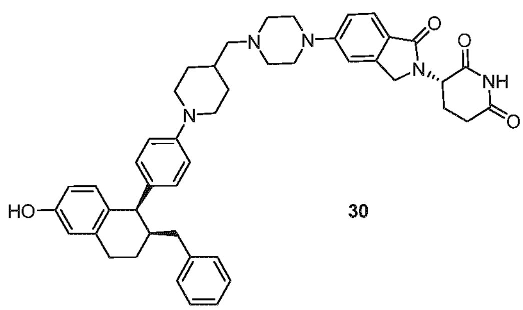

30

,

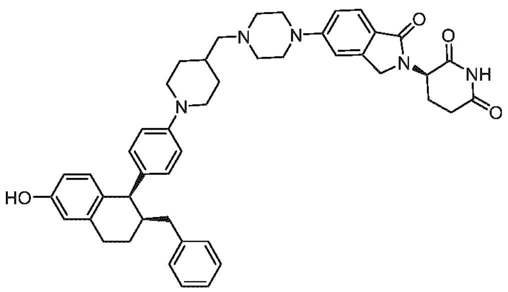,

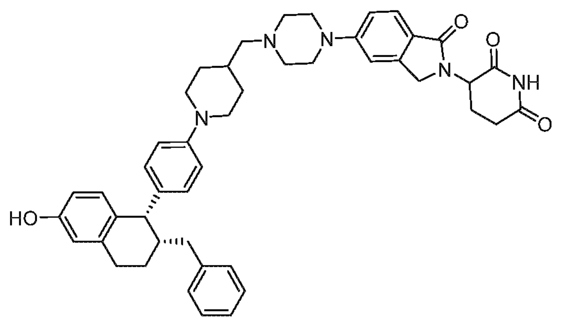,

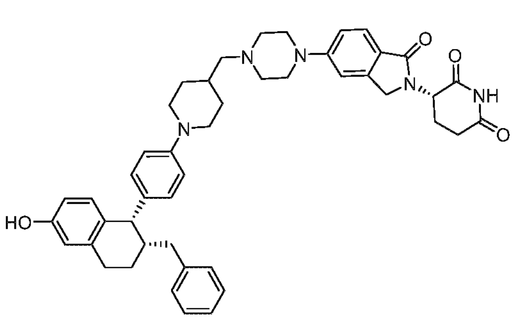,

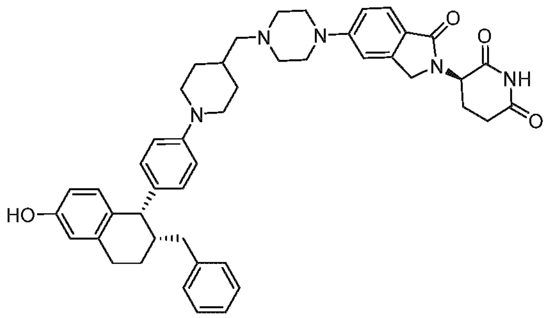,

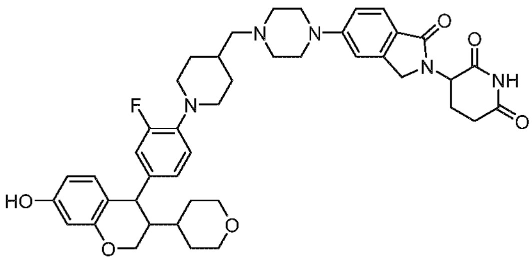
,
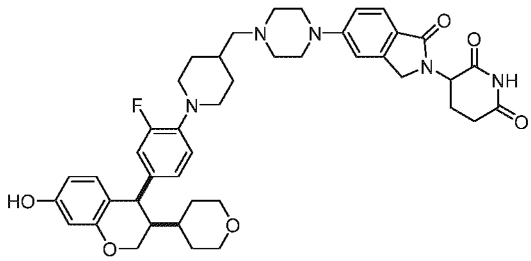
,
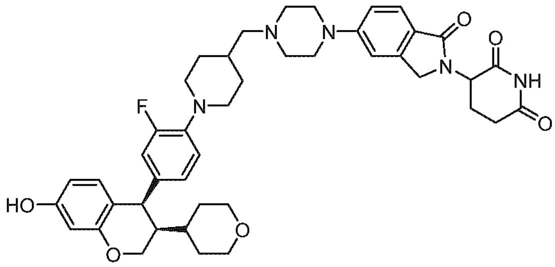
,
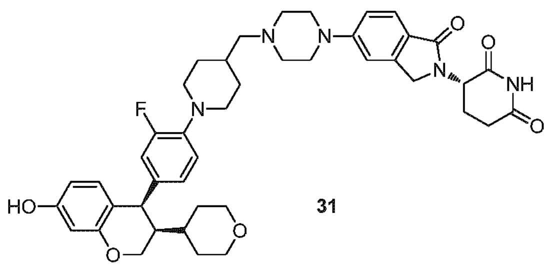
31
,
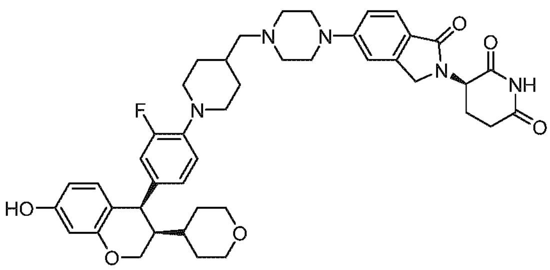
,
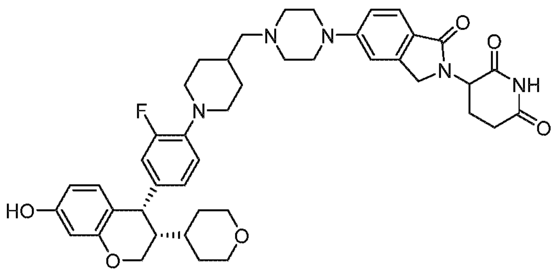
,
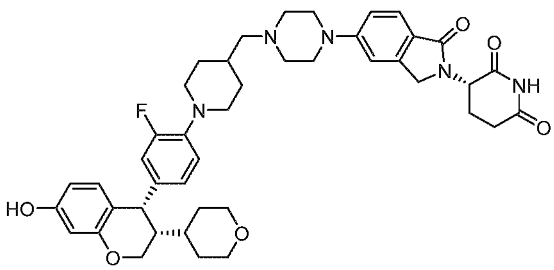
,
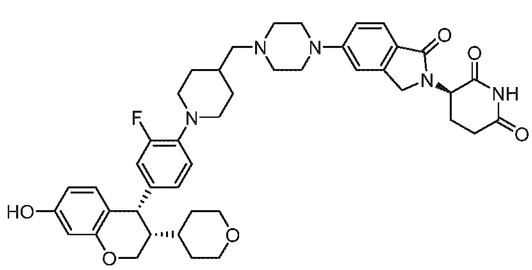
,

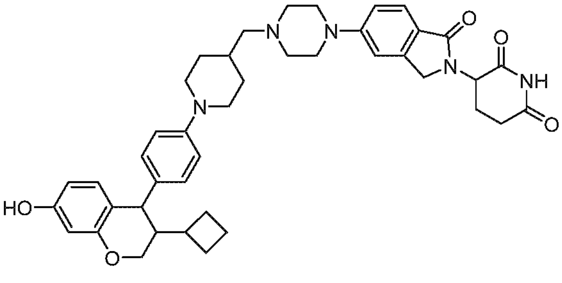
,

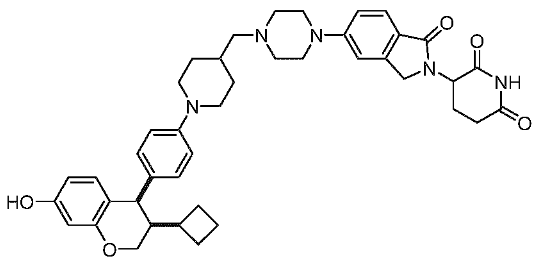
,

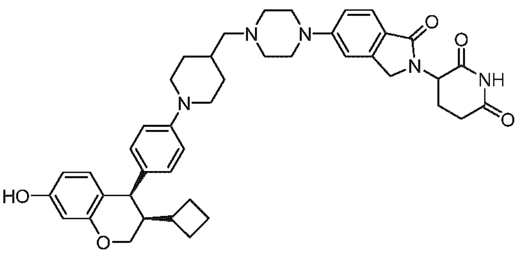
,

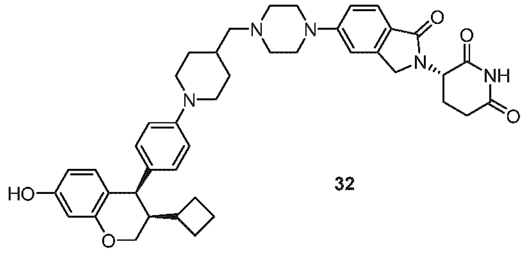
32
,

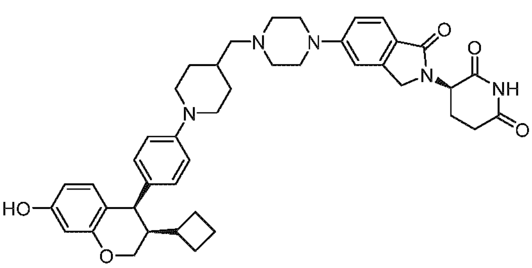
,

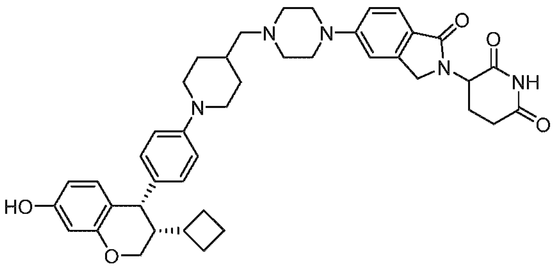
,

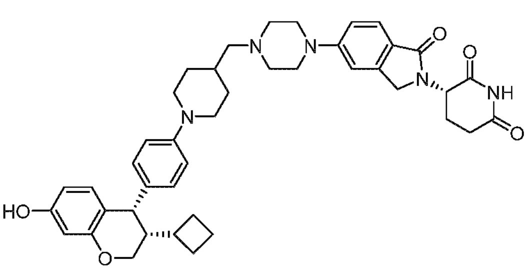
,

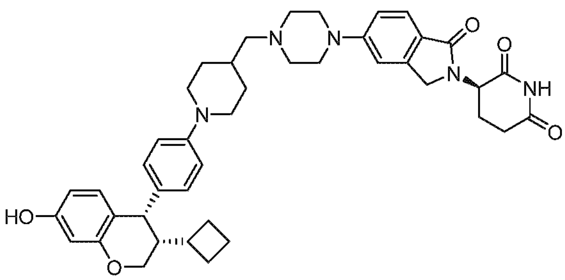
,

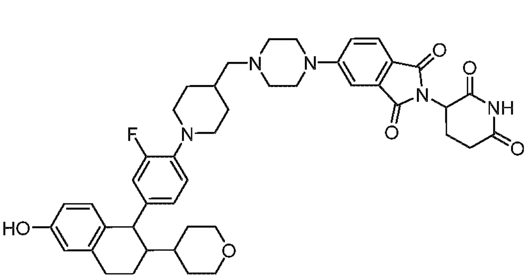
,

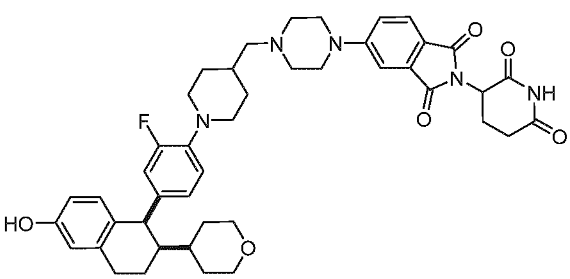
,

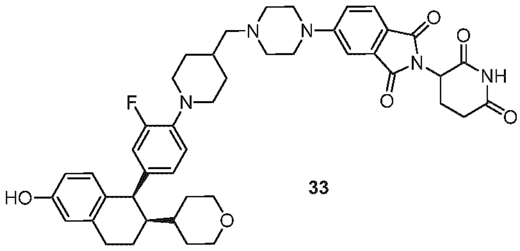

33

,

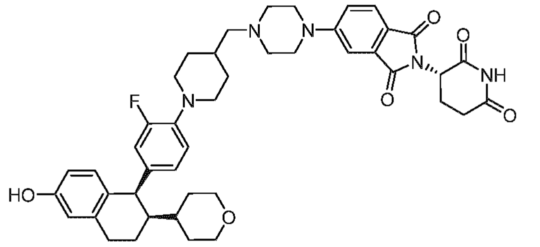

,

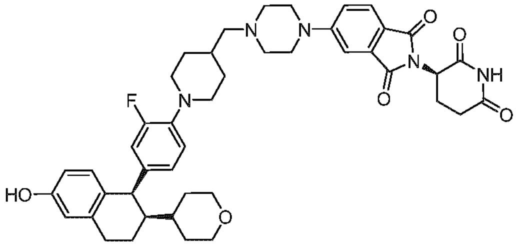

,

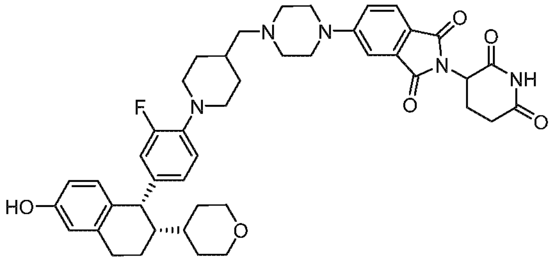

,

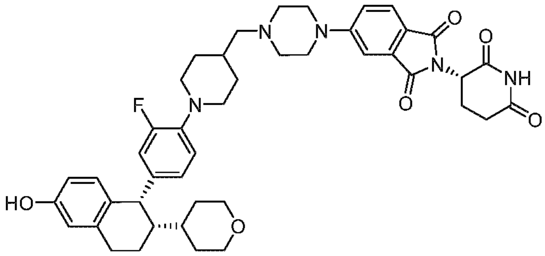

,

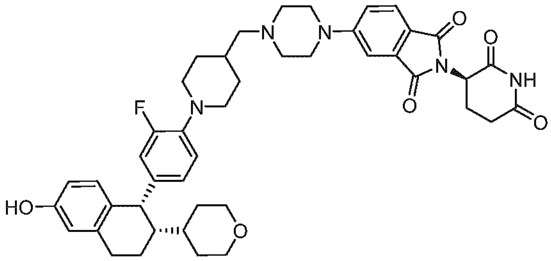

,

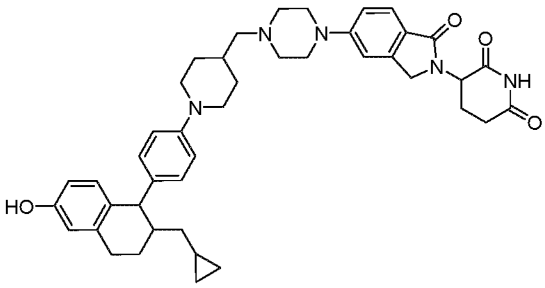

,

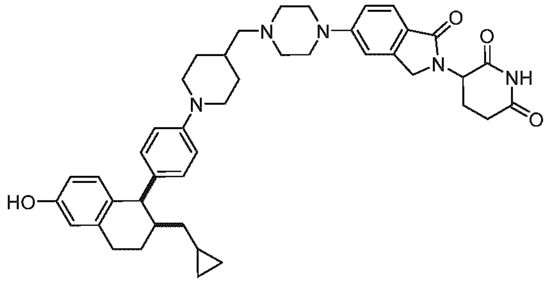

,

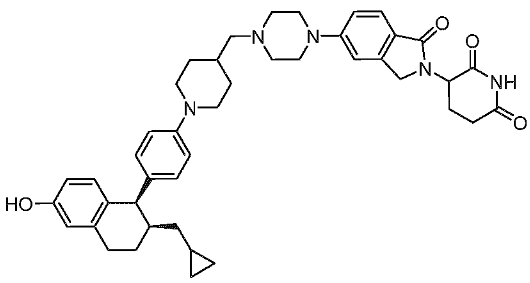

,

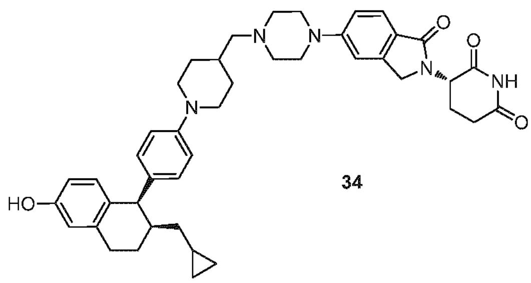

34

,

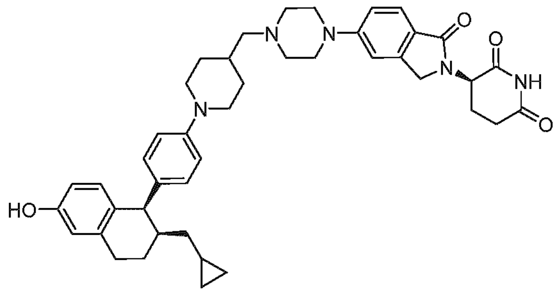

,

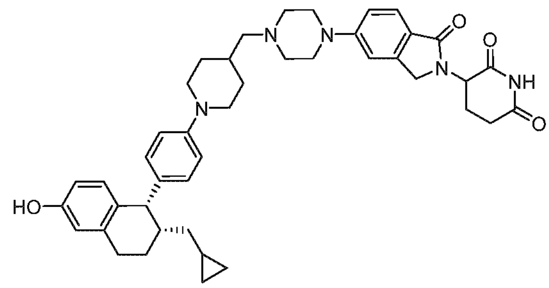

,

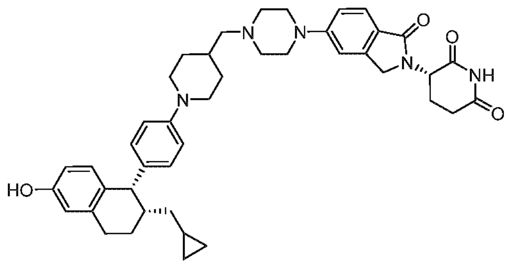

,

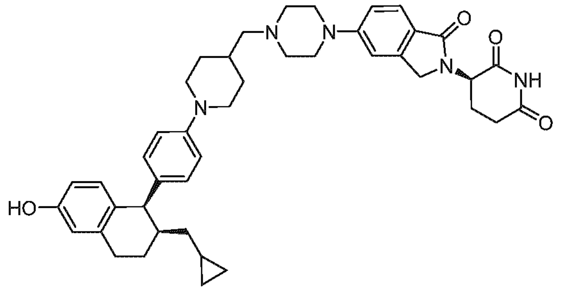

,

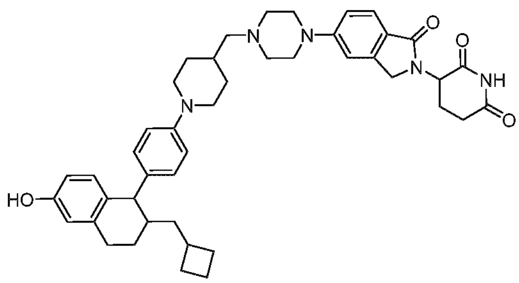

,

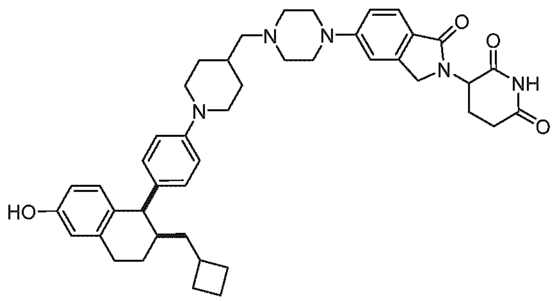

,

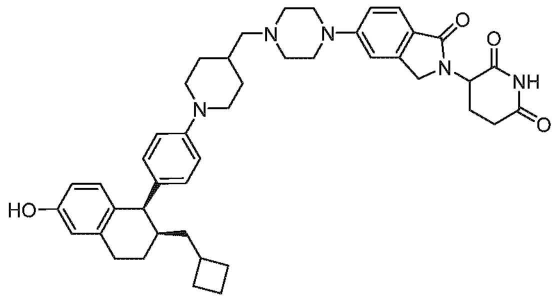

,

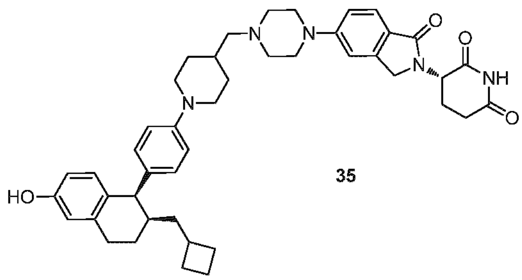

35

,

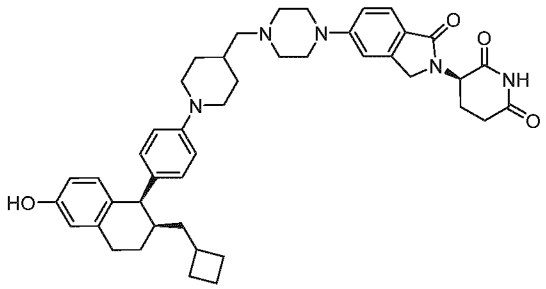

,

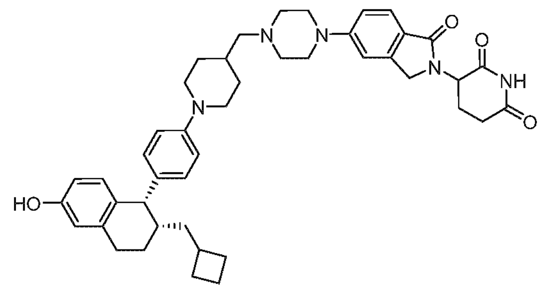

,

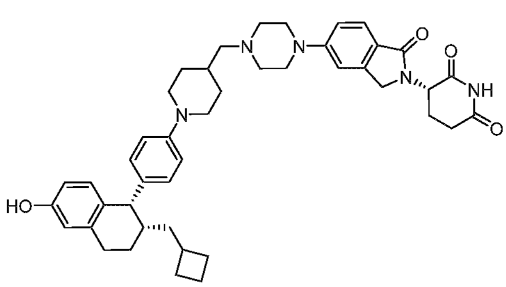

,

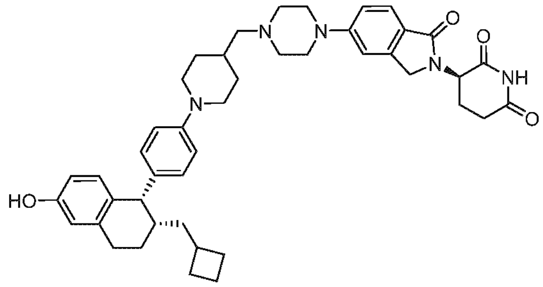

,

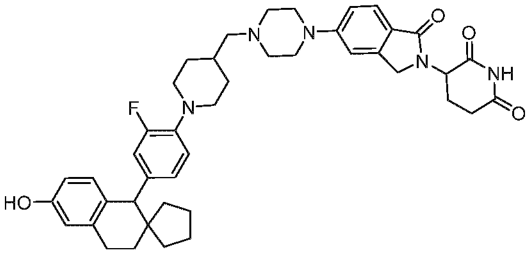

,

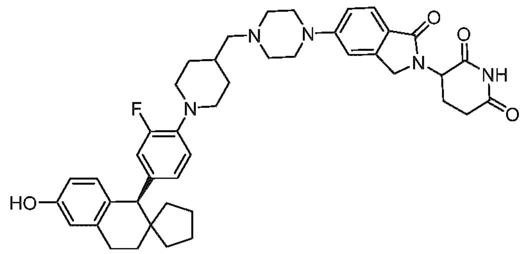

,

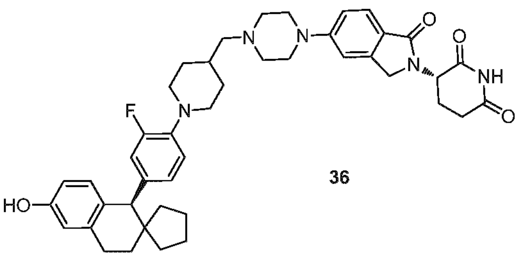

36

,

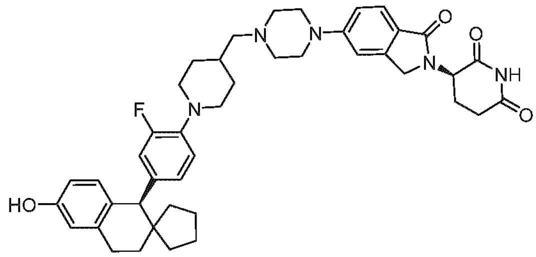

,

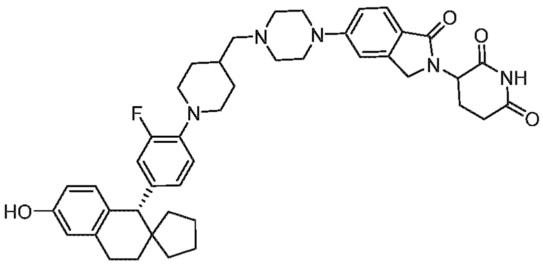

,

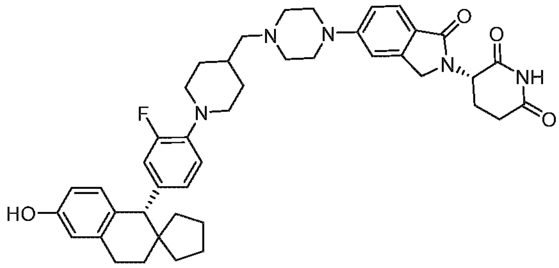

,

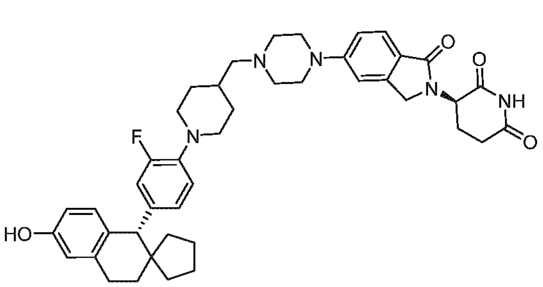

,

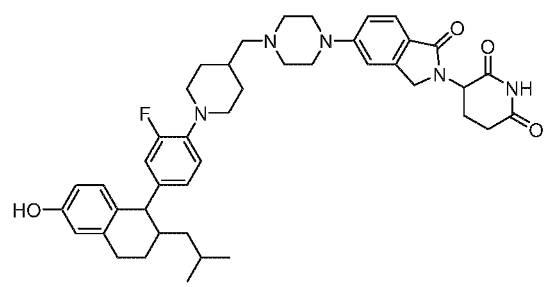

,

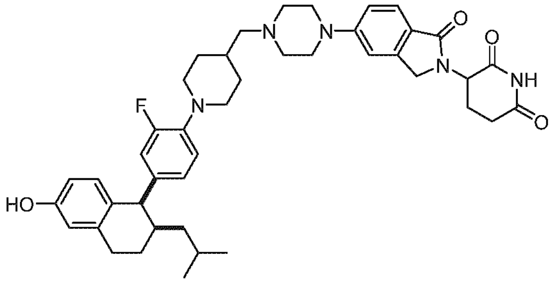
,
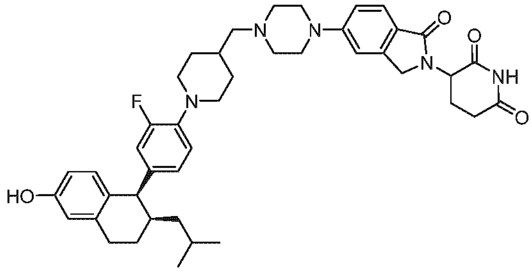
,
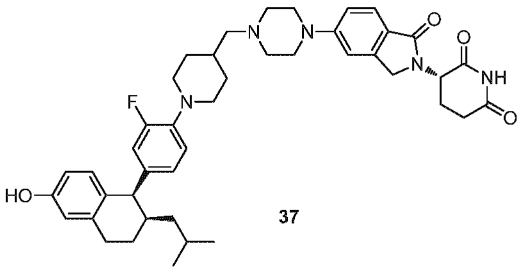
37
,
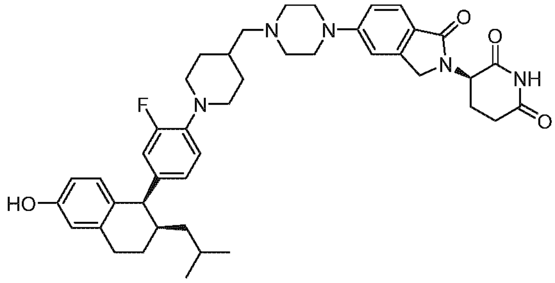
,
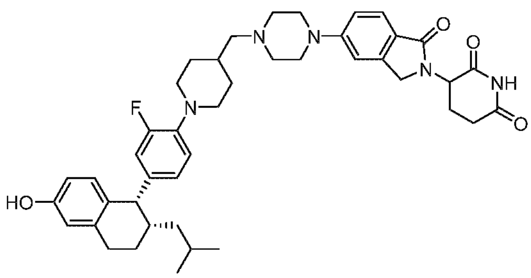
,
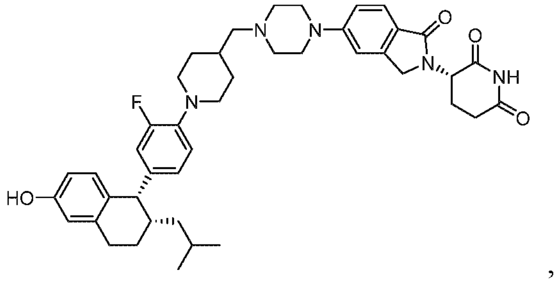
,
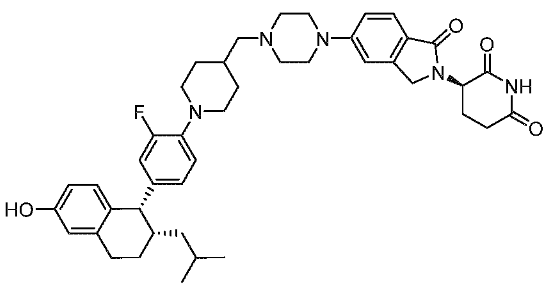
,
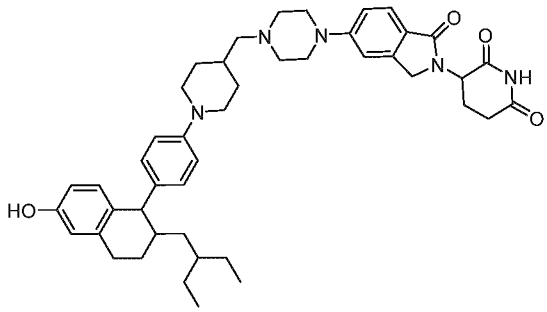
,
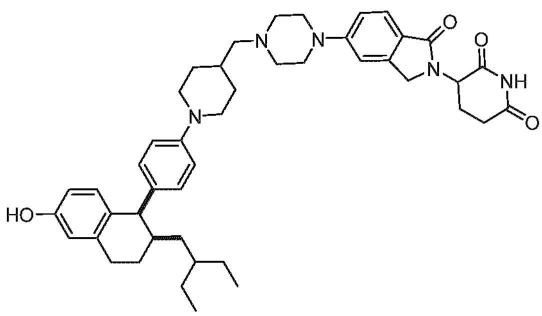
,
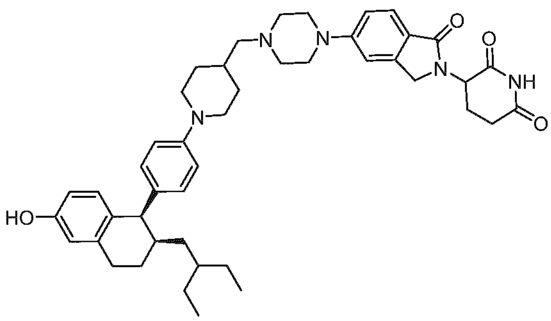
,

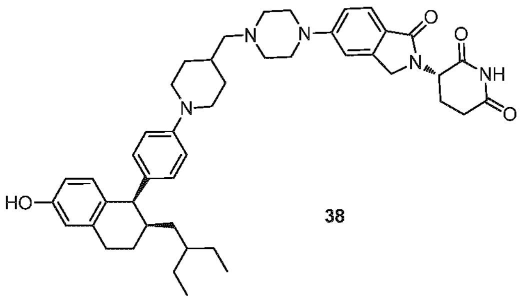

38

,

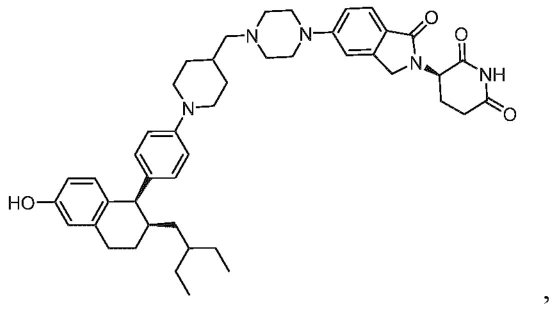

,

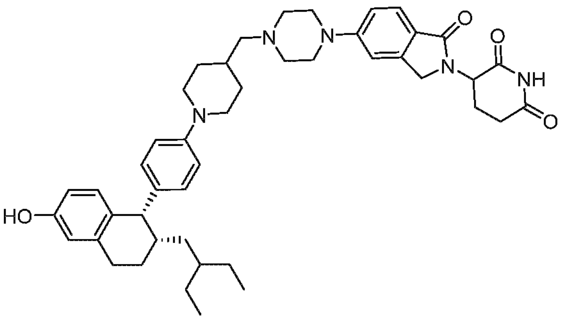

,

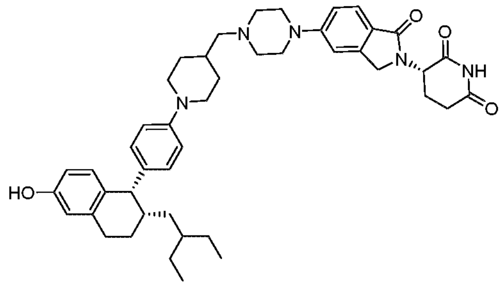

,

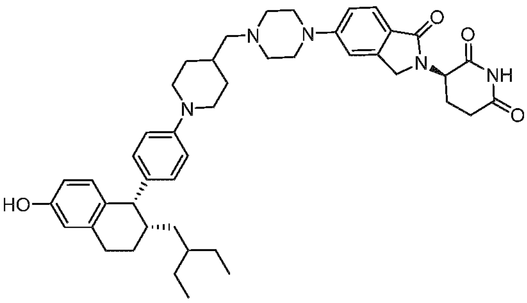

,

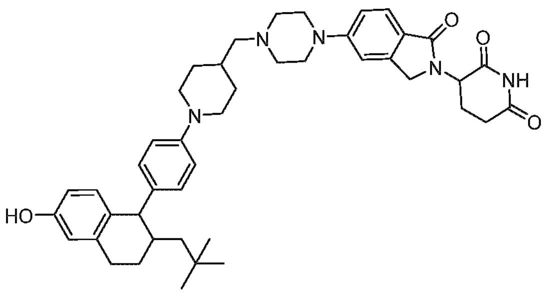

,

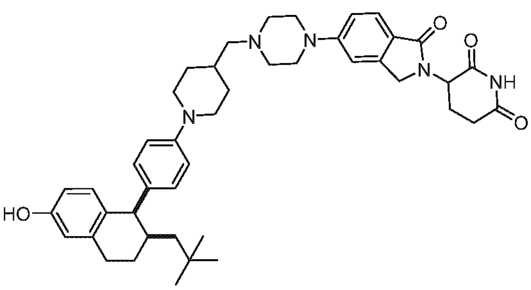

,

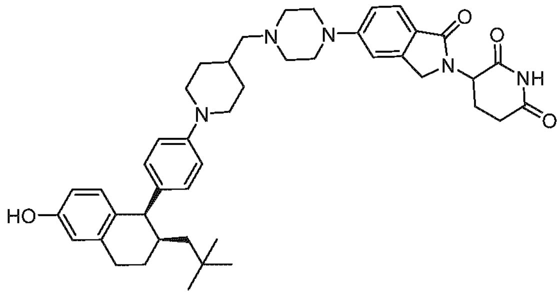

,

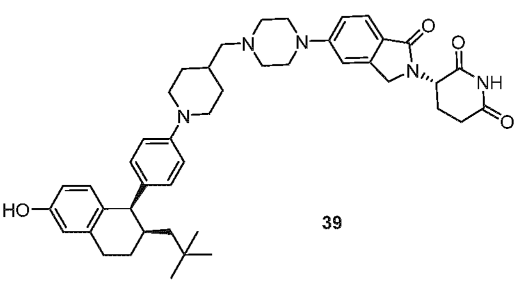

39

,

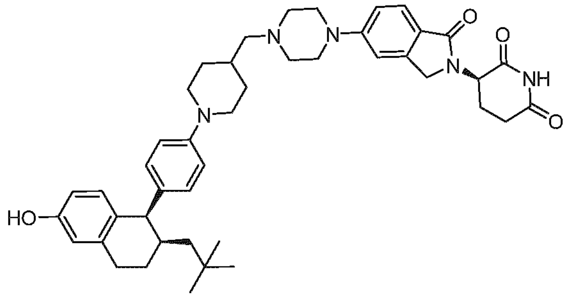

,

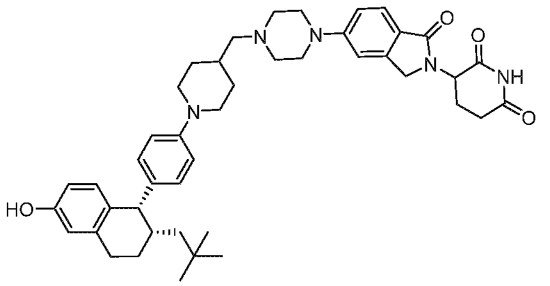

,

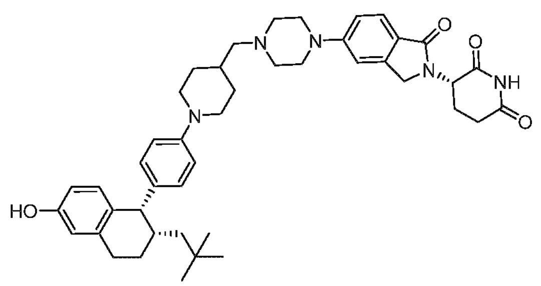

,

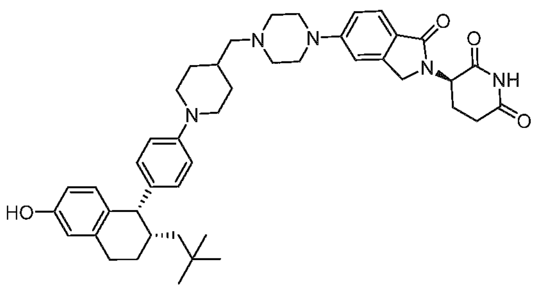

,

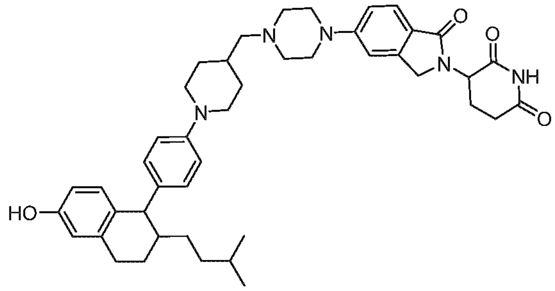

,

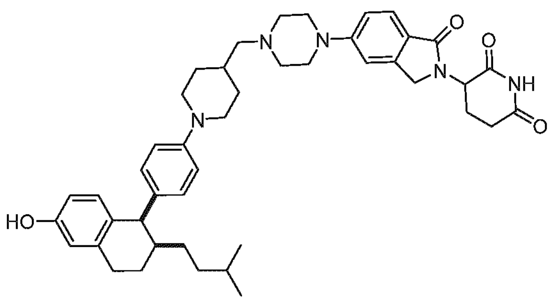

,

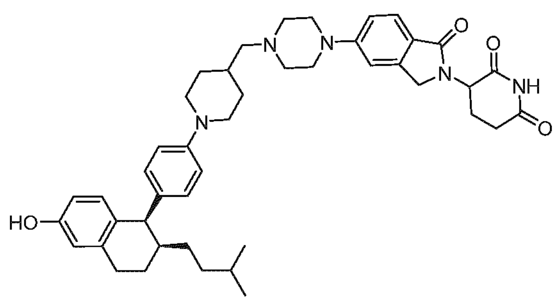

,

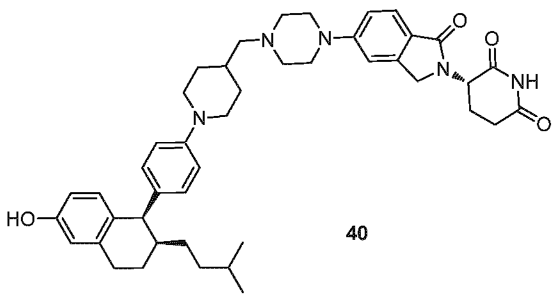

40

,

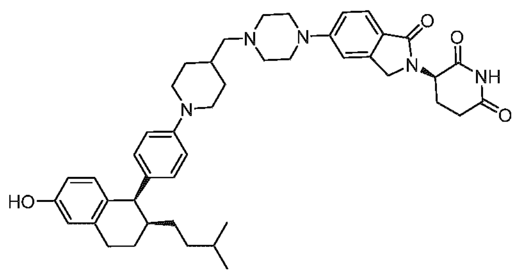

,

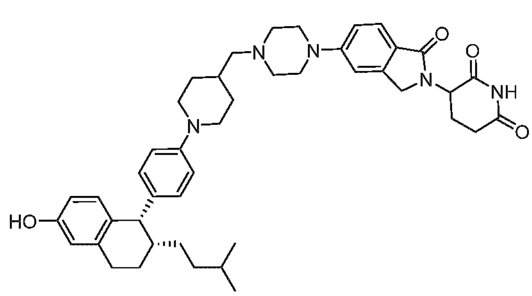

,

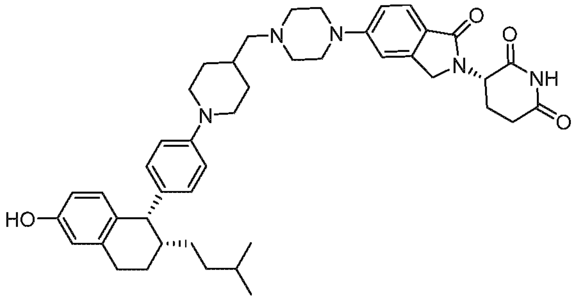

,

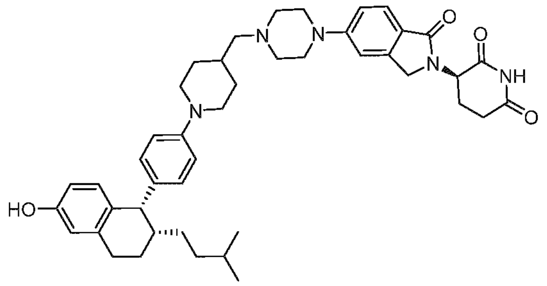

,

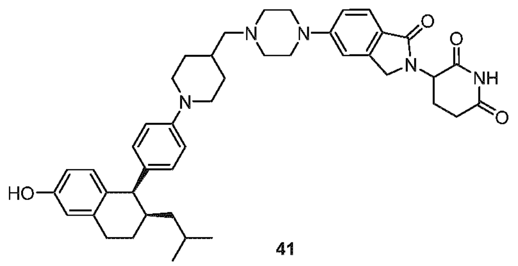

41

,

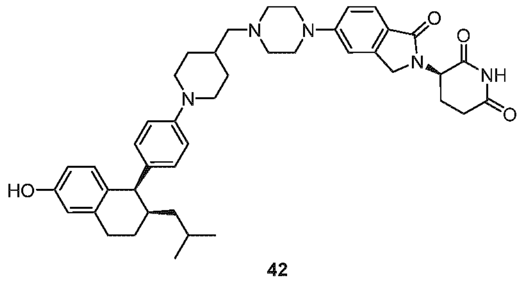

42

and

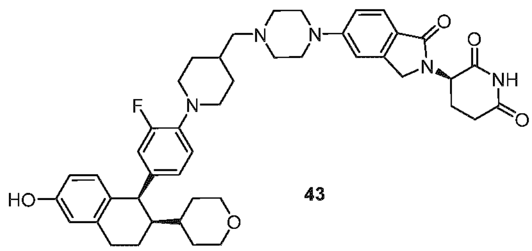

43

.

**17.** A compound of general formula (IIIa) or a salt thereof,

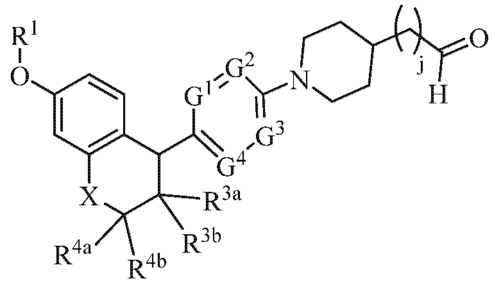

**( IIIa )**

wherein:

X, $G^1$ to $G^4$, $R^1$, $R^{3a}$, $R^{3b}$, $R^{4a}$, $R^{4b}$ and j are as defined in claim 8.

**18.** The compound of general formula (IIIa) or the salt thereof according to claim 17, being selected from the group consisting of the following compounds:

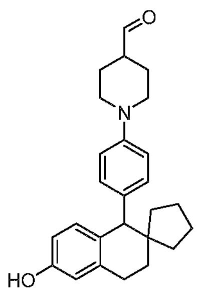

1n

,

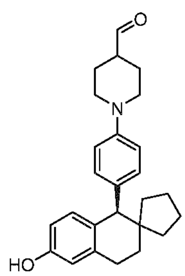

2b-1

,

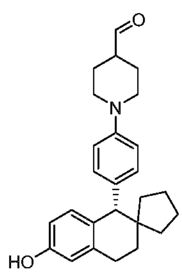

2b-2

,

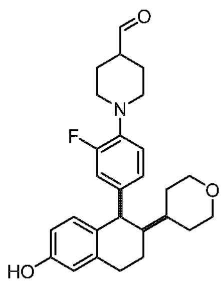

3o

,

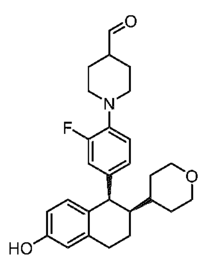

4b-1

,

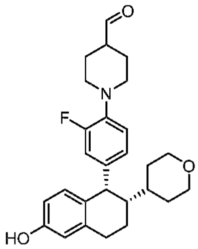
4b-2 ,
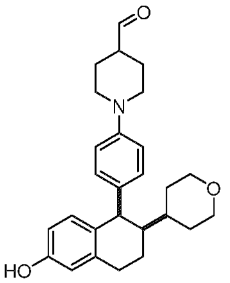
7f ,
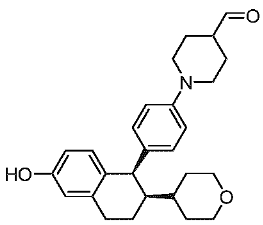
,
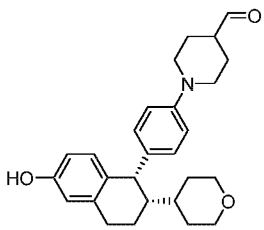
,

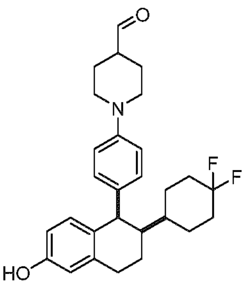
9c ,
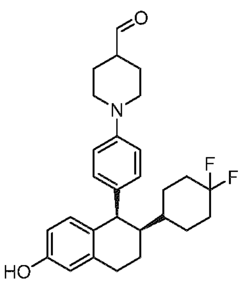
10b ,
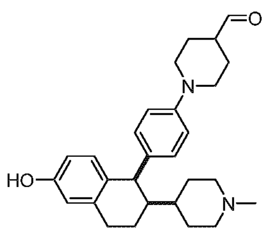
,
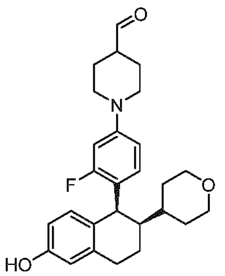
12h-1 ,

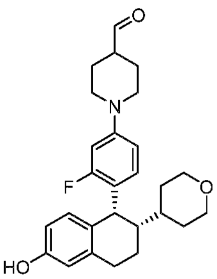
12h-2 ,
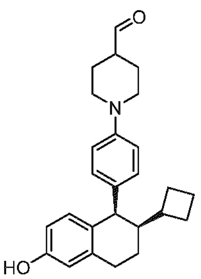
13c-1 ,
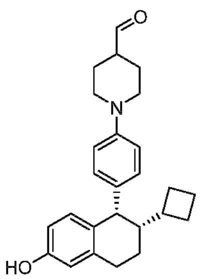
13c-2 ,
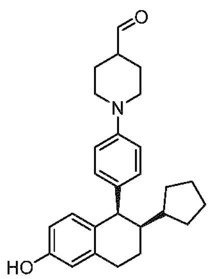
14c-1 ,
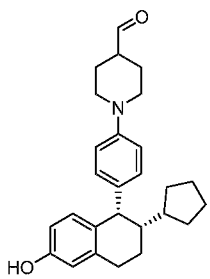
14c-2 ,

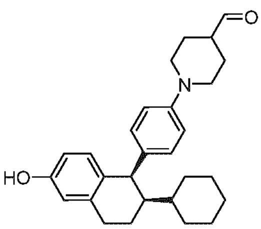
,
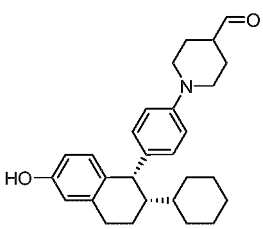
,
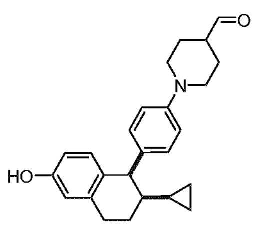
,
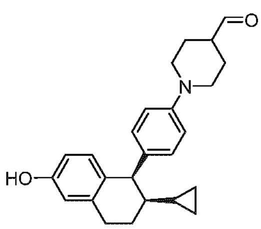
,

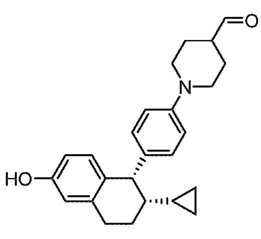
,
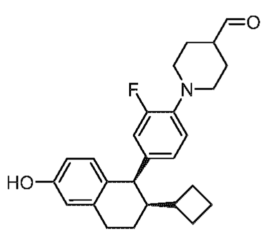
,
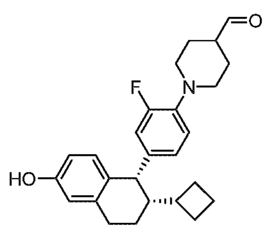
,
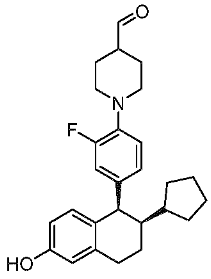
19d ,

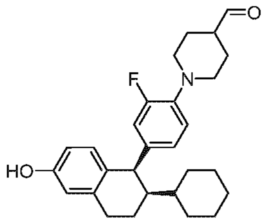 ,

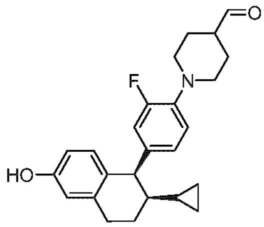 ,

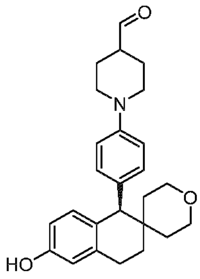

22g ,

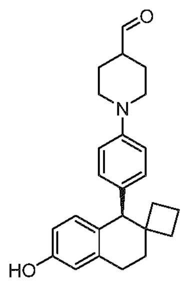

23j-1 ,

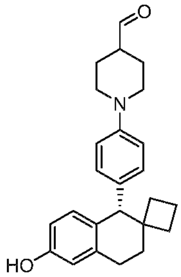

23j-2 ,

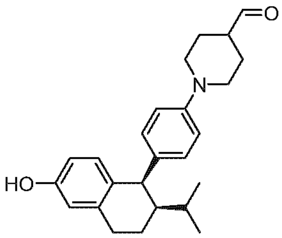 ,

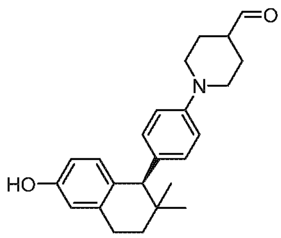 ,

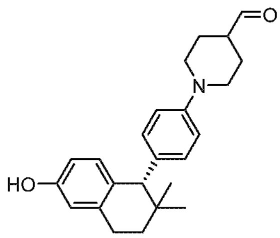 ,

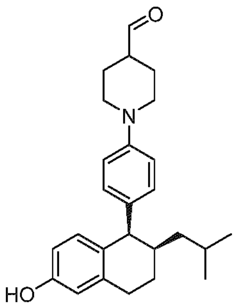

26c-1 ,

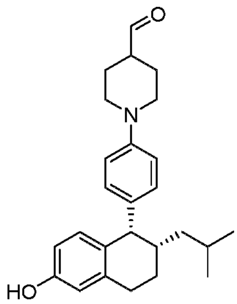

26c-2 ,

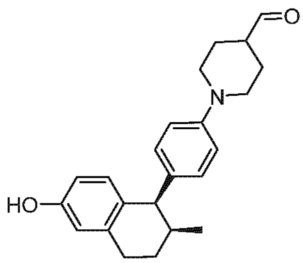 ,

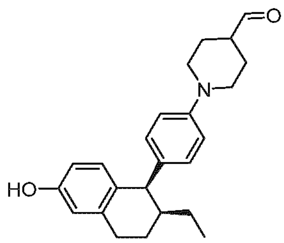 ,

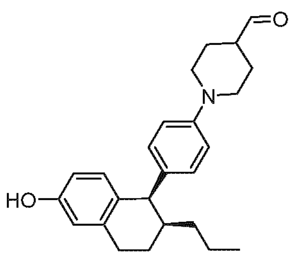 ,

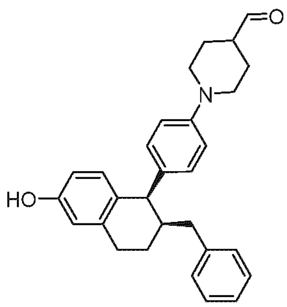 ,

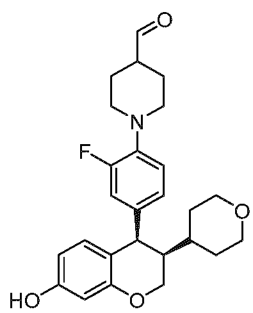

31i ,

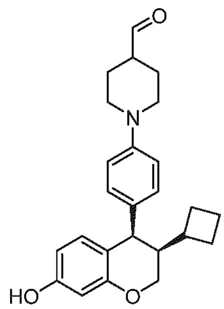

32i ,

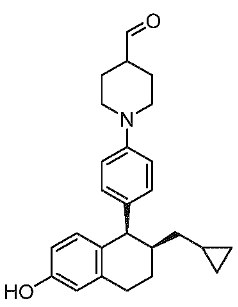
34e ,

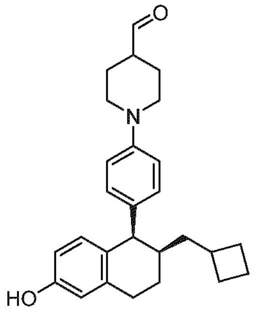
35g ,

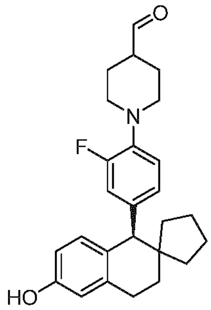
36e ,

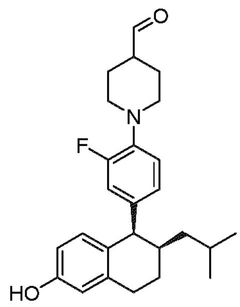
37d ,

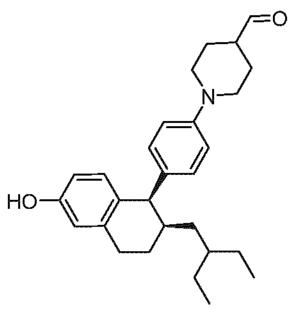
,

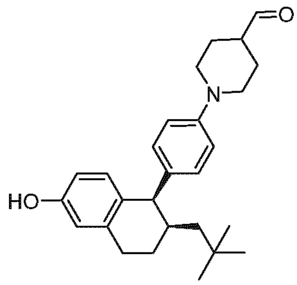
and

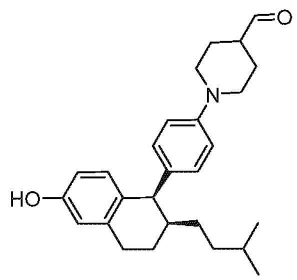
.

**19.** A method for preparing a compound of general formula (III) or a pharmaceutically acceptable salt thereof, comprising:

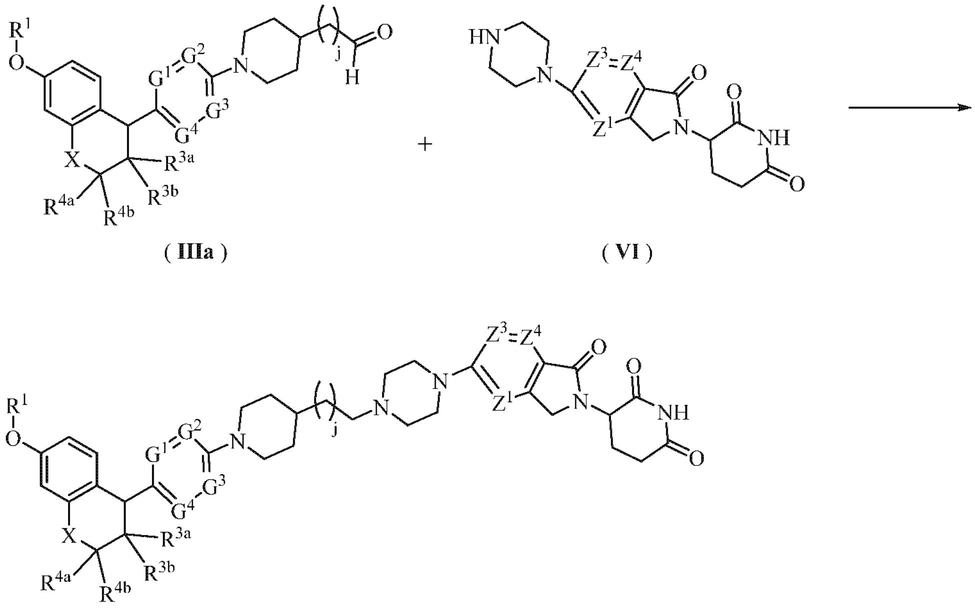
( IIIa ) ( VI )

( III )

conducting a reductive amination reaction of a compound of general formula (IIIa) with a compound of general formula (VI) or a salt thereof to give the compound of general formula (III) or the pharmaceutically acceptable salt thereof;

wherein:

X, $G^1$ to $G^4$, $Z^1$, $Z^3$, $Z^4$, $R^1$, $R^{3a}$, $R^{3b}$, $R^{4a}$, $R^{4b}$ and j are as defined in claim 8.

**20.** A pharmaceutical composition, wherein the pharmaceutical composition comprises the compound of general formula (I) or the pharmaceutically acceptable salt thereof according to any one of claims 1 to 16, and one or more pharmaceutically acceptable carriers, diluents or excipients.

**21.** Use of the compound of general formula (I) or the pharmaceutically acceptable salt thereof according to any one of claims 1 to 16 or the pharmaceutical composition according to claim 20 in the preparation of a medicament for treating and/or preventing a disease or disorder by degrading a target protein.

**22.** The use according to claim 21, wherein the disease or disorder is selected from the group consisting of abnormal cell proliferation, a tumor, an immune disease, diabetes, a cardiovascular disease, an infectious disease and an inflammatory disease, preferably a tumor and an infectious disease.

**23.** The use according to claim 22, wherein the tumor is a cancer preferably selected from the group consisting of breast cancer, endometrial cancer, testicular cancer, cervical cancer, prostate cancer, ovarian cancer, fallopian tube tumors, leukemia, skin cancer, squamous cell carcinoma, basal cell carcinoma, bladder cancer, colorectal cancer, esophageal cancer, head and neck cancer, kidney cancer, liver cancer, lung cancer, pancreatic cancer, gastric cancer, lymphoma, melanoma, sarcoma, peripheral neuroepithelioma, neuroglioma, astrocytoma, ependymoma, glioblastoma, neuroblastoma, gangliocytoma, medulloblastoma, pineocytoma, meningioma, neurofibroma, neurilemmoma, thyroid cancer, Wilms tumor and teratocarcinoma; and more preferably selected from the group consisting of breast cancer, endometrial cancer, testicular cancer, cervical cancer, prostate cancer, ovarian cancer and fallopian tube tumors.

**24.** The use according to claim 22, wherein the infectious disease is selected from the group consisting of viral pneumonia, influenza, avian influenza, meningitis, gonorrhea, and diseases caused by infection with HIV, HBV, HCV, HSV, HPV, RSV, CMV, ebolaviruses, flaviviruses, pestiviruses, rotaviruses, coronaviruses, EBV, drug-resistant viruses, RNA viruses, DNA viruses, adenoviruses, poxviruses, picornaviruses, togaviruses, orthomyxoviruses, retroviruses, hepadnaviruses, gram-negative bacteria, gram-positive bacteria, atypical bacteria, staphylococci, streptococci, *Escherichia coli, Salmonella, Helicobacter pylori, Chlamydiaceae, Mycoplasmataceae,* fungi, protozoa, helminths, worms, prions and parasites.

**25.** Use of the compound of general formula (I) or the pharmaceutically acceptable salt thereof according to any one of claims 1 to 16 or the pharmaceutical composition according to claim 20 in the preparation of a medicament for treating and/or preventing an estrogen receptor mediated or dependent disease or disorder, wherein the estrogen receptor mediated or dependent disease or disorder is a tumor, preferably a cancer, more preferably selected from the group consisting of breast cancer, endometrial cancer, testicular cancer, cervical cancer, prostate cancer, ovarian cancer and fallopian tube tumors, and most preferably breast cancer.

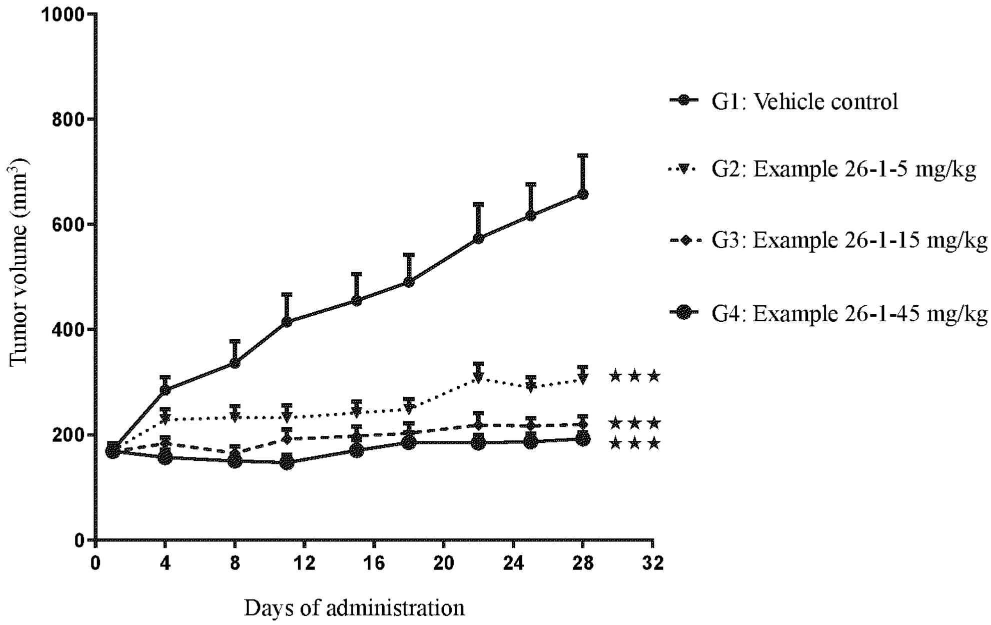
1000
800
600
400
200
0
Tumor volume (mm³)
0
4
8
12
16
20
24
28
32
Days of administration
G1: Vehicle control
G2: Example 26-1-5 mg/kg
G3: Example 26-1-15 mg/kg
G4: Example 26-1-45 mg/kg
★★★
★★★
★★★

FIG. 1

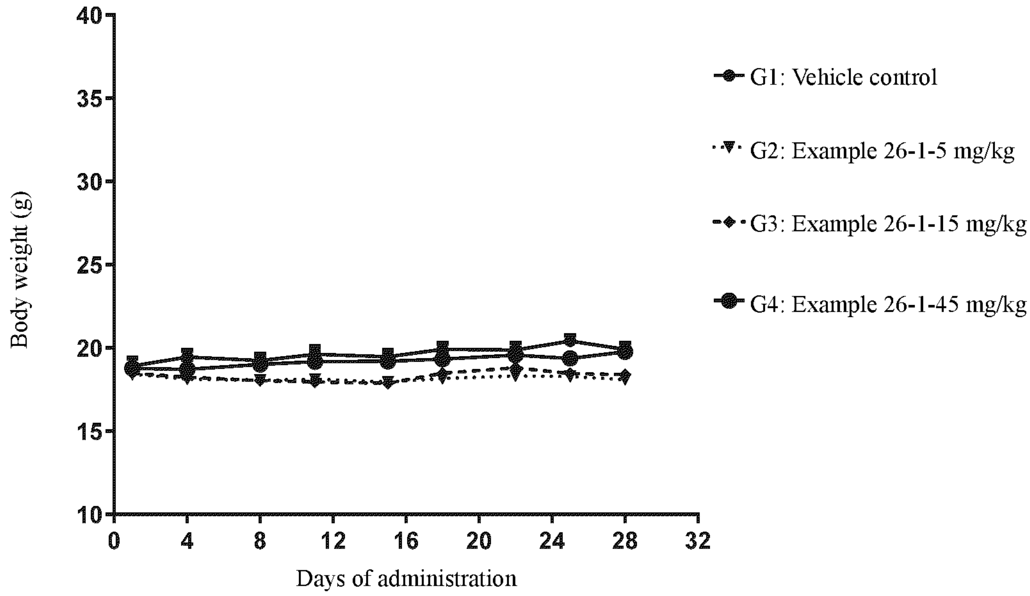
40
35
30
25
20
15
10
Body weight (g)
0
4
8
12
16
20
24
28
32
Days of administration
G1: Vehicle control
G2: Example 26-1-5 mg/kg
G3: Example 26-1-15 mg/kg
G4: Example 26-1-45 mg/kg

FIG. 2

# INTERNATIONAL SEARCH REPORT

International application No.
**PCT/CN2022/083597**

**A. CLASSIFICATION OF SUBJECT MATTER**

C07D 401/14(2006.01)i; C07D 403/14(2006.01)i; A61P 35/00(2006.01)i; A61K 31/4725(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

WPI, STN, EPODOC, CNPAT, CNKI, 万方, 百度学术, 恒瑞医药, 四氢萘, 雌激素受体, 肿瘤, 癌症, ER, tetrahydronaphthalene, estrogen receptor, cancer, tumor, structural formula search in STN.

**C. DOCUMENTS CONSIDERED TO BE RELEVANT**

| Category* | Citation of document, with indication, where appropriate, of the relevant passages | Relevant to claim No. |
|---|---|---|
| X | CN 110291087 A (ARVINAS OPERATIONS, INC.) 27 September 2019 (2019-09-27) claims 1-31, description paragraphs [2438]-[2439] | 1-9, 11-15, 17, 19-25 |
| X | CN 112262134 A (ARVINAS OPERATIONS, INC.) 22 January 2021 (2021-01-22) claims 2-38, description paragraphs [1102] and [1917] | 1-9, 11-15, 17, 19-25 |
| X | US 2021060008 A1 (ARVINAS OPERATIONS INC.) 04 March 2021 (2021-03-04) claims 1-31, description paragraph [0185] | 1-9, 11-15, 17, 19-25 |
| A | CN 110291087 A (ARVINAS OPERATIONS, INC.) 27 September 2019 (2019-09-27) claims 1-31, description paragraphs [2438]-[2439] | 10, 16, 18 |
| A | CN 112262134 A (ARVINAS OPERATIONS, INC.) 22 January 2021 (2021-01-22) claims 2-38, description paragraphs [1102] and [1917] | 10, 16, 18 |
| A | US 2021060008 A1 (ARVINAS OPERATIONS INC.) 04 March 2021 (2021-03-04) claims 1-31, description paragraph [0185] | 10, 16, 18 |
| A | CN 106458993 A (ARVINAS INC.) 22 February 2017 (2017-02-22) claims 2-7, 9-34, description paragraphs [0686]-[0712] | 1-25 |
| A | CN 110612294 A (ARVINAS OPERATIONS, INC.) 24 December 2019 (2019-12-24) claims 2-34, description paragraphs [1660]-[1661] | 1-25 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 May 2022** | **27 June 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/083597**

| Patent document cited in search report | Publication date (day/month/year) | Patent family member(s) | Publication date (day/month/year) |
|---|---|---|---|
| CN 110291087 A | 27 September 2019 | EP 3689868 A1 | 05 August 2020 |
| | | IL 266842 D0 | 31 July 2019 |
| | | EP 3548483 A1 | 09 October 2019 |
| | | WO 2018102725 A1 | 07 June 2018 |
| | | CO 2019007091 A2 | 09 October 2019 |
| | | BR 112019011200 A2 | 08 October 2019 |
| | | AU 2020201793 A1 | 26 March 2020 |
| | | RU 2019120120 A | 28 December 2020 |
| | | KR 20190082989 A | 10 July 2019 |
| | | CA 3042968 A1 | 07 June 2018 |
| | | US 2021139458 A1 | 13 May 2021 |
| | | JP 2020504089 A | 06 February 2020 |
| | | AU 2021204549 A1 | 05 August 2021 |
| | | AU 2017366693 A1 | 18 April 2019 |
| | | IL 272527 D0 | 31 March 2020 |
| | | JP 2020100659 A | 02 July 2020 |
| | | IL 284424 D0 | 29 July 2021 |
| | | AU 2021257895 A1 | 18 November 2021 |
| | | US 10899742 B1 | 26 January 2021 |
| | | US 2018155322 A1 | 07 June 2018 |
| | | MX 2019006402 A | 11 September 2019 |
| | | KR 20200020988 A | 26 February 2020 |
| | | RU 2020106142 A | 13 April 2020 |
| | | CN 111454248 A | 28 July 2020 |
| | | JP 2022023103 A | 07 February 2022 |
| | | JP 2021169479 A | 28 October 2021 |
| CN 112262134 A | 22 January 2021 | None | |
| | | JP 2021521192 A | 26 August 2021 |
| | | WO 2019199816 A1 | 17 October 2019 |
| | | KR 20210003804 A | 12 January 2021 |
| | | EP 3774772 A1 | 17 February 2021 |
| | | AU 2019251223 A1 | 26 November 2020 |
| | | IL 277934 D0 | 30 November 2020 |
| | | CA 3095912 A1 | 17 October 2019 |
| US 2021060008 A1 | 04 March 2021 | CO 2022002729 A2 | 08 April 2022 |
| | | CA 3152401 A1 | 04 March 2021 |
| | | IL 290789 D0 | 01 April 2022 |
| | | AU 2020336309 A1 | 17 March 2022 |
| | | WO 2021041348 A1 | 04 March 2021 |
| CN 106458993 A | 22 February 2017 | KR 20210132233 A | 03 November 2021 |
| | | US 2015291562 A1 | 15 October 2015 |
| | | MX 2016013563 A | 09 May 2017 |
| | | JP 2021008507 A | 28 January 2021 |
| | | BR 112016024016 A2 | 23 January 2018 |
| | | RU 2020139890 A | 18 January 2022 |
| | | KR 20170002446 A | 06 January 2017 |
| | | EP 3131588 A2 | 22 February 2017 |
| | | KR 20200097827 A | 19 August 2020 |
| | | AU 2015247817 A1 | 27 October 2016 |
| | | JP 2017513862 A | 01 June 2017 |

Form PCT/ISA/210 (patent family annex) (January 2015)

**INTERNATIONAL SEARCH REPORT**
**Information on patent family members**

International application No.
**PCT/CN2022/083597**

| Patent document cited in search report | Publication date (day/month/year) | Patent family member(s) | Publication date (day/month/year) |
|---|---|---|---|
| | | CA 2945975 A1 | 22 October 2015 |
| | | WO 2015160845 A2 | 22 October 2015 |
| | | AU 2019240589 A1 | 24 October 2019 |
| | | US 2022089570 A1 | 24 March 2022 |
| | | AU 2021236430 A1 | 14 October 2021 |
| | | RU 2016144289 A | 18 May 2018 |
| CN 110612294 A | 24 December 2019 | KR 20190116315 A | 14 October 2019 |
| | | JP 2020506922 A | 05 March 2020 |
| | | CO 2019009424 A2 | 28 February 2020 |
| | | BR 112019015484 A2 | 28 April 2020 |
| | | EP 3577109 A1 | 11 December 2019 |
| | | CA 3050309 A1 | 09 August 2018 |
| | | IL 268069 D0 | 26 September 2019 |
| | | MX 2019009046 A | 30 October 2019 |
| | | RU 2019123462 A | 27 January 2021 |
| | | AU 2018215212 A1 | 11 July 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015160845 A2
- WO 2016197032 A1
- WO 2016105518 A1
- WO 2017197046 A1
- WO 2017197051 A1
- WO 2018144649 A1
- US 10800770 B1
- WO 2018102725 A1
- WO 2019199816 A1
- US 20180155322 A1


### Non-patent literature cited in the description


- *J. Biol. Chem.,* 2006, vol. 14, 9607-9615
- *Science,* 2010, vol. 327, 1345
- *Science,* 2014, vol. 343, 301
- *Science,* 2014, vol. 343, 305